# EUROPEAN PATENT APPLICATION

(11) **EP 1 044 985 A1**
(43) Date of publication of application: **18.10.2000**
(21) Application number: 98959141.7
(22) Date of filing: 09.12.1998
(51) Int. Cl.: C07H 17/08, A61K 31/71

(54) **ERYTHROMYCIN DERIVATIVES**

(30) Priority: 11.12.1997 JP 36263497
(71) Applicant: HOKURIKU SEIYAKU CO., LTD., Katsuyama-shi, Fukui 911-0813 (JP)
(72) Inventor: KATO, Hideo, Katsuyama-shi, Fukui 911-0813 (JP); KADO, Noriyuki, Katsuyama-shi, Fukui 911-0813 (JP); YOSHIDA, Toshihiko, Katsuyama-shi, Fukui 911-0813 (JP); NISHINO, Hiroyuki, Katsuyama-shi, Fukui 911-0813 (JP); NISHIMOTO, Akemi, Katsuyama-shi, Fukui 911-0813 (JP)
(74) Representative: Sternagel, Fleischer, Godemeyer & Partner Patentanwälte
(86) International application number: JP9805570
(87) International publication number: WO9929709

(57) **Abstract**

Novel erythromycin derivatives represented by general formula (I) and drugs containing these derivatives as the active ingredient and being useful in treating atypical acid-fast bacterium infection, wherein R¹ represents hydrogen or lower alkyl; R² represents alkyl, cycloalkyl, aryl, aralkyl, -X-R⁴, etc. (wherein X represents oxygen or amino; and R⁴ represents alkyl or aryl); and R³ represents alkyl, cycloalkyl, alkenyl or - (CH₂ )ₙ -Y-R⁵ (wherein Y represents methylene, oxygen, sulfur, sulfinyl, carbonyl, etc.; R⁵ represents aryl; and n is an integer of 1 to 5).

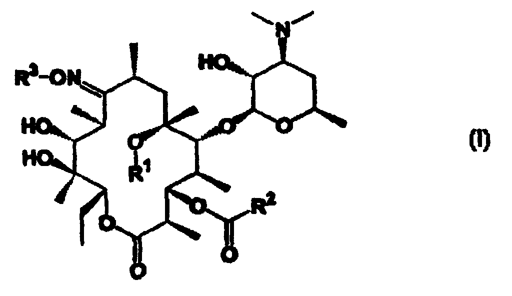

## Description

### Technical Field

The present invention relates to erythromycin derivatives useful as antibacterial agents. In particular, the invention relates to novel erythromycin derivatives and salts thereof which are useful for treatment of atypical acid-fast mycobacteriosis (non-tuberculous acid-fast mycobacteriosis).

### Background Art

Atypical acid-fast mycobacteria have low sensitivity to various antibacterial agents including antituberculosis agents, and for this reason, atypical acid-fast mycobacteriosis is an extremely intractable disease. Rifampicin (The Merck Index, 12th edition, 8382) and the like are known as compounds that can be applied to diseases similar to those treatable by the compounds of the present invention. As macrolide derivatives that have similar chemical structures to those of the compounds of the present invention, clarithromycin (The Merck Index, 12th edition, 2400), roxithromycin (The Merck Index, 12th edition, 8433) as a 9-oxime type compound and the like are known. Furthermore, as compounds converted from 3-cladinose, International Publication WO93/13116 and other publications disclose ester-type compounds, and International Publication WO93/13115 discloses carbamate-type compounds. Clinical application of clarithromycin has been approved in the United State and other countries, which is considered as the most promising agent for the treatment of atypical acid-fast mycobacteriosis among those macrolide derivatives. However, even clarithromycin fails to have sufficient antibacterial activity, and therefore, development of more potent antibacterial agents has been desired.

### Disclosure of the Invention

In recent years, increase of opportunistic infections has become a big social problem. Causes of the increase of the opportunistic infections may include increase of compromised hosts with degraded biophylaxis mechanism such as patients infected by human immunodeficiency virus (HIV), patients of cancer and diabetes, and elderly persons, and increase of multiple drug-resistant bacteria whose typical examples are Methicillin-resistant *Staphylococcus aureus,* or may also include microbial substitution. For these causes, chemotherapy of opportunistic infections becomes more difficult. Opportunistic infections caused by atypical acid-fast mycobacteria also become a problem. Atypical acid-fast mycobacteria proliferate slowly, and even when they are captured by phagocytes, they can survive in the cells for a long period of time. Therefore, prolonged chemotherapy is required to treat infections by these bacteria.

In particular, among the atypical acid-fast mycobacteria, few effective antibacterial agents are available against *Mycobacterium avium* complex (MAC), and accordingly, surgical treatment has been studied for the treatment of this infection at present. Moreover, even the aforementioned clarithromycin lacks selectivity as an agent for therapeutic treatment of atypical acid-fast mycobacteriosis, and a problem also arises that clarithromycin resistant MACs have already emerged. As explained above, there are various problems in chemotherapy of atypical acid-fast mycobacteriosis, for example, low sensitivity of the bacteria to known antibacterial agents, and conditions of high possibility of microbial substitution or emergence of resistant bacteria. An object of the present invention is to provide a compound that has selective and excellent antibacterial activity against atypical acid-fast mycobacteria.

The inventors of the present invention eagerly conducted researches to achieve the aforementioned object. As a result, they found that the novel erythromycin derivatives or salts thereof according to the present invention were useful as antibacterial agents having the aforementioned characteristics, and that they were excellent in antibacterial activity particularly against atypical acid-fast mycobacteria. The present invention was achieved on the basis of these findings.

The present invention thus relates to novel erythromycin derivatives represented by the following general formula (1) or salts thereof: 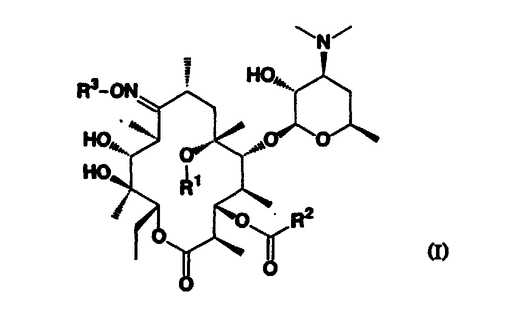 wherein R¹ represents a hydrogen atom or a lower alkyl group; R² represents an alkyl group which may be substituted, a cycloalkyl group which may be substituted, a (cycloalkyl)alkyl group which may be substituted, an aryl group which may be substituted, an aralkyl group which may be substituted, a styryl group which may be substituted, or a group represented by the formula -X-R⁴ wherein X represents an oxygen atom or an amino group and R⁴ represents an alkyl group which may be substituted or an aryl group which may be substituted; and R³ represents an alkyl group which is substituted with carboxyl group, an alkoxycarbonyl group, an aryloxycarbonyl group or an aralkyloxycarbonyl group, a cycloalkyl group which may be substituted, a (cycloalkyl)alkyl group which may be substituted, an alkenyl group which may be substituted, or a group represented by the formula -(CH2)ₙ-Y-R⁵ wherein Y represents a methylene group which may be substituted, an oxygen atom, a sulfur atom, a sulfinyl group, a sulfonyl group, an amino group which may be substituted with an alkyl group, or a carbonyl group, R⁵ represents an aryl group which may be substituted, and n represents an integer of from 1 to 5.

According to another embodiment of the present invention, there are provided compounds represented by the aforementioned general formula (I) or salts thereof wherein R¹ is a hydrogen atom.

According to another aspect of the present invention, there are provided medicaments which comprise a compound represented by the aforementioned general formula (I) or a salt thereof as an active ingredient. The medicaments are useful as antibacterial agents, and can be used as, for example, agents for therapeutic treatment of infections by various microorganisms. Preferably, they are useful for treatment of atypical acid-fast mycobacteriosis, and more preferably, they are useful for treatment of infection by *Mycobacterium avium* complex.

According to further aspects of the present invention, there are provided use of the compounds represented by the aforementioned general formula (I) or salts thereof for the manufacture of the aforementioned medicaments; and methods for therapeutic treatment of infectious diseases, preferably atypical acid-fast mycobacterioses, more preferably infections by *Mycobacterium avium* complex, which comprise a step of administering to a patient a therapeutically effective amount of a compound represented by the aforementioned general formula (I) or a salt thereof.

### Best Mode for Carrying out the Invention

In the aforementioned general formula (I) of the present invention, the lower alkyl group represented by R¹ may be, for example, methyl group, ethyl group, n-propyl group, n-butyl group and the like. The alkyl group represented by R², R³, or R⁴, or the alkyl group which may be a substituent of the amino group represented by Y may be a linear or branched alkyl group having 1 to 10 carbon atoms, and those alkyl groups may contain one or more hetero atoms selected from the group consisting of an oxygen atom, a sulfur atom, and a nitrogen atom. Examples thereof include, for example, methyl group, ethyl group, n-propyl group, isopropyl group, n-butyl group, isobutyl group, sec-butyl group, tert-butyl group, n-pentyl group, isopentyl group, neopentyl group, tert-pentyl group, n-hexyl group, n-heptyl group, n-octyl group, n-nonyl group, n-decyl group, methoxyethyl group, ethoxyethyl group, methoxypropyl group, methoxybutyl group, methoxypentyl group, methoxyhexyl group, methylthioethyl group, ethylthioethyl group, methylthiopropyl group, methylthiobutyl group, methylthiopentyl group, methylthiohexyl group, methylaminoethyl group, ethylaminoethyl group, methylaminopropyl group, methylaminobutyl group, methylaminopentyl group, methylaminohexyl group, dimethylaminoethyl group, dimethylaminopropyl group, dimethylaminobutyl group, dimethylaminopentyl group, dimethylaminohexyl group and the like. The cycloalkyl group represented by R² or R³ may be a cycloalkyl group having 3 to 7 carbon atoms, for example, cyclopropyl group, cyclobutyl group, cyclopentyl group, cyclohexyl group, cycloheptyl group. The (cycloalkyl)alkyl group represented by R² or R³ may be an alkyl group which is substituted with a monocyclic or polycyclic cycloalkyl group at any position and may contain an oxygen atom, a sulfur atom or a nitrogen atom. Examples thereof include, for example, (cyclopropyl)methyl group, (cyclobutyl)methyl group, (cyclopentyl)methyl group, (cyclohexyl)methyl group, (cycloheptyl)methyl group, (cyclopropyl)ethyl group, (cyclobutyl)ethyl group, (cyclopentyl)ethyl group, (cyclohexyl)ethyl group, (cycloheptyl)ethyl group, (cyclohexyl)propyl group, (cyclohexyl)butyl group, (cyclohexyl)pentyl group, (cyclohexyl)hexyl group, (cyclohexyl)heptyl group, (cyclohexyl)octyl group, (cyclohexyl)nonyl group, (cyclohexyl)decyl group, (2,3-dihydrobenzofuran-2-yl)methyl group, (2,3-dihydrobenzofuran-3-yl)methyl group, (3,4-dihydrobenzo[b]pyran-2-yl)methyl group, (3,4-dihydrobenzo[b]pyran-3-yl)methyl group, (3,4-dihydrobenzo[b]-pyran-4-yl)methyl group, (2,3-dihydro-1,4-benzodioxin-2-yl)methyl group and the like. The alkenyl group represented by R³ may be an alkenyl group having 3 to 7 carbon atoms, for example, allyl group, butenyl group, pentenyl group, hexenyl group, heptenyl group and the like.

As the alkoxycarbonyl group contained in the alkyl group which is substituted with an alkoxycarbonyl group, an aryloxycarbonyl group, or an aralkyloxycarbonyl group represented by R³, examples include methoxycarbonyl group, ethoxycarbonyl group, n-propoxycarbonyl group, isopropoxycarbonyl group, n-butoxycarbonyl group, isobutoxycarbonyl group, sec-butoxycarbonyl group, tert-butoxycarbonyl group, n-pentyloxycarbonyl group, isopentyloxycarbonyl group, neopentyloxycarbonyl group and the like. Examples of the aryloxycarbonyl group include, for example, phenoxycarbonyl group, pyridyloxycarbonyl group, thienyloxycarbonyl group and the like. Examples of the aralkyloxycarbonyl group include, for example, benzyloxycarbonyl group, pyridylmethyloxycarbonyl group, thenyloxycarbonyl group and the like.

In the aforementioned general formula (I) of the present invention, examples of the aryl group represented by R², R⁴, or R⁵ include, for example, monocyclic or polycyclic aromatic rings such as phenyl group, pyridyl group, pyrimidyl group, pyrazinyl group, imidazolyl group, naphthyl group, furyl group, benzofuranyl group, benzo[b]thiophenyl group, benzimidazolyl group, indolyl group, thienyl group, pyrrolyl group, quinolyl group, isoquinolyl group, 1,2,3,4-tetrahydronaphthalen-5-yl group and 1,2,3,4-tetrahydronaphthalen-6-yl group and the like. The aralkyl group represented by R² may be the aforementioned aryl group which is substituted with a linear or branched alkyl group having 1 to 6 carbon atoms at any position and may contain an oxygen atom, a sulfur atom, or a nitrogen atom. Examples thereof include benzyl group, pyridylmethyl group, pyrimidylmethyl group, pyrazinylmethyl group, naphthylmethyl group, furfuryl group, benzofuranylmethyl group, thenyl group, pyrrolylmethyl group, quinolylmethyl group, isoquinolylmethyl group, phenethyl group, phenylpropyl group, phenylbutyl group, phenylpentyl group, phenylhexyl group, phenyloxymethyl group, pyridyloxymethyl group, pyrimidyloxymethyl group, pyrazinyloxymethyl group, naphthyloxymethyl group, tetrahydronaphthyloxyethyl group, phenylthiomethyl group, pyridylthiomethyl group, pyrimidylthiomethyl group, pyrazinylthiomethyl group, naphthylthiomethyl group, phenylaminomethyl group and the like.

Substituents for the optionally substituted alkyl group represented by R² or R⁴, the optionally substituted cycloalkyl group and the optionally substituted (cycloalkyl)alkyl group represented by R² or R³, the optionally substituted aryl group represented by R², R⁴ or R⁵, the optionally substituted aralkyl group and the optionally substituted styryl group represented by R², the optionally substituted alkenyl group represented by R³, and the optionally substituted methylene group represented by Y may be any kinds of substituents so long as they can bind as substituents to those groups. Number and kind of the substituents are not particularly limited. When two or more substituents are present, they may be the same or different. Examples of the substituents include, for example, a hydroxyl group which may be protected, an alkoxyl group, an amino group which may be substituted, a carbamoyl group which may be substituted, a halogen atom, an alkyl group, trifluoromethyl group, an alkanoyl group, a cycloalkyl group, an aryl group, an aryloxy group, cyano group, nitro group, guanidino group, amidino group, carboxyl group, an alkoxycarbonyl group, an aryloxycarbonyl group, an aralkyloxycarbonyl group and the like. As the protective group of hydroxyl group, any kind of protective group may be used so long as the protective group is substantially inactive in a system in which the hydroxyl group should not participate in a reaction and easily cleaved under a particular deprotection condition. Examples of the protective group include, for example, an alkanoyl group, a halogenoalkanoyl group, a trialkylsilyl group, benzyl group and the like. Examples of the alkanoyl group as the protective group of hydroxyl group include, for example, formyl group, acetyl group, propionyl group, butyryl group, trimethylacetyl group and the like, and examples of the halogenoalkanoyl group as the protective group of hydroxyl group include, for example, trifluoroacetyl group, trichloroacetyl group and the like. Examples of the trialkylsilyl group as the protective group of hydroxyl group include, for example, trimethylsilyl group, triethylsilyl group and the like. The alkoxyl group may be a linear or branched alkoxyl group having 1 to 6 carbon atoms, and examples thereof include, for example, methoxy group, ethoxy group, n-propoxy group, isopropoxy group, n-butoxy group, isobutoxy group, sec-butoxy group, tert-butoxy group, n-pentyloxy group, isopentyloxy group, neopentyloxy group, tert-pentyloxy group, n-hexyloxy group and the like. Examples of the amino group which may be substituted include, for example, amino group, methylamino group, ethylamino group, n-propylamino group, isopropylamino group, n-butylamino group, isobutylamino group, sec-butylamino group, tert-butylamino group, n-pentylamino group, isopentylamino group, neopentylamino group, tert-pentylamino group, n-hexylamino group, N,N-dimethylamino group, N,N-diethylamino group and the like. Examples of the carbamoyl group which may be substituted include, for example, carbamoyl group, N-methylcarbamoyl group, N-ethylcarbamoyl group, N-n-propylcarbamoyl group, N-isopropylcarbamoyl group, N-n-butylcarbamoyl group, N-isobutylcarbamoyl group, N-sec-butylcarbamoyl group, N-tert-butylcarbamoyl group, N-n-pentylcarbamoyl group, N-isopentylcarbamoyl group, N-neopentylcarbamoyl group, N-n-hexylcarbamoyl group, N,N-dimethylcarbamoyl group, N,N-diethylcarbamoyl group and the like. The halogen atom may be any of fluorine atom, chlorine atom, bromine atom and iodine atom. Examples of the alkanoyl group include, for example, acetyl group, propionyl group, butyryl group and the like, and examples of the aryloxy group include, for example, phenyloxy group, pyridyloxy group, pyrimidyloxy group, pyrazinyloxy group, naphthyloxy group, furyloxy group, benzofuranyloxy group, thienyloxy group, pyrrolyloxy group, quinolyloxy group, isoquinolyloxy group and the like.

As the alkyl group, alkanoyl group, cycloalkyl group, aryl group, alkoxycarbonyl group, aryloxycarbonyl group, and aralkyloxycarbonyl group which may be substituted, examples include those groups mentioned above.

The compounds represented by the aforementioned general formula (I) of the present invention may exist as stereoisomers such as optical isomers, diastereoisomers, geometrical isomers and the like. Any of these isomers and any mixtures thereof as well as salts thereof also fall within the scope of the present invention.

The compounds represented by the aforementioned general formula (I) of the present invention can be converted into salts, preferably pharmacologically acceptable salts, if desired, and the resulting salts can also be converted into compounds in free forms. Examples of salts of the compounds represented by the aforementioned general formula (I) of the present invention include, for example, acid addition salts or alkali addition salts. Examples of the acid addition salts include, for example, mineral acid salts such as hydrochlorides, hydrobromides, nitrates, sulfates, hydroiodides, and phosphates, organic acid salts such as acetates, propionates, butyrates, formates, trifluoroacetates, maleates, tartrates, citrates, stearates, succinates, lactobionates, gluconates, benzoates, methanesulfonates, ethanesulfonates, 2-hydroxyethanesulfonates, benzenesulfonates, p-toluenesulfonates, laurylsulfates, gluceptates, malates, aspartates, glutamates, adipates, oxalates, nicotinates, picrates, thiocyanates, undecanoates, mandelates, fumarates, 10-camphorsulfonates, lactates, 5-oxotetrahydrofuran-2-carboxylates and 2-hydroxyglutarates. Examples of the alkali addition salts include, for example, inorganic alkali salts such as sodium salts, potassium salts, calcium salts, magnesium salts and ammonium salts, and organic base salts such as ethanolamine salts and N,N-dialkylethanolamine salts, and the like.

The compounds represented by the aforementioned general formula (I) or salts thereof according to the present invention can exist in the forms of various crystals or hydrates or solvates with organic solvents depending on the manufacturing conditions. Any of these crystals, hydrates, solvates, and mixtures thereof also fall within the scope of the present invention.

Preferred compounds of the present invention include the compounds listed below. However, the present invention is not limited to these examples. In the tables, Me represents methyl group, Et represents ethyl group, n-Pr represents n-propyl group, i-Pr represents isopropyl group, n-Bu represents n-butyl group, n-Pent represents n-pentyl group, n-Hex represents n-hexyl group, n-Hept represents n-heptyl group, n-Oct represents n-octyl group, n-Non represents n-nonyl group, n-Dec represents n-decyl group, Ph represents phenyl group, and Bn represents benzyl group.

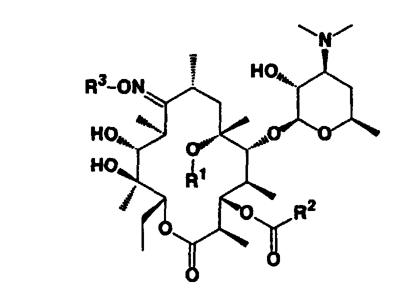

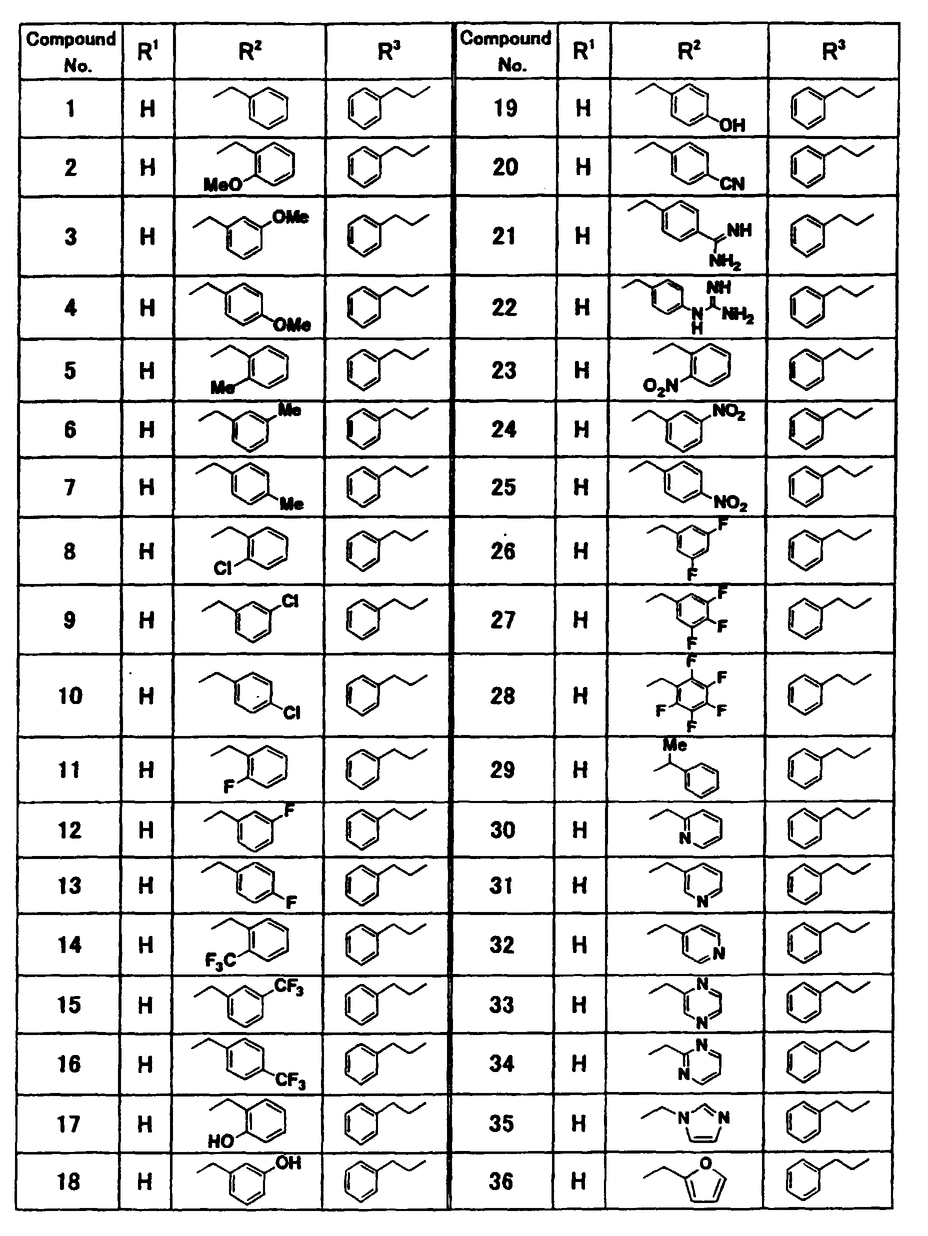

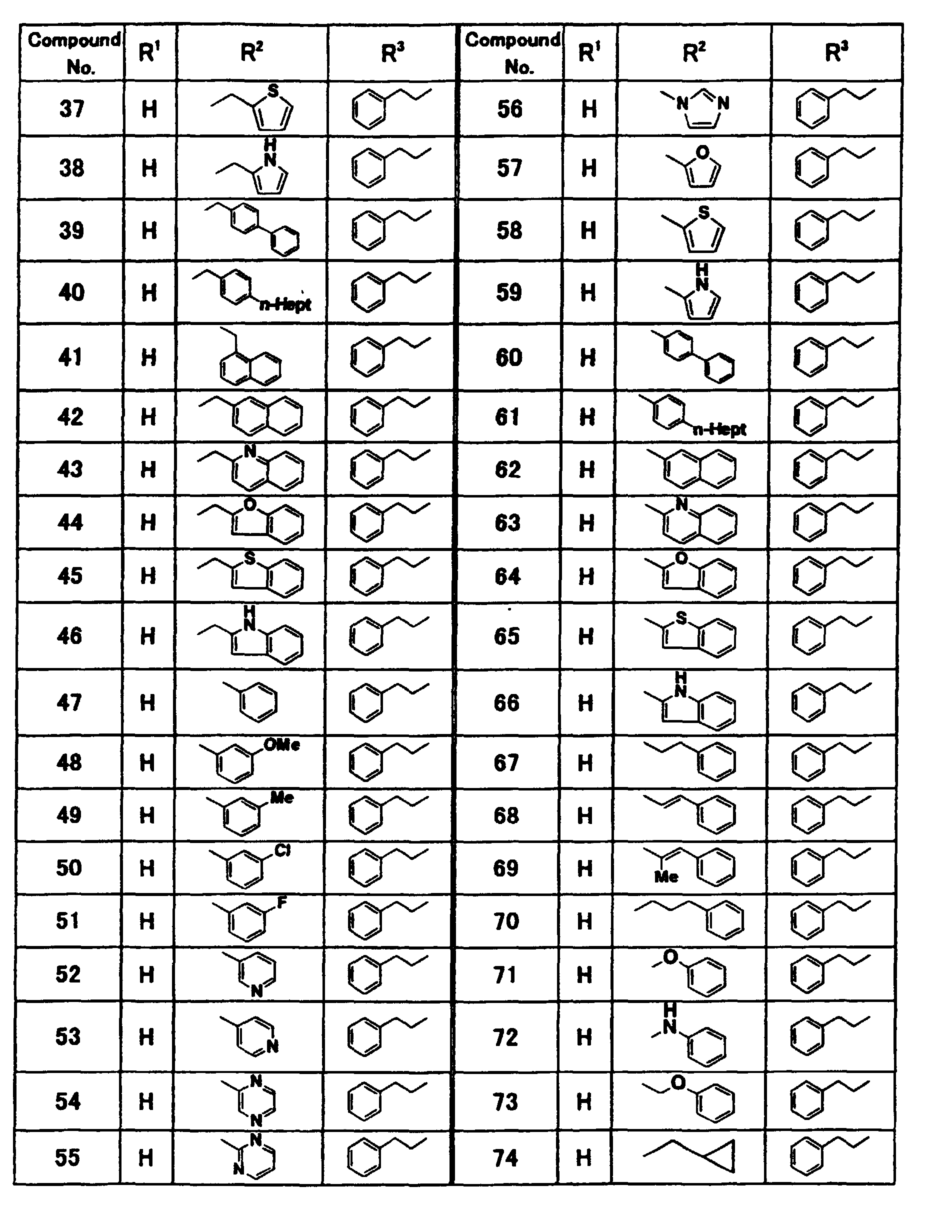

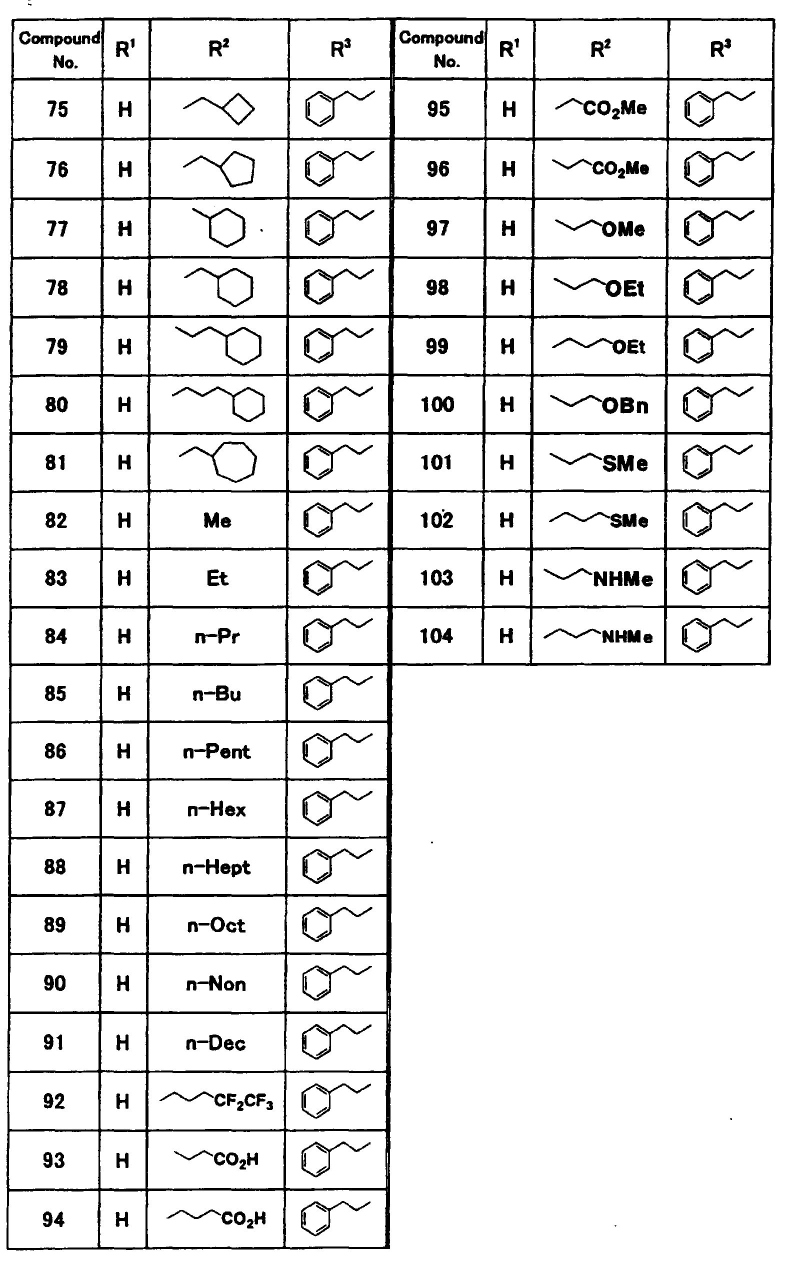

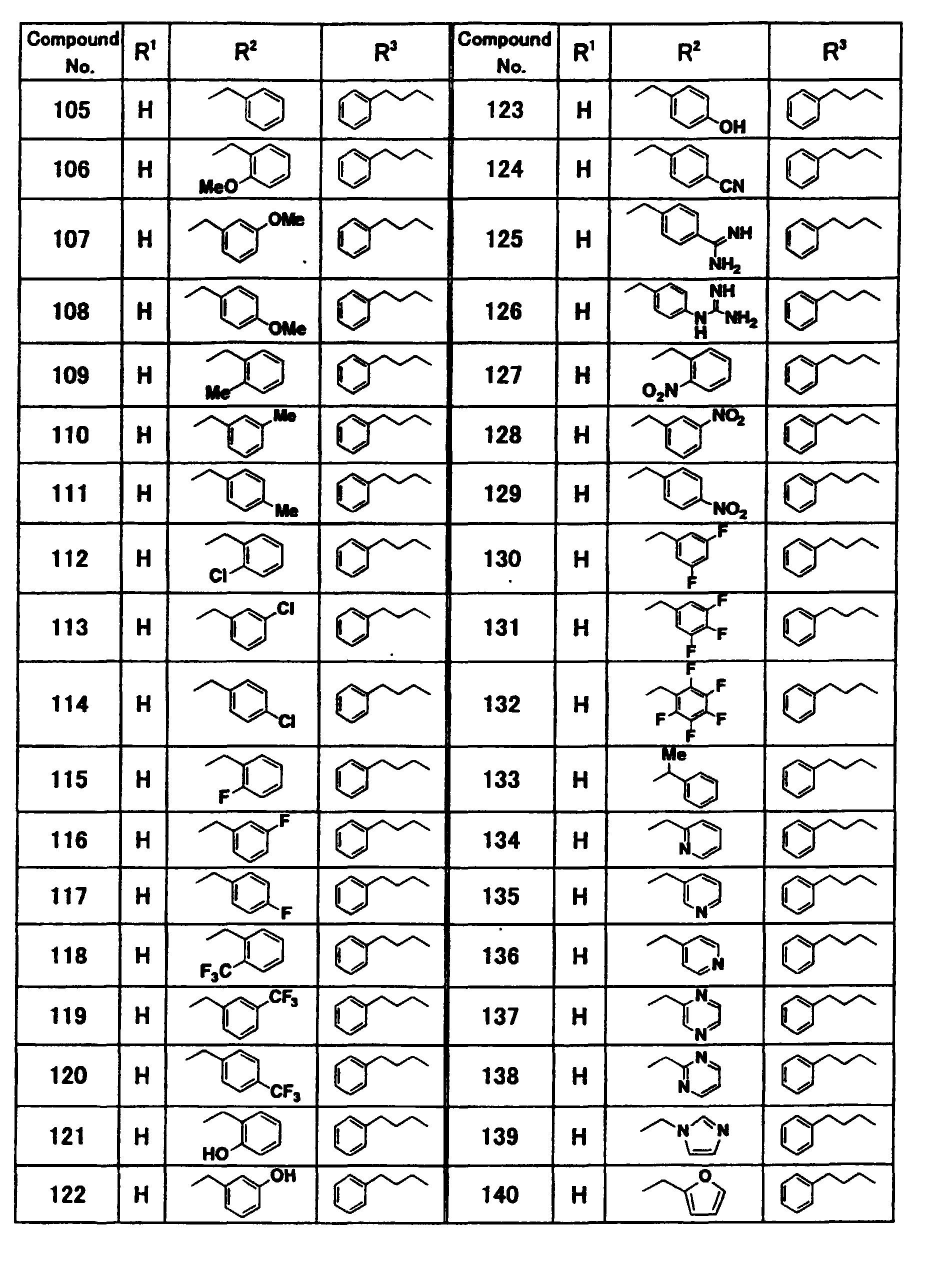

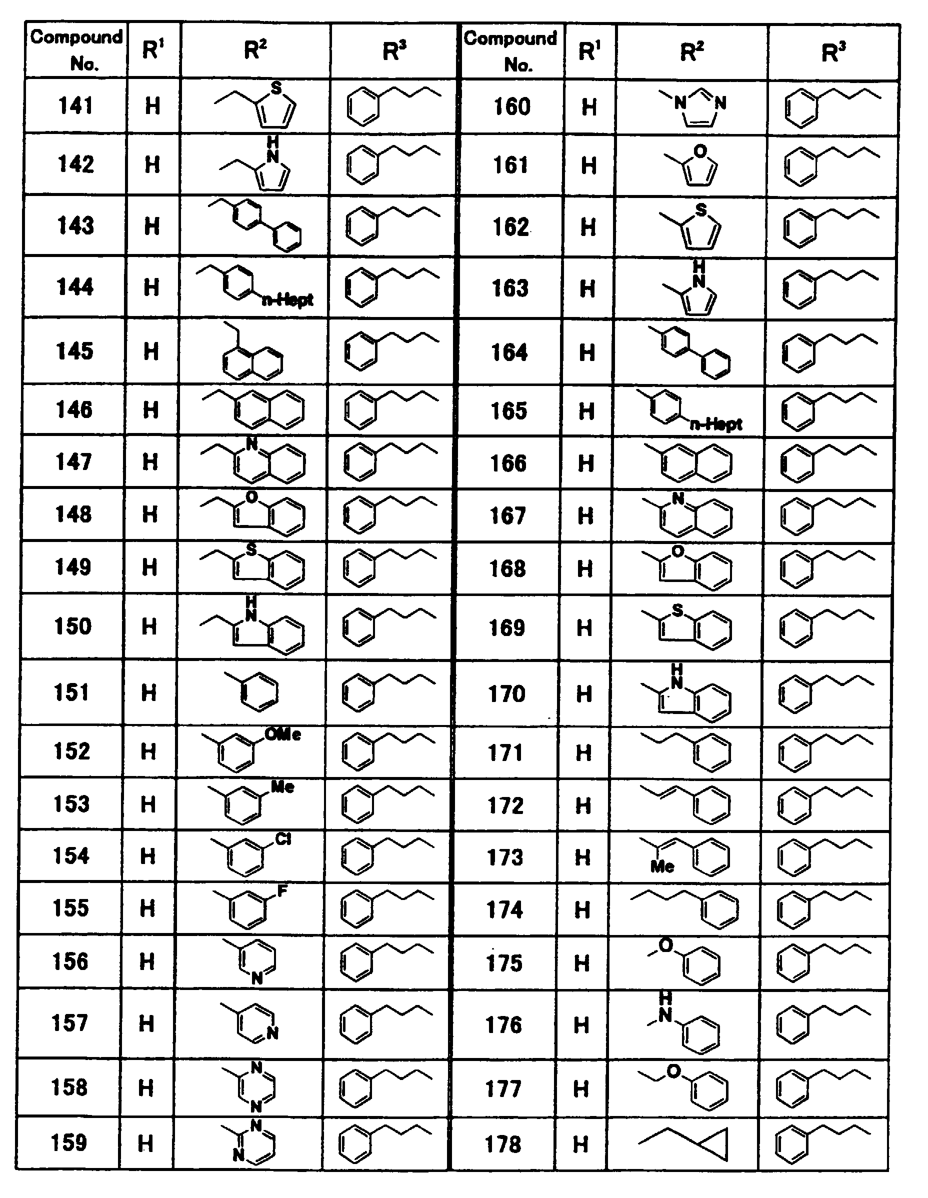

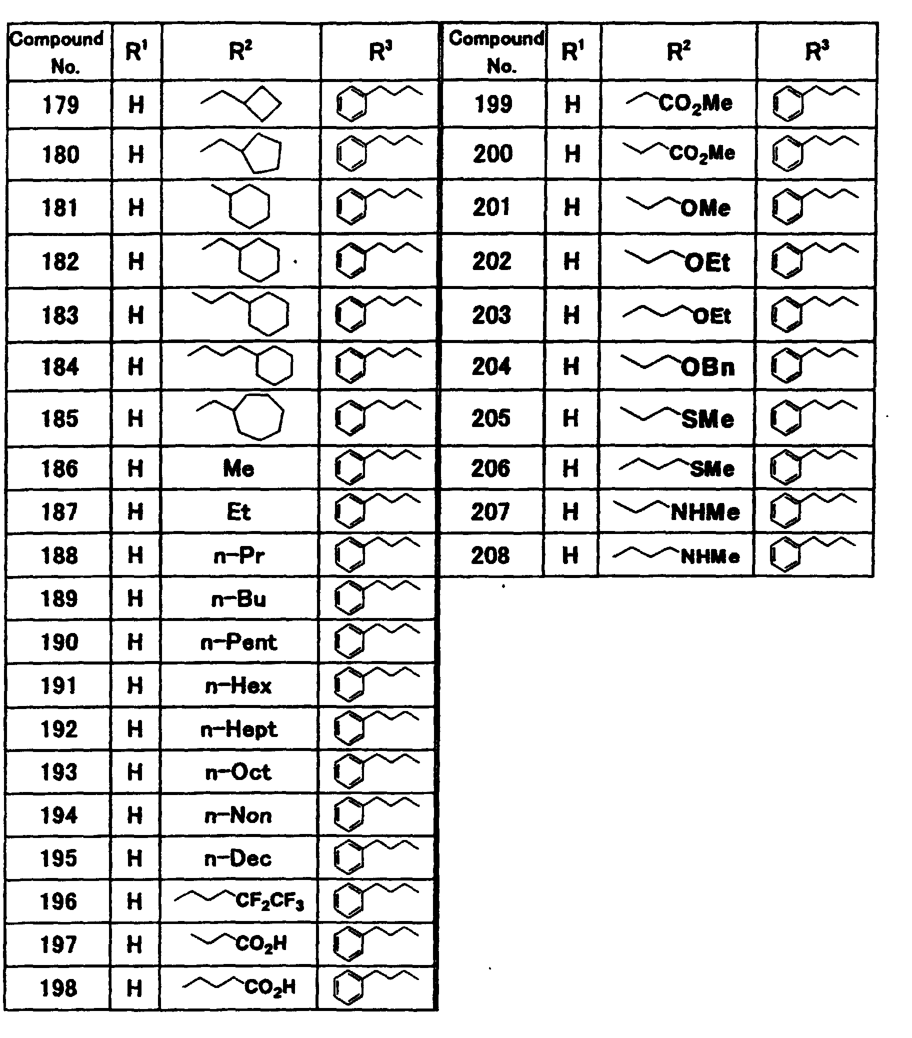

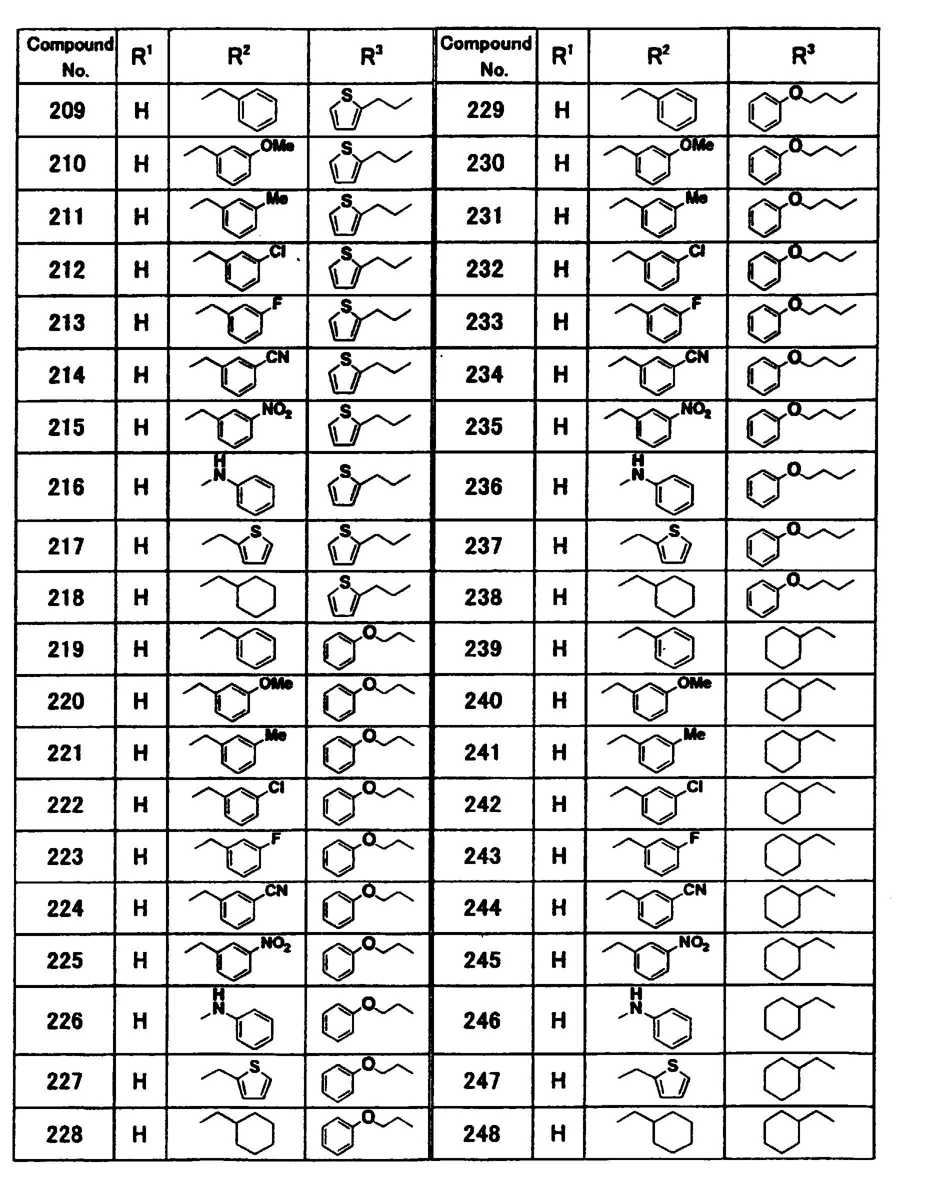

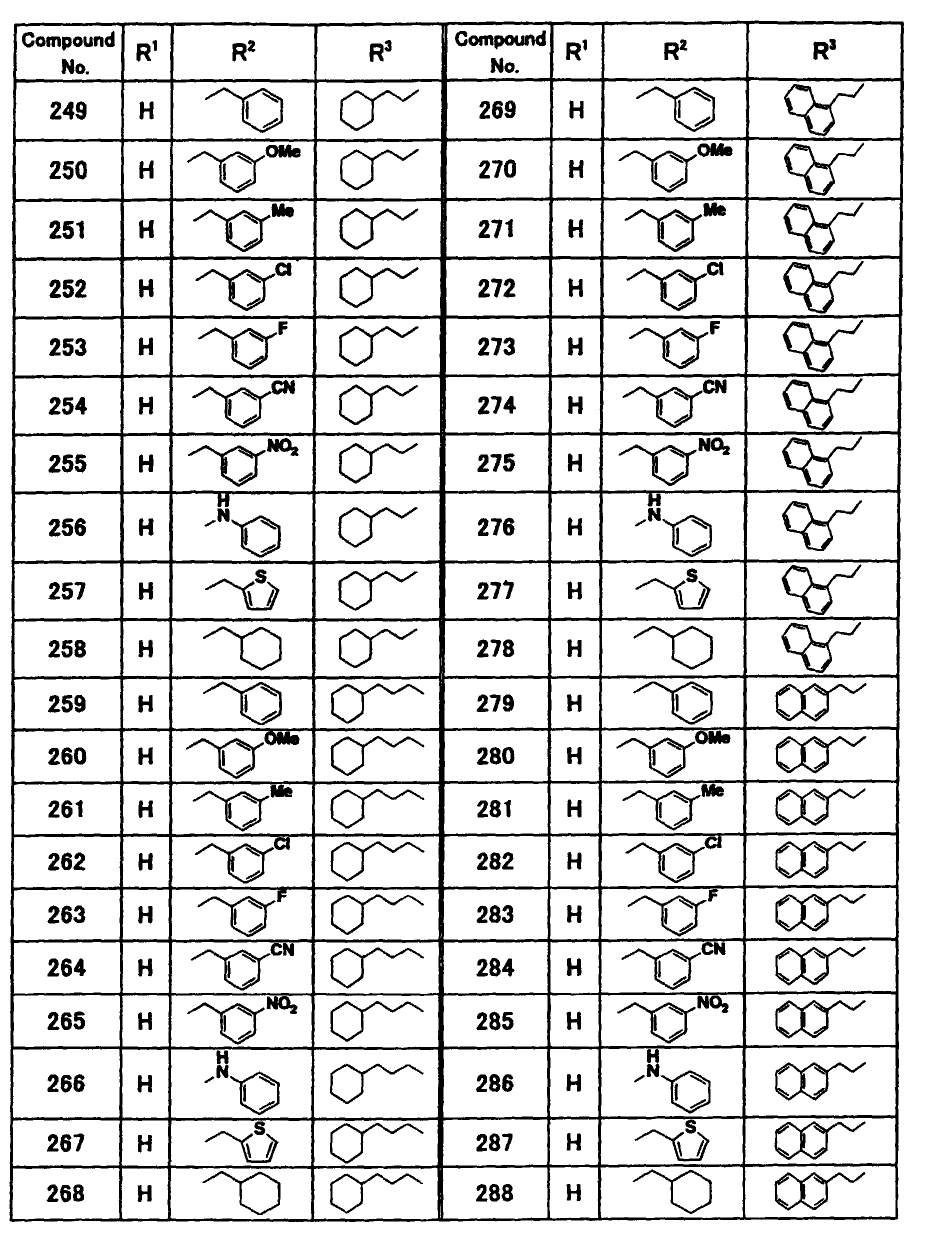

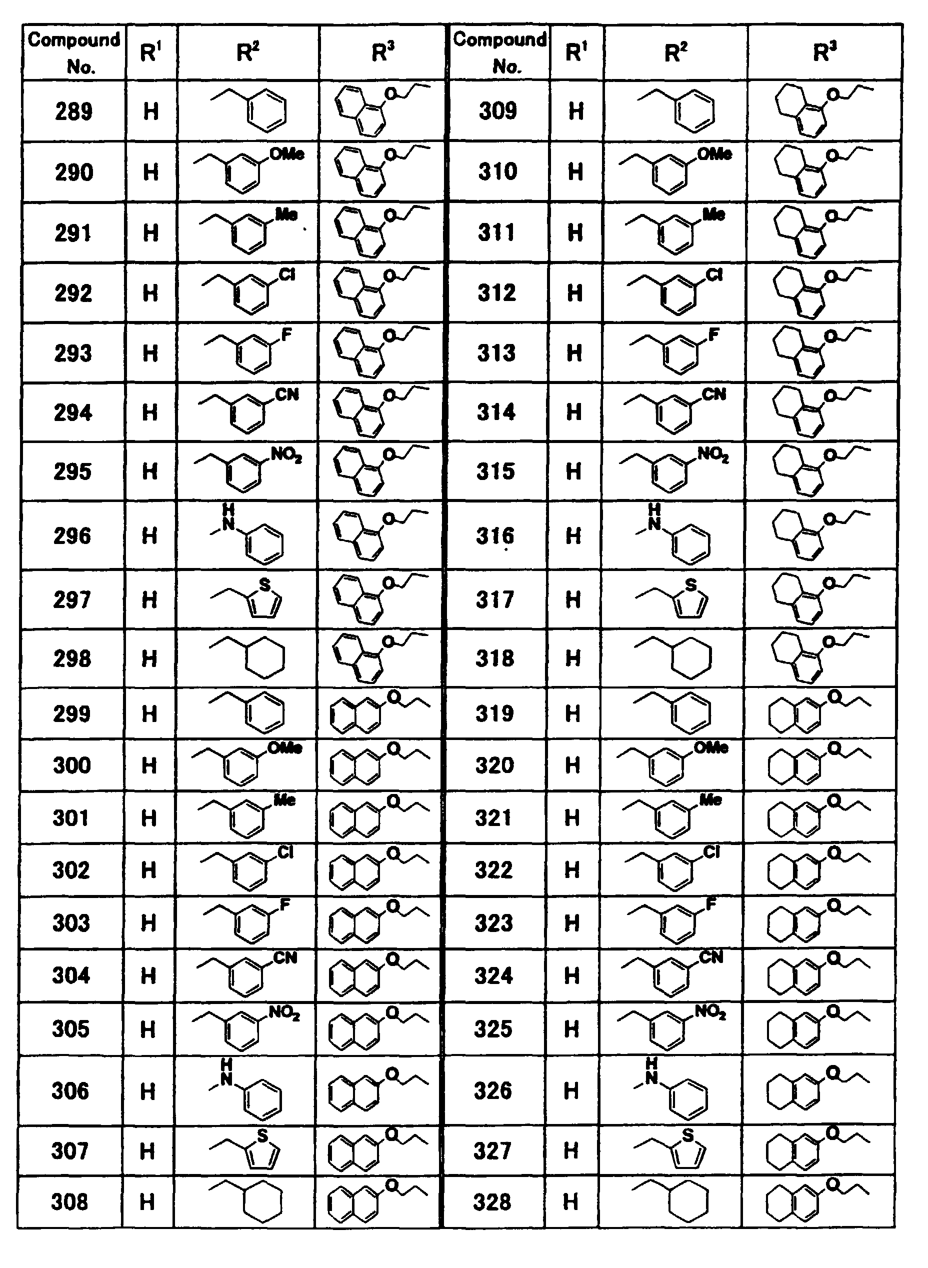

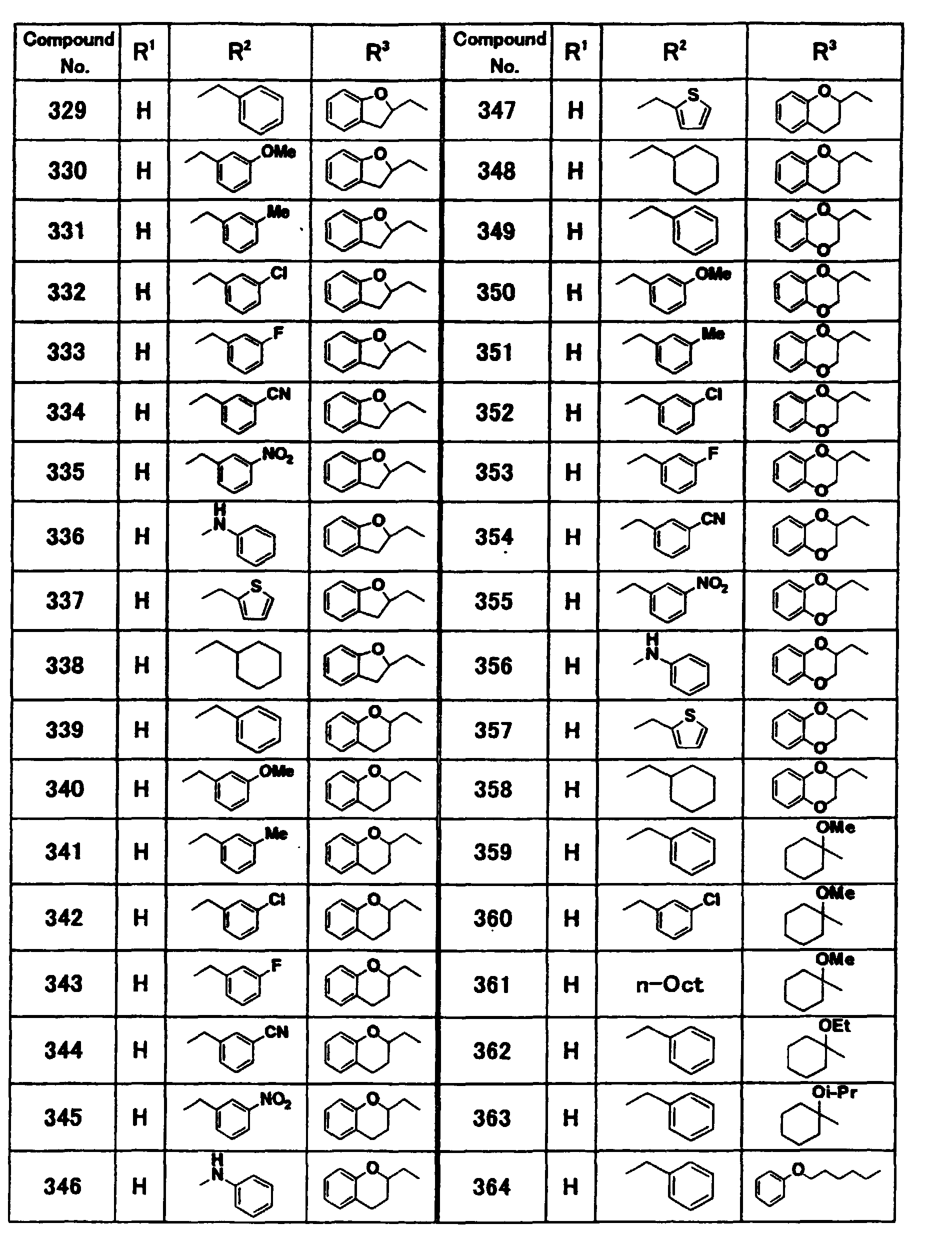

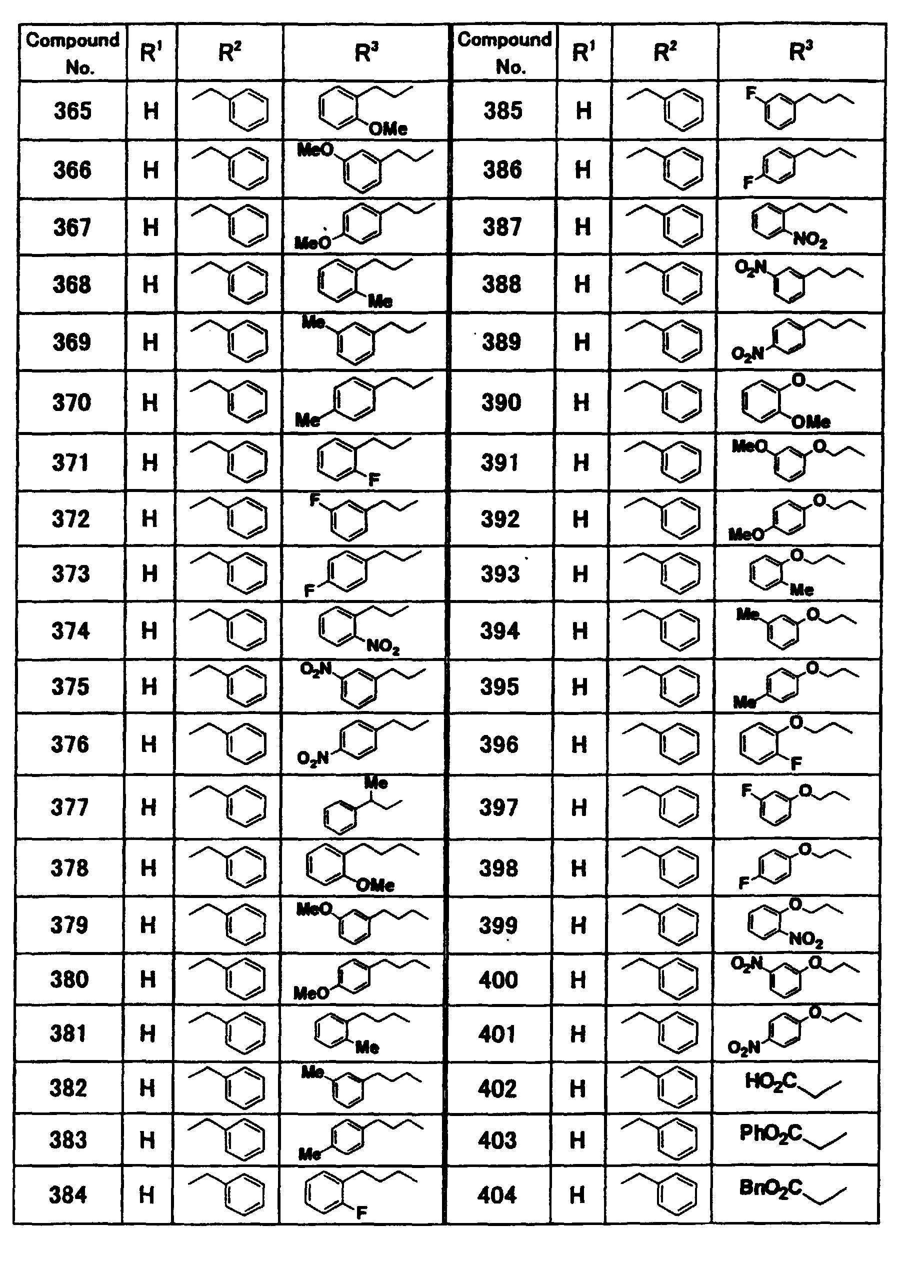

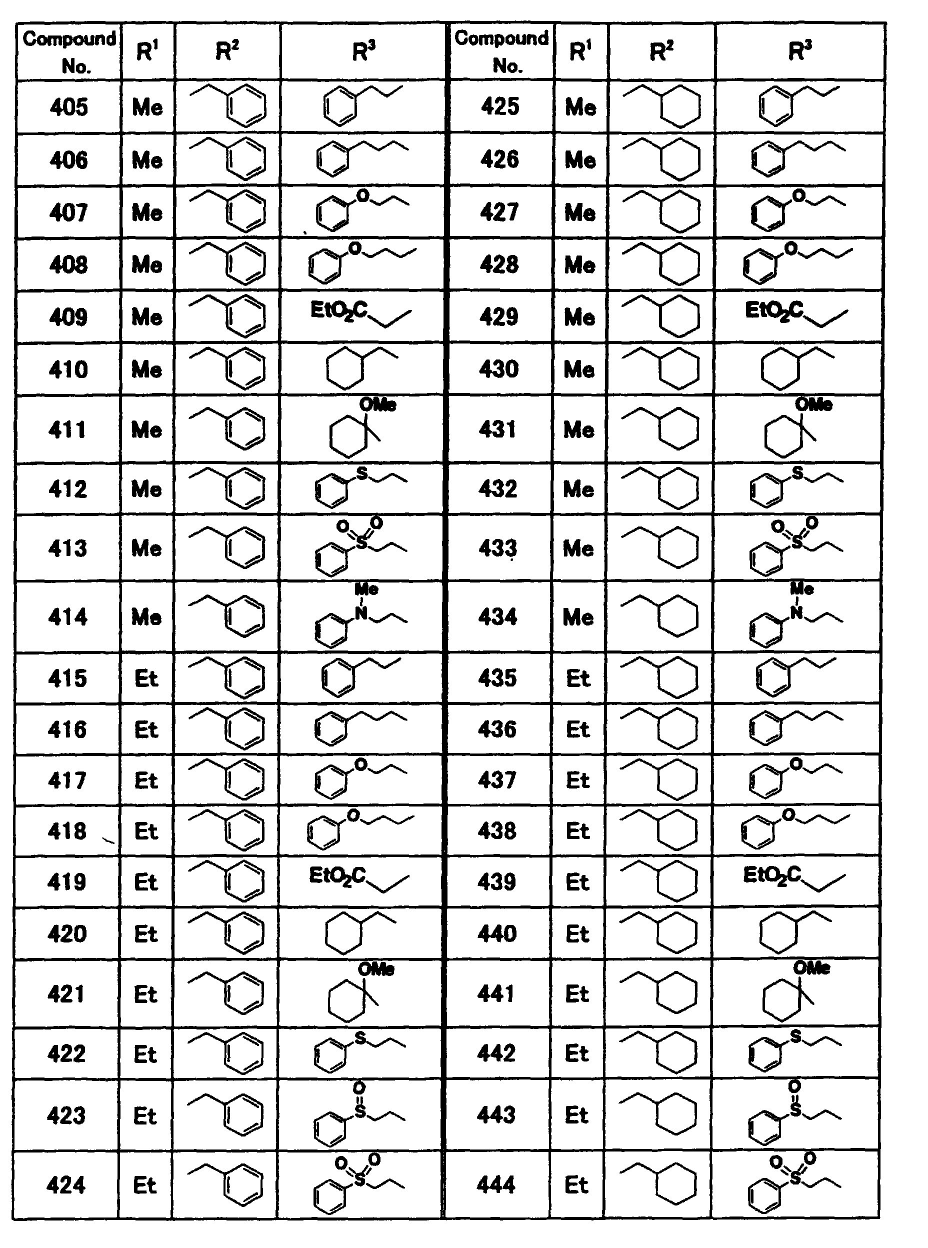

The novel erythromycin derivatives represented by the aforementioned general formula (I) of the present invention can be prepared by, for example, the methods explained below. However, the methods for preparing the compounds of the present invention are not limited to these methods.

According to the first embodiment of the method for preparing the compounds of the present invention, the compounds represented by the aforementioned general formula (I) can be prepared by reacting a compound represented by the following general formula (II): 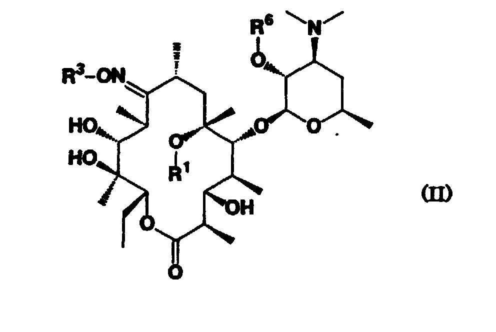 wherein R¹ and R³ have the same meanings as those defined above and R⁶ represents a hydrogen atom or a protective group of hydroxyl group, with a carboxylic acid derivative represented by the following general formula (III):

R²-CO₂H (III)

wherein R² has the same meaning as that defined above, together with a condensing agent in the presence or absence of a base without a solvent or in a solvent, and then eliminating the protective group, if necessary, or alternatively, with an acid anhydride represented by the following general formula (IV):

R²-CO₂U (IV)

wherein R² has the same meaning as that defined above and U represents an acid anhydride residue, or an acid halide or a halogenated carbonate derivative represented by the following general formula (V):

R²-COW (V)

wherein R² has the same meaning as that defined above and W represents a halogen atom, or an isocyanate derivative represented by the following general formula (VI):

R⁴-N=C=O (VI)

wherein R⁴ has the same meaning as that defined above, in the presence or absence of a base without a solvent or in a solvent, and then eliminating the protective group, if necessary.

Example of the condensing agent used in the above preparation include, for example, 1,3-dicyclohexylcarbodiimide, 1-ethyl-3-(3-dimethylaminopropyl)carbrodiimide hydrochloride, 1,1'-carbonyldiimidazole, Woodward reagent K (2-ethyl-5-phenylisoxazolium-3'-sulfonic acid) and the like. Example of the base to be used include, for example, organic bases such as triethylamine, pyridine, diisopropylethylamine, 4-dimethylaminopyridine, 1,8-diazabicyclo[5.4.0]-7-undecene and 1,2,2,6,6-pentamethylpiperidine, inorganic bases such as sodium carbonate, potassium carbonate, sodium hydrogencarbonate and potassium hydrogencarbonate and the like. The solvent used in the above preparation may be any solvent so long as the solvent per se is inert in the reaction and does not inhibit the reaction. Examples of the solvent include, for example, halogenated hydrocarbon solvents such as dichloromethane, 1,2-dichloroethane and chloroform, aromatic hydrocarbon solvents such as benzene and toluene, aprotic polar solvents such as acetone, acetonitrile, N,N-dimethylformamide, N-methyl-2-pyrrolidone, dimethyl sulfoxide, tetramethylene sulfolan, tetramethylene sulfoxide and hexamethylenephosphoric triamide, ester solvents such as methyl acetate and ethyl acetate, ether solvents such as tetrahydrofuran, diethyl ether and 1,4-dioxane, organic base solvents such as pyridine, picoline, lutidine and collidine, and mixed solvents thereof. The reaction is performed in a temperature ranging from ice-cooling to 200°C.

Deprotection in the above preparation of the present invention can be performed by various methods depending on the type of protective group R⁶ of hydroxyl group.

For example, where R⁶ is a protective group that forms an ester such as an alkanoyl group, a halogenoalkanoyl group and an arylcarbonyl group, desired compounds can be manufactured by hydrolysis in the presence or absence of an acid or an alkali without a solvent or in a solvent. The hydrolysis of an ester may be performed according to a process known per se, and an acid such as hydrochloric acid and sulfuric acid can be used for acidic hydrolysis, and an alkali such as sodium hydrogencarbonate, sodium carbonate, sodium hydroxide, lithium hydroxide, barium hydroxide, sodium methylate, sodium ethylate, sodium tert-butoxide and potassium tert-butoxide can be used for alkaline hydrolysis. Although these acids and alkalis may be used as aqueous solutions, they can be used in a solvent, for example, alcoholic solvent such as methanol, ethanol, n-propanol, isopropanol, n-butanol, sec-butanol and tert-butanol, aprotic polar solvents such as acetone, acetonitrile, N,N-dimethylformamide, dimethyl sulfoxide, tetramethylene sulfolan, tetramethylene sulfoxide and hexamethylenephosphoric triamide, ether solvents such as tetrahydrofuran and 1,4-dioxane, and water-containing solvents of these solvents. The reaction is performed in a temperature ranging from ice-cooling to 200°C.

Where R⁶ is a protective group of hydroxyl group that forms a carbonate such as an alkoxycarbonyl group, an aryloxycarbonyl group, and an aralkyloxycarbonyl group, desired compounds can be manufactured by deprotection using an acid in the presence or absence of a cation scavenger without a solvent or in a solvent, or by hydrogenolysis in the presence of a catalyst in a solvent.

The solvent to be used may be any solvent so long as the solvent per se is inert in the reaction and does not inhibit the reaction. Examples of the solvent include, for example, water, acetic acid, alcoholic solvent such as methanol, ethanol, n-propanol, isopropanol, n-butanol, sec-butanol and tert-butanol, halogenated hydrocarbon solvents such as dichloromethane, 1,2-dichloroethane and chloroform, aromatic hydrocarbon solvents such as benzene and toluene, aprotic polar solvents such as acetone, acetonitrile, N,N-dimethylformamide, N-methyl-2-pyrrolidone, dimethyl sulfoxide, tetramethylene sulfolan, tetramethylene sulfoxide and hexamethylenephosphoric triamide, ester solvents such as methyl acetate and ethyl acetate, ether solvents such as tetrahydrofuran, diethyl ether and 1,4-dioxane, and mixed solvents thereof and the like. Examples of the cation scavenger include, for example, anisole, thioanisole, thioethanol and the like. Examples of the acid to be used include, for example, hydrochloric acid, hydrobromic acid, trifluoroacetic acid, acetic acid, sulfuric acid and the like. The reaction is performed in a temperature ranging from ice-cooling to 200°C.

Examples of the catalyst used for the hydrogenolysis include, for example, palladium catalysts such as 5% palladium/carbon, 10% palladium/carbon and 20% palladium hydroxide/carbon, or platinum oxide and the like. Examples of a hydrogen source include cyclohexene, 1,3-cyclohexadiene, formic acid, ammonium formate and the like, as well as hydrogen gas. The solvent to be used may be any solvent so long as the solvent per se is inert in the reaction and does not inhibit the reaction. Examples of the solvent include, for example, water, acetic acid, alcoholic solvent such as methanol, ethanol, n-propanol, isopropanol, n-butanol, sec-butanol and tert-butanol, halogenated hydrocarbon solvents such as dichloromethane, 1,2-dichloroethane and chloroform, aromatic hydrocarbon solvents such as benzene and toluene, aprotic polar solvents such as acetone, acetonitrile, N,N-dimethylformamide, N-methyl-2-pyrrolidone, dimethyl sulfoxide, tetramethylene sulfolan, tetramethylene sulfoxide and hexamethylenephosphoric triamide, ester solvents such as methyl acetate and ethyl acetate, ether solvents such as tetrahydrofuran, diethyl ether and 1,4-dioxane, and mixed solvents thereof and the like. The reaction is performed in a temperature ranging from room temperature to 200°C under a hydrogen pressure of from ordinary pressure to 200 kgf/cm².

Where R⁶ is a protective group of hydroxyl group that forms a trialkylsilyl type, the desired compounds can be manufactured by deprotection by using an acid or tetrabutylammonium fluoride without a solvent or in a solvent.

The solvent to be used may be any solvent so long as the solvent per se is inert in the reaction and does not inhibit the reaction. Examples of the solvent include, for example, water, acetic acid, alcoholic solvent such as methanol, ethanol, n-propanol, isopropanol, n-butanol, sec-butanol and tert-butanol, halogenated hydrocarbon solvents such as dichloromethane, 1,2-dichloroethane and chloroform, aromatic hydrocarbon solvents such as benzene and toluene, aprotic polar solvents such as acetone, acetonitrile, N,N-dimethylformamide, N-methyl-2-pyrrolidone, dimethyl sulfoxide, tetramethylene sulfolan, tetramethylene sulfoxide and hexamethylenephosphoric triamide, ester solvents such as methyl acetate and ethyl acetate, ether solvents such as tetrahydrofuran, diethyl ether and 1,4-dioxane, and mixed solvents thereof and the like. Examples of the acid to be used include, for example, mineral adds such as hydrofluoric acid, hydrochloric acid, hydrobromic acid and sulfuric acid, organic acids such as trifluoroacetic acid, acetic acid, p-toluenesulfonic acid, citric acid and oxalic acid and the like. The reaction is performed in a temperature ranging from ice-cooling to 200°C.

According to the second embodiment of the method for preparing the compounds of the present invention, the compounds represented by the aforementioned general formula (I) can be prepared by reacting a compound represented by the following general formula (VII): 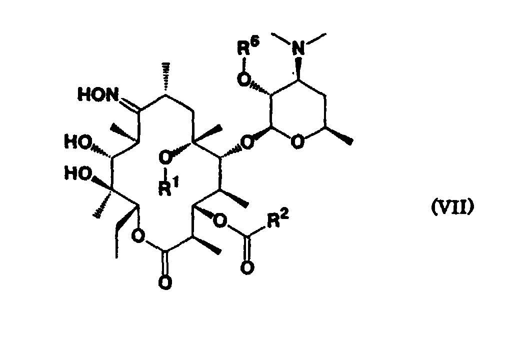 wherein R¹, R², and R⁶ have the same meanings as those defined above, with a compound represented by the following general formula (VIII):

R³-Z (VIII)

wherein R³ has the same meaning as that defined above and Z represents a halogen atom, methanesulfonyloxy group or p-toluenesulfonyloxy group, and with tetrabutylammonium iodide in the presence or absence of sodium iodide or a base without a solvent or in a solvent, and then eliminating the protective group, if necessary.

Example of the base used in the above preparation include, for example, organic bases such as triethylamine, diisopropylethylamine, 4-dimethylaminopyridine, 1,8-diazabicyclo[5.4.0]-7-undecene and 1,2,2,6,6-pentamethylpiperidine, inorganic bases such as sodium carbonate, potassium carbonate, sodium hydrogencarbonate, potassium hydrogencarbonate, sodium hydride, sodium hydroxide and potassium hydroxide and the like. The solvent to be used may be any solvent so long as the solvent per so is inert in the reaction and does not inhibit the reaction. Examples of the solvent include, for example, halogenated hydrocarbon solvents such as dichloromethane, 1,2-dichloroethane and chloroform, aromatic hydrocarbon solvents such as benzene and toluene, aprotic polar solvents such as acetone, acetonitrile, N,N-dimethylformamide, N-methyl-2-pyrrolidone, dimethyl sulfoxide, tetramethylene sulfolan, tetramethylene sulfoxide and hexamethylenephosphoric triamide, ester solvents such as methyl acetate and ethyl acetate, ether solvents such as tetrahydrofuran, diethyl ether and 1,4-dioxane, organic base solvents such as pyridine, picoline, lutidine and collidine, and mixed solvents thereof and the like. The reaction is performed in a temperature ranging from ice-cooling to 200°C.

The deprotection in the above preparation can be performed by various methods depending on the kind of the protective group R⁶ of hydroxyl group, and can be performed according to the method explained in the aforementioned first embodiment of the preparation.

According to the third embodiment of the method for preparing the compounds of the present invention, among the compounds represented by the aforementioned general formula (I), the compounds wherein R³ is a cycloalkyl group which may be substituted can be prepared by reacting a compound represented by the aforementioned general formula (VII), with a cycloalkyl compound represented by the following general formula (IX): 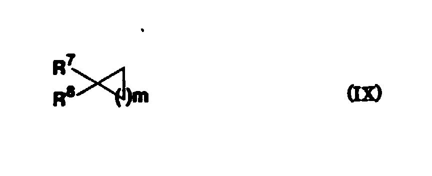 wherein R⁷ and R⁸ represent an alkoxyl group, and m represents an integer of from 1 to 5, or a cycloalkene compound represented by the following general formula (X): 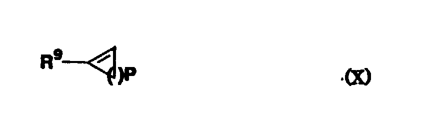 wherein R⁹ represents an alkoxyl group, and p represents an integer of from 1 to 5, in the presence or absence of an acid catalyst without a solvent or in a solvent, and then eliminating the protective group, if necessary.

Example of the acid used in the above preparation include, for example, pyridinium hydrochloride, pyridinium trifluoroacetate, pyridinium p-toluenesulfonate and the like. The solvent to be used may be any solvent so long as the solvent per se is inert in the reaction and does not inhibit the reaction. Examples of the solvent include, for example, halogenated hydrocarbon solvents such as dichloromethane, 1,2-dichloroethane and chloroform, aromatic hydrocarbon solvents such as benzene and toluene, aprotic polar solvents such as acetone, acetonitrile, N,N-dimethylformamide, N-methyl-2-pyrrolidone, dimethyl sulfoxide, tetramethylene sulfolan, tetramethylene sulfoxide and hexamethylenephosphoric triamide, ester solvents such as methyl acetate and ethyl acetate, ether solvents such as tetrahydrofuran, diethyl ether and 1,4-dioxane, and mixed solvents thereof and the like. The reaction is performed in a temperature ranging from ice-cooling to 200°C.

The deprotection in the above preparation can be performed by various methods depending on the kind of the protective group R⁶ of hydroxyl group, and can be performed according to the methods explained in the aforementioned first embodiment of the preparation.

According to the fourth embodiment of the method for preparing the compounds of the present invention, the compounds represented by the aforementioned general formula (I) can be prepared by reacting a compound represented by the following general formula (XI): 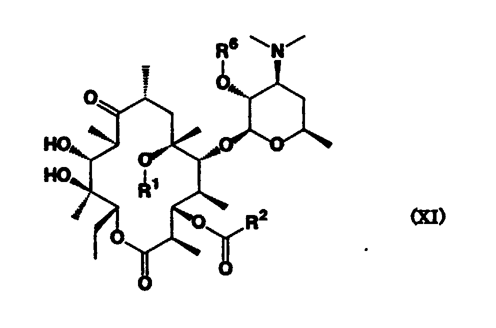 wherein R¹, R² and R⁶ have the same meanings as those defined above, with a hydroxylamine derivative represented by the following general formula (XII) or a salt thereof.

R³-O-NH₂ (XII)

wherein R³ has the same meaning as that defined above, in the presence or absence of a base without a solvent or in a solvent, and eliminating the protective group, if necessary.

Example of the base used in the above preparation include, for example, organic bases such as triethylamine, pyridine, imidazole, diisopropylethylamine, 4-dimethylaminopyridine, 1,8-diazabicyclo[5.4.0]-undecene and 1,2,2,6,6-pentamethylpiperidine, inorganic bases such as sodium carbonate, potassium carbonate, sodium hydrogencarbonate and potassium hydrogencarbonate. The solvent to be used may be any solvent so long as the solvent per se is inert in the reaction and does not inhibit the reaction. Examples of the solvent include, for example, alcoholic solvent such as methanol, ethanol, n-propanol, isopropanol, n-butanol, sec-butanol and tert-butanol, halogenated hydrocarbon solvents such as dichloromethane, 1,2-dichloroethane and chloroform, aromatic hydrocarbon solvents such as benzene and toluene, aprotic polar solvents such as acetone, acetonitrile, N,N-dimethylformamide, N-methyl-2-pyrrolidone, dimethyl sulfoxide, tetramethylene sulfolan, tetramethylene sulfoxide and hexamethylenephosphoric triamide, ester solvents such as methyl acetate and ethyl acetate, ether solvents such as tetrahydrofuran, diethyl ether and 1,4-dioxane, organic base solvents such as pyridine, picoline, lutidine and collidine, and mixed solvents thereof and the like. The reaction is performed in a temperature ranging from ice-cooling to 200°C.

The deprotection in the above preparation can be performed by various methods depending on the kind of the protective group R⁶ of hydroxyl group, and can be performed according to the methods explained in the aforementioned first embodiment for preparation.

According to the fifth embodiment of the method for preparing the compounds of the present invention, among the compounds represented by the aforementioned general formula (I), the compounds wherein R² is represented by -X-R⁴ wherein X is oxygen atom and R⁴ is an alkyl group which may be substituted can be prepared by reacting a compound wherein X is an oxygen atom and R⁴ is an aryl group which may be substituted, which is prepared according to the first embodiment of the preparation, with an alcohol derivative represented by the following general formula (XIII):

R¹⁰-OH (XIII)

wherein R¹⁰ represents an alkyl group which may be substituted, without a solvent or in a solvent, and then eliminating the protective group, if necessary.

The solvent used in the method for preparing may be any solvent so long as the solvent per se is inert in the reaction and does not inhibit the reaction. Examples of the solvent include, for example, halogenated hydrocarbon solvents such as dichloromethane, 1,2-dichloroethane and chloroform, aromatic hydrocarbon solvents such as benzene and toluene, aprotic polar solvents such as acetone, acetonitrile, N,N-dimethylformamide, N-methyl-2-pyrrolidone, dimethyl sulfoxide, tetramethylene sulfolan, tetramethylene sulfoxide and hexamethylenephosphoric triamide, ester solvents such as methyl acetate and ethyl acetate, ether solvents such as tetrahydrofuran, diethyl ether and 1,4-dioxane, organic base solvents such as pyridine, picoline, lutidine and collidine, and mixed solvents thereof and the like. The reaction is performed in a temperature ranging from ice-cooling to 200°C.

The deprotection in the above preparation can be performed by various methods depending on the kind of the protective group R⁶ of hydroxyl group, and can be performed according to the methods explained in the aforementioned first embodiment of the preparation.

The compounds represented by the aforementioned general formulas (II) and (VII) used as starting materials in the methods for preparation according to the present invention are partly known compounds, which are disclosed in, for example, Japanese Patent Unexamined Publication {Kokai} Nos. 63-264495/1988, 8-104640/1996, International Publication WO93/13116 and the like, and for example, they can be prepared as shown below. Among the compounds represented by the aforementioned general formulas (II) and (VII), the compounds wherein R⁶ is a protective group of hydroxyl group can be prepared by introducing a protective group into a compound whose R⁶ is a hydrogen atom according to the process for preparation described below. As for novel compounds among the compounds, preparations thereof are described in Reference Examples. 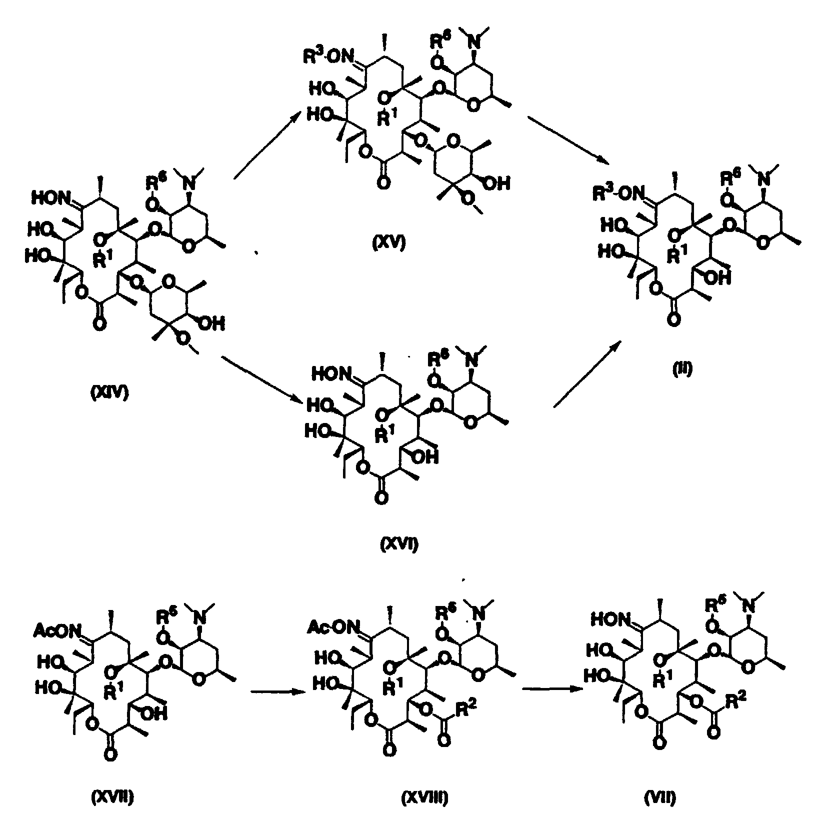 (In the formulas, Ac represents acetyl group and R¹, R², R³, and R⁶ have the same meanings as those defined above.)

The medicaments comprising as an active ingredient at least one of the novel erythromycin derivatives represented by the aforementioned general formula (I) or salts thereof thus prepared can be used for therapeutic treatment of infectious diseases, preferably atypical acid-fast mycobacteriosis, and most preferably infections by *Mycobacterium avium* complex. The medicaments may also be used for preventive treatment of these infectious diseases. The aforementioned medicaments are usually administered as preparations for oral administration such as capsules, tablets, subtilized granules, granules, powders, and syrups, or preparations such as injections, suppositories, eye drops, ophthalmologic ointments, ear drops, and dermatological preparations. As the active ingredient, hydrates and solvates of the aforementioned erythromycin derivatives or salts thereof may also be used. These preparations can be manufactured in a conventional manner by admixing with pharmacologically and pharmaceutically acceptable additives. For the preparation of orally available preparations and suppositories, additives such as, for example, excipients such as lactose, D-mannitol, corn starch and crystalline cellulose; disintegrating agents such as carboxymethylcellulose and calcium carboxymethylcellulose; binders such as hydroxypropylcellulose, hydroxypropylmethylcellulose and polyvinylpyrrolidone; lubricants such as magnesium stearate, and talc; coating agents such as hydroxypropylmethylcellulose, sucrose and titanium oxide; plasticizers such as polyethylene glycol; bases such as polyethylene glycol and hard fat and the like may be used. For injections, eye drops and ear drops, additives such as, for example, dissolving agents and dissolving aids that can constitute aqueous preparations or preparations to be dissolved upon use such as distilled water for injection, physiological saline and propylene glycol; pH modifiers such as inorganic or organic acids and bases, isotonic agents such as sodium chloride, glucose and glycerin, stabilizers and the like may be used. For ophthalmologic ointments and dermatological preparations, additives suitable for ointments, creams and patches such as white soft paraffin, macrogol, glycerin, liquid paraffin and cotton cloth may be used.

Doses of the medicaments of the present invention are not particularly limited, and generally, they can be administered to an adult in an amount of about 10 to 2000 mg as an active ingredient per day for oral administration, or about 1 to 1000 mg per day for parenteral administration, which daily doses may be administered at one time, or several times as divided portions. However, it is desirable that the doses are suitably increased or decreased depending on the purpose of the administration, i.e., therapeutic or preventive treatment, a region of infection and the type of pathogenic bacteria, the age and symptoms of a patient and the like.

### Examples

The present invention will be further explained with reference to the following reference examples and examples. However, the present invention is not limited to these examples. In the following tables, Me represents methyl group, Et represents ethyl group, n-Pr represents n-propyl group, i-Pr represents isopropyl group, n-Bu represents n-butyl group, n-Pent represents n-pentyl group, n-Hept represents n-heptyl group, n-Non represents n-nonyl group, Bn represents benzyl group, Ac represents acetyl group, HR-MS is an abbreviation of High-Resolution Mass-Spectrum, and Anal. Calcd. indicates values of elemental analysis.

### Reference Example 1: Erythromycin A 9-[O-(phenethyl)oxime]

To a mixture of 5.00g of erythromycin A 9-oxime, 0.13 g of tetrabutylammomnium iodide and 1.49 g of (2-bromoethyl)benzene in 30 ml of tetrahydrofuran, 0.53 g of powdered potassium hydroxide was added at room temperature with stirring, and the reaction mixture was stirred at room temperature for 3.5 hours. And then 1.49 g of (2-bromoethyl)benzene and 0.50 g of powdered potassium hydroxide were added to the reaction mixture, and the mixture was stirred at room temperature for 2.5 hours. The reaction mixture was poured into ice-water, and the mixture was made alkaline with saturated aqueous sodium hydrogencarbonate solution and extracted with ethyl acetate. The extract was washed with water, dried over sodium sulfate, and the solvent was removed under reduced pressure. The residue was added with diisopropyl ether for solidification to give 5.30 g of a pale yellowish brown solid.
NMR spectrum δ (CDCl₃) ppm: 0.85 (3H, t, J=7.5Hz), 0.98-1.70 (34H, m), 1.88-2.00 (2H, m), 2.20 (1H,d, J=10.5Hz), 2.28 (6H,s), 2.37 (1H,d, J=16Hz), 2.37-2.46 (1H, m), 2.60-2.69 (1H, m), 2.85-2.99 (3H, m), 3.03 (1H, t, J=10Hz), 3.12 (1H, s), 3.20 (1H, dd, J=10.5, 7.5Hz), 3.32 (3H, s), 3.37 (1H, s), 3.42-3.53 (2H, m), 3.54-3.63 (1H, m), 3.71 (1H, s), 3.95-4.05 (2H, m), 4.28 (2H, t, J=6.5Hz), 4.39 (1H,d, J=7.5Hz), 4.46 (1H, s), 4.93 (1H, d, J=5Hz), 5.14 (1H, dd, J=11, 2.5Hz), 7.17-7.34 (5H, m)

Compounds of Reference Examples 2 through 37 were obtained in the same manner as that described in Reference Example 1.

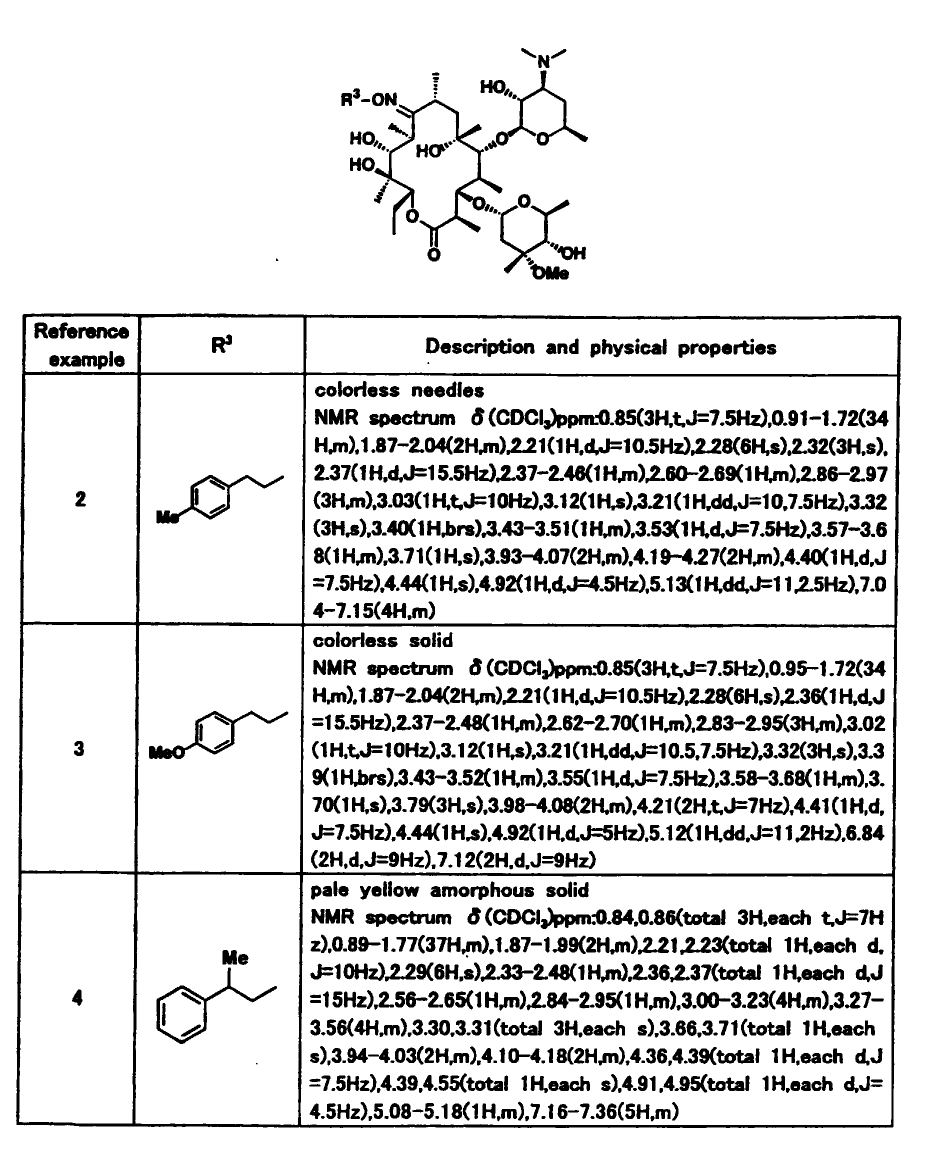

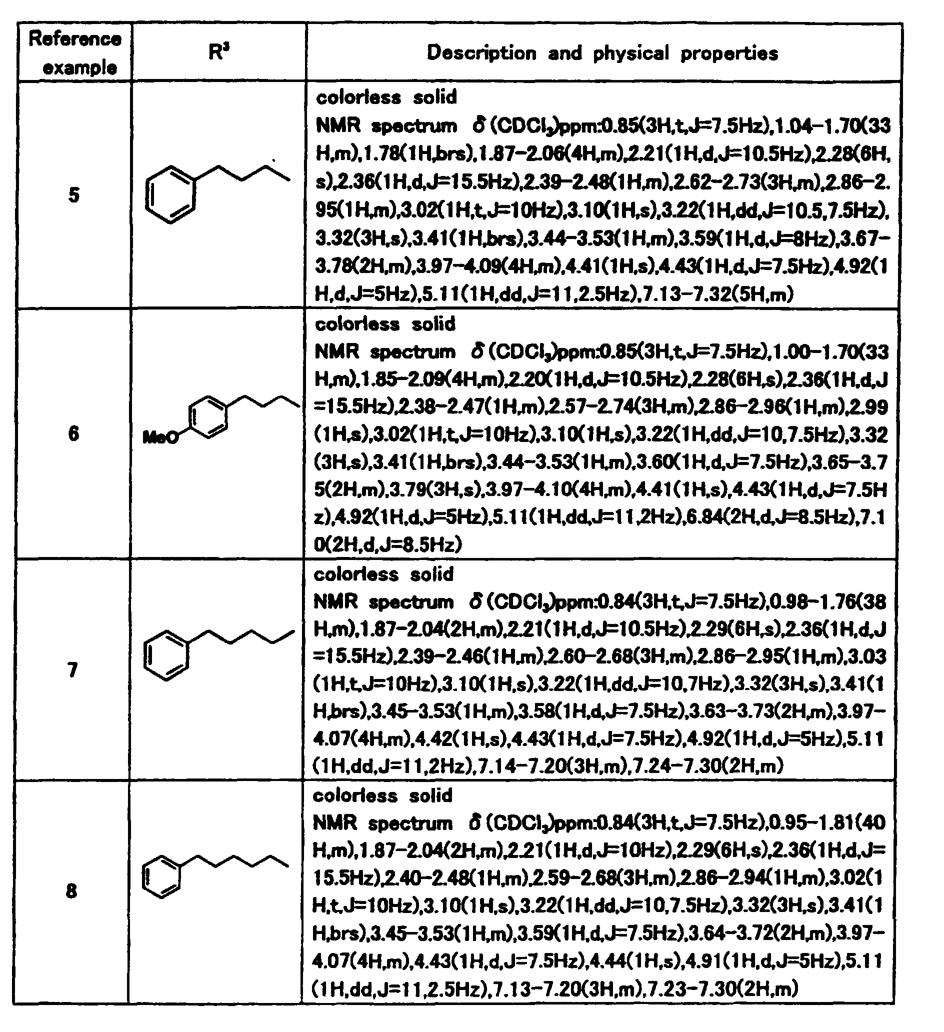

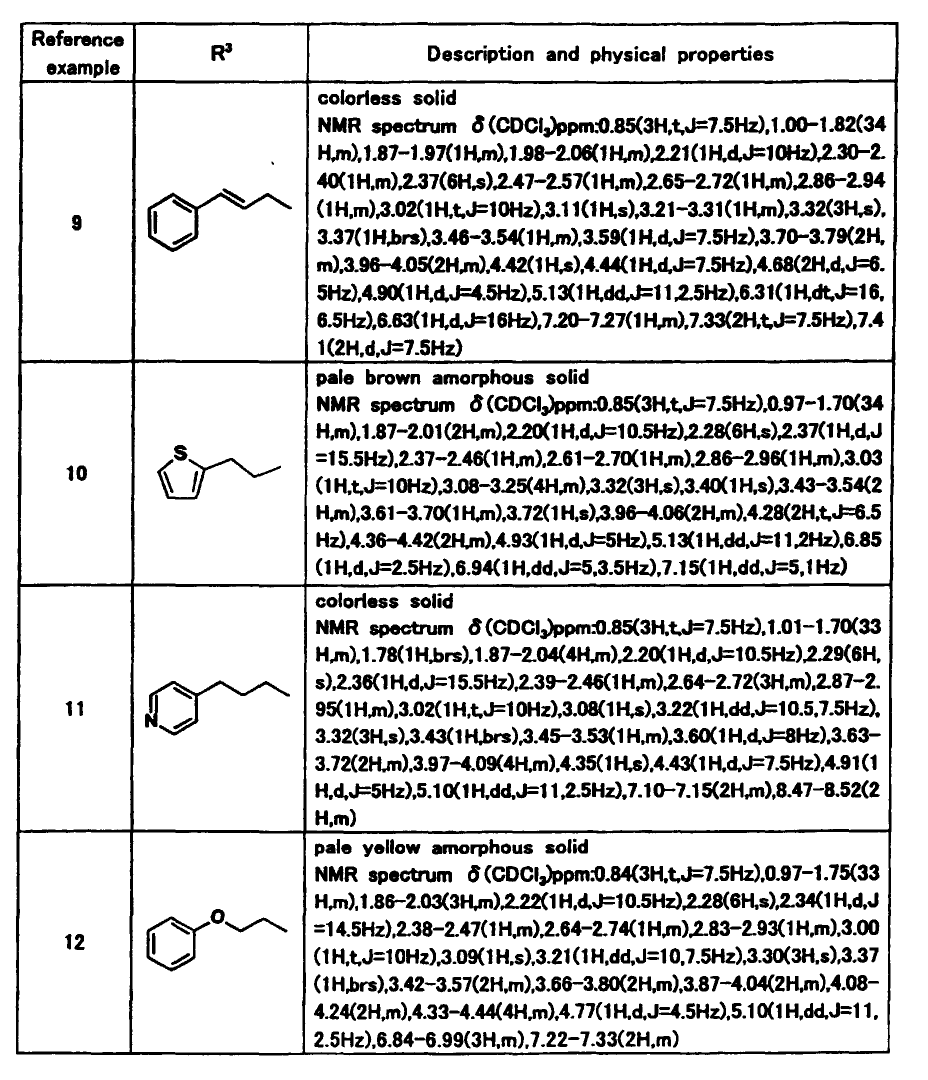

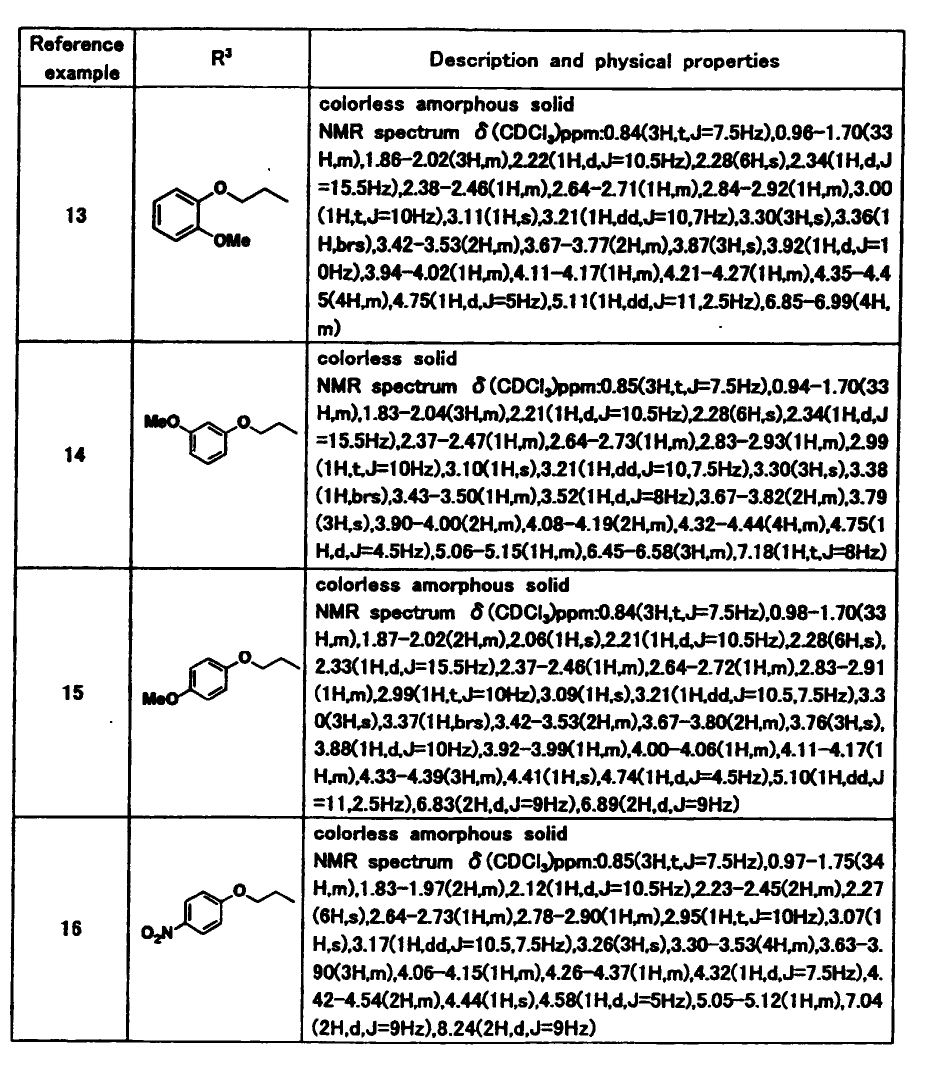

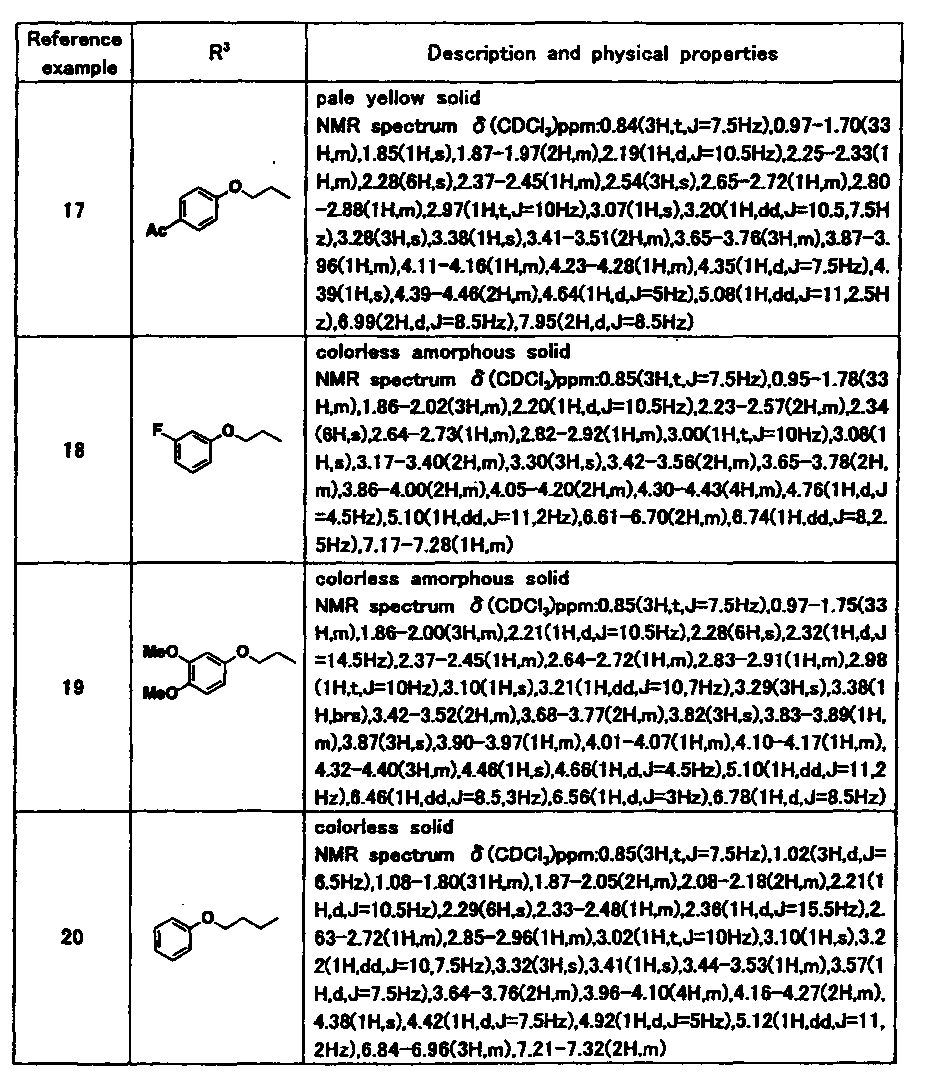

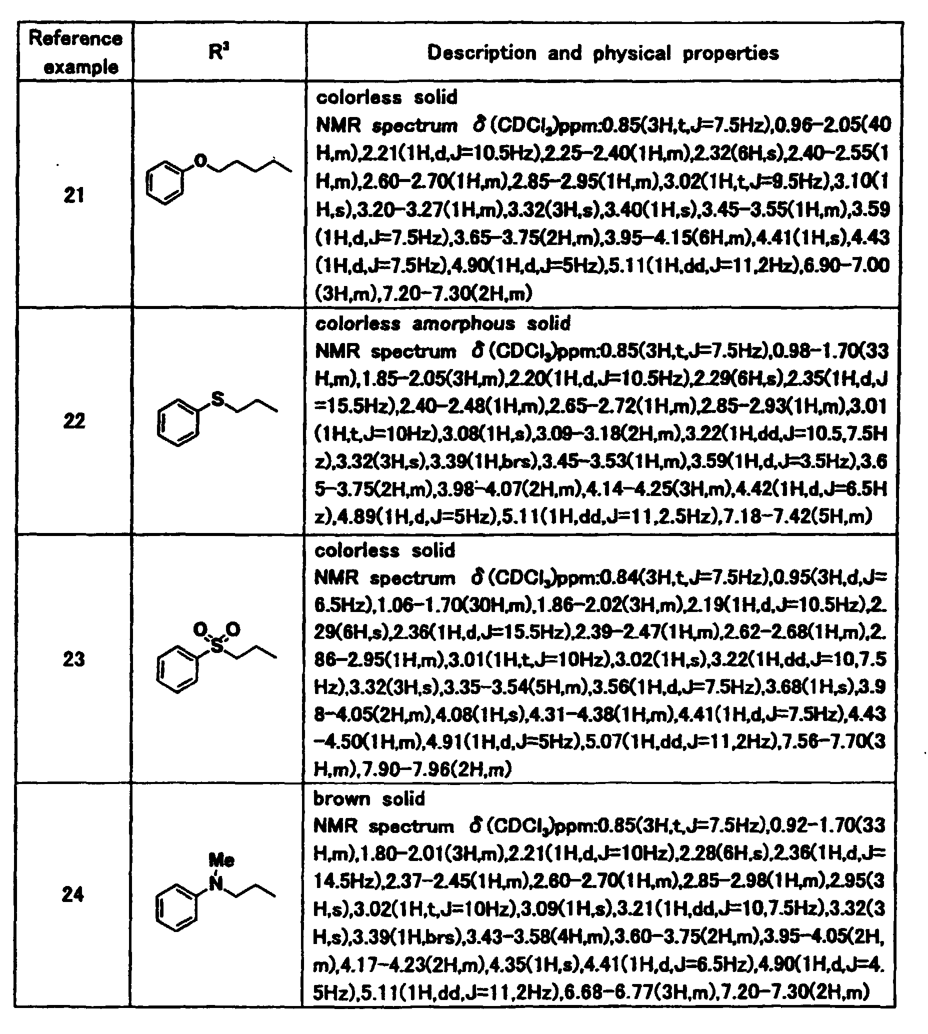

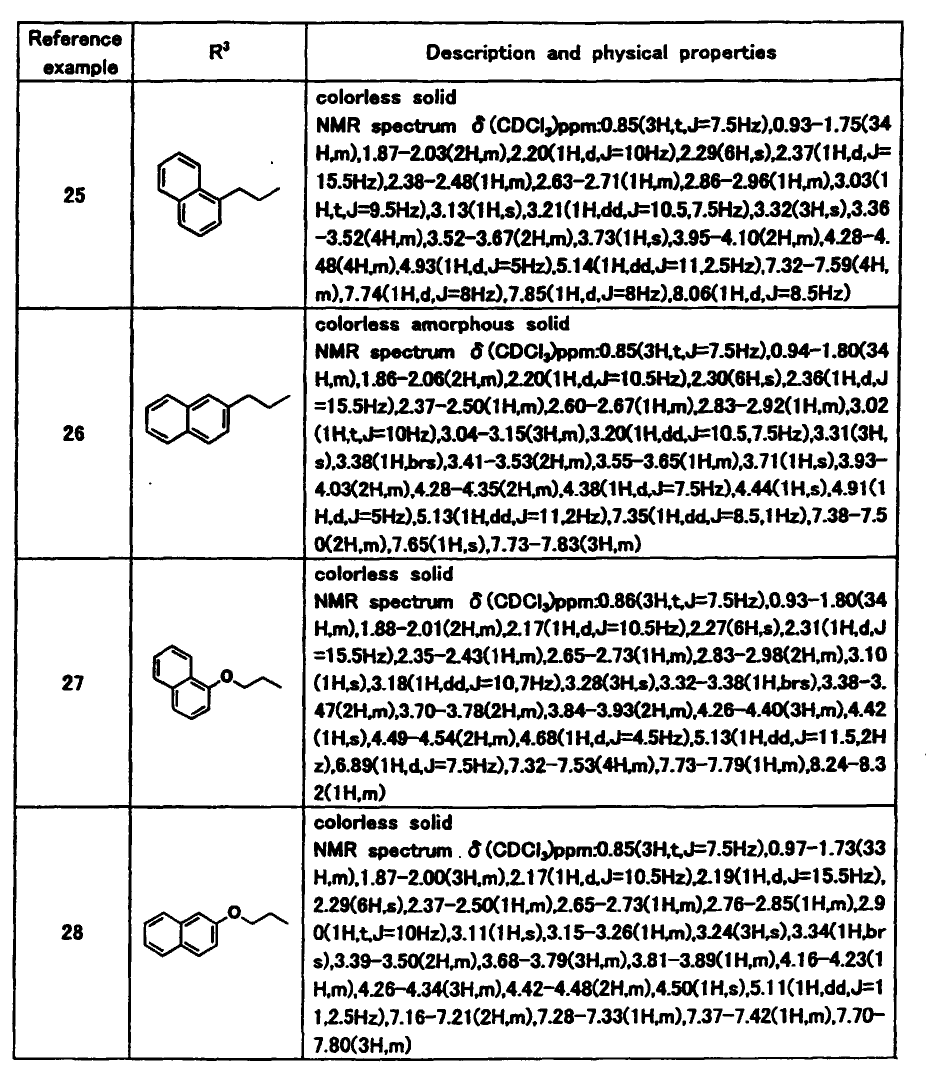

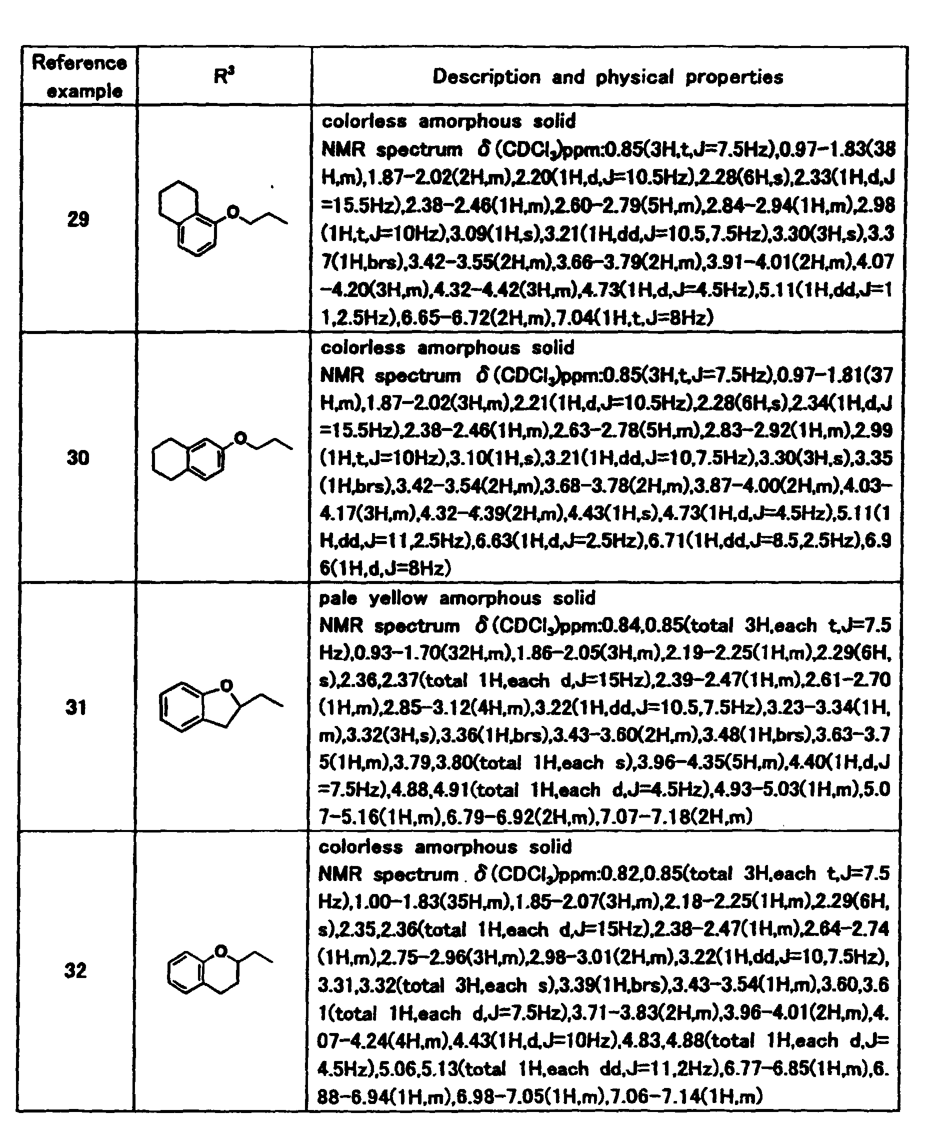

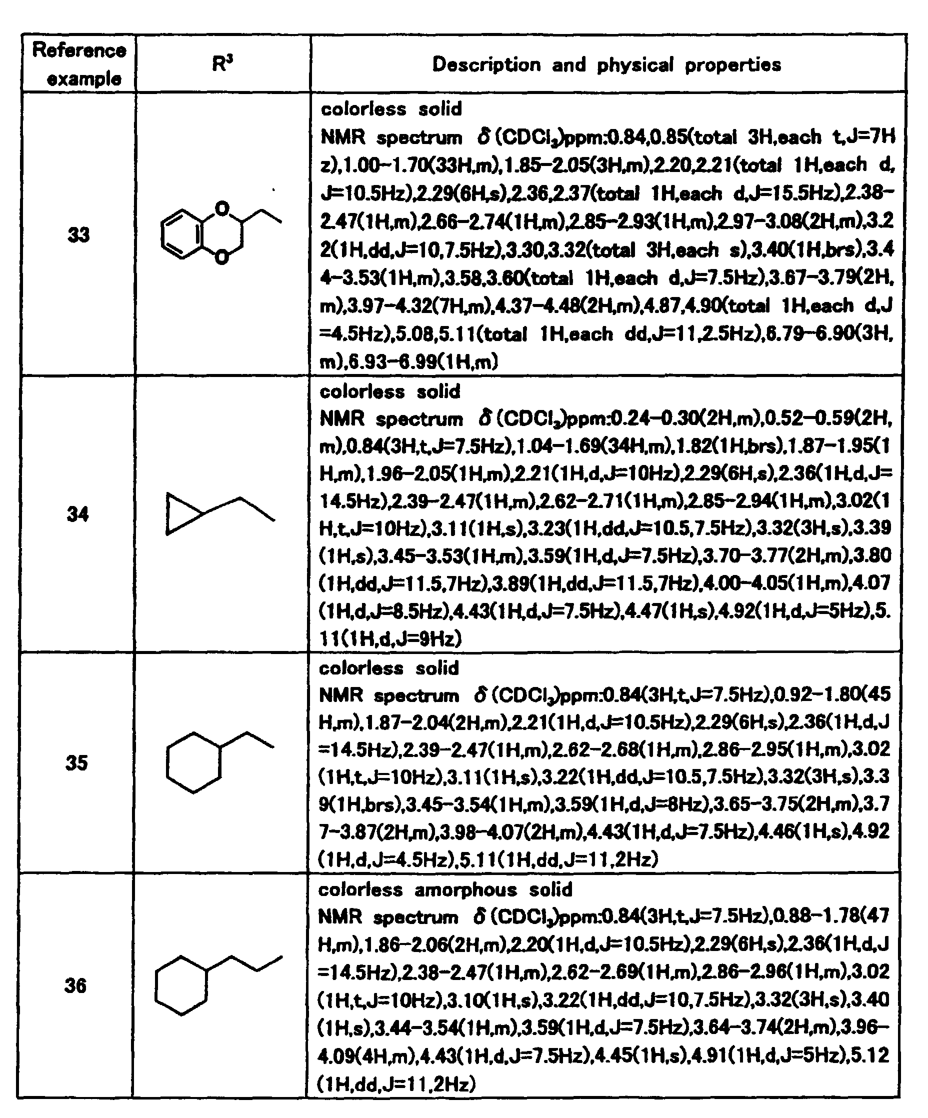

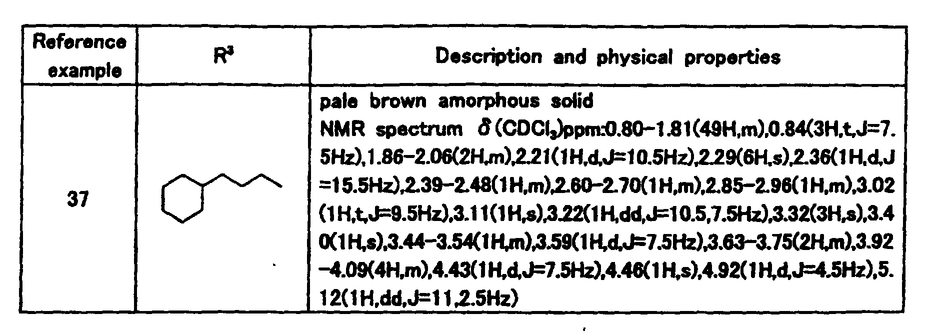

### Reference Example 38: 5-O-Desosaminylerythronolide A 9- [O-(phenethyl)oxime]

To a suspension of 4.80 g of erythromycin A 9-[O- (phenethyl)oxime] in 40 ml of 1 N hydrochloric acid, 50 ml of methanol was added at room temperature with stirring, and the reaction mixture was stirred at room temperature for 3.5 hours. The reaction mixture was concentrated under reduced pressure, and then the resulting residue was added with ice-water, and the mixture was made alkaline with 10% aqueous sodium hydroxide solution and extracted with diethyl ether. The extract was washed with water, and dried over sodium sulfate, and the solvent was removed under reduced pressure. The residue was purified by column chromatography (silica gel, dichloromethane:methanol = 50:1 → 25:1) to give 3.43 g of a pale yellowish brown amorphous solid.
NMR spectrum δ (CDCl₃) ppm: 0.85 (3H, t, J=7.5Hz), 1.01 (3H, d, J=6.5Hz), 1.08 (3H, d, J=7.5Hz), 1.10-1.75 (20H, m), 1.88-1.99 (1H, m), 2.01-2.11 (2H, m), 2.25 (6H, s), 2.42-2.52 (1H, m), 2.59-2.71 (2H, m), 2.90-3.00 (2H, m), 3.18 (1H, s), 3.23 (1H, dd, J=10.5, 7.5Hz), 3.40-3.70 (6H, m), 3.83 (1H, brs), 4.21-4.31 (2H, m), 4.38 (1H, d, J=7.5Hz), 4.38 (1H, s), 5.23 (1H, dd, J=11, 2.5Hz), 7.16-7.31 (5H, m)
HR-MS m/z 694.43983 [Calcd. for C₃₇H₆₂N₂O₁₀ (M⁺): 694.44045]

Compounds of Reference Examples 39 through 76 were obtained in the same manner as that described in Reference Example 38.

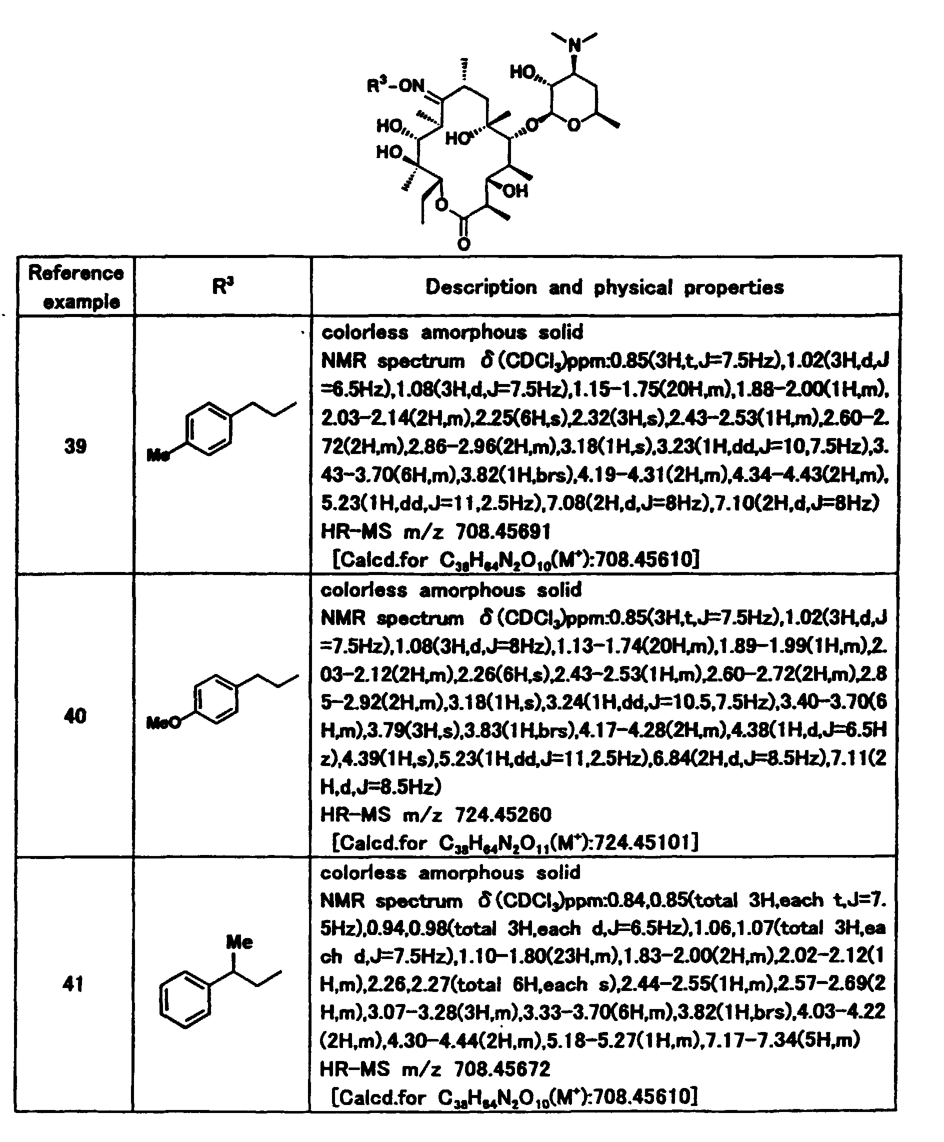

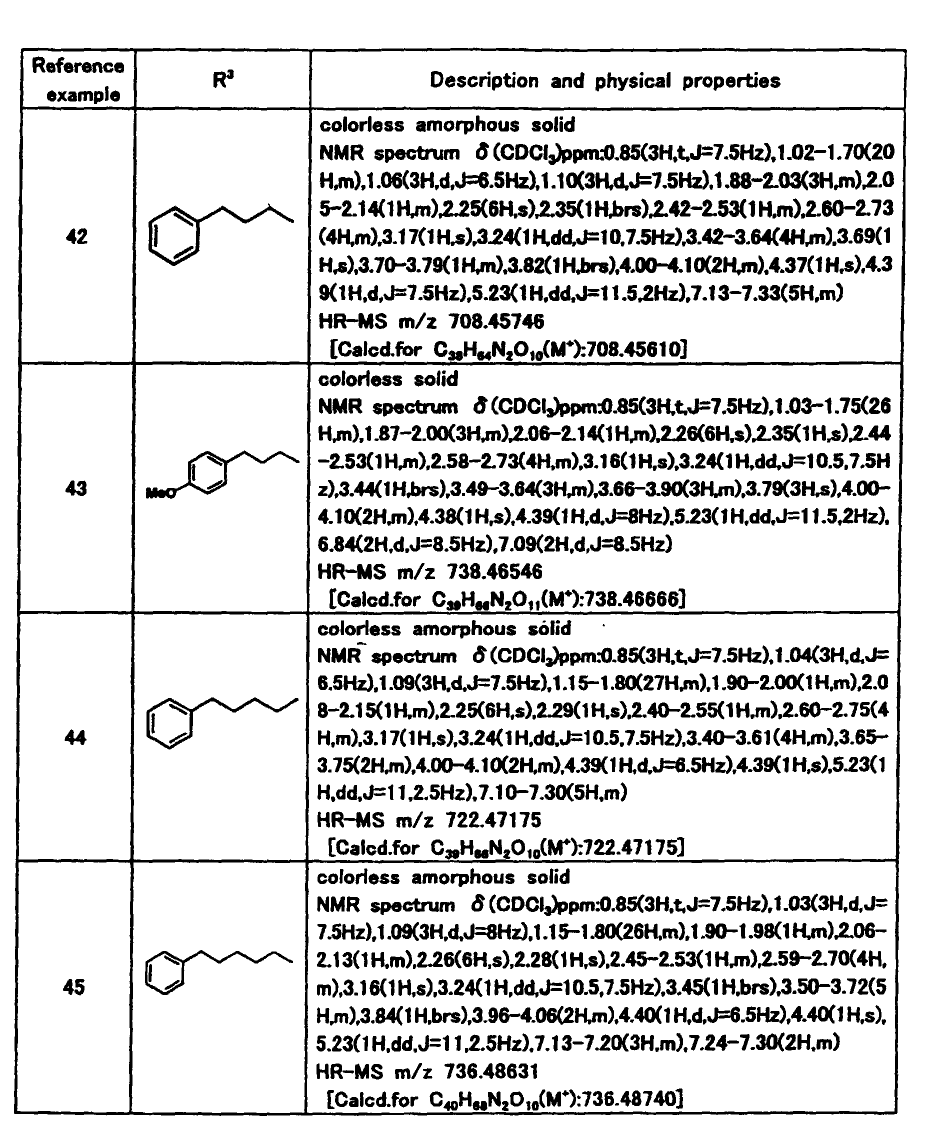

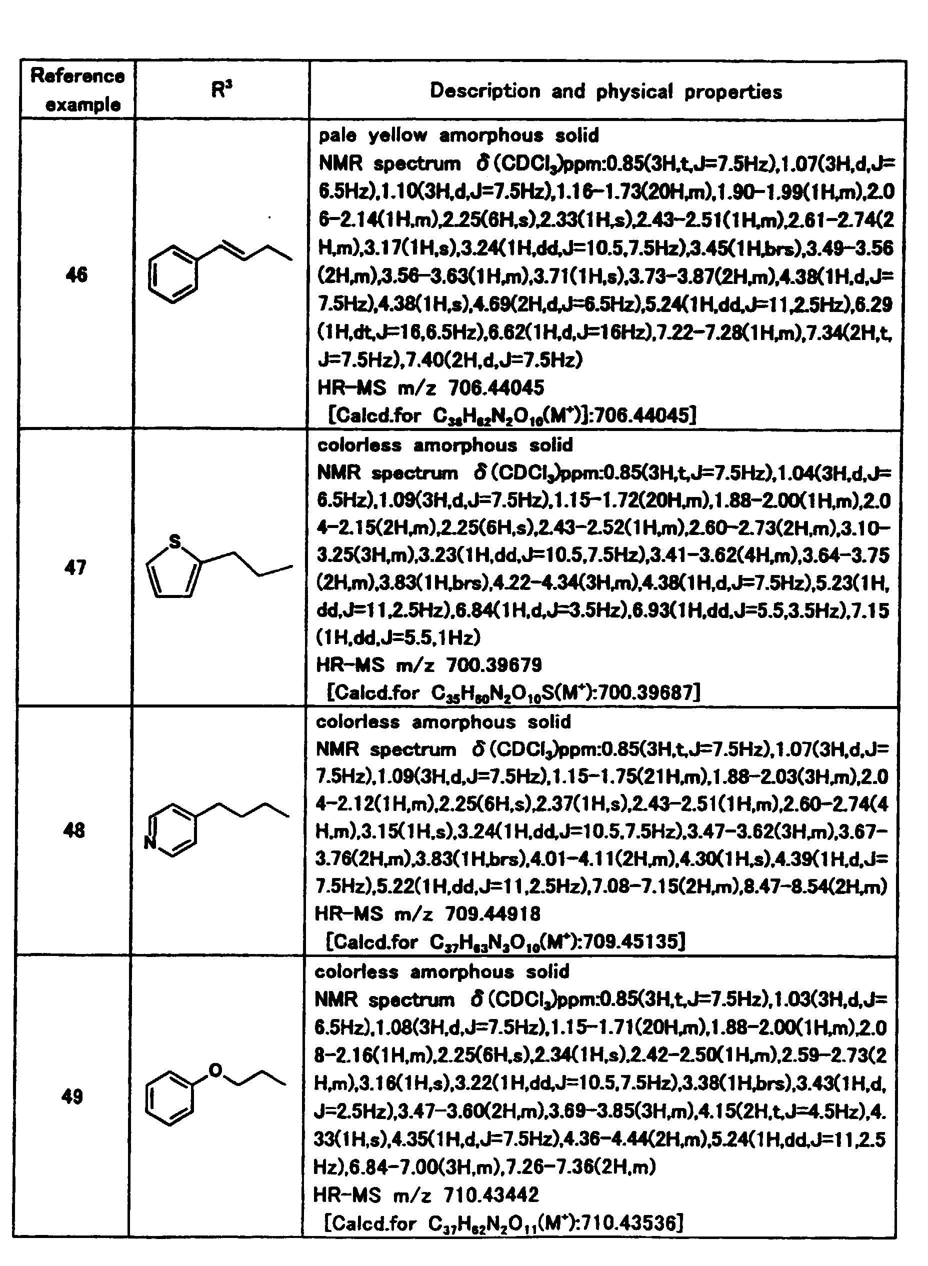

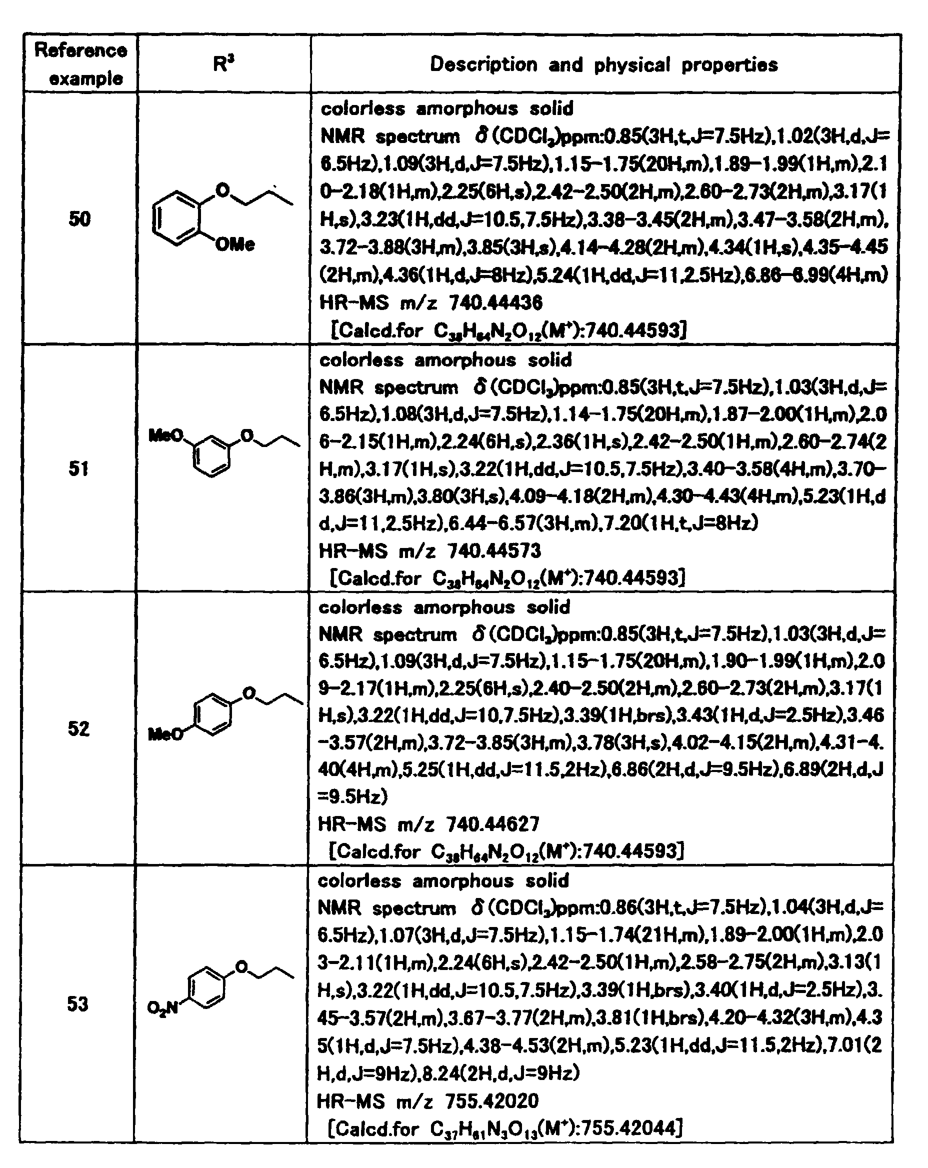

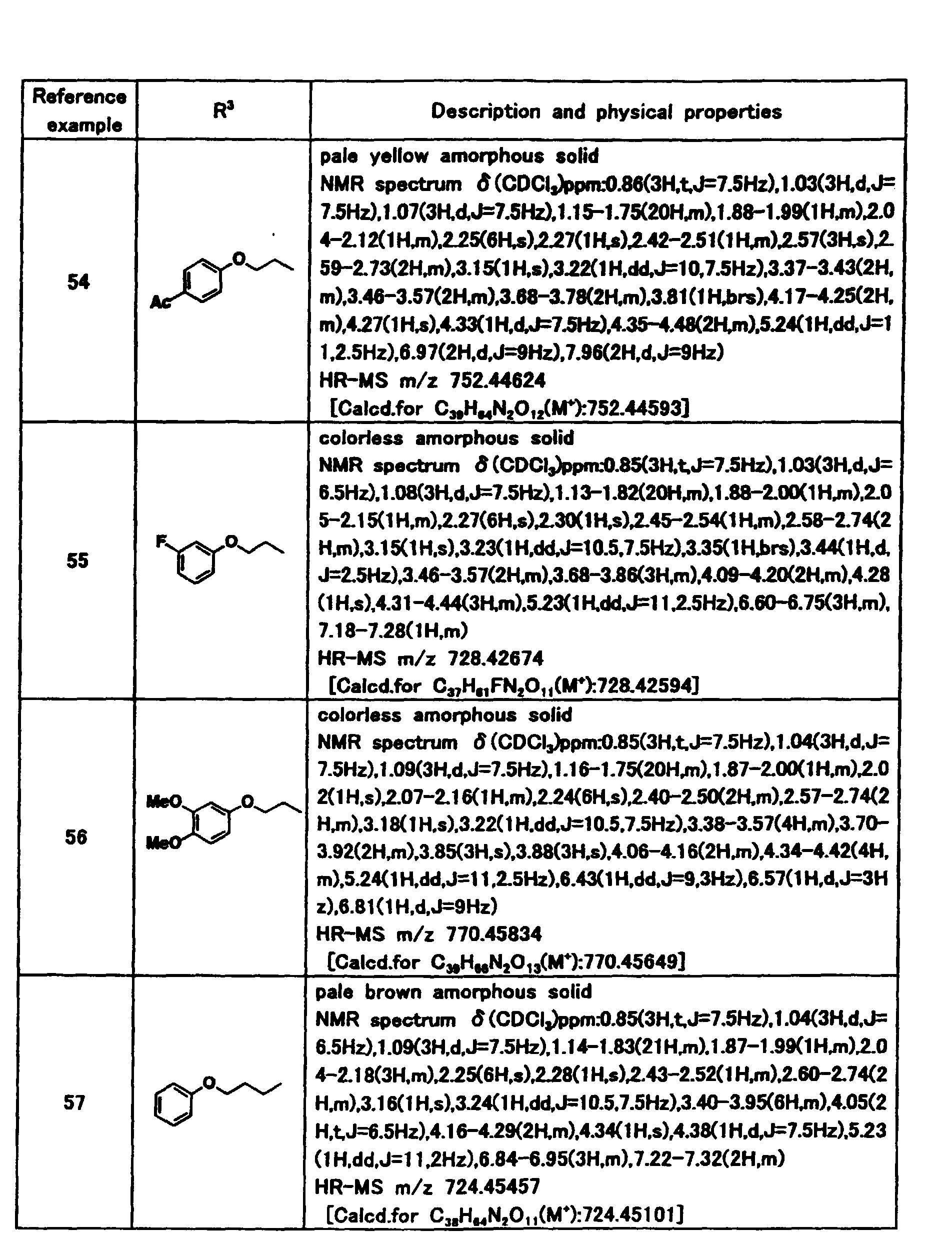

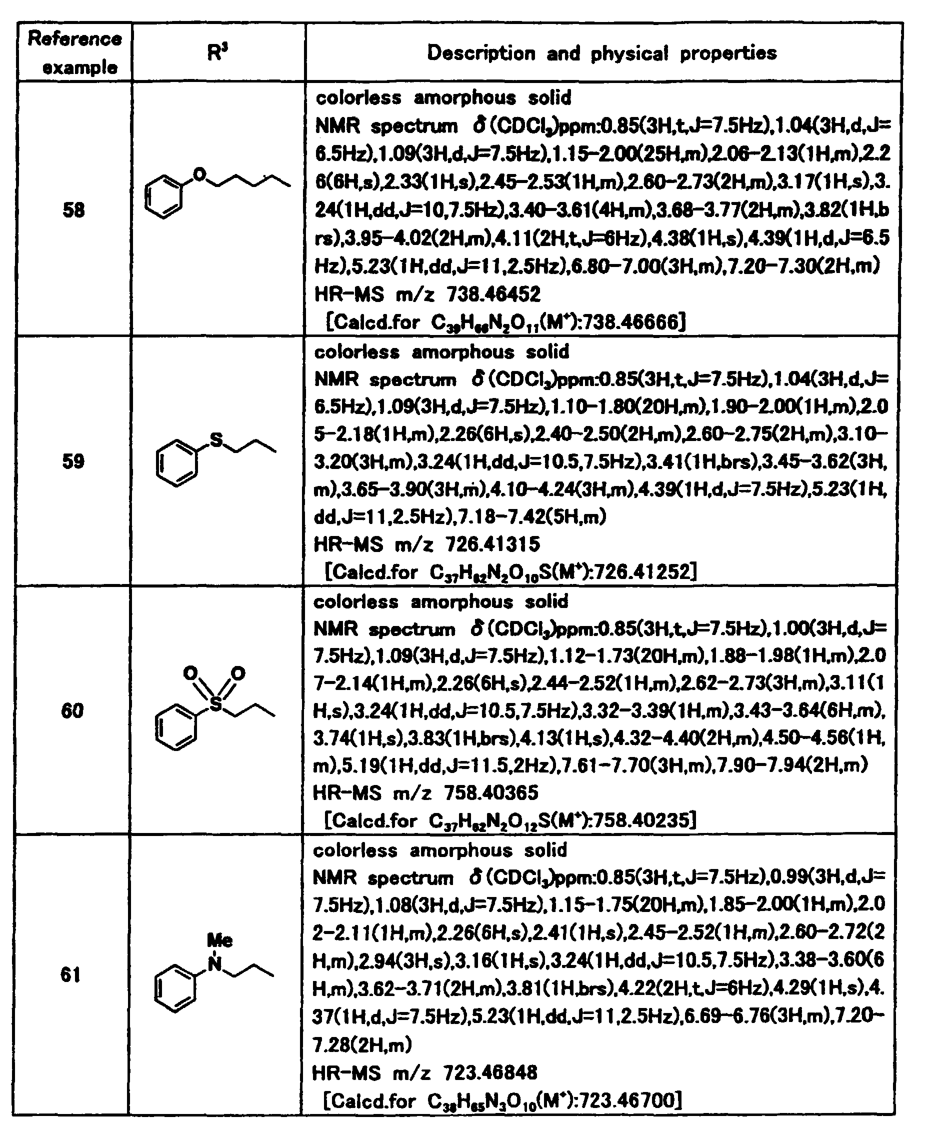

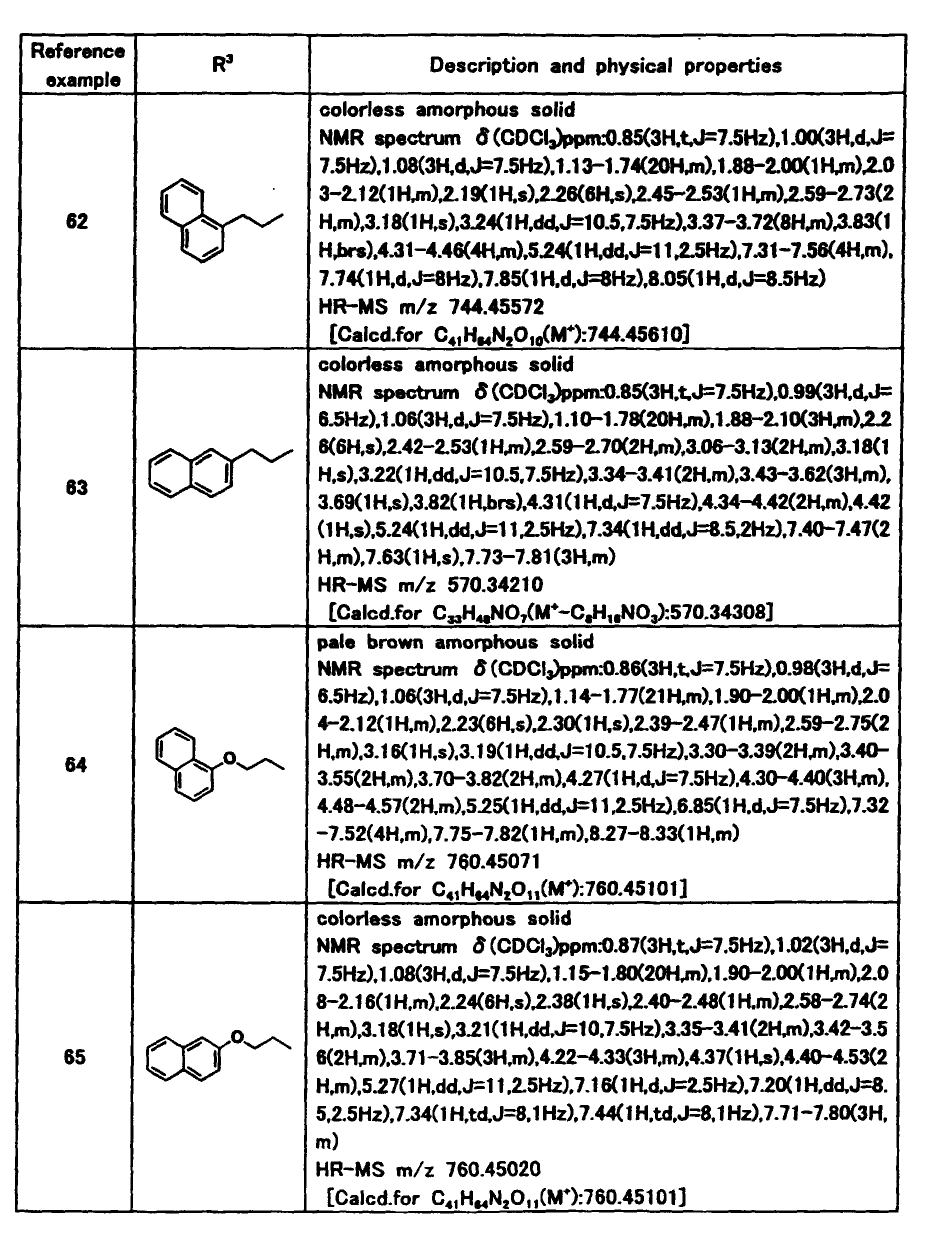

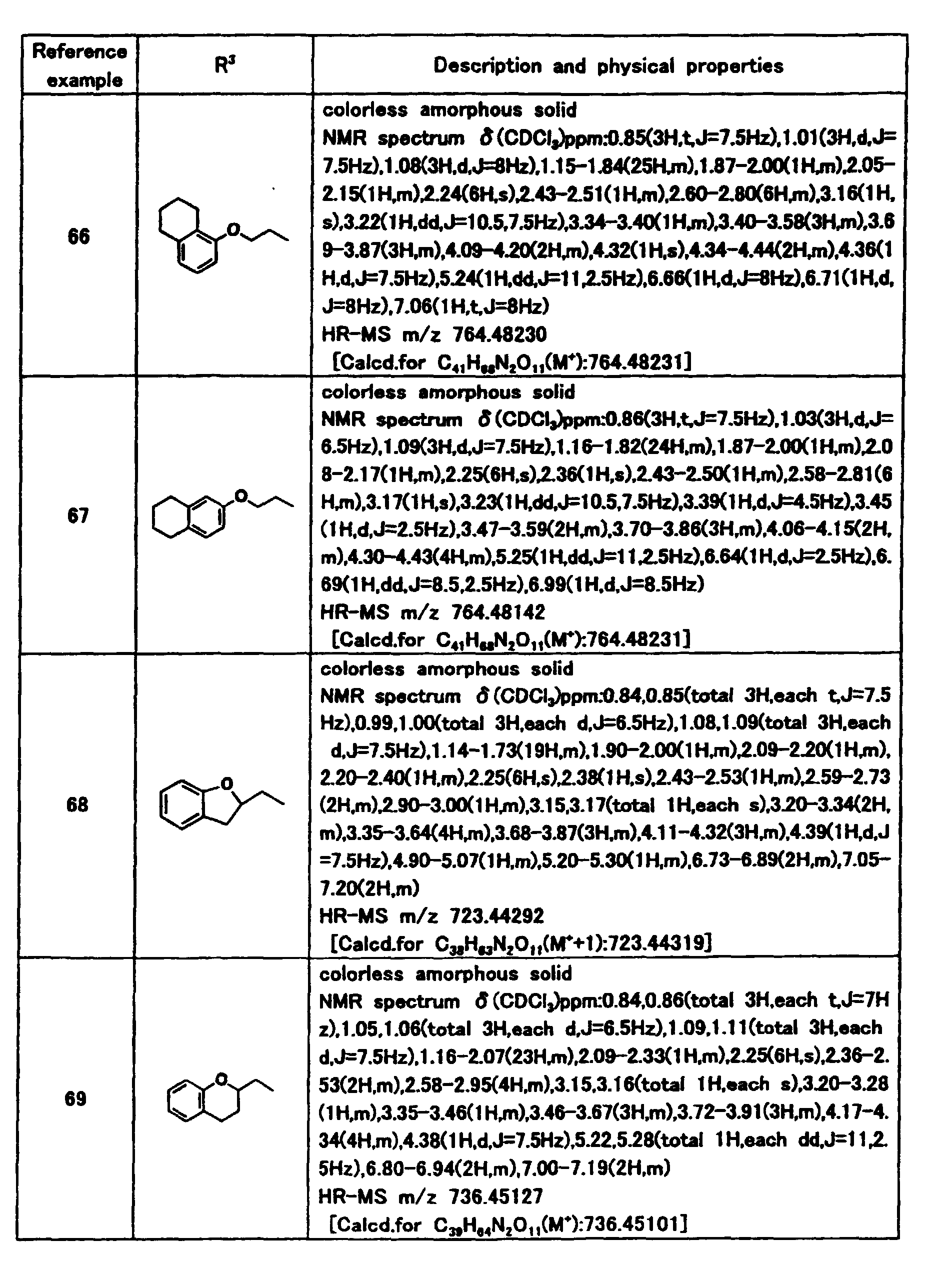

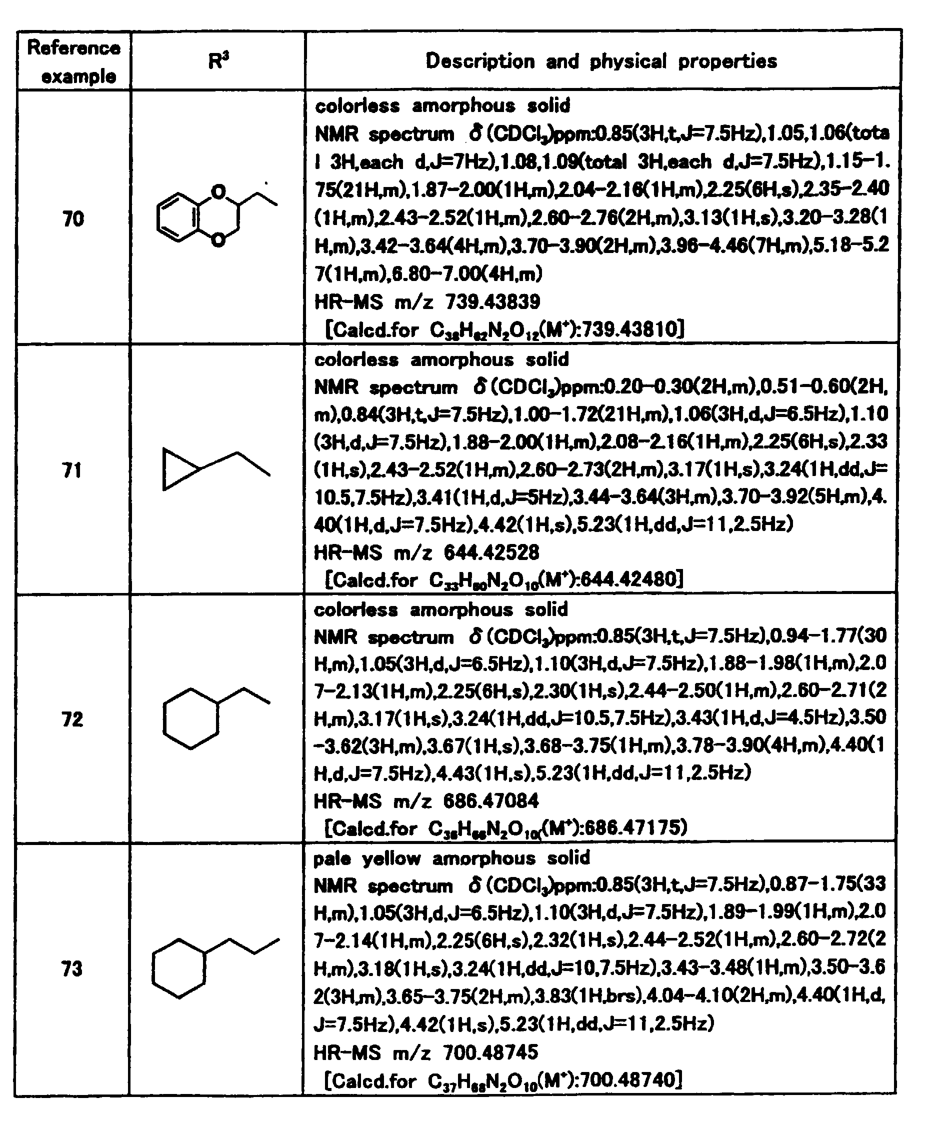

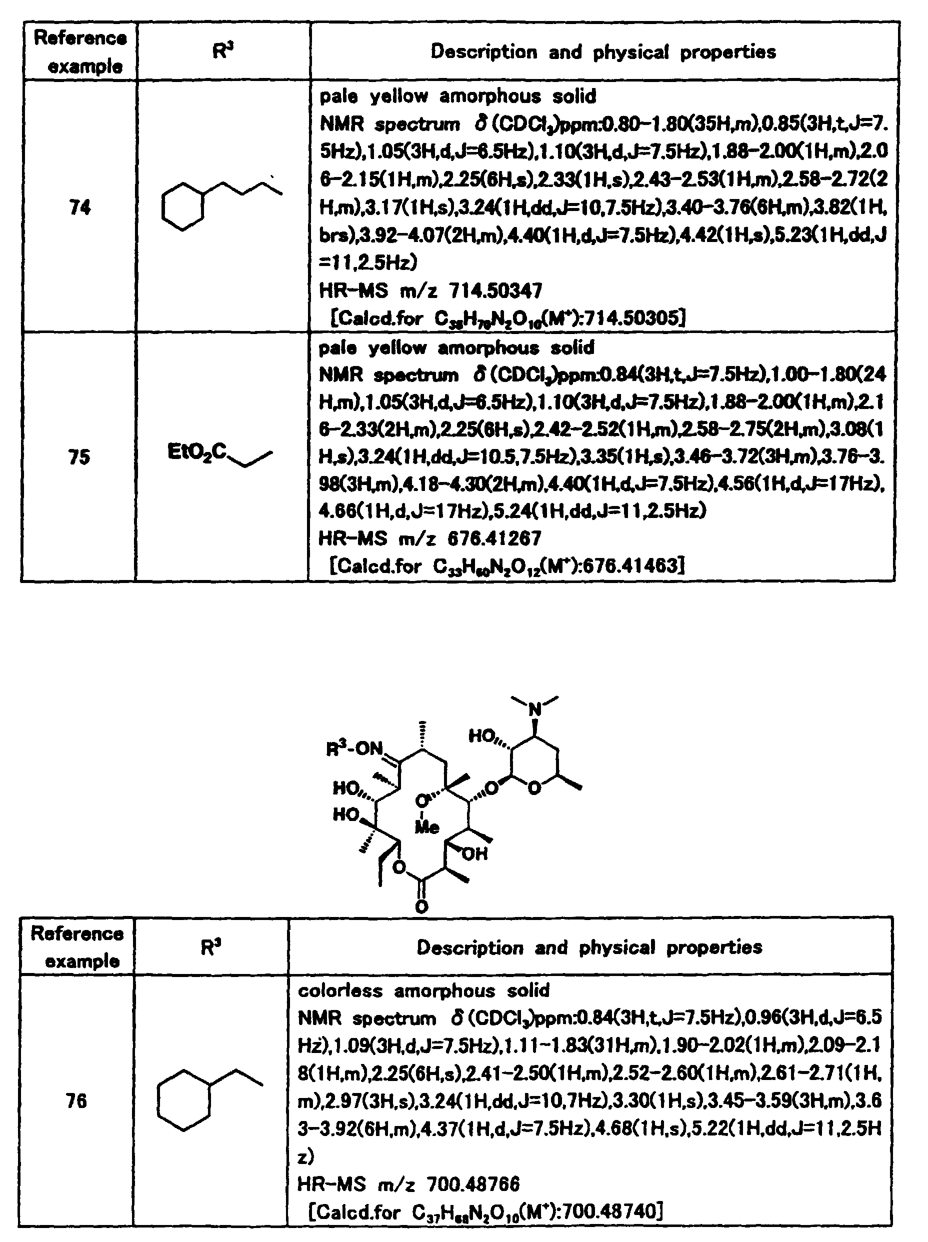

### Reference Example 77: 5-O-Desosaminyl-6-O-methylerythronolide A 9-[O-(1-methoxycyclohexyl)oxime]

To a mixture of 1.10 g of 5-O-desosaminyl-6-O-methylerythronolide A 9-oxime and 0.32 g of pyridinium hydrochloride in 11 ml of dichloromethane, a solution of 1.4 ml of 1,1-dimethoxycyclohexane in 4 ml of dichloromethane was added dropwise at room temperature with stirring, and the reaction mixture was stirred at room temperature for 19 hours and then refluxed for 18 hours. The reaction mixture was added with water, and the mixture was made alkaline with saturated aqueous sodium hydrogencarbonate solution and extracted with diethyl ether. The extract was washed with saturated brine, dried over sodium sulfate, and the solvent was removed under reduced pressure. The residue was purified by column chromatography (silica gel, ethyl acetate:methanol:aqueous ammonia = 20:1:0.1 → 10:1:0.1) and the resulting solid was washed with diisopropyl ether to give 0.37 g of a colorless amorphous solid.
NMR spectrum δ (CDCl₃) ppm: 0.84 (3H, t, J=7.5Hz), 0.99 (3H, d, J=7.5Hz), 1.10 (3H, d, J=7.5Hz), 1.07-2.02 (31H, m), 2.09-2.20 (1H, m), 2.25 (6H, s), 2.43-2.52 (1H, m), 2.57-2.72 (2H, m), 3.00 (3H, s), 3.21 (3H, s), 3.24 (1H, dd, J=10.5, 7.5Hz), 3.33 (1H, s), 3.48-3.60 (3H, m), 3.68-3.93 (4H, m), 4.38 (1H, d, J=7.5Hz), 4.54 (1H, s), 5.23 (1H, dd, J=11, 2.5Hz)
HR-MS m/z 716.47970 [Calcd. for C₃₇H₆₈N₂O₁₁ (M⁺): 716.48231]

Compounds of Reference Examples 78 through 80 were obtained in the same manner as that described in Reference Example 77.

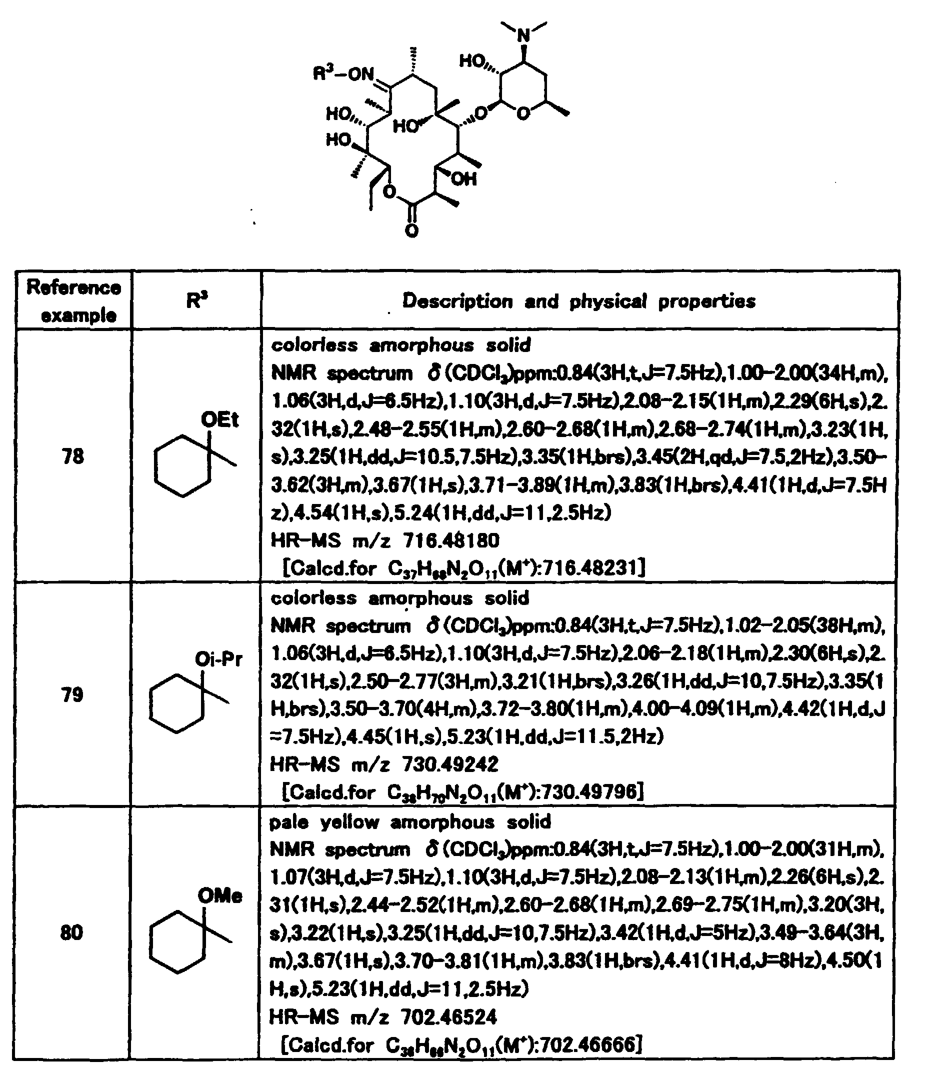

### Reference Example 81: 2'-O-Acetyl-5-O-desosaminylerythronolide A 9-[O-(phenethyl)oxime]

To a solution of 3.25 g of 5-O-desosaminylerythronolide A 9-[O-(phenethyl)oxime] in 40 ml of acetone, 0.53 ml of acetic anhydride was added at room temperature with stirring, and the reaction mixture was stirred at room temperature for 4 hours. The reaction mixture was concentrated under reduced pressure, and the residue was added with water. The reaction mixture was made alkaline with saturated aqueous sodium hydrogencarbonate solution and extracted with dichloromethane. The extract was washed with water, dried over sodium sulfate, and the solvent was removed under reduced pressure to give 3.45 g of a pale yellow amorphous solid.
NMR spectrum δ (CDCl₃) ppm: 0.84 (3H, t, J=7.5Hz), 0.91 (3H, d, J=7.5Hz), 0.99 (3H, d, J=6.5Hz), 1.12-1.52 (19H, m), 1.70-1.76 (2H, m), 1.90-2.00 (1H, m), 2.06 (3H, s), 2.05-2.14 (1H, m), 2.28 (6H, s), 2.61-2.68 (2H, m), 2.73-2.83 (1H, m), 2.94 (2H, t, J=6.5Hz), 3.16 (1H,brs), 3.42-3.60 (5H, m), 3.68 (1H, s), 4.26 (2H, m), 4.46 (1H,brs), 4.56 (1H, d, J=7.5Hz), 4.77 (1H, dd, J=10.5, 8Hz), 5.24 (1H, dd, J=11, 2.5Hz), 7.17-7.31 (5H, m)
HR-MS m/z 736.45008 [Calcd. for C₃₉H₆₄N₂O₁₁ (M⁺): 736.45101]

Compounds of Reference Examples 82 through 123 were obtained in the same manner as that described in Reference Example 81.

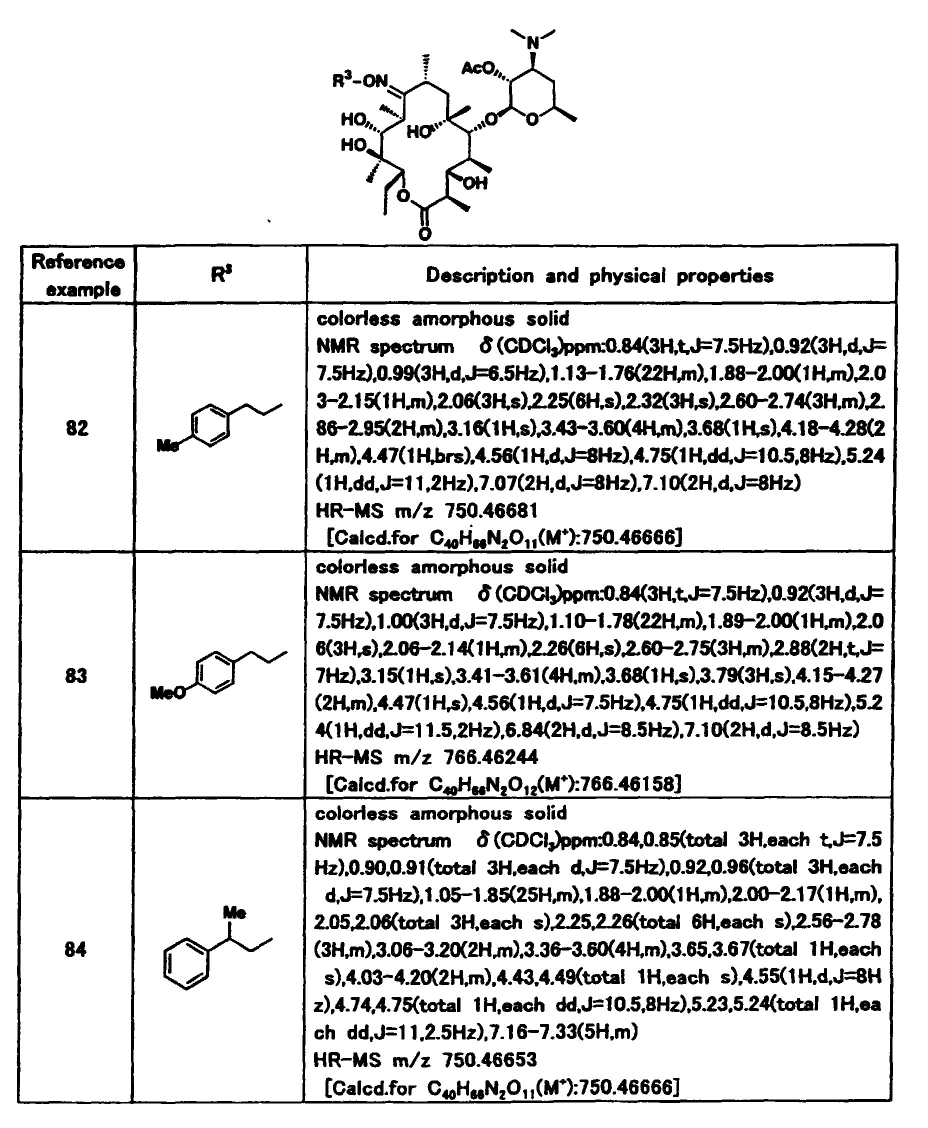

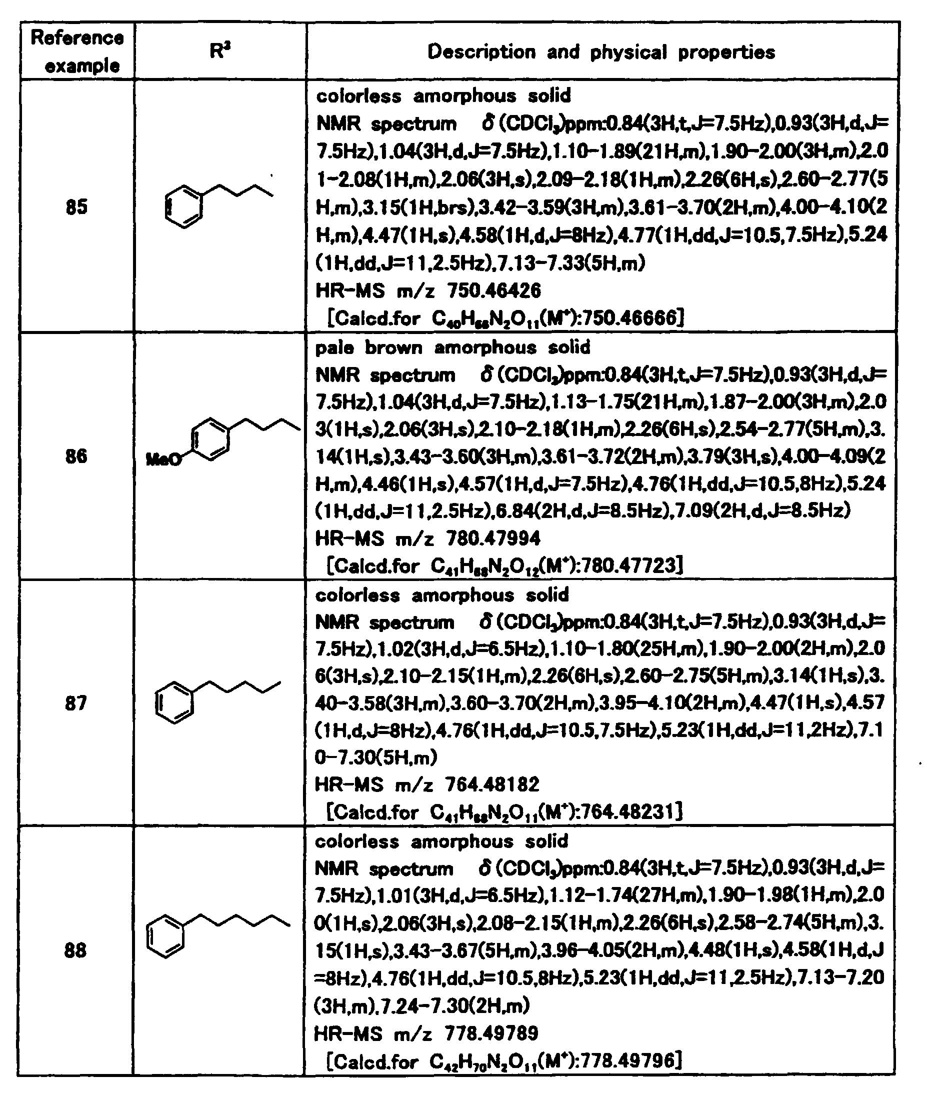

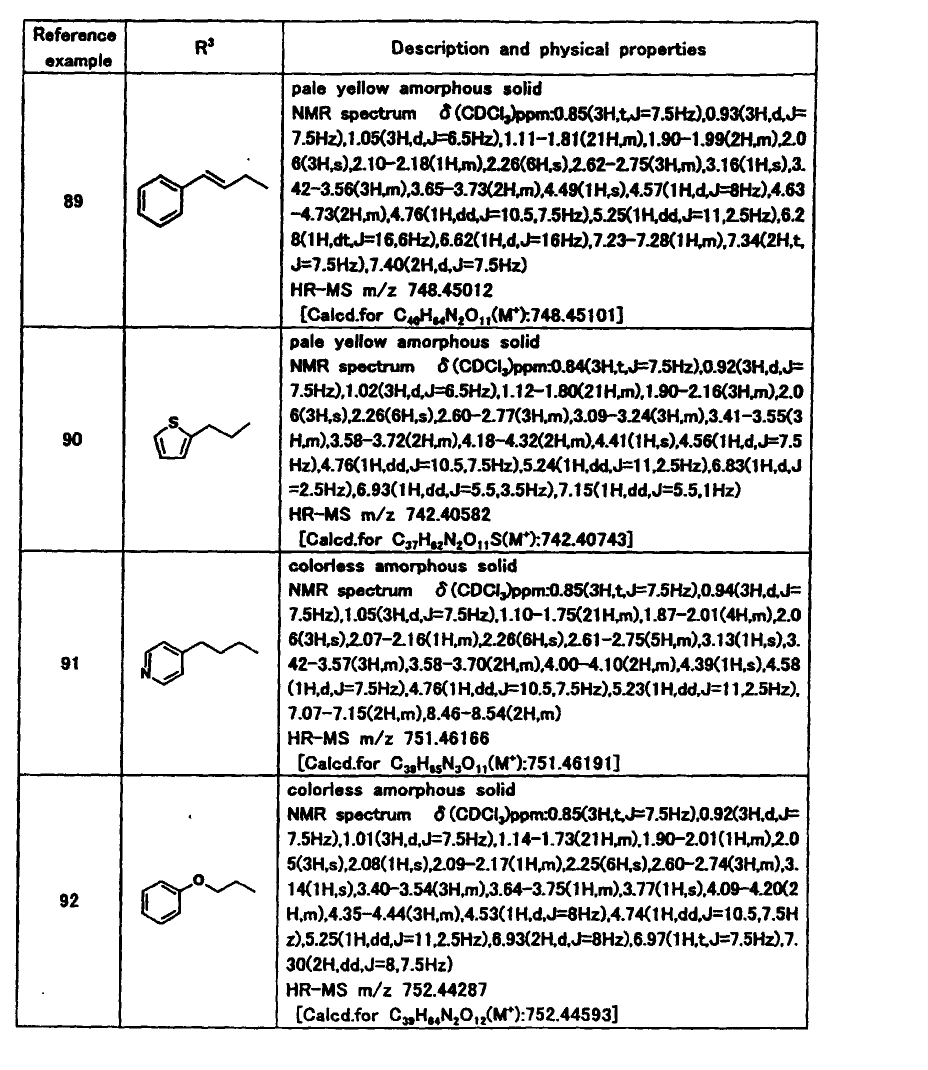

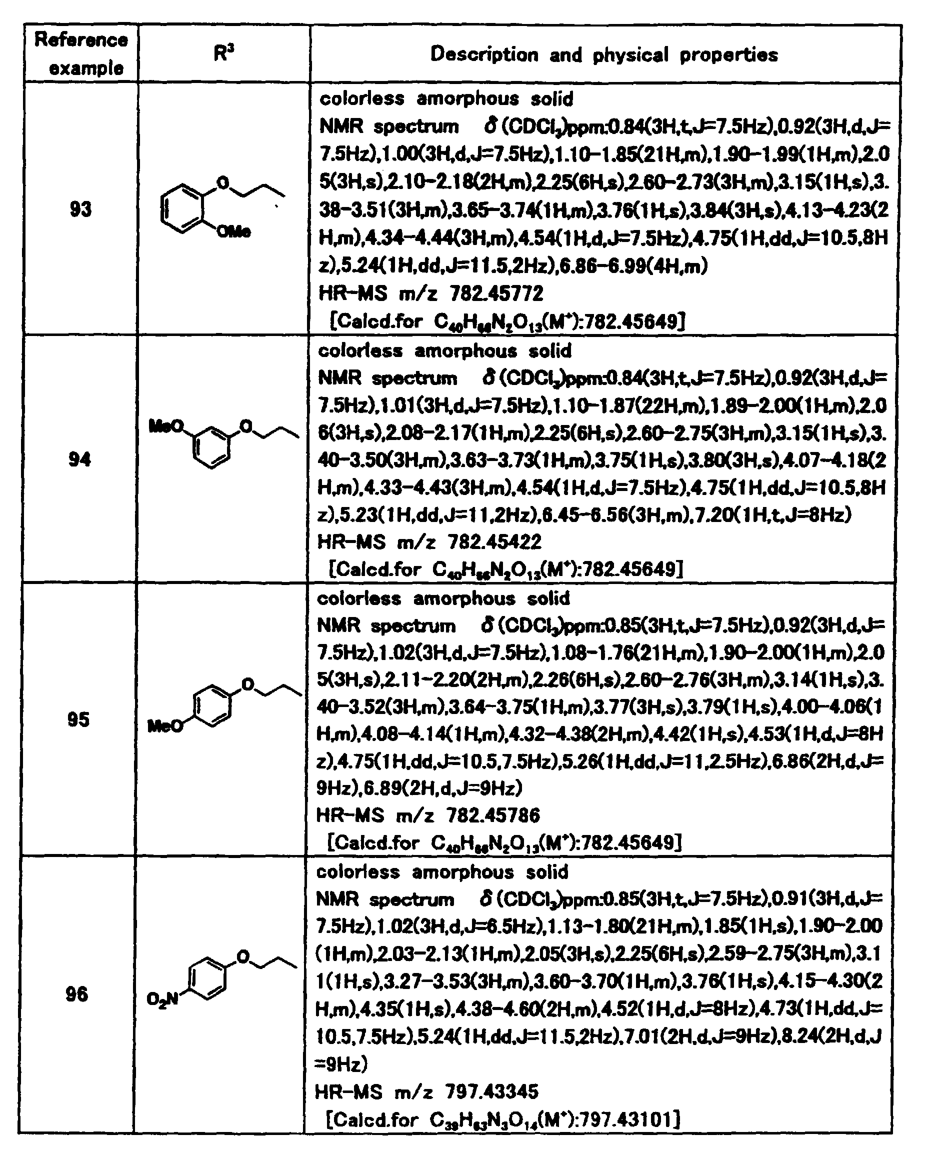

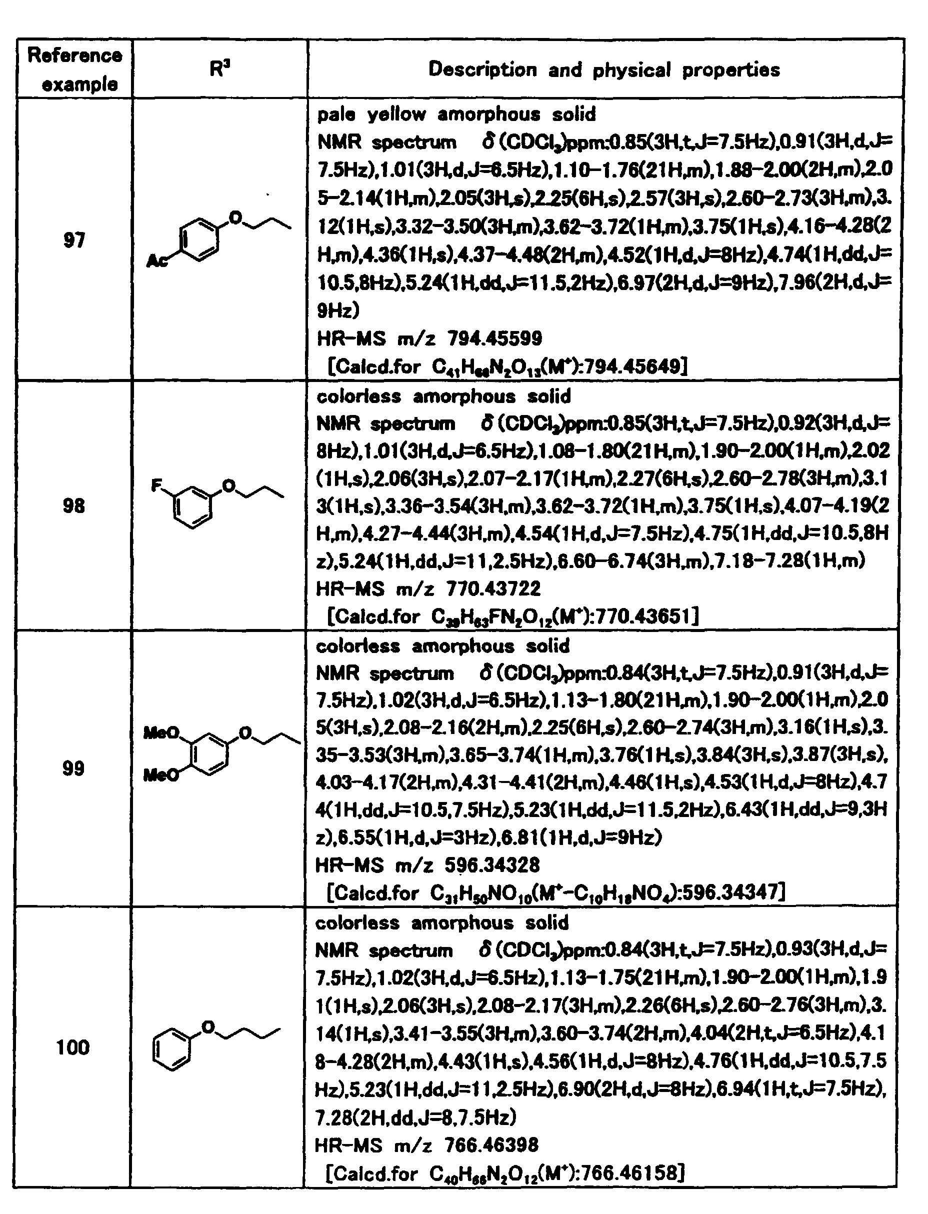

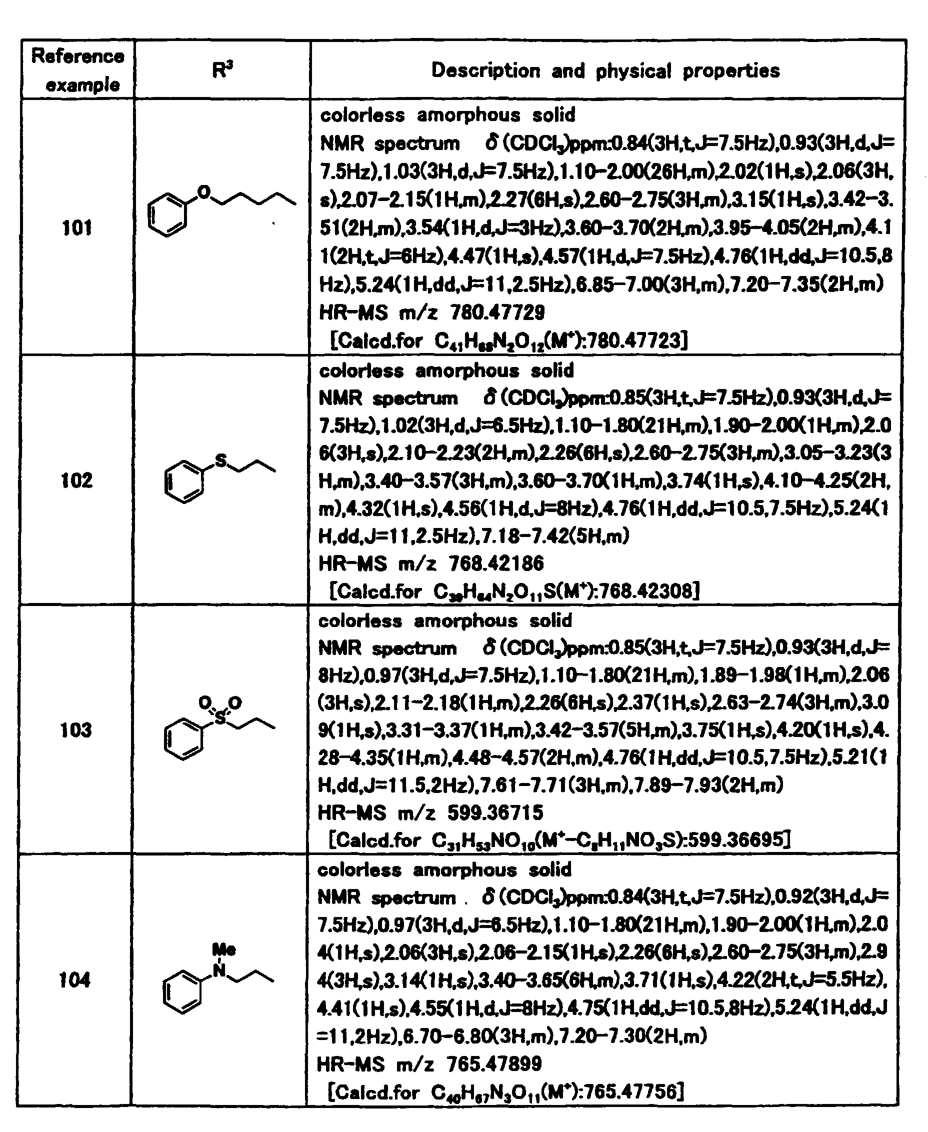

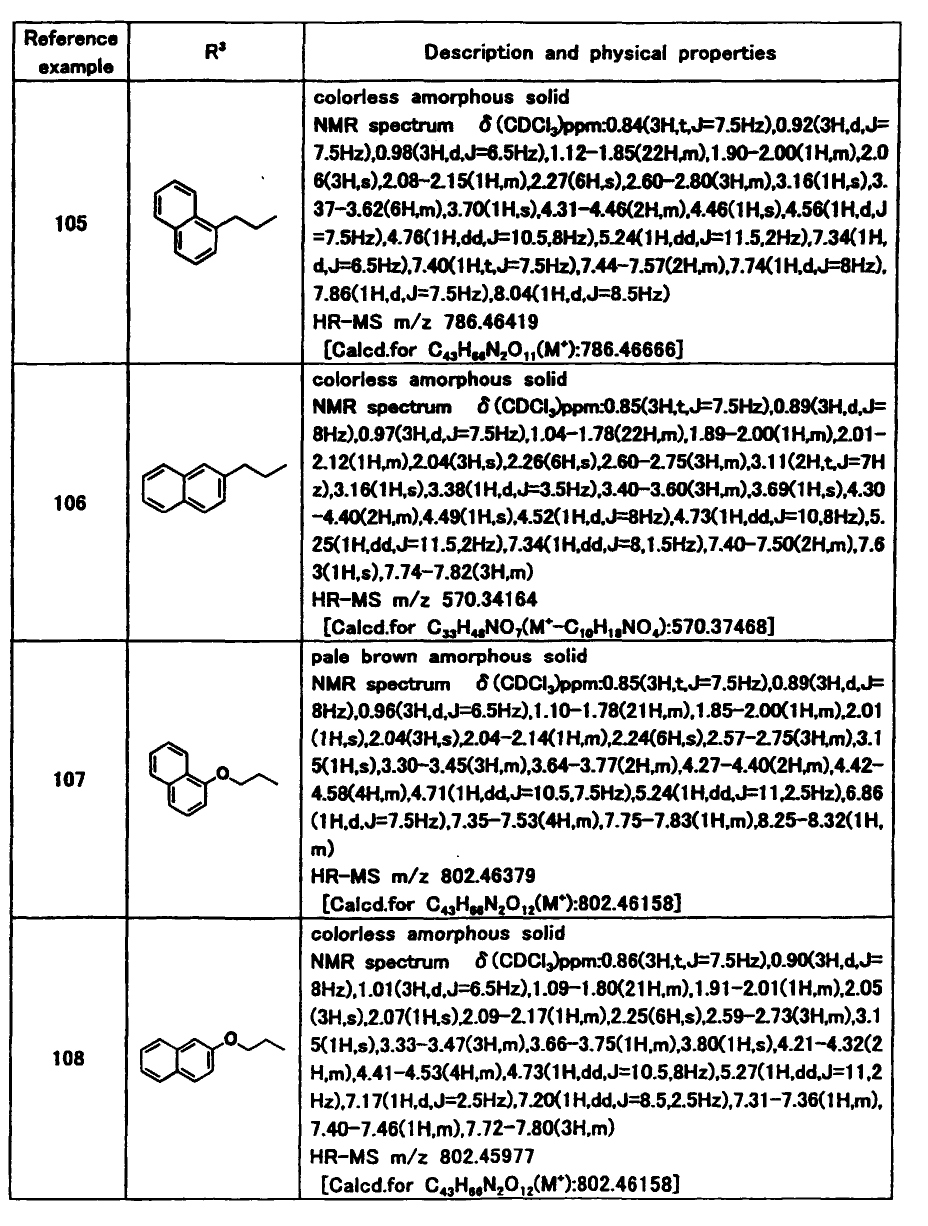

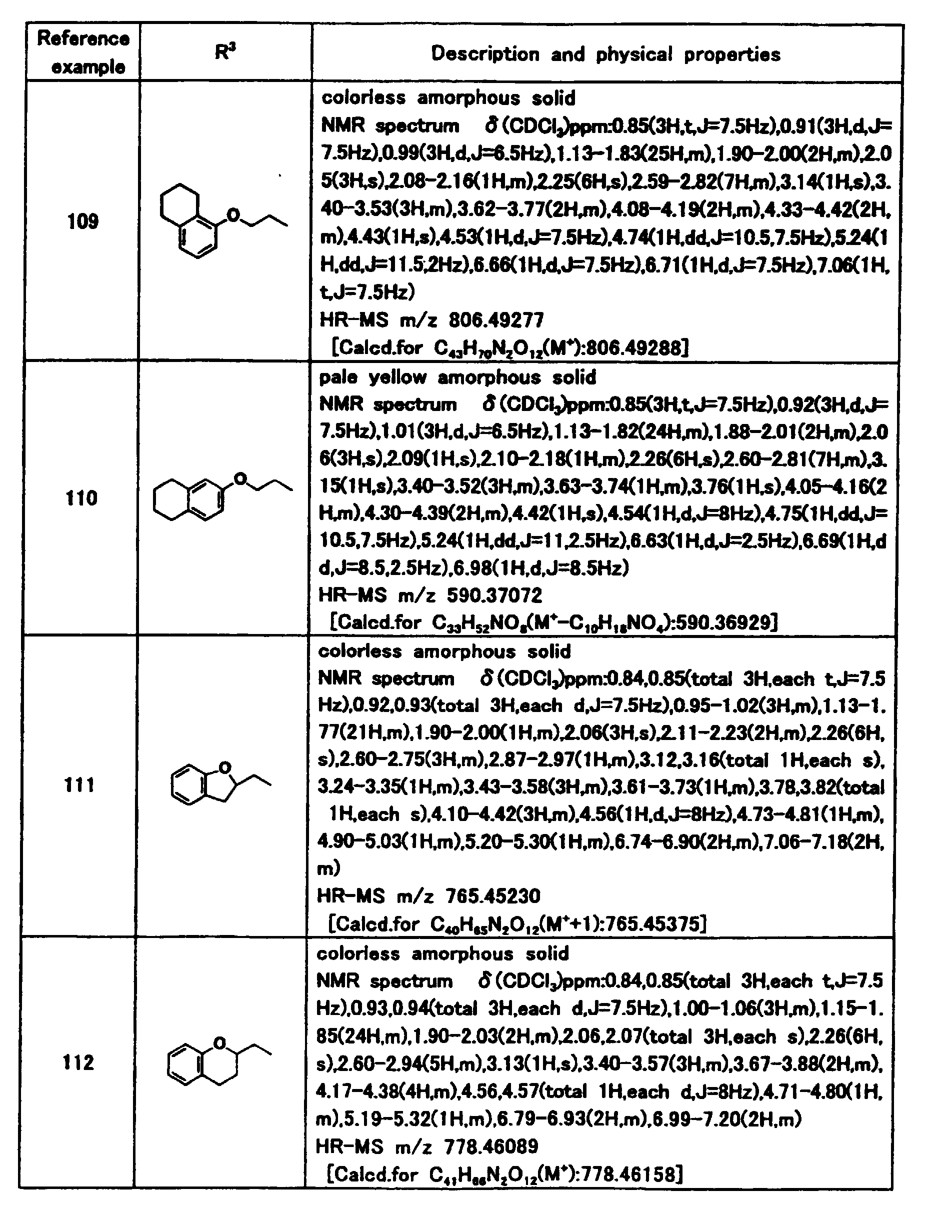

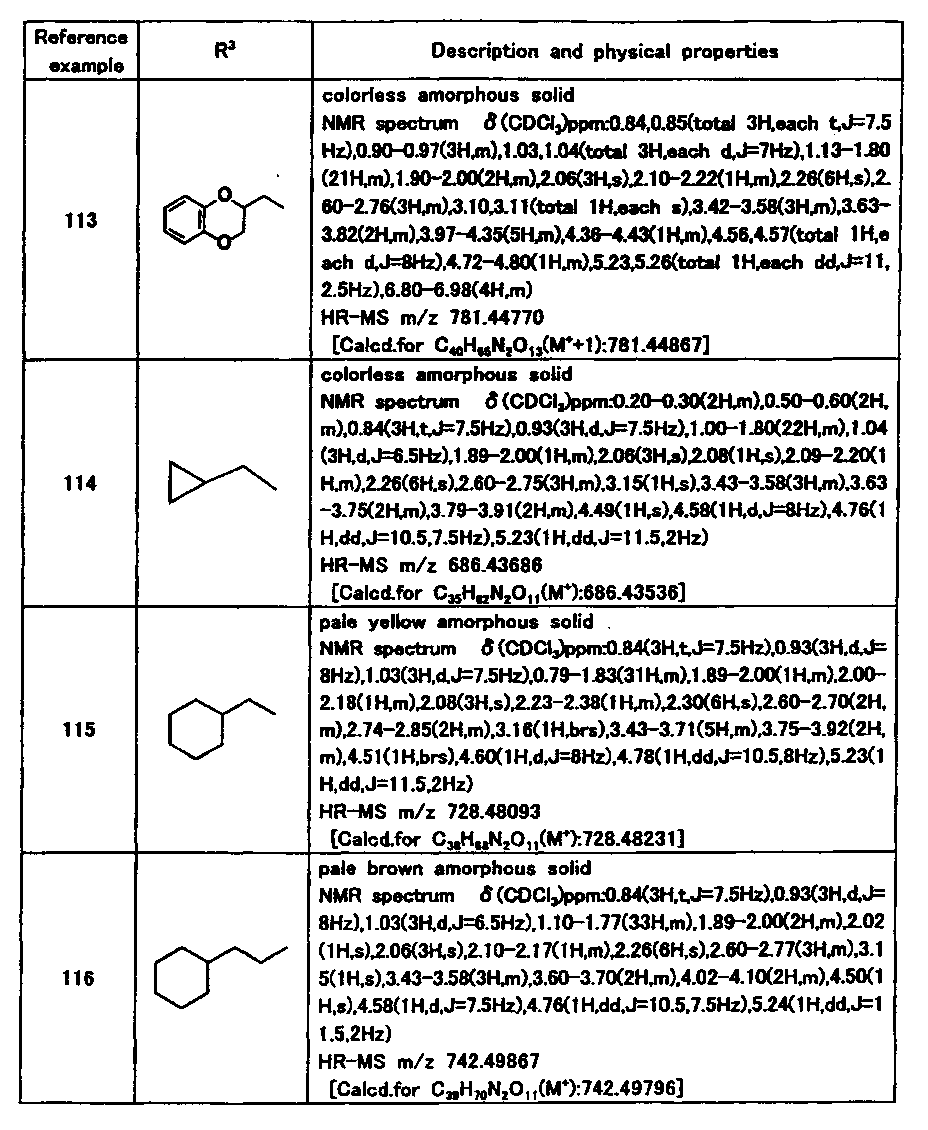

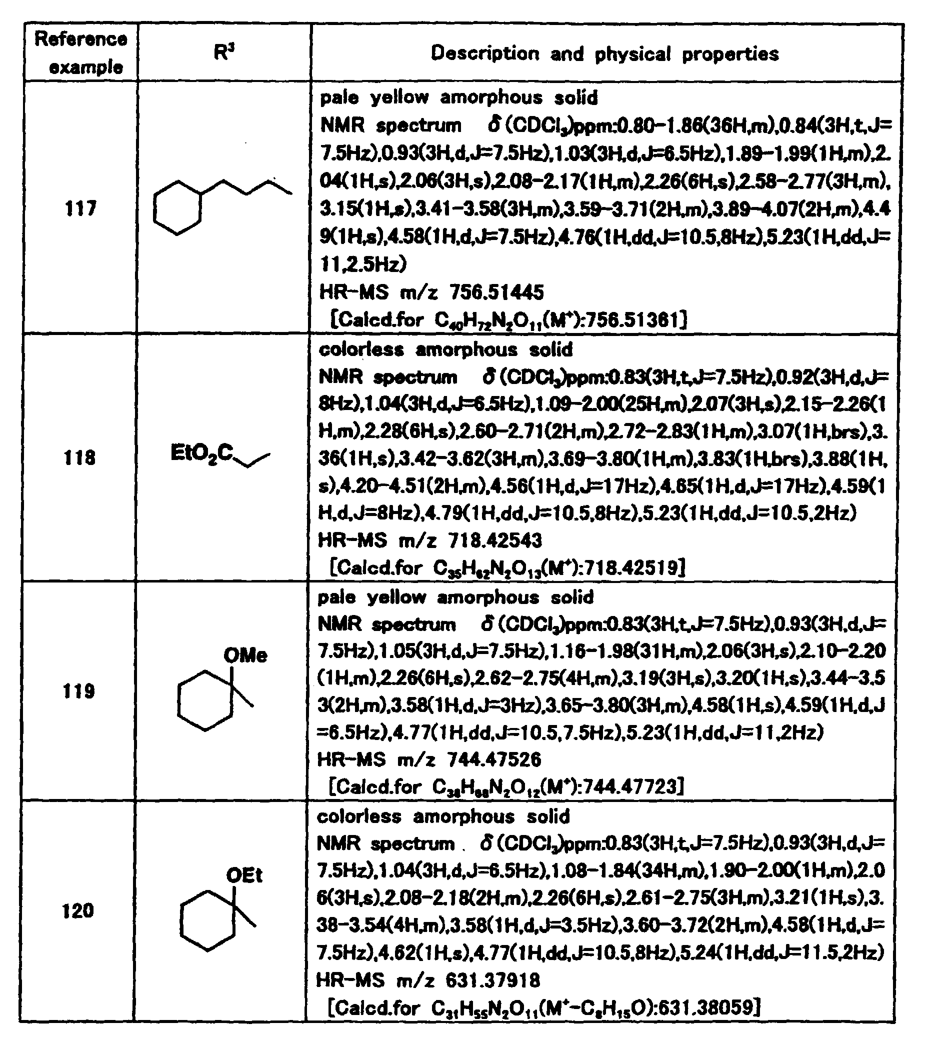

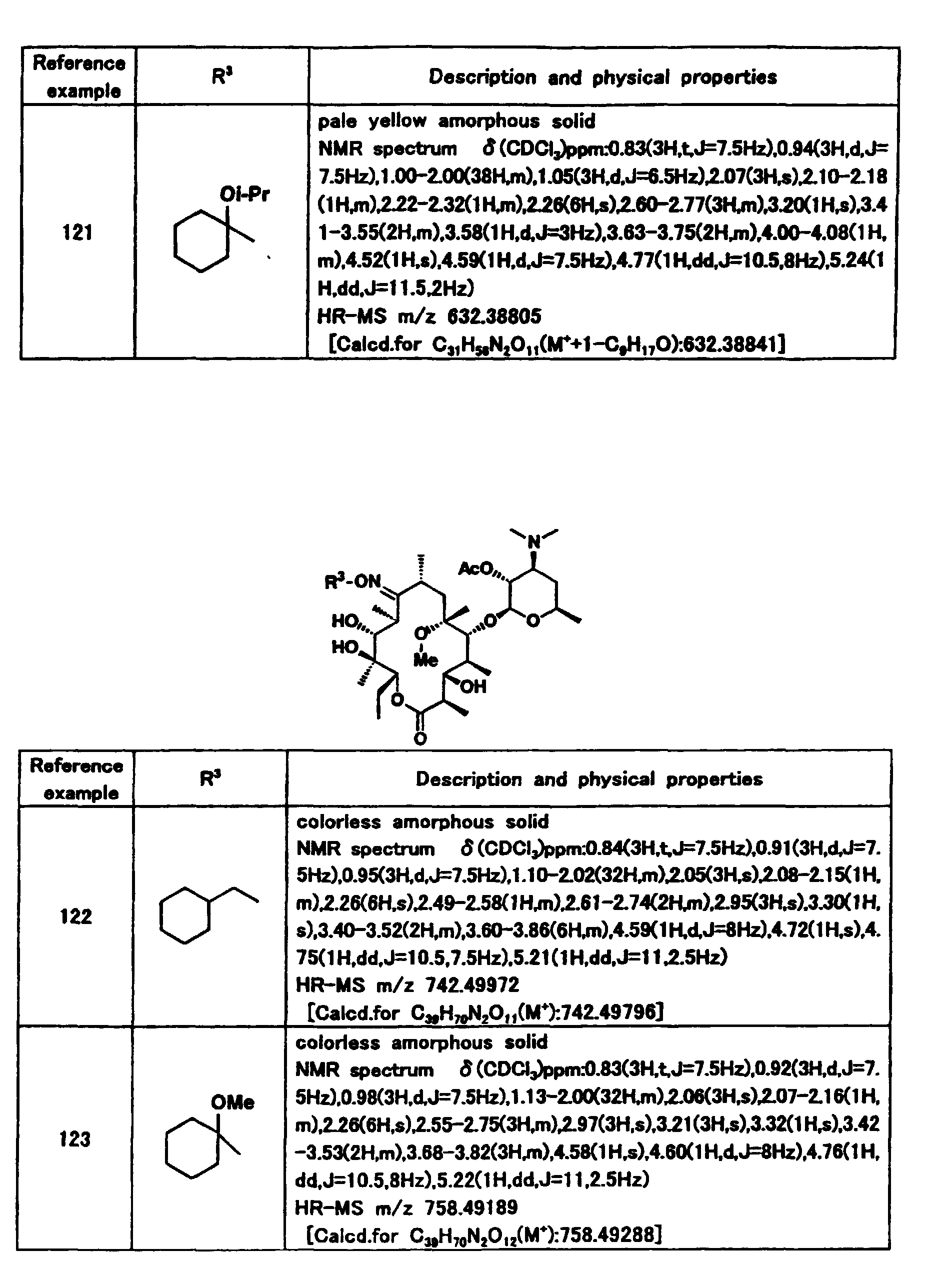

### Example 1: 5-O-Desosaminyl-3-O-phenylacetylerythronolide A 9-[O-(1-methoxycyclohexyl)oxime]

To a solution of 1.11 g of phenylacetic acid and 1.2 ml of triethylamine in 40 ml of dichloromethane, 1.0 ml of pivaloyl chloride was added dropwise under ice-cooling with stirring, and the reaction mixture was stirred at the same temperature for 1 hour. To the reaction mixture, 2.2 ml of pyridine and a solution of 2.00 g of 2'-O-acetyl-5-O-desosaminylerythronolide A 9-[O-(1-methoxycyclohexyl)oxime] in 15 ml of dichloromethane were successively added dropwise under ice-cooling with stirring, and the reaction mixture was stirred at room temperature for 1 day. The reaction mixture was added with ice-water, and the mixture was made alkaline with saturated aqueous sodium hydrogencarbonate solution and extracted with dichloromethane. The extract was washed with water, dried over sodium sulfate, and the solvent was removed under reduced pressure. The residue was purified by column chromatography (silica gel, dichloromethane:methanol = 33:1) to give 1.38 g of yellowish brown viscous oil. A solution of 1.20 g of the resulting yellowish brown viscous oil in 50 ml of methanol was stirred at room temperature for 1 day. The reaction mixture was concentrated under reduced pressure, and the residue was purified by column chromatography (silica gel, dichloromethane methanol = 100:1 → 50:3) to give 0.72 g of a colorless amorphous solid.
NMR spectrum δ (CDCl₃) ppm: 0.80 (3H, t, J=7.5Hz), 0.86 (3H, d, J=6.5Hz), 1.03 (3H, d, J=6.5Hz), 1.12-1.98 (32H, m), 2.22-2.31 (1H, m), 2.30 (6H, s), 2.33-2.42 (1H, m), 2.60 (1H, brs), 2.64-2.72 (1H, m), 2.75-2.85 (1H, m), 3.04-3.21 (3H, m), 3.19 (3H, s), 3.50 (1H, d, J=4.5Hz), 3.64-3.78 (2H, m), 3.65 (1H, d, J=14.5Hz), 3.71 (1H, d, J=14.5Hz), 3.96 (1H, d, J=7.5Hz), 4.57 (1H, s), 5.14 (1H, d, J=10.5Hz), 5.20 (1H, dd, J=11, 2.5Hz), 7.24-7.38 (5H, m)
HR-MS m/z 820.50550 [Calcd. for C₄₄H₇₂N₂O₁₂ (M⁺): 820.50853]

Compounds of Examples 2 and 3 were obtained in the same manner as that described in Example 1.

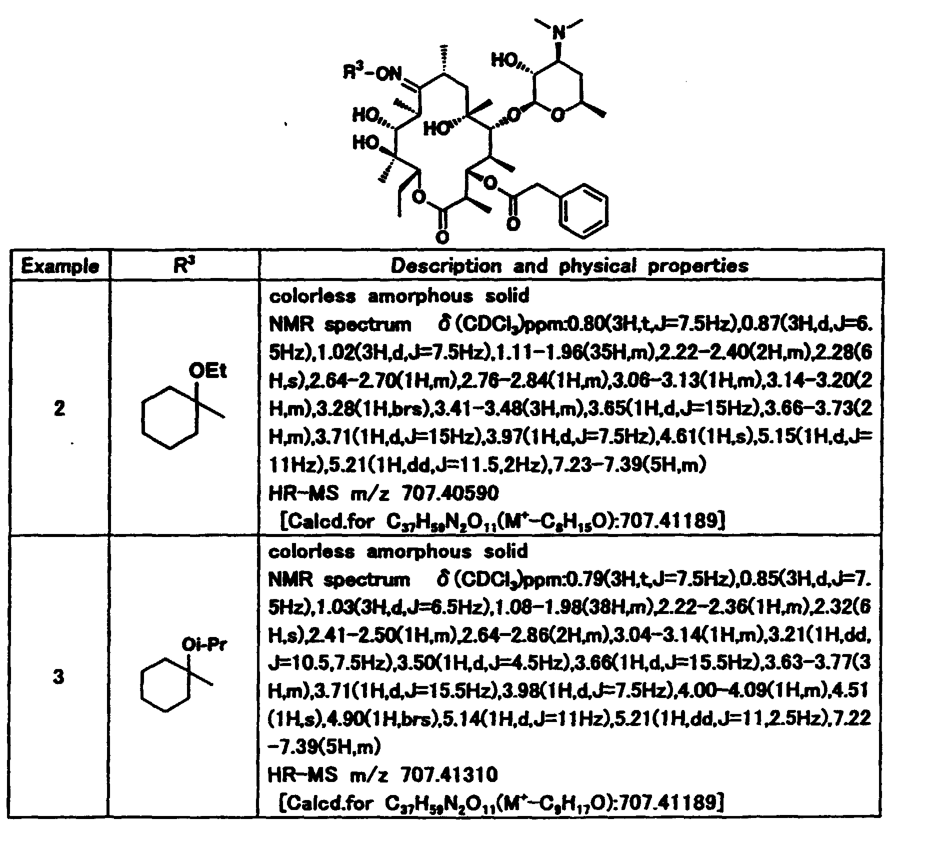

### Example 4: 5-O-Desosaminyl-3-O-phenylacetyl-6-O-methylerythronolide A 9-[O-(1-methoxycyclohexyl)oxime]

To a solution of 0.05 g of phenylacetic acid and 0.03 ml of oxalyl chloride in 0.6 ml of dichloromethane, 1 drop of N,N-dimethylformamide was added with stirring at room temperature. Then, the reaction mixture was stirred at room temperature for 30 minutes, and concentrated under reduced pressure. To a solution of 0.10 g of 2'O-acetyl-5-O-desosaminyl-6-O-methylerythronolide A 9-[O(1-methoxycyclohexyl)oxime] and 0.09 ml of pyridine in 0.8 ml of dichloromethane, a solution of the resulting residue in 2.5 ml of dichloromethane was added dropwise at room temperature with stirring, and the reaction mixture was stirred at room temperature for 1 hour. The reaction mixture was added with ice-water, and the mixture was made alkaline with saturated aqueous sodium hydrogencarbonate solution and extracted with diethyl ether. The extract was washed with water, dried over sodium sulfate, and the solvent was removed under reduced pressure. A solution of the resulting residue in 4 ml of methanol was stirred at room temperature for 20 hours. The reaction mixture was concentrated under reduced pressure, and the residue was added with ice-water. The mixture was made alkaline with saturated aqueous sodium hydrogencarbonate solution and extracted with diethyl ether. The extract was washed with water, dried over sodium sulfate, and the solvent was removed under reduced pressure. The residue was purified by column chromatography (silica gel, dichloromethane:methanol = 20:1) to give 0.05 g of a colorless amorphous solid.
NMR spectrum δ (CDCl₃) ppm: 0.80 (3H, t, J=7.5Hz), 0.89 (3H, d, J=6.5Hz), 0.97 (3H, d, J=7.5Hz), 1.00-2.00 (31H, m), 2.10-2.39 (2H, m), 2.28 (6H, s), 2.52-2.64 (1H, m), 2.76-2.88 (1H, m), 2.90-3.35 (4H, m), 3.07 (3H, s), 3.21 (3H, s), 3.37-3.50 (1H, m), 3.67 (1H, d, J=15Hz), 3.73 (1H, d, J=15Hz), 3.68-3.82 (2H, m), 3.90 (1H, d, J=7.5Hz), 4.58 (1H, s), 5.07 (1H, d, J=11Hz), 5.20 (1H, dd, J=11, 2Hz), 7.17-7.40 (5H, m)
HR-MS m/z 721.42800 [Calcd. for C₃₈H₆₁N₂O₁₁ (M⁺-C₇H₁₃O): 721.42754]

Compounds of Examples 5 through 22 were obtained in the same manner as that described in Example 4.

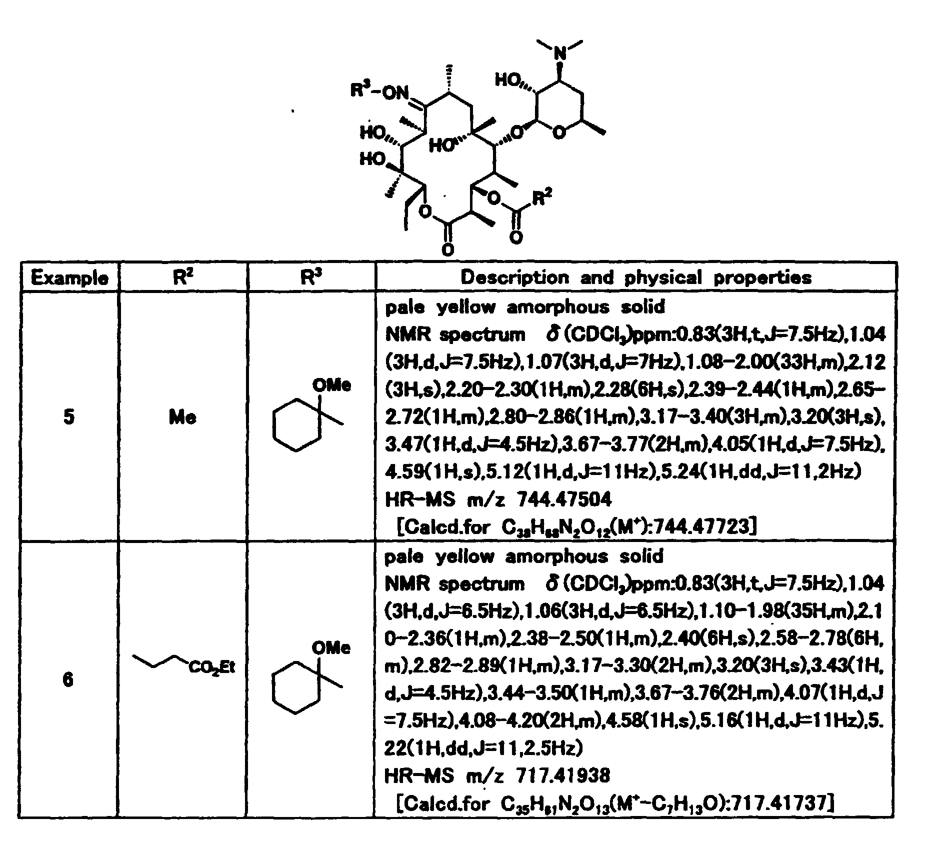

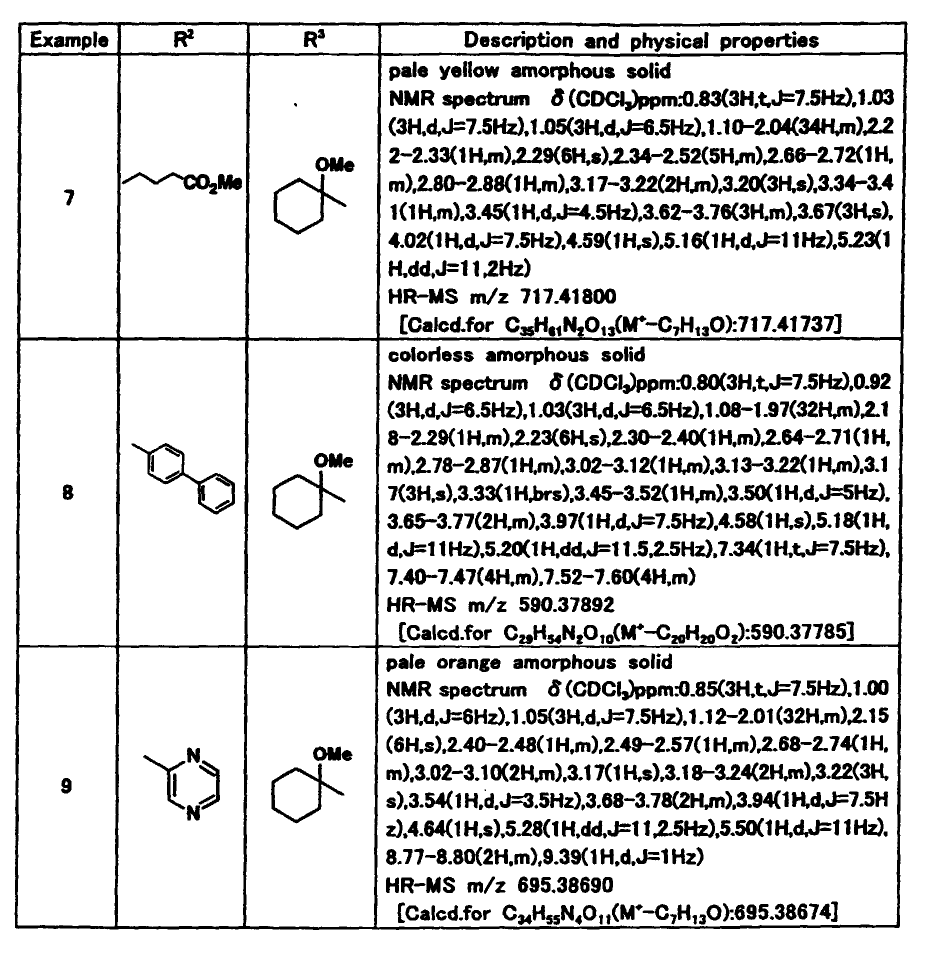

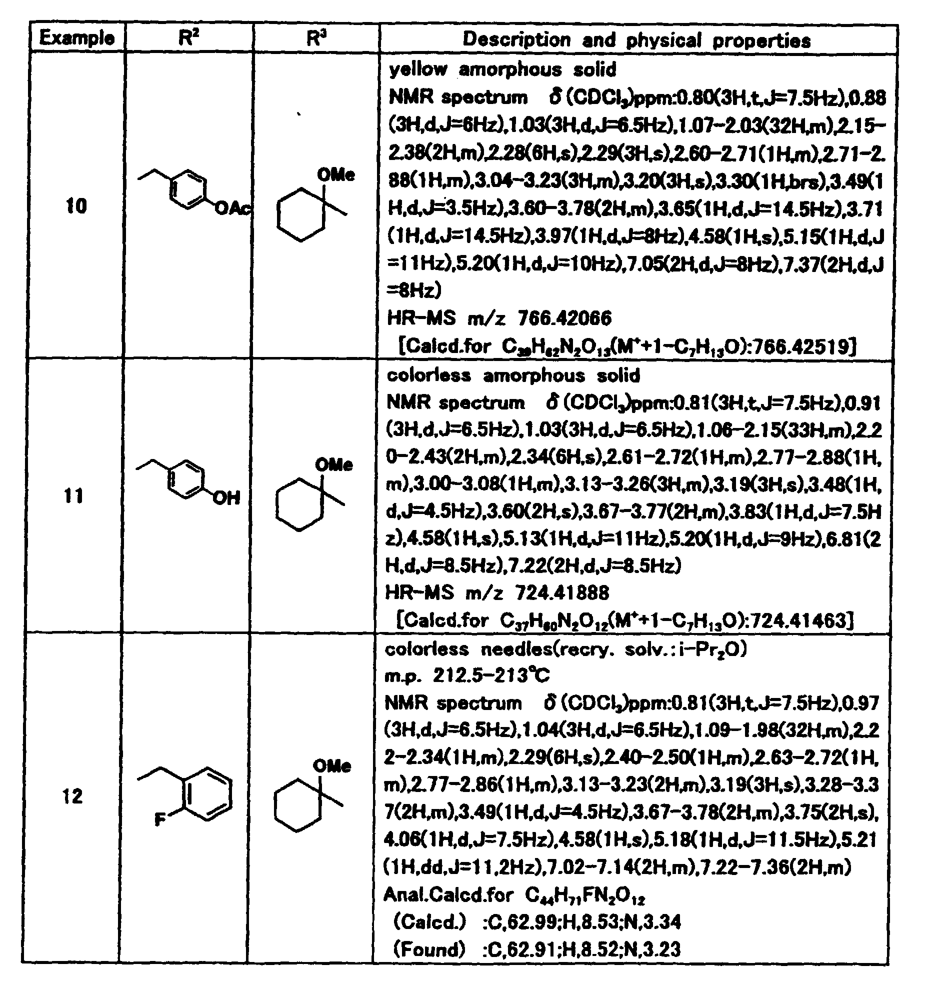

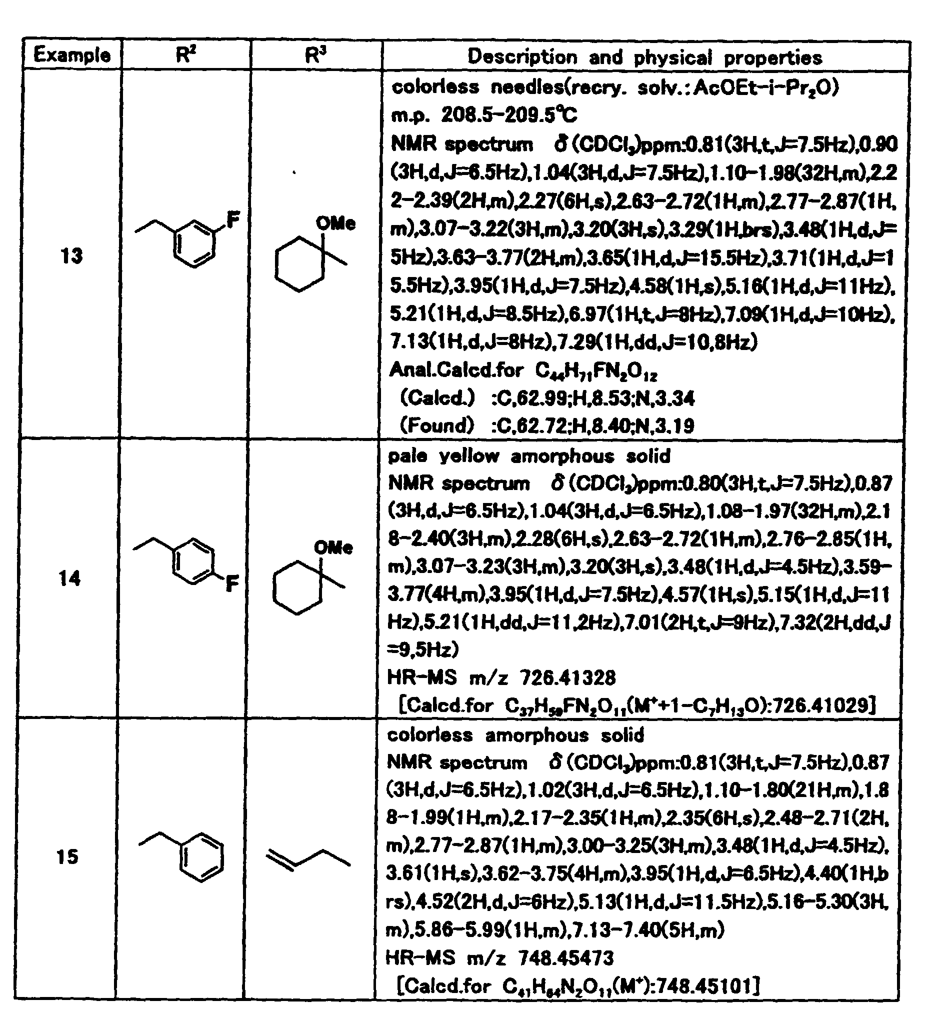

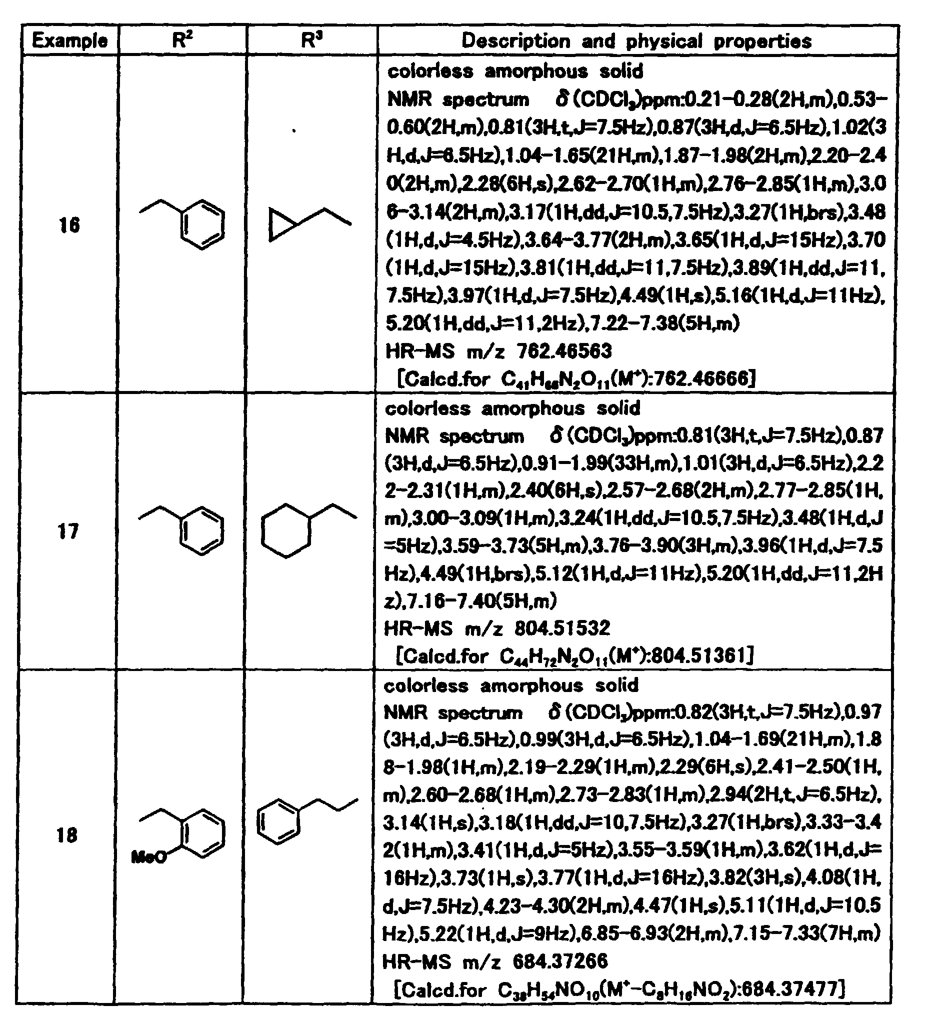

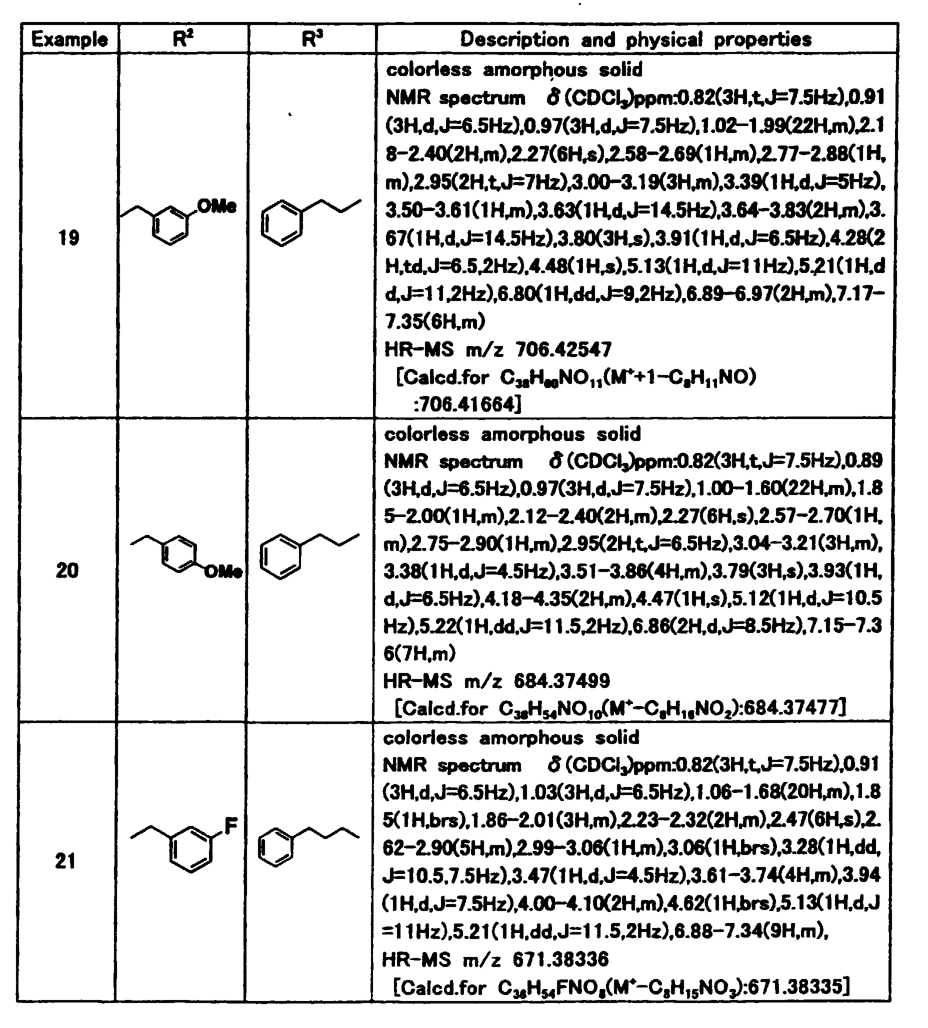

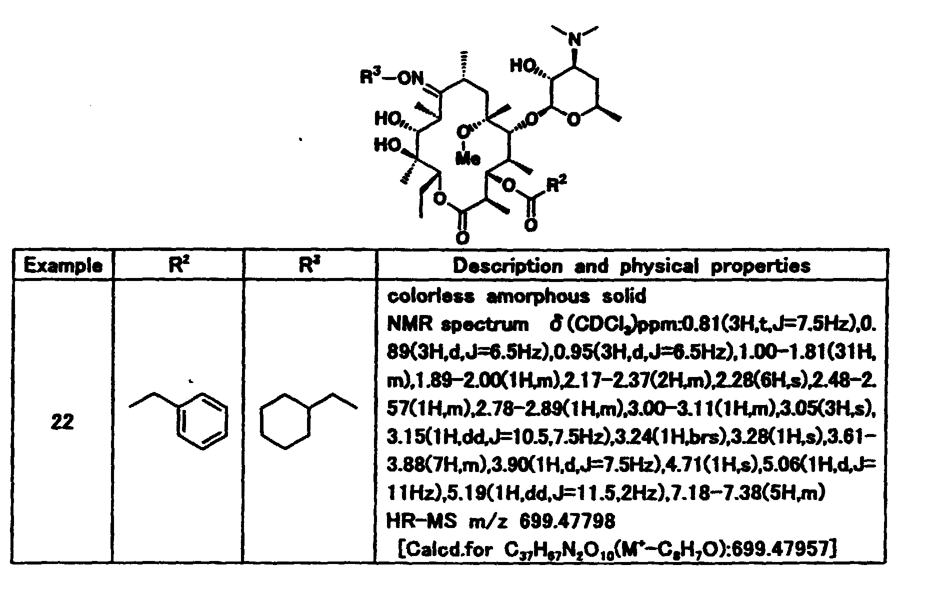

### Example 23: 5-O-Desosaminyl-3-O-phenylacetylerythronolide A 9-[O-(phenethyl)oxime]

A mixture of 0.50 g of 2'-O-acetyl-5-O-desosaminylerythronolide A 9-[O-(phenethyl)oxime], 0.28 g of phenylacetic acid, 0.40 g of 1-ethyl-3-(3-dimethylaminopropyl)carbodiimide hydrochloride and 0.11 g of 4-dimethylaminopyridine in 5 ml of dichloromethane was stirred at room temperature for 4 hours. The reaction mixture was added with water, and the mixture was made alkaline with saturated aqueous sodium hydrogencarbonate solution and extracted with dichloromethane. The extract was washed with water, dried over sodium sulfate, and the solvent was removed under reduced pressure. A solution of the resulting residue in 40 ml of methanol was stirred at room temperature for 2.6 days. The reaction mixture was concentrated under reduced pressure and the residue was purified by column chromatography (silica gel, dichloromethane:methanol = 50:1 → 25:1) to give 0.32 g of a pale yellow amorphous solid.
NMR spectrum δ (CDCl₃) ppm: 0.81 (3H, t, J=7.5Hz), 0.88 (3H, d, J=6.5Hz), 0.97 (3H, d, J=7.5Hz), 1.05-1.65 (21H, m), 1.87-1.97 (1H, m), 2.19-2.39 (2H, m), 2.27 (6H, a), 2.60-2.68 (1H, m), 2.76-2.85 (1H, m), 2.95 (2H, t, J=7Hz), 3.06-3.19 (3H, m), 3.26 (1H, brs), 3.39 (1H, d, J=4.5Hz), 3.52-3.61 (1H, m), 3.65 (1H, d, J=14.5Hz), 3.70 (1H, d, J=14.5Hz), 3.72 (1H, s), 3.94 (1H, d, J=7.5Hz), 4.27 (2H, td, J=7, 2Hz), 4.47 (1H, s), 5.13 (1H, d, J=11Hz), 5.22 (1H, dd, J=11, 2Hz), 7.14-7.38 (10H, m)
HR-MS m/z 675.40315 [Calcd. for C₃₇H₅₇NO₁₀ (M⁺-C₈H₁₁NO): 675.39825]

Compounds of Examples 24 through 202 were obtained in the same manner as that described in Example 23.

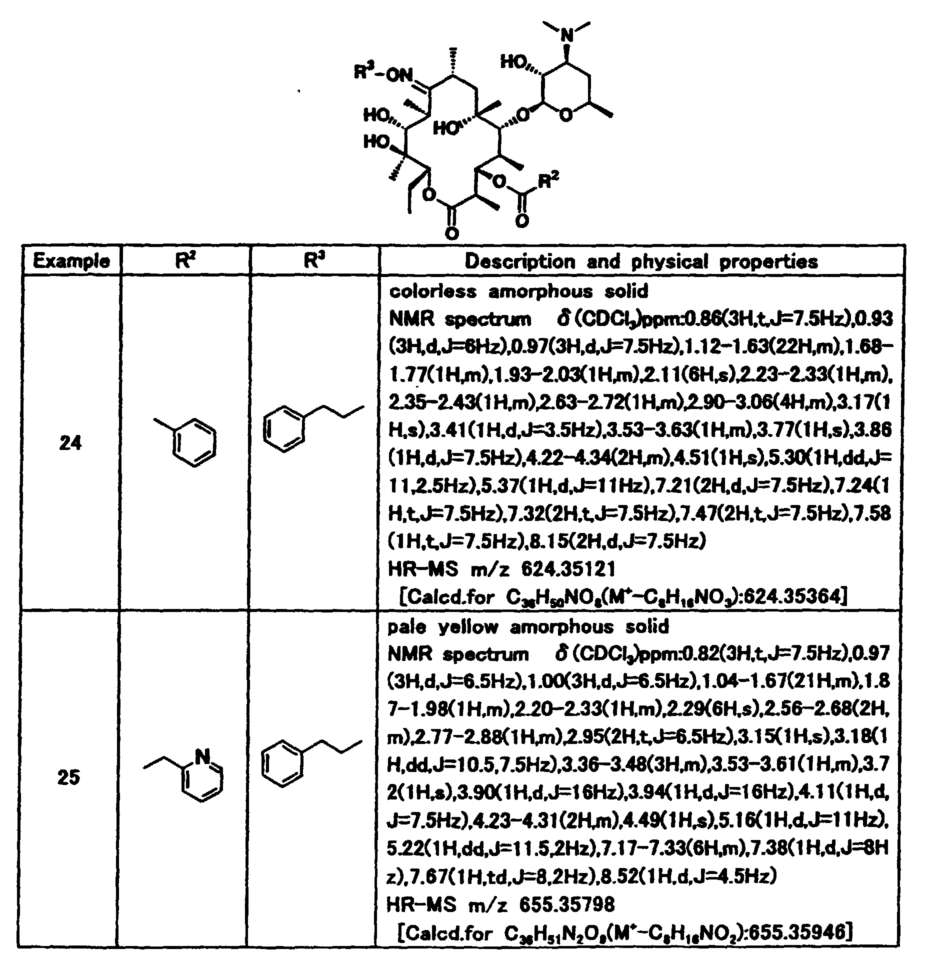

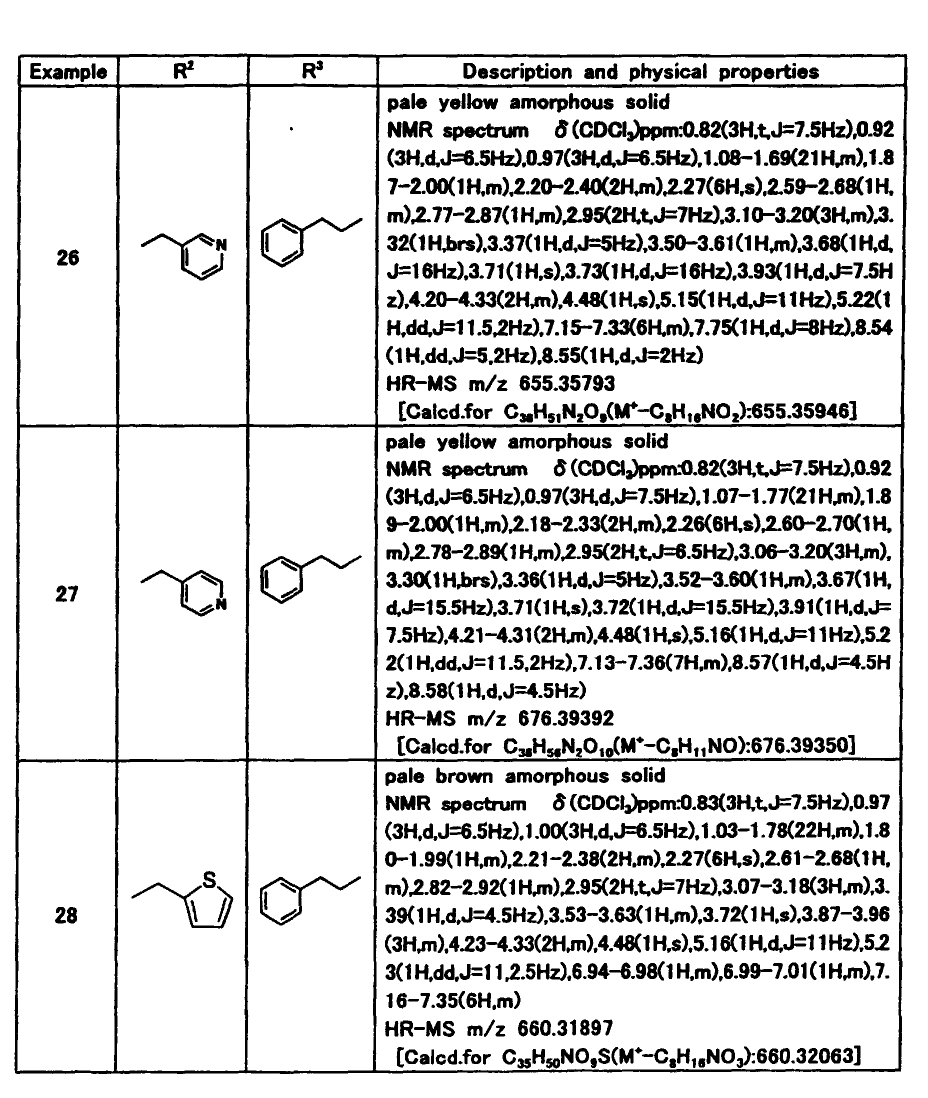

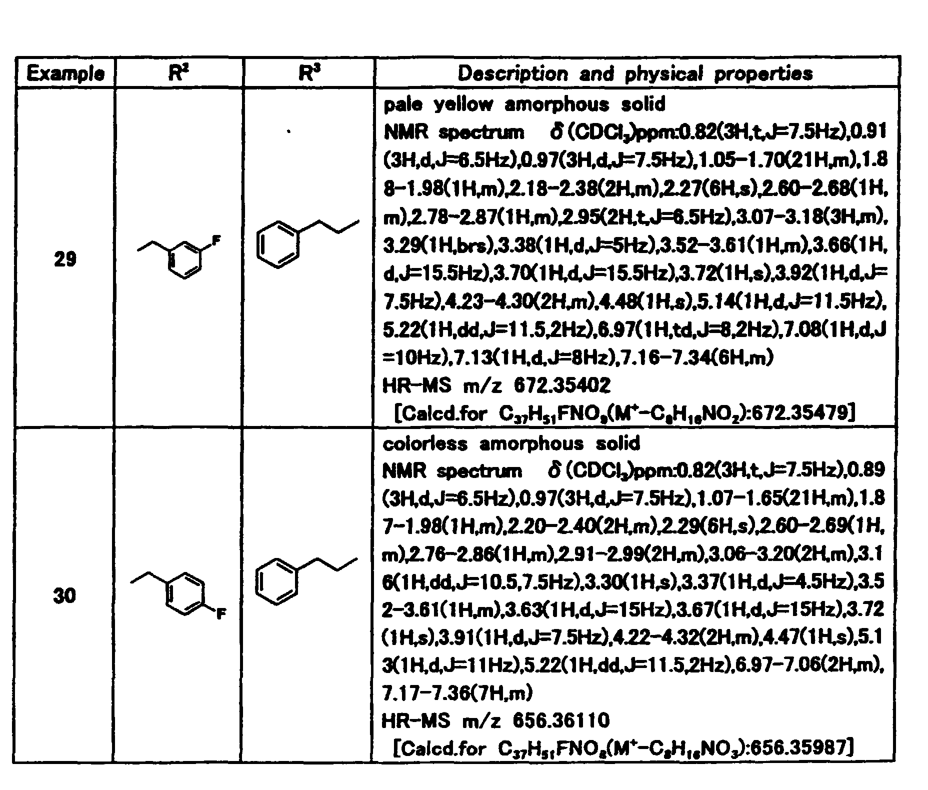

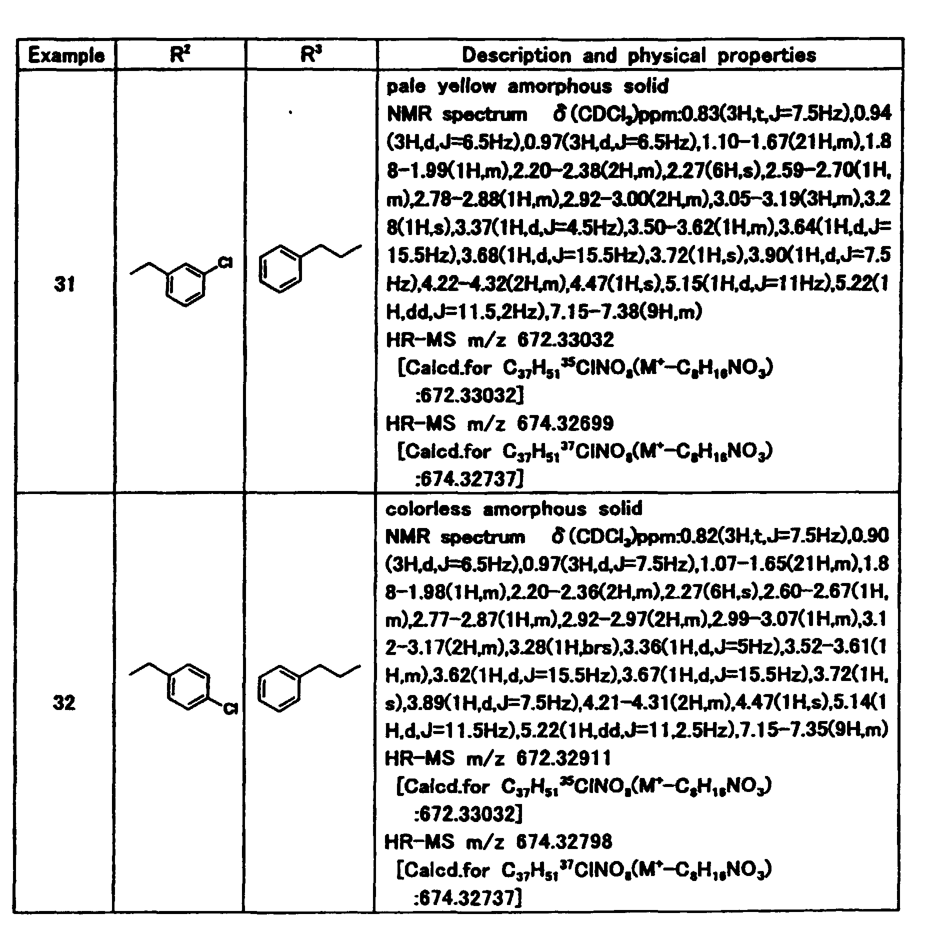

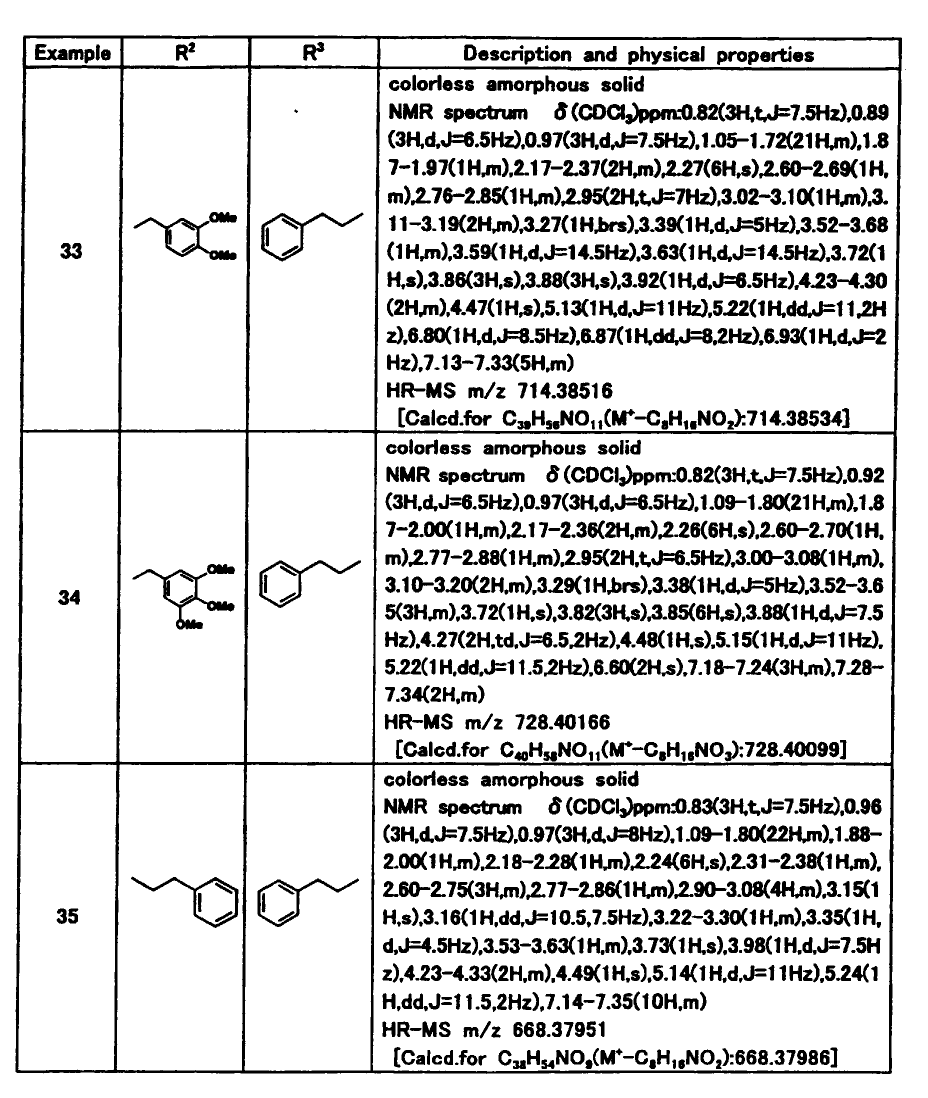

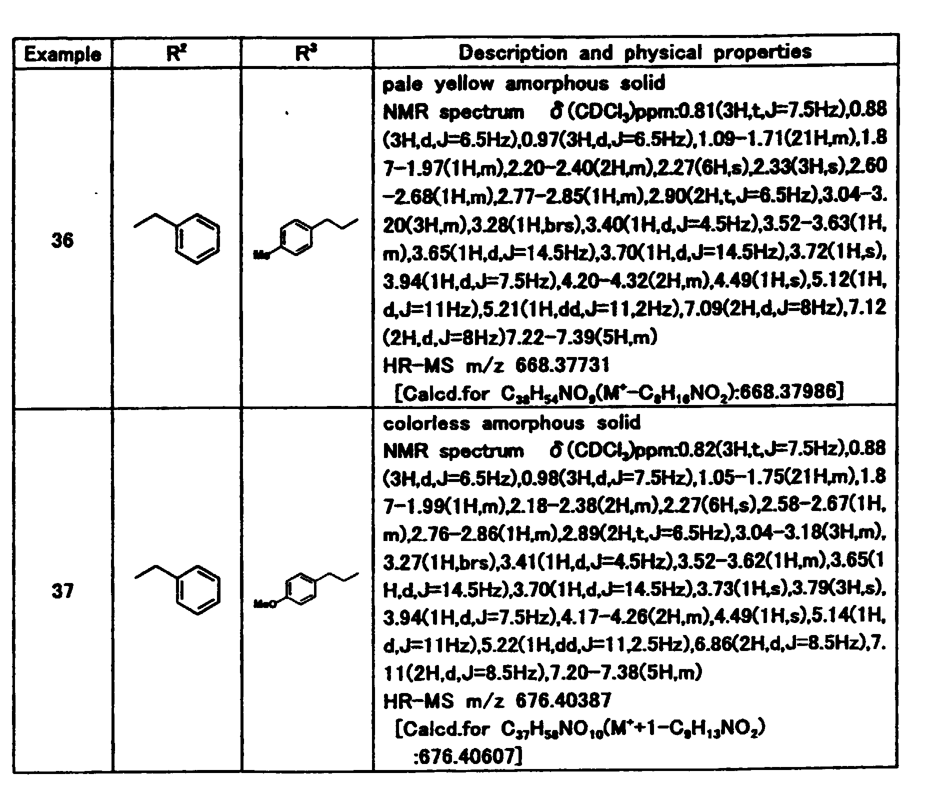

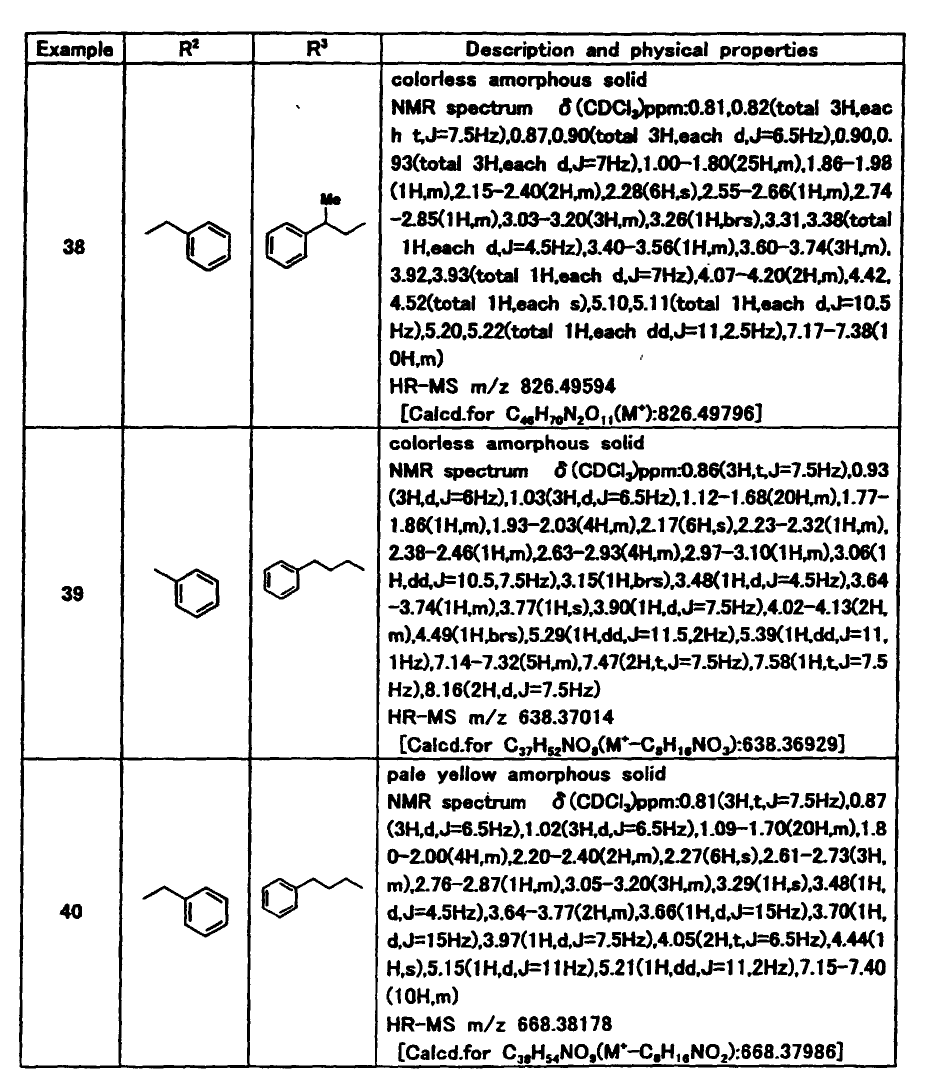

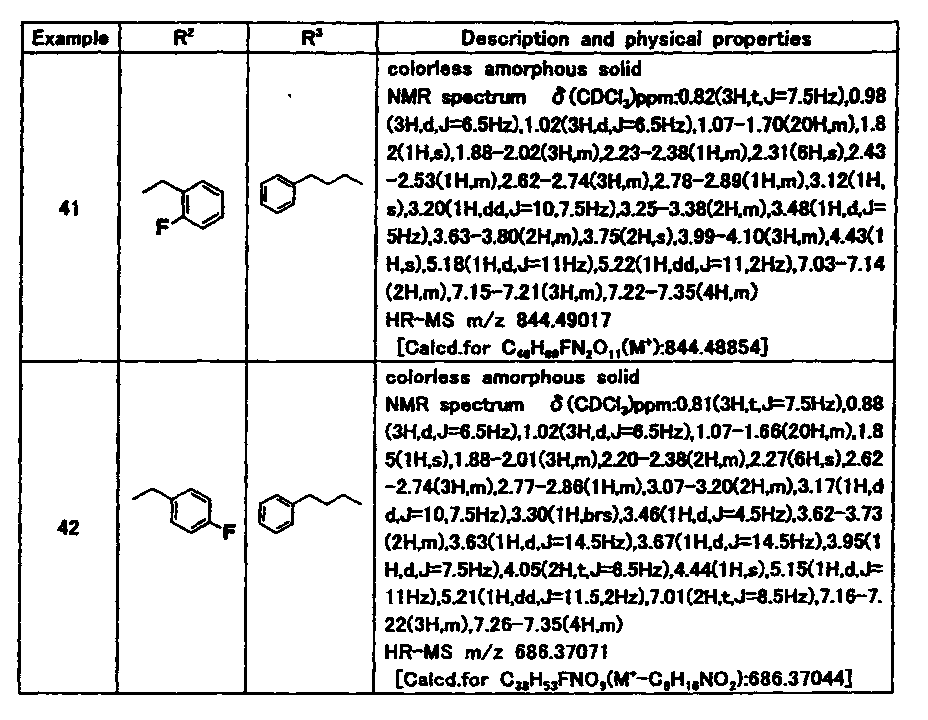

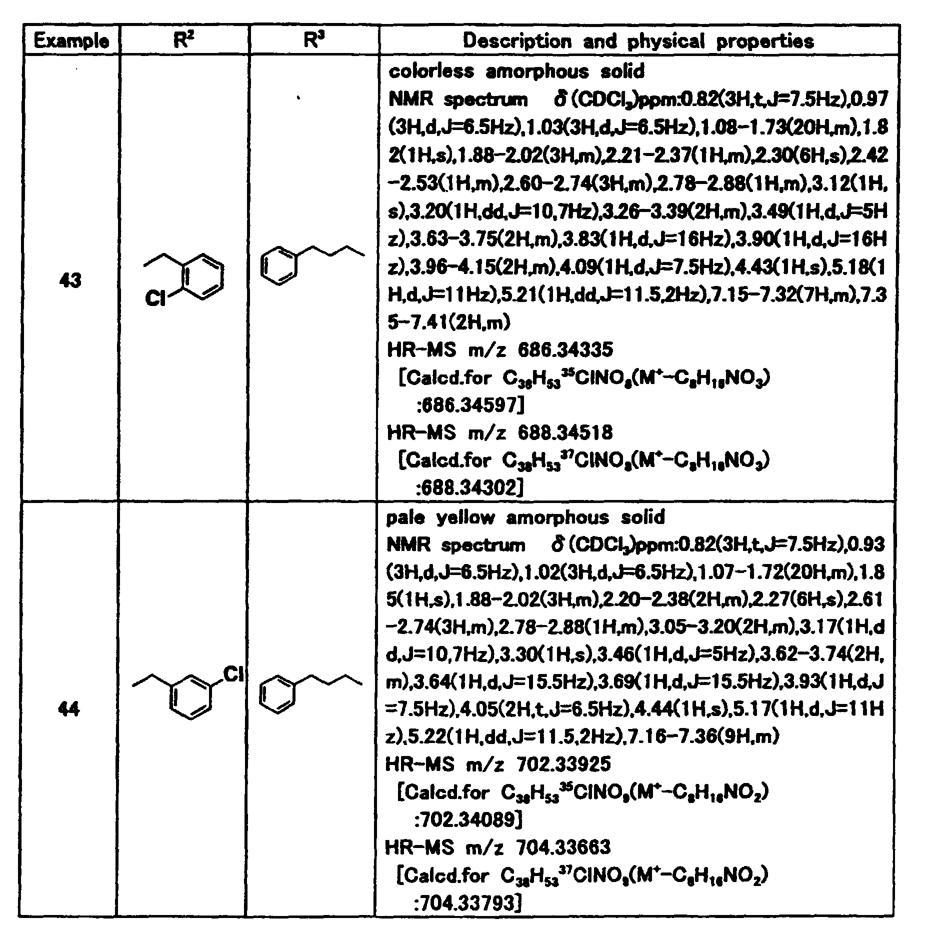

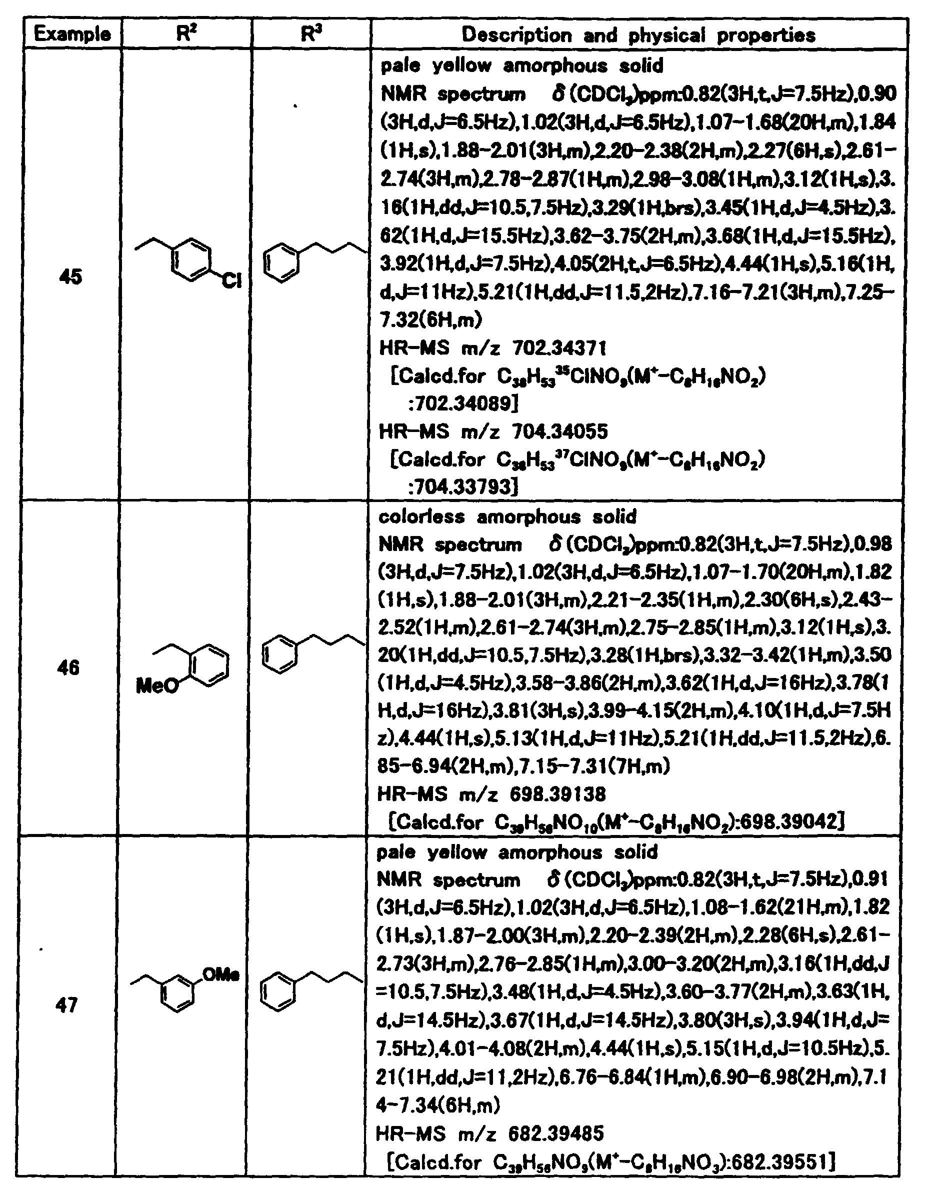

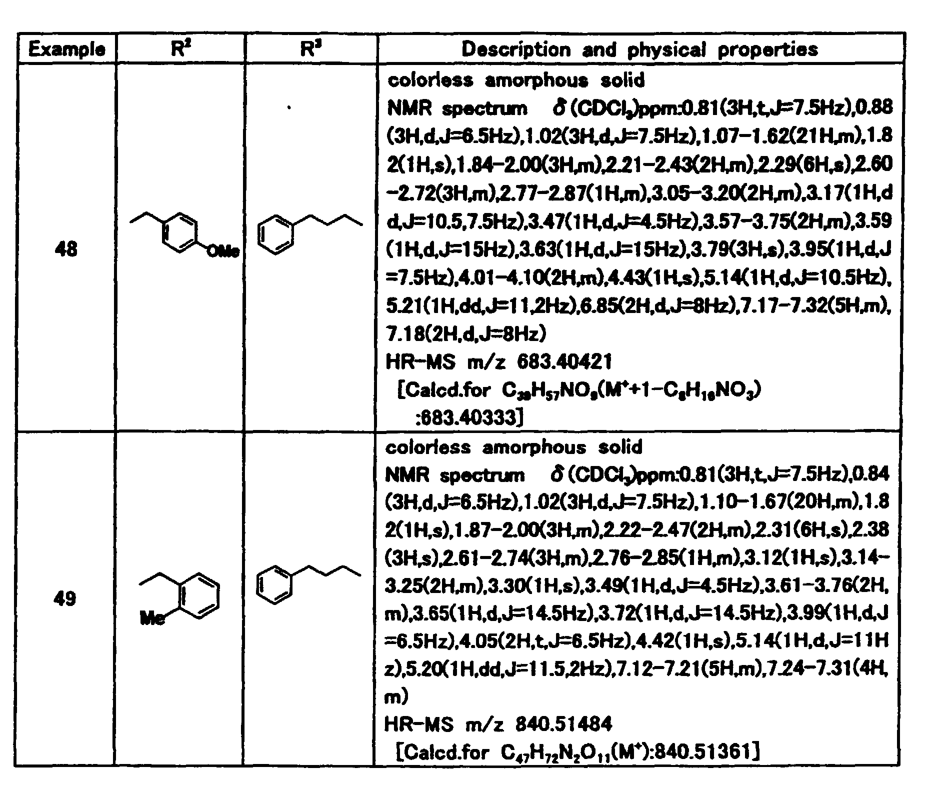

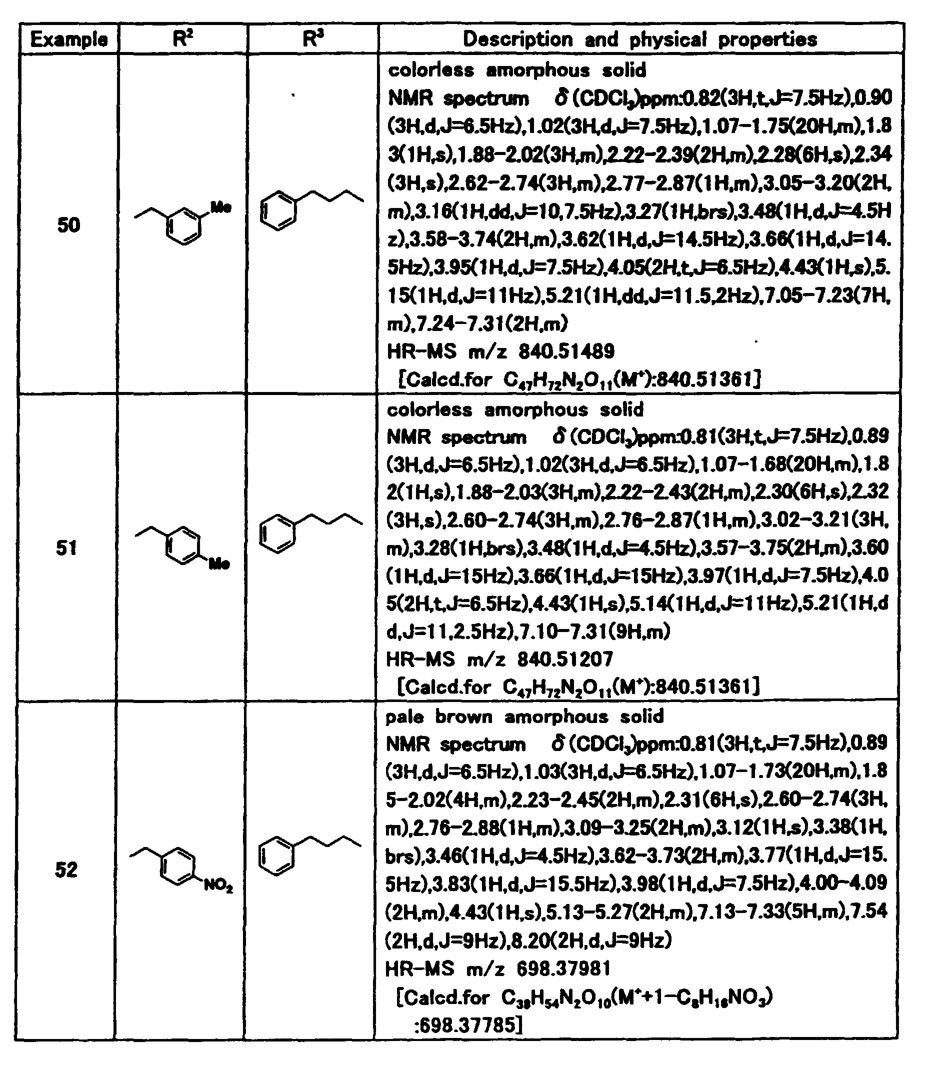

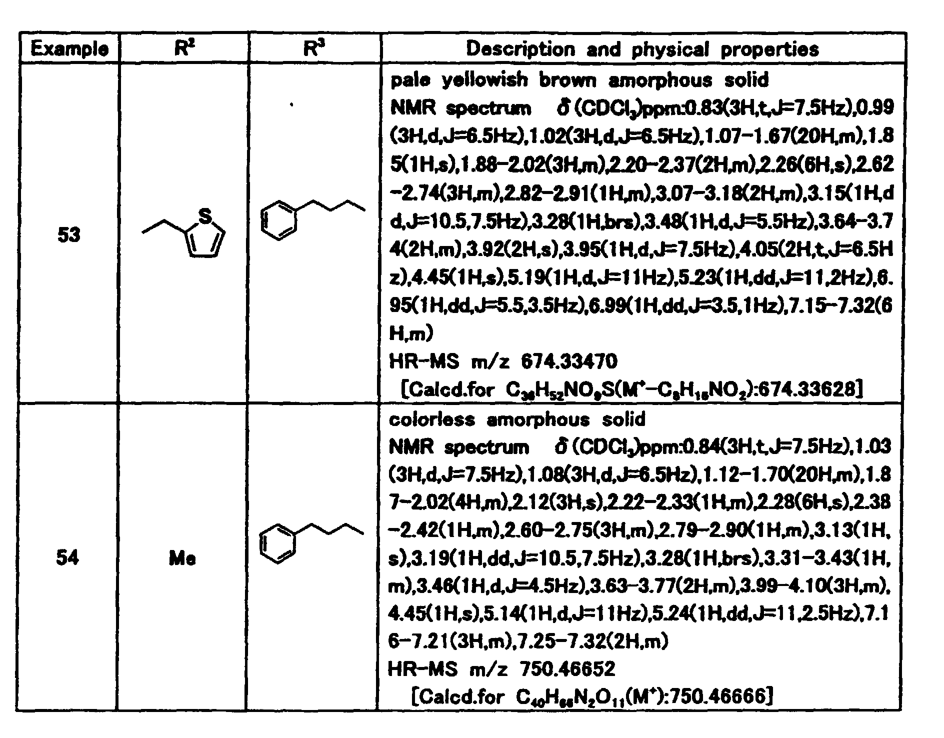

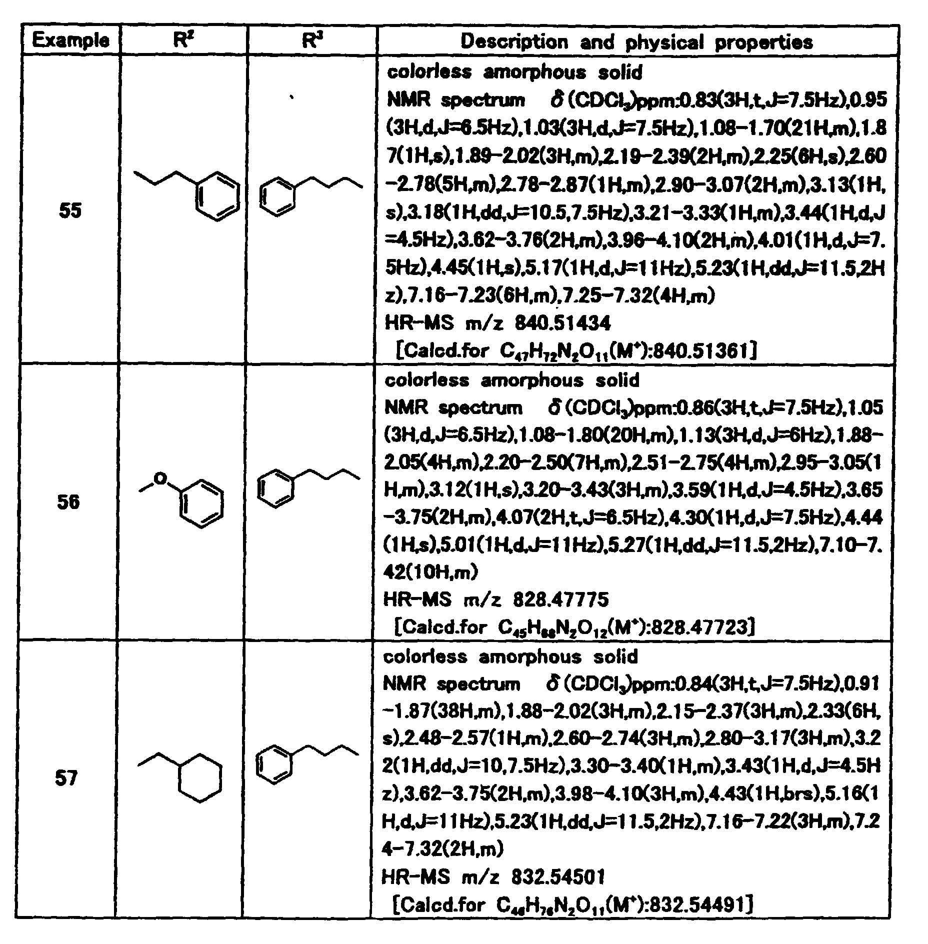

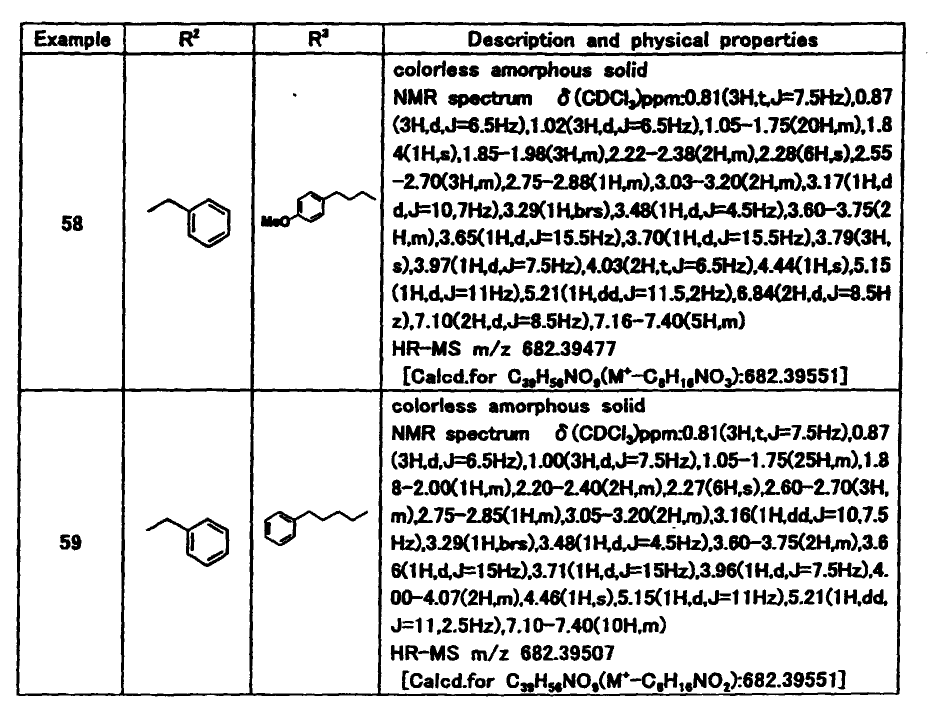

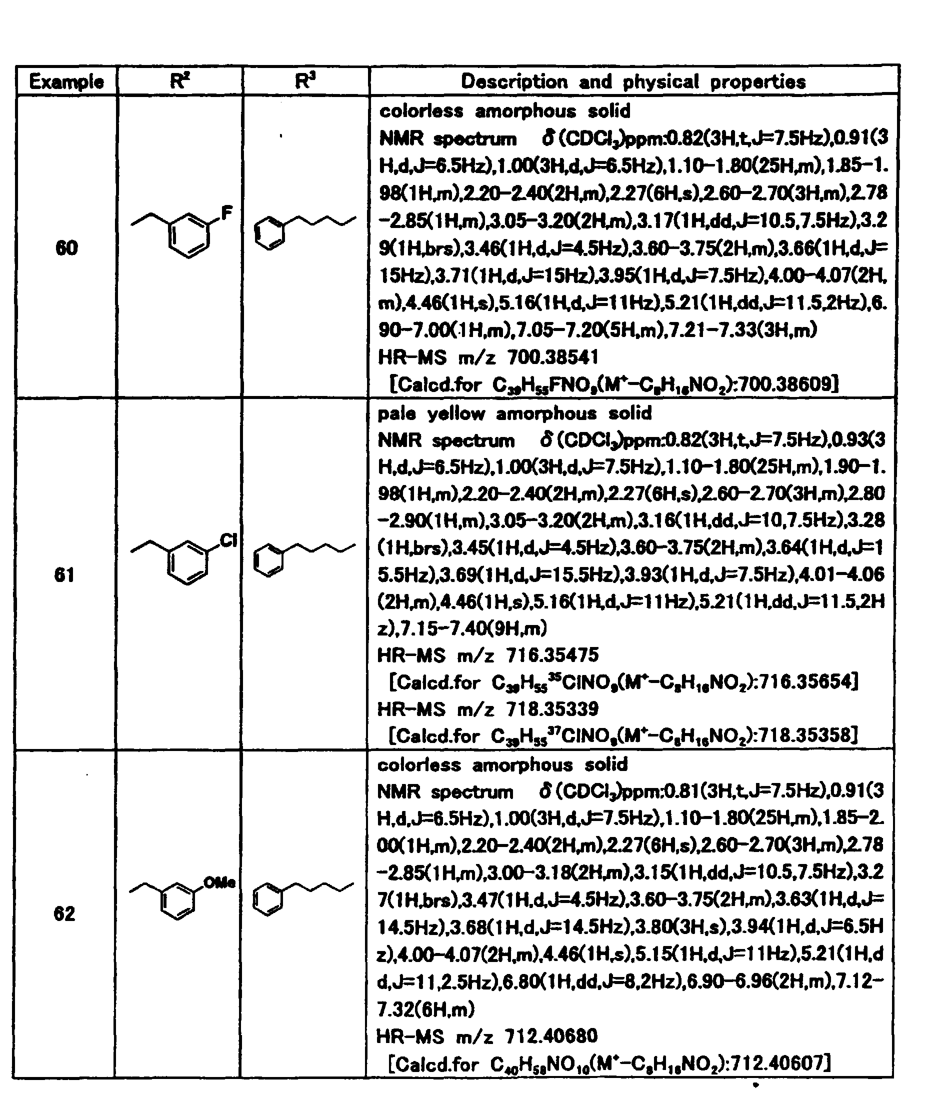

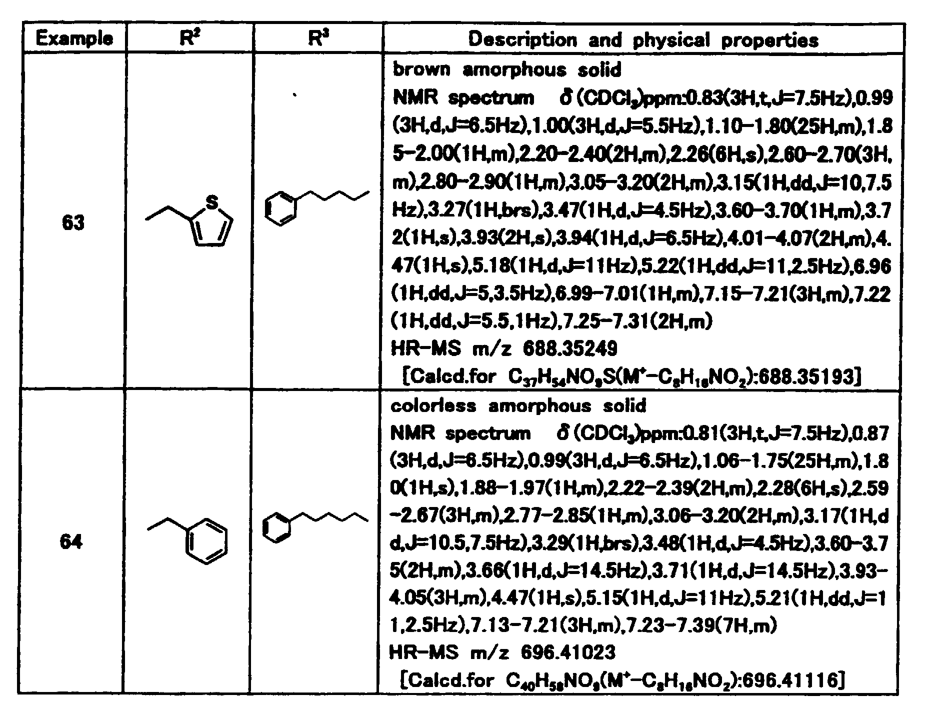

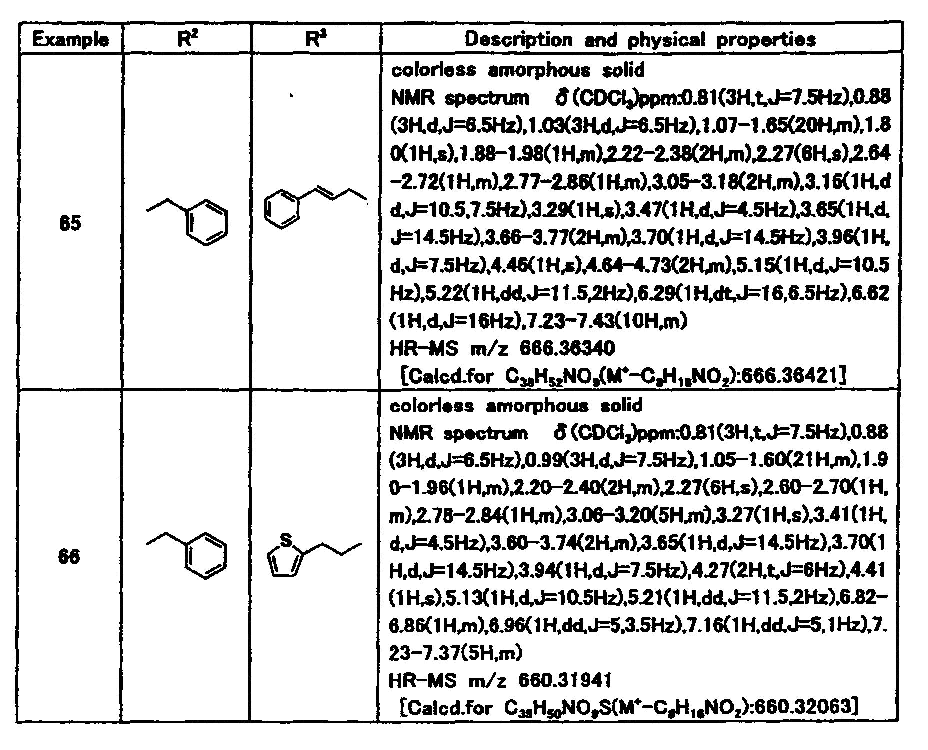

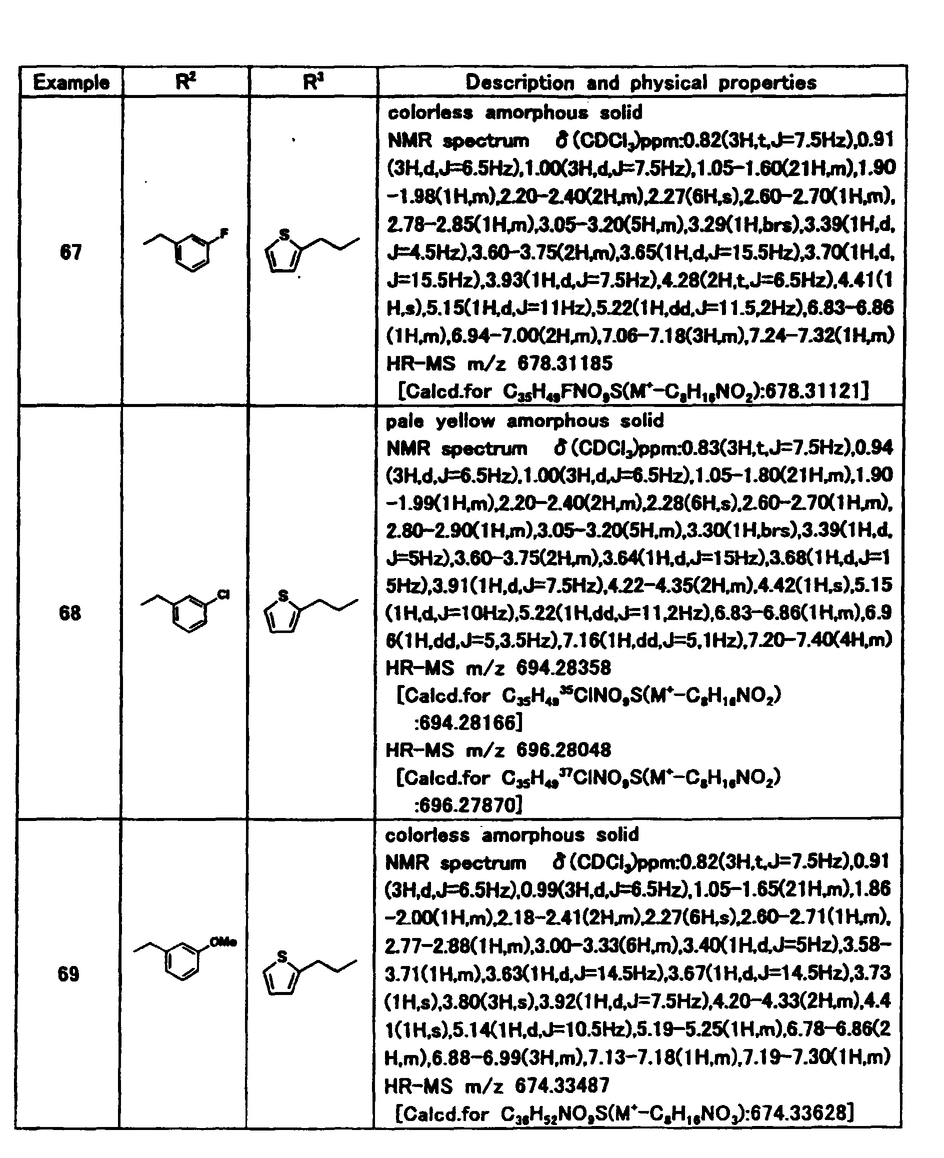

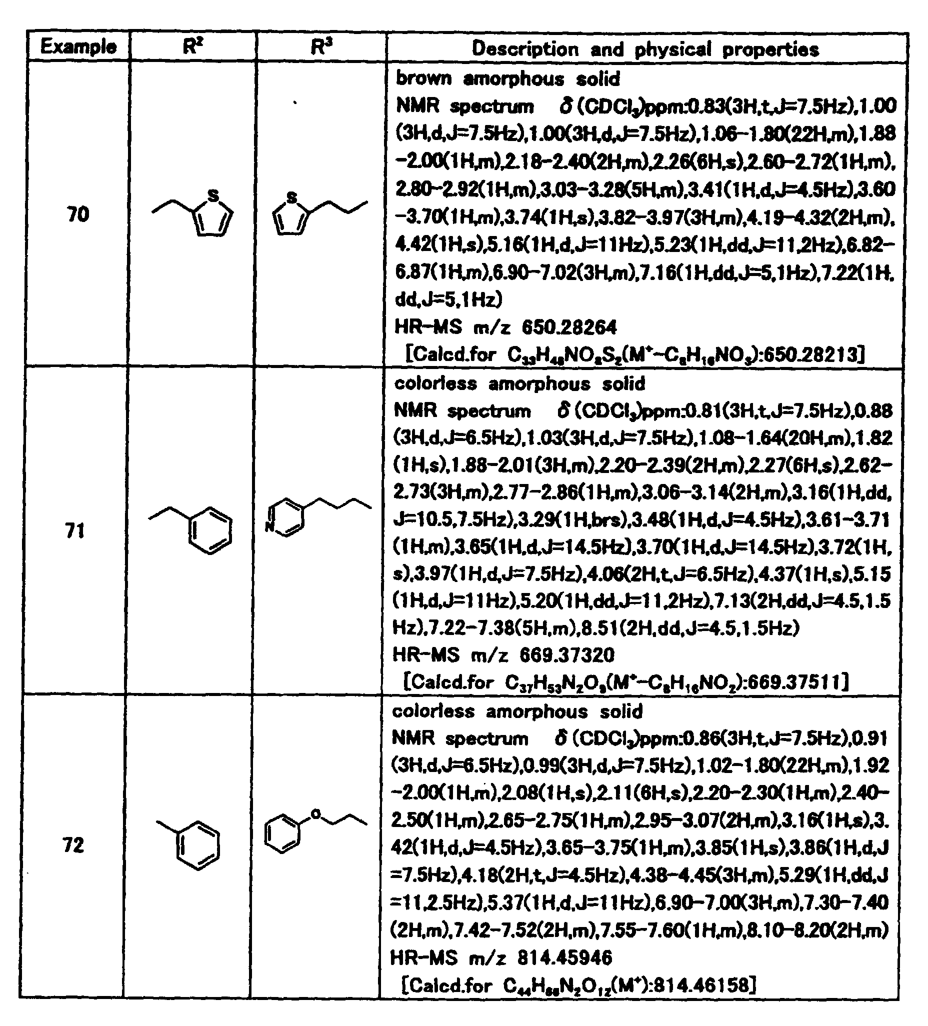

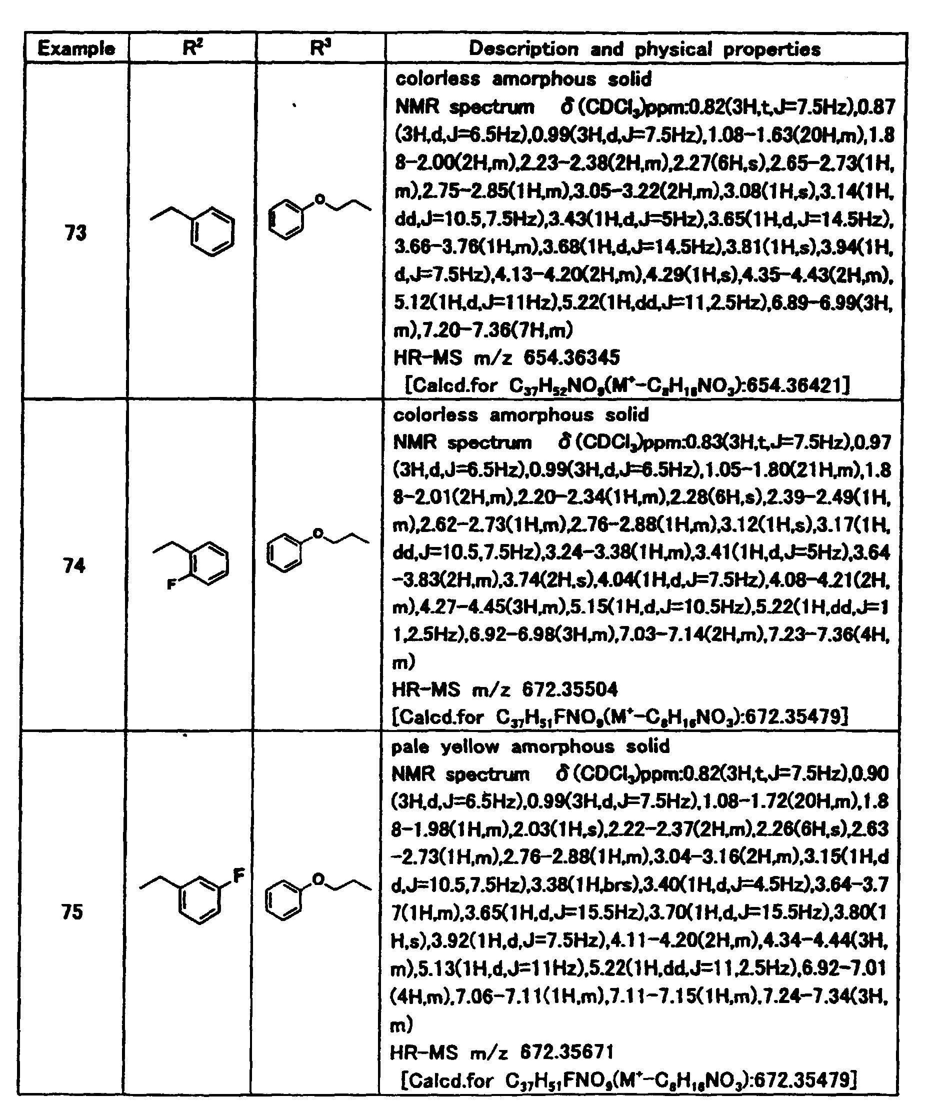

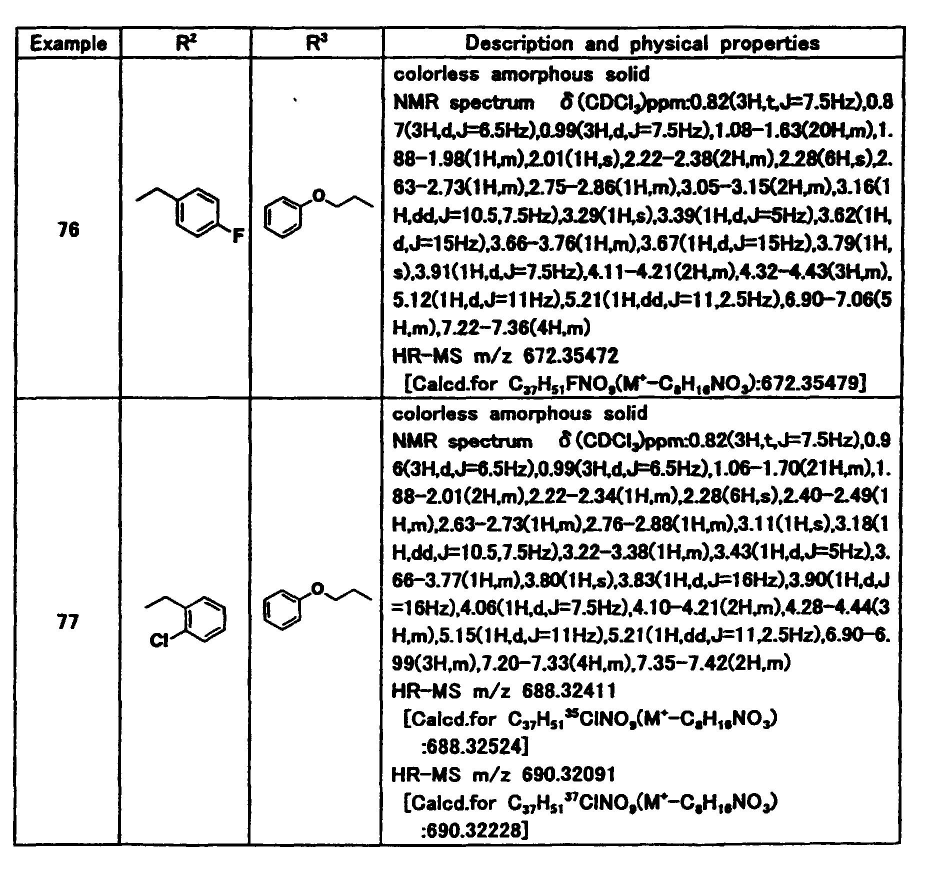

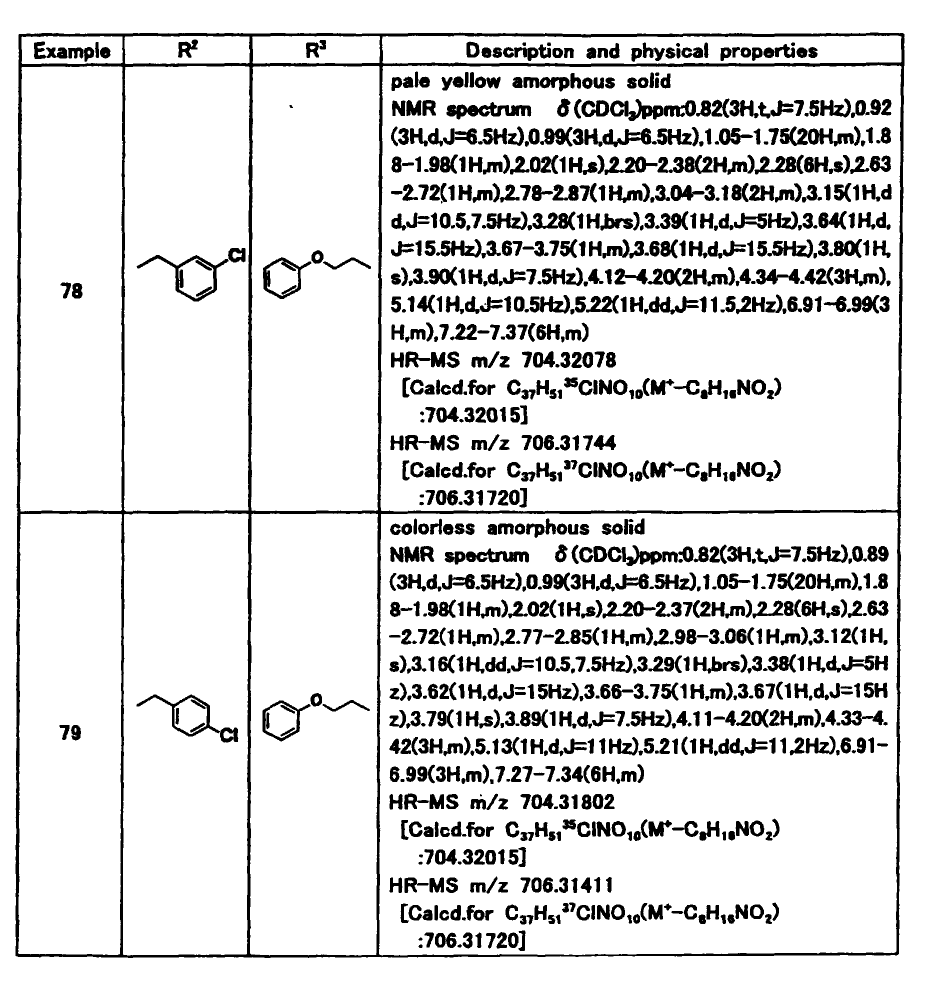

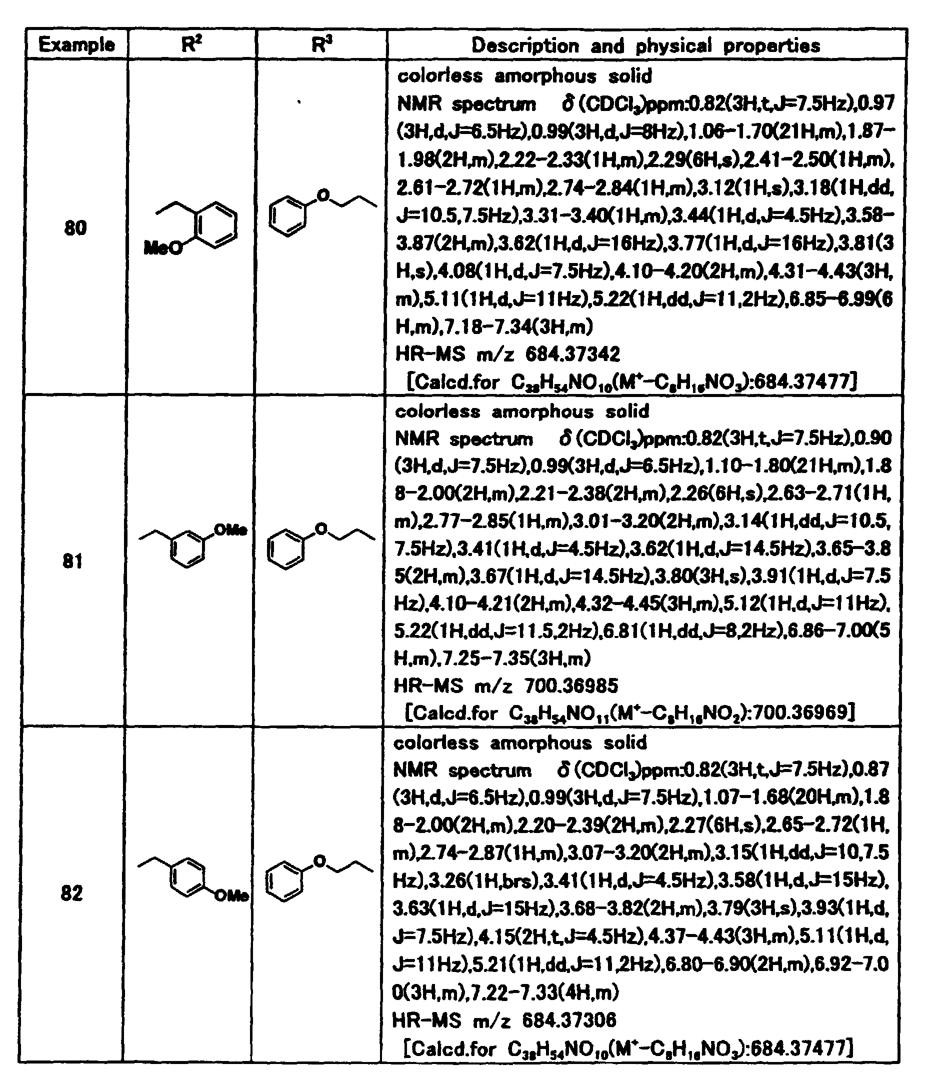

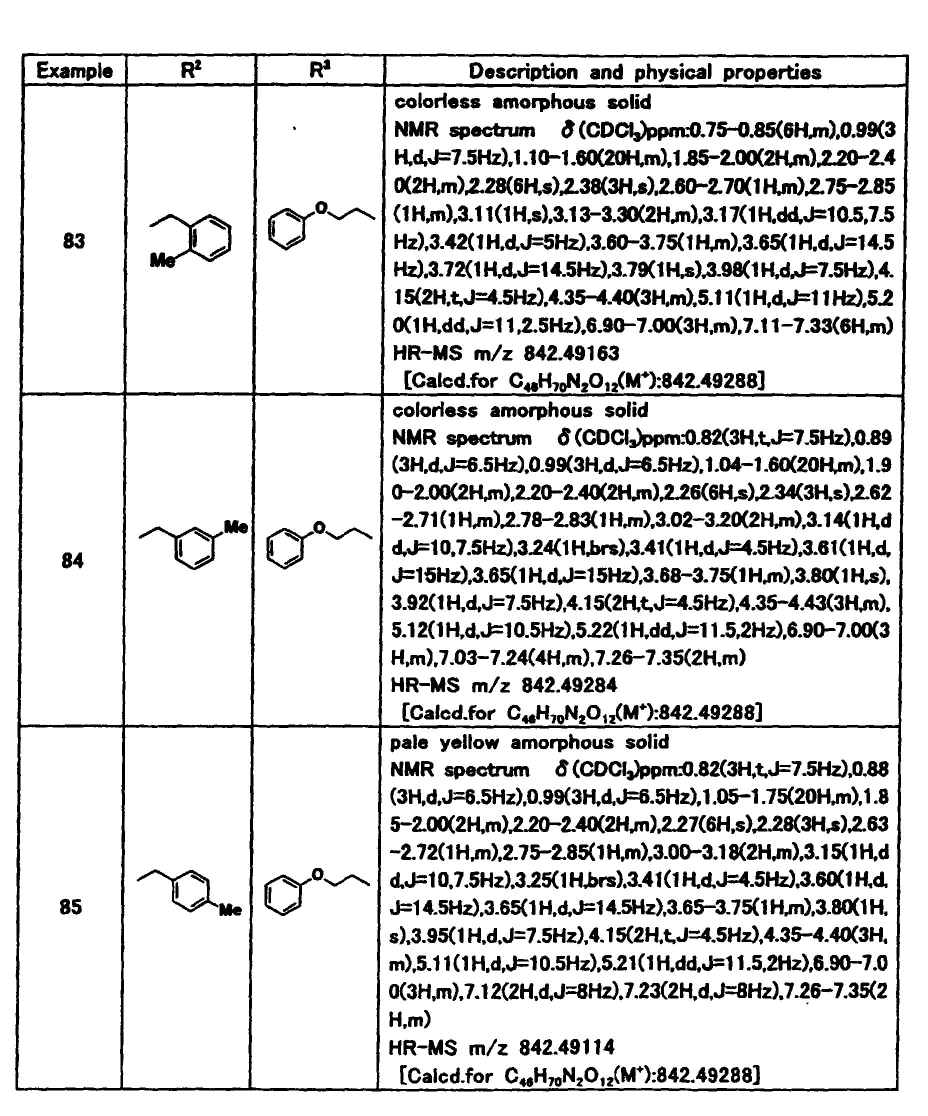

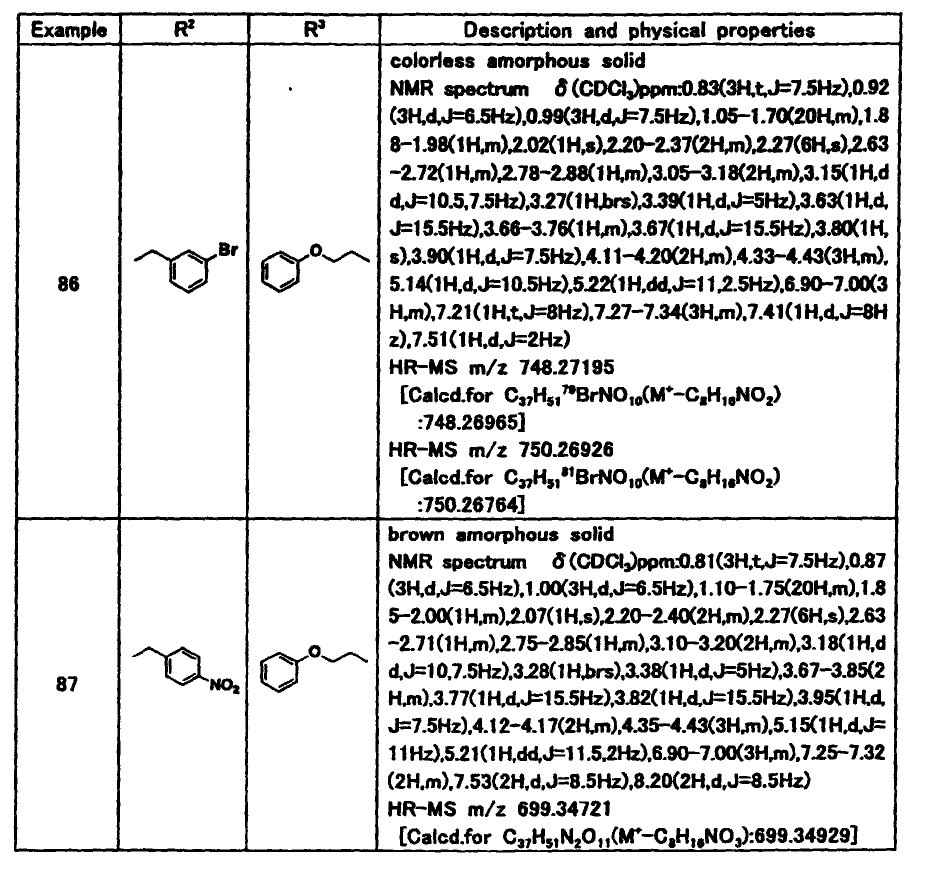

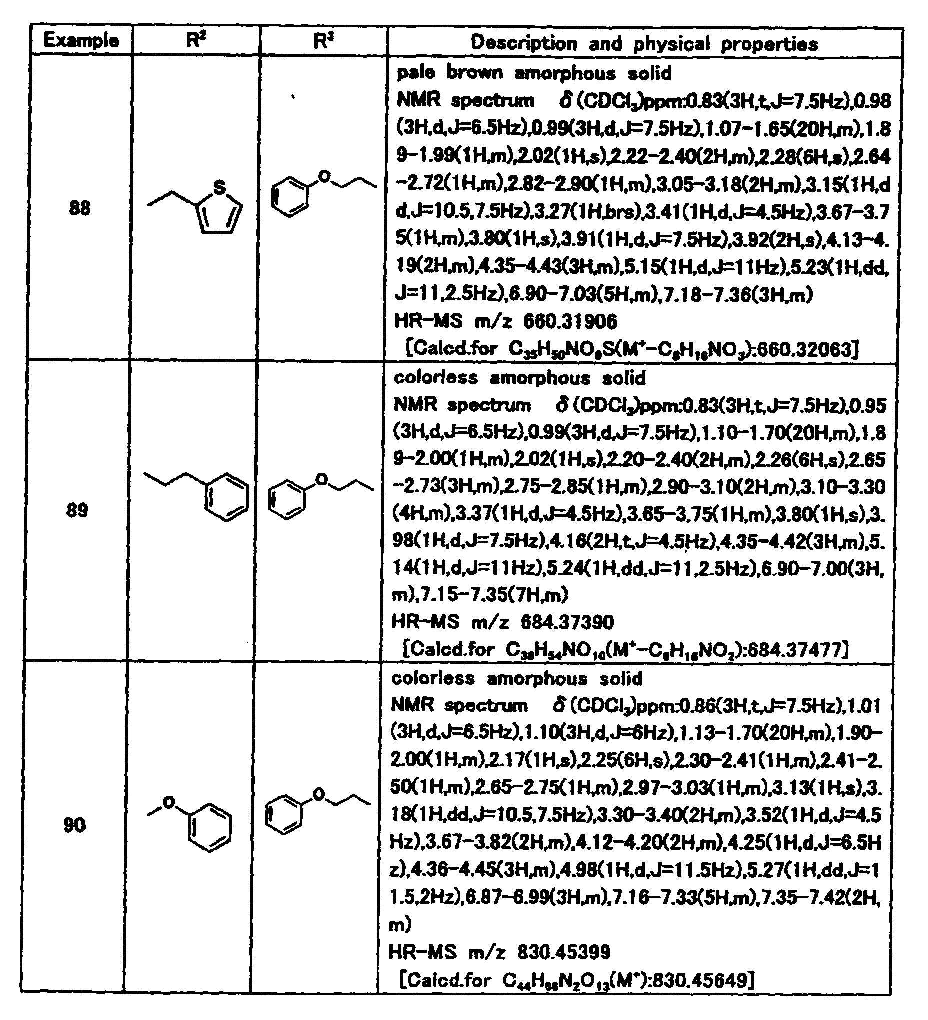

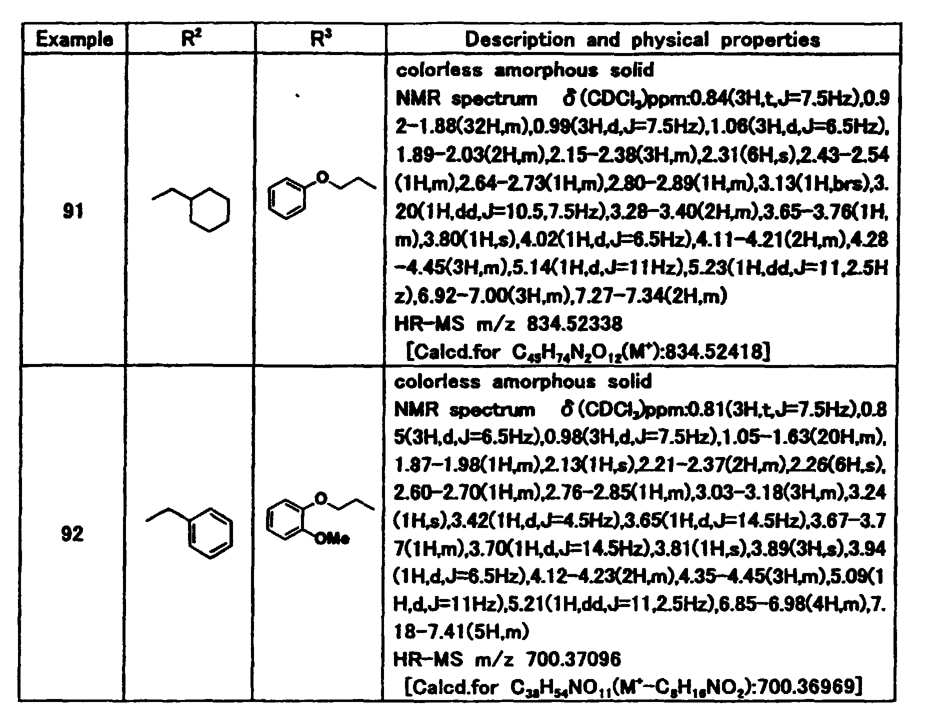

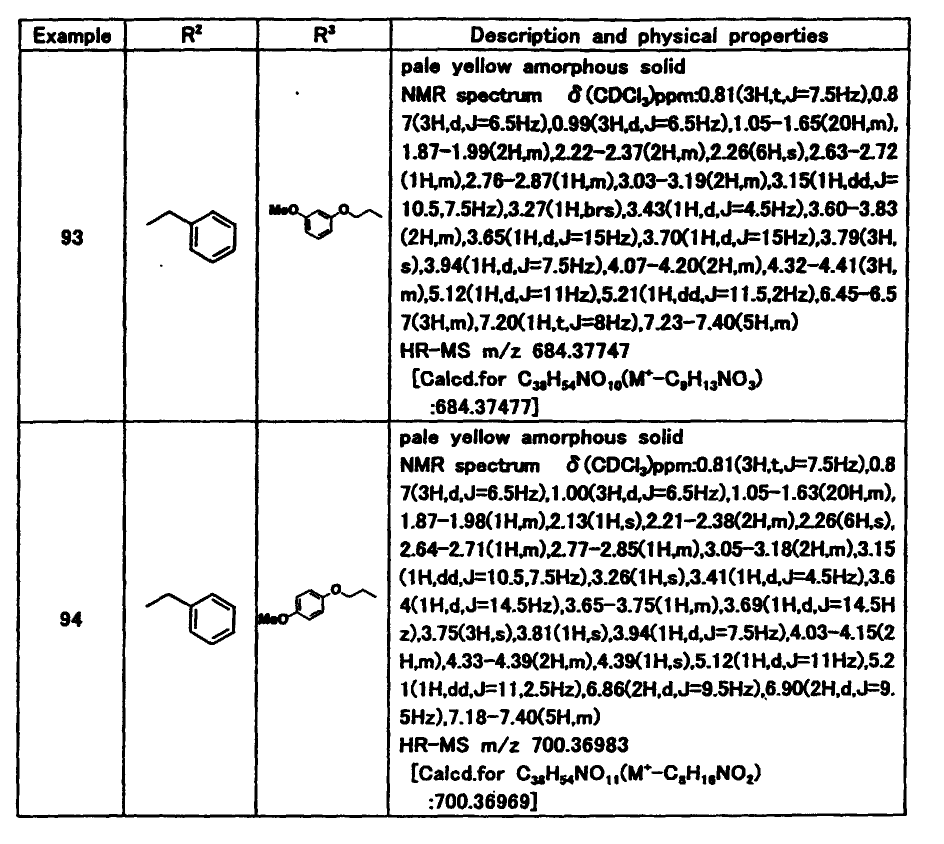

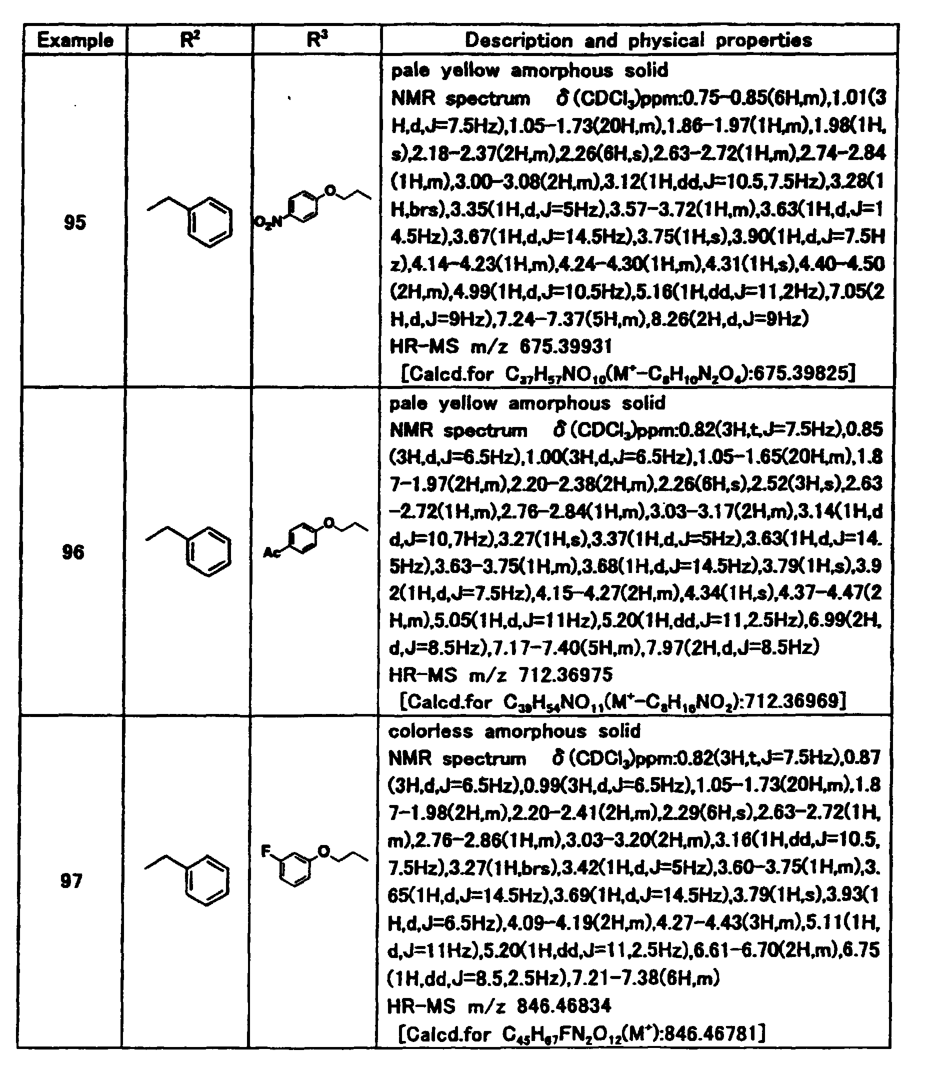

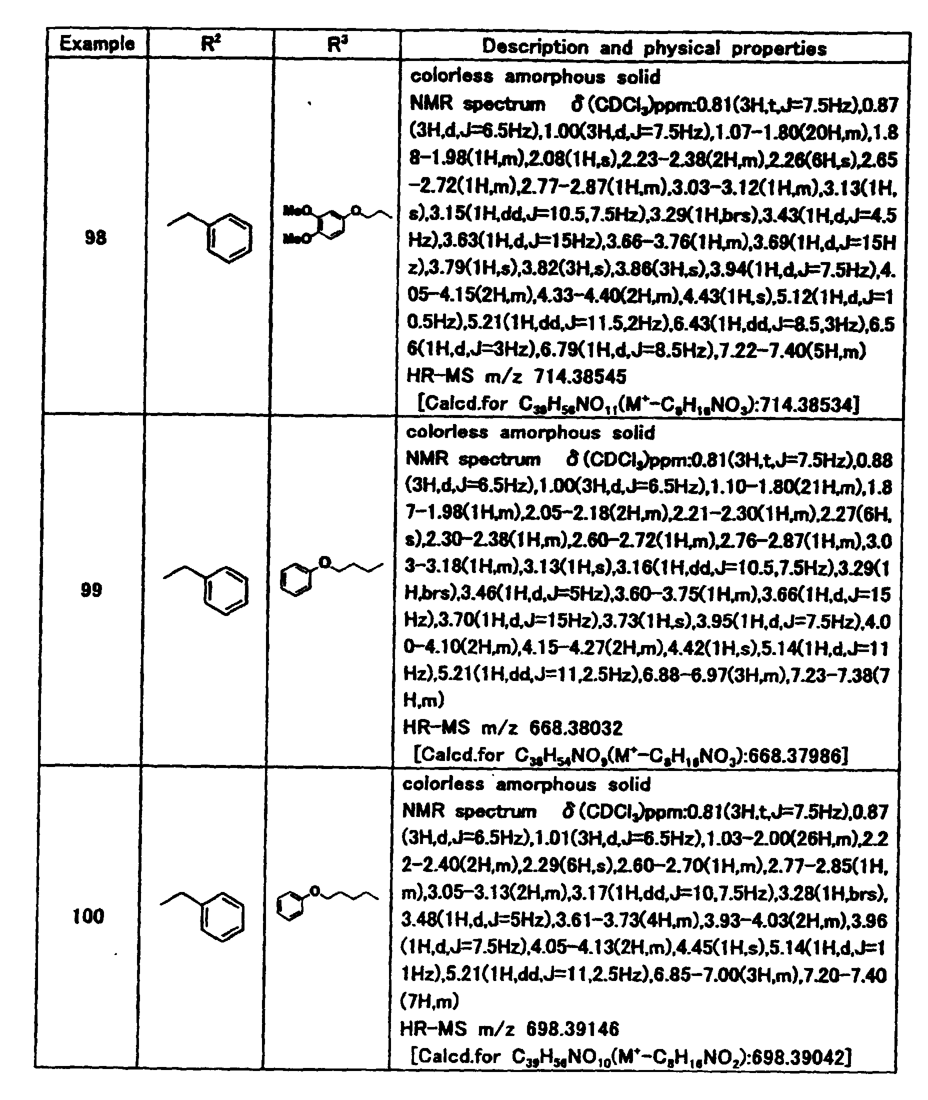

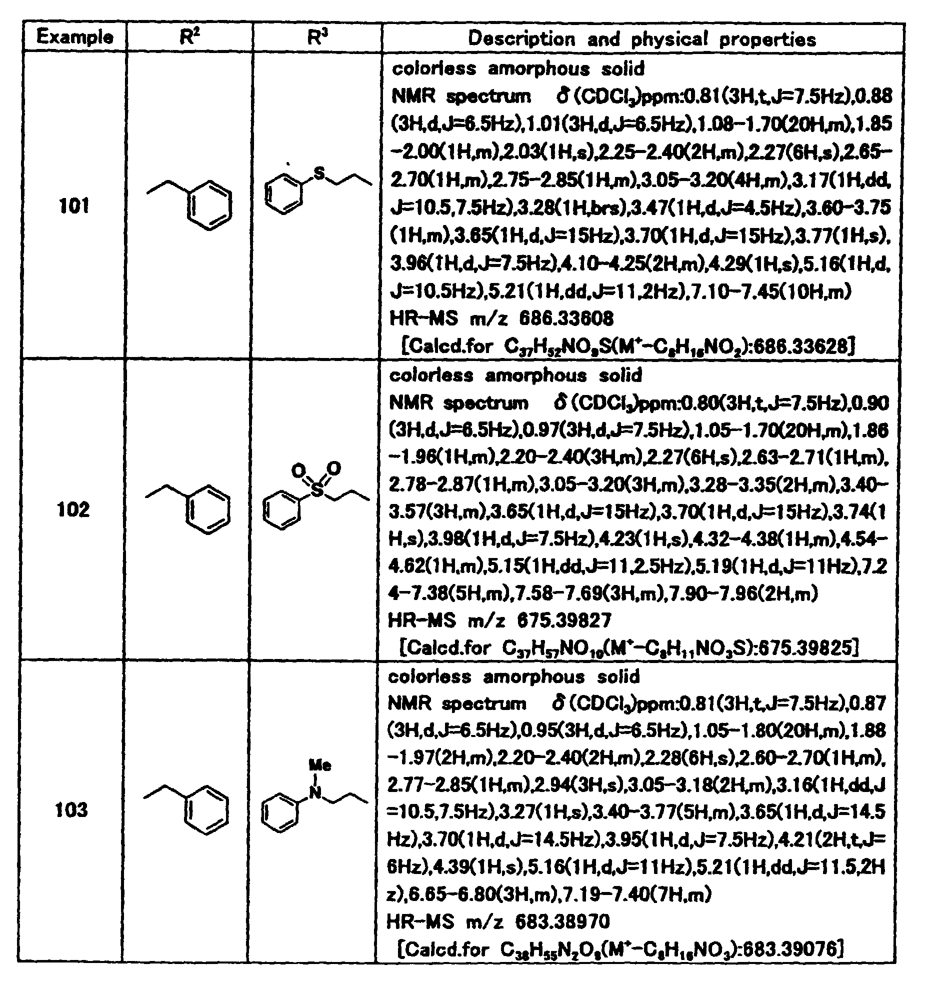

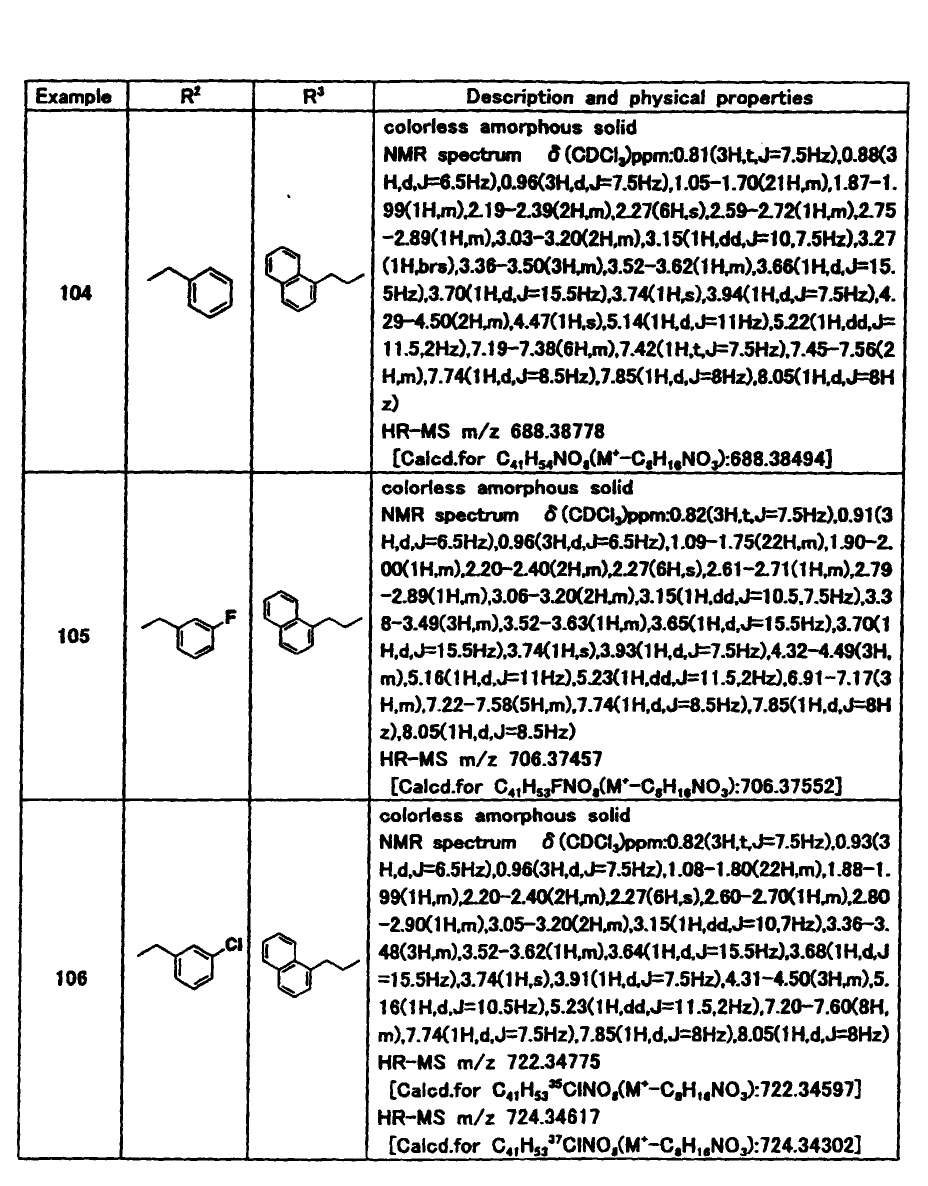

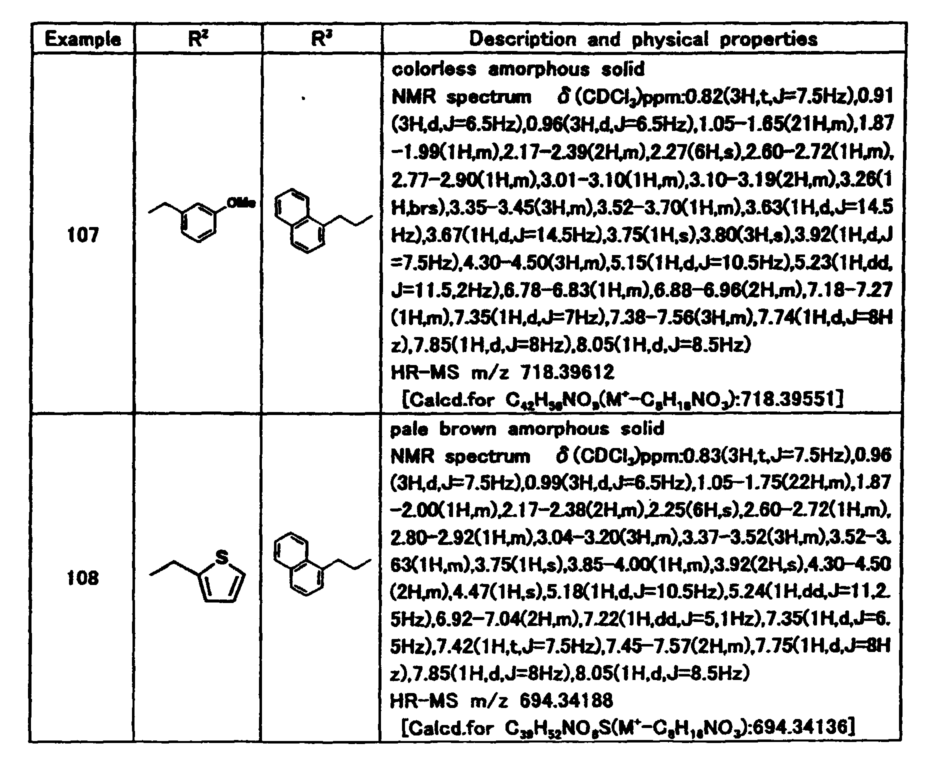

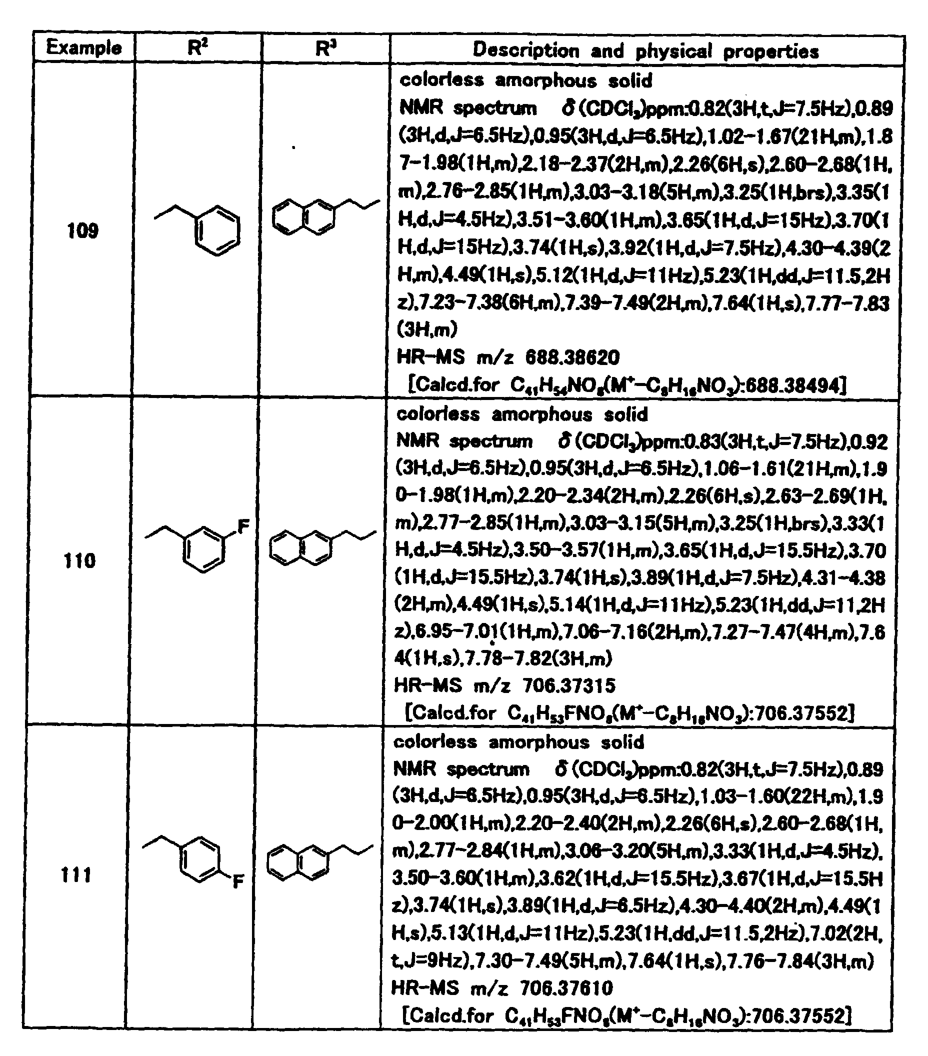

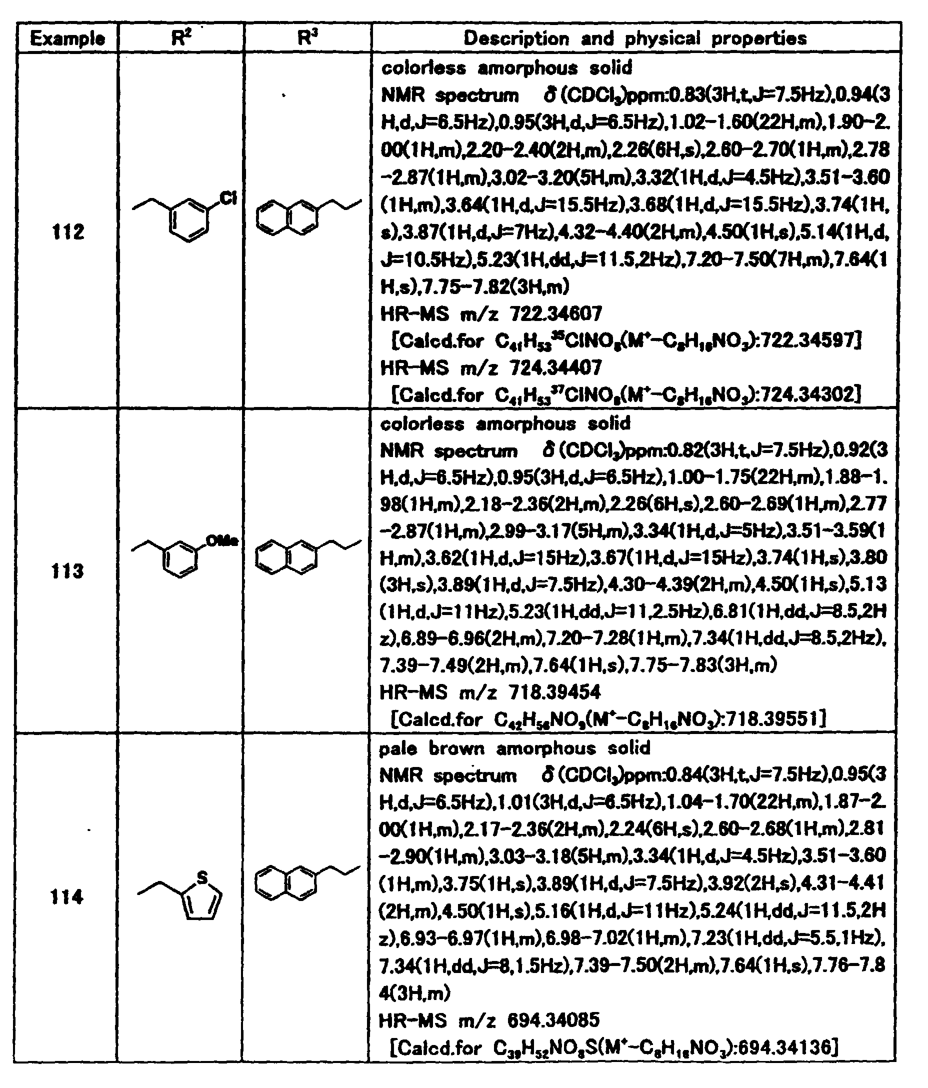

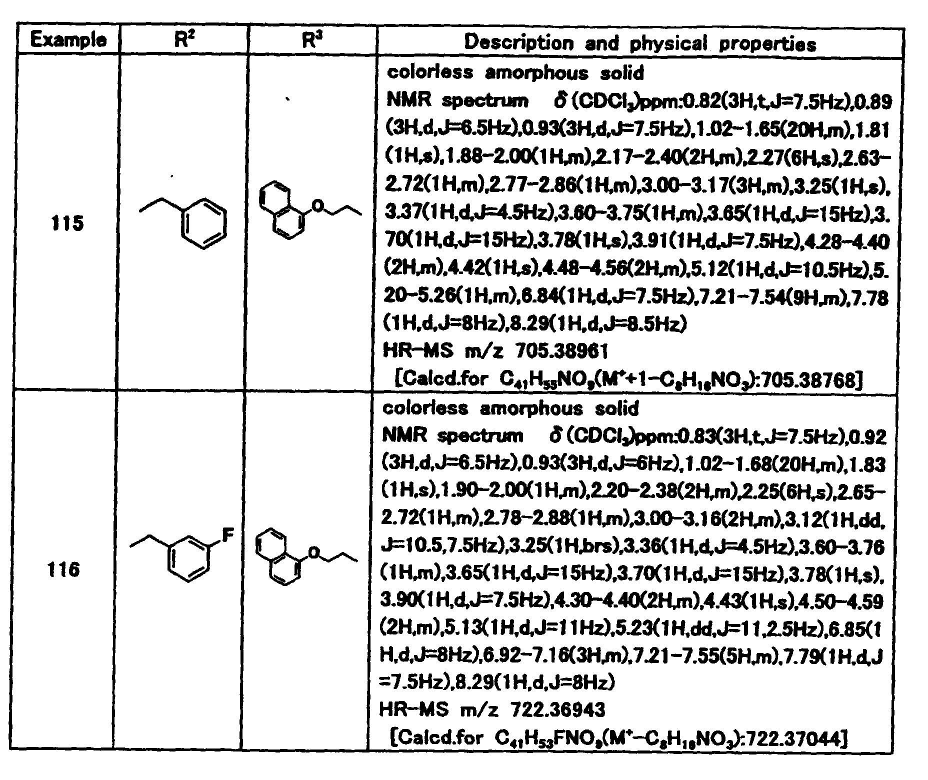

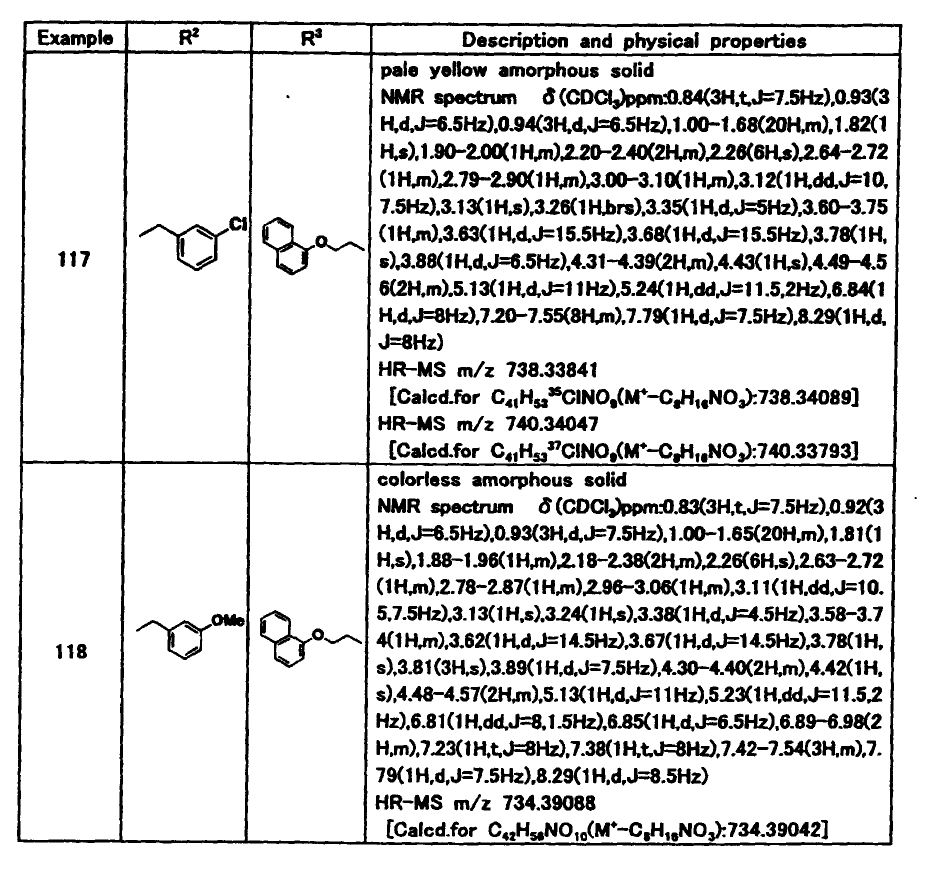

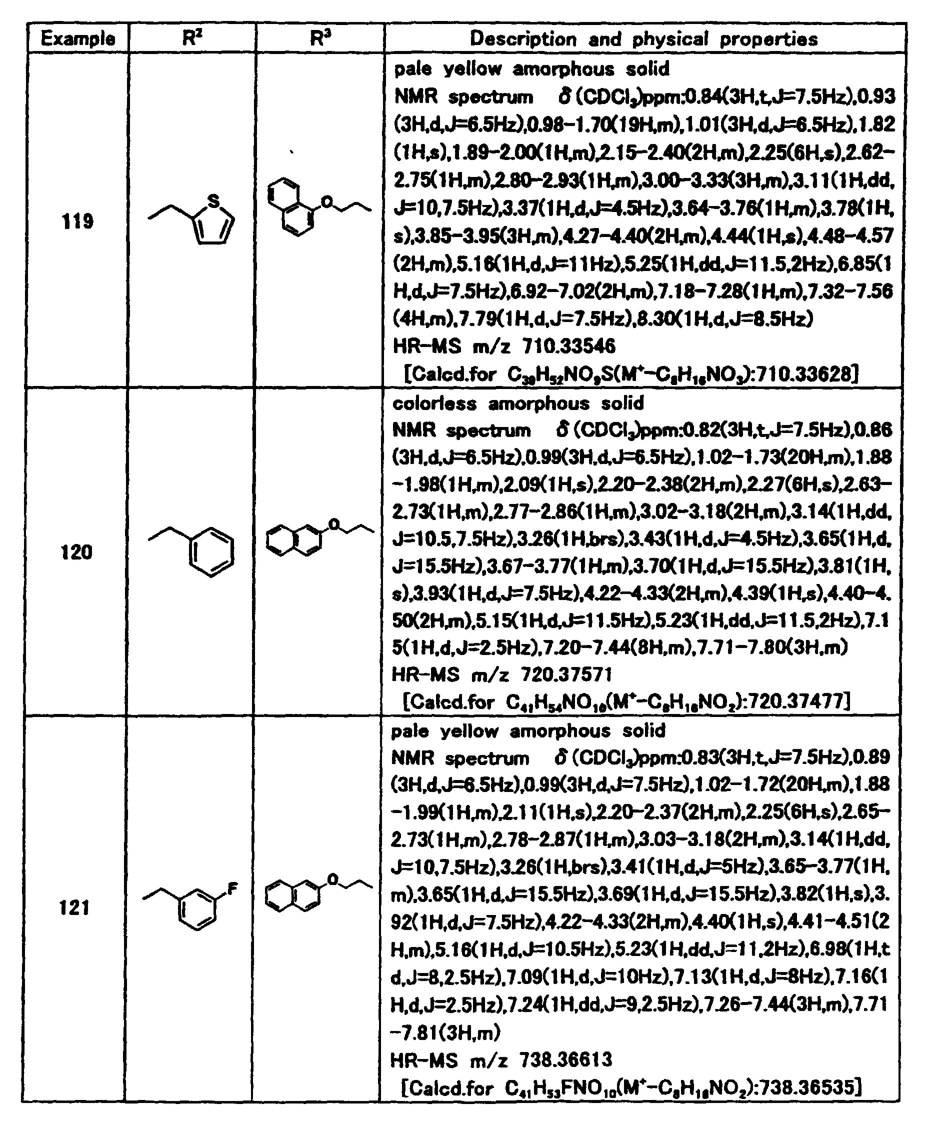

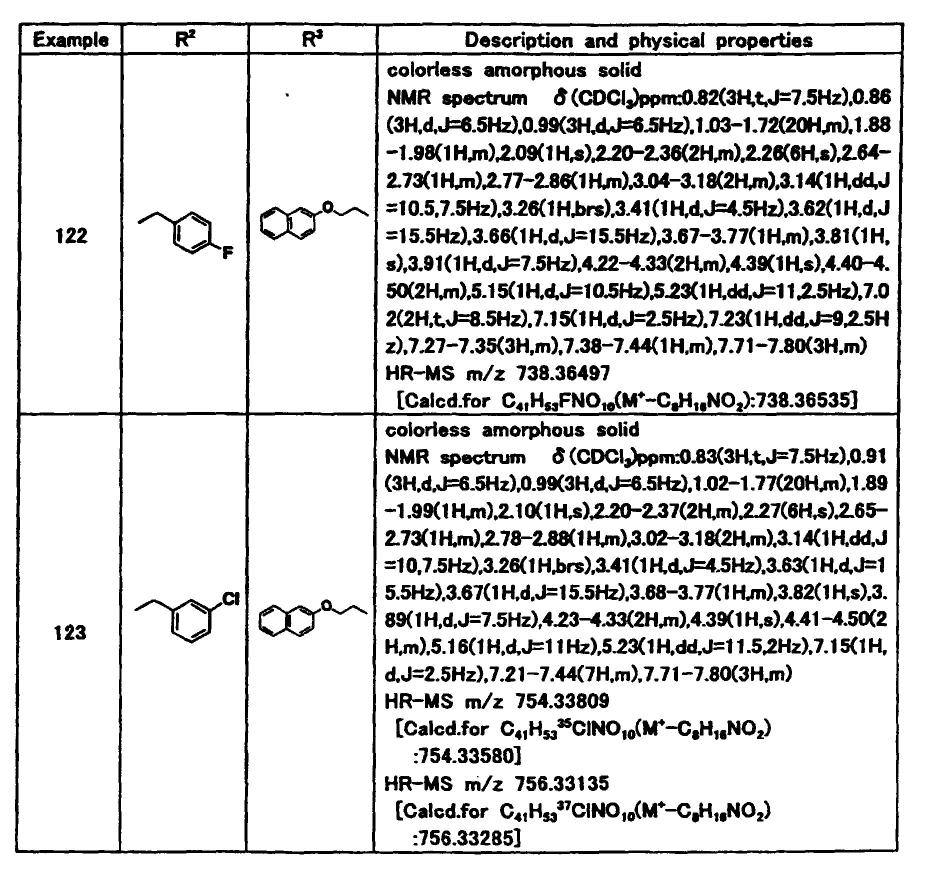

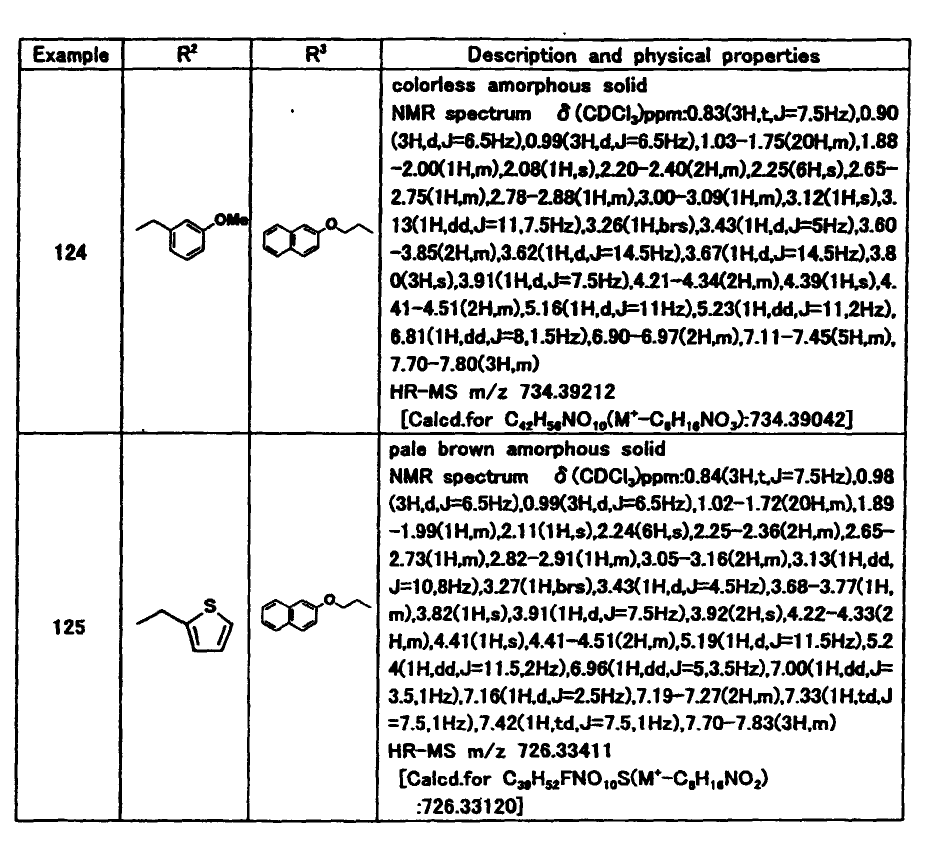

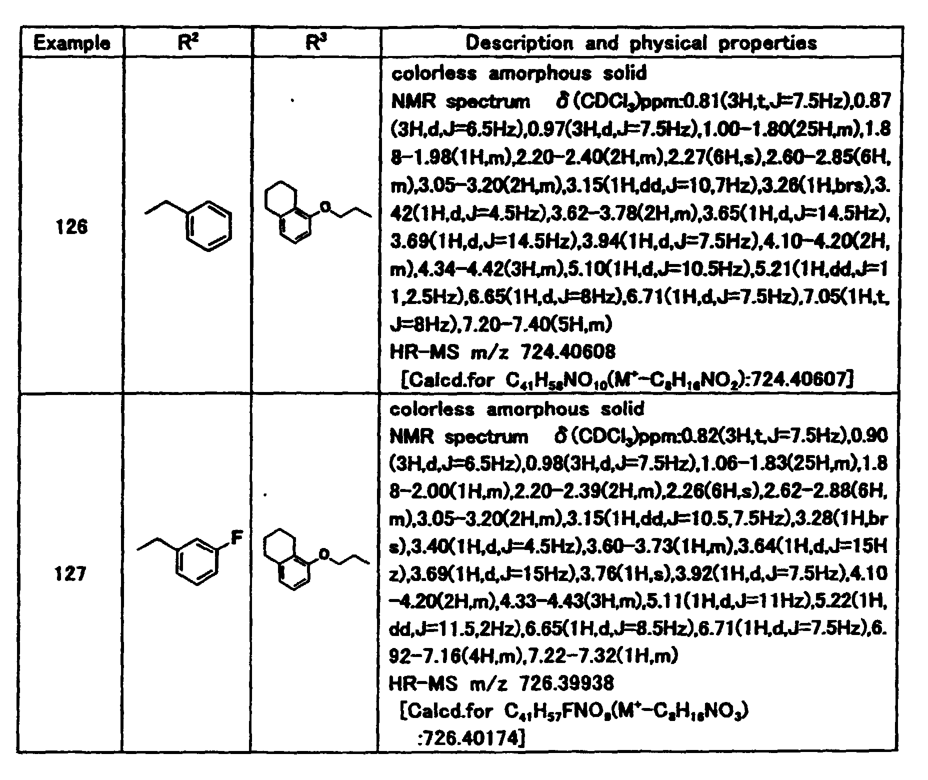

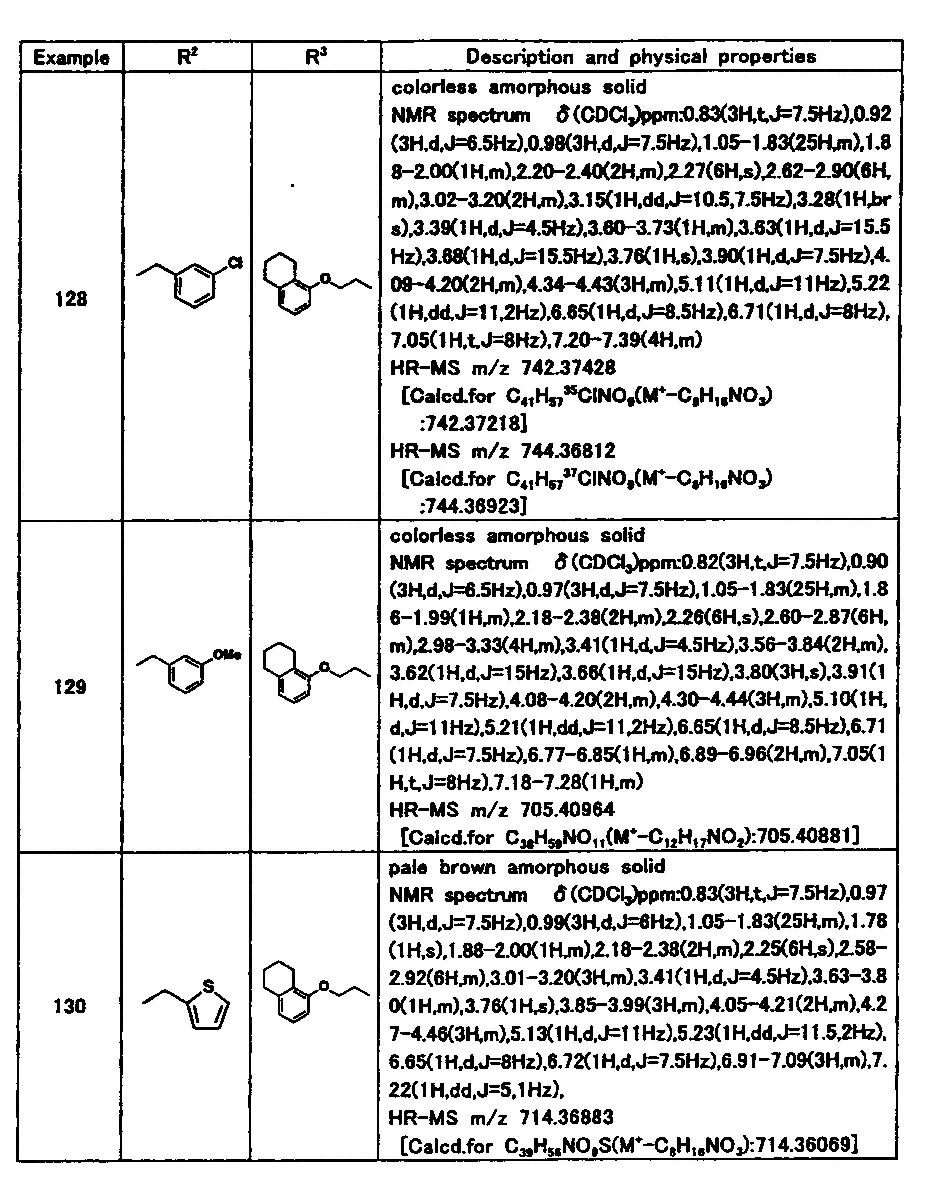

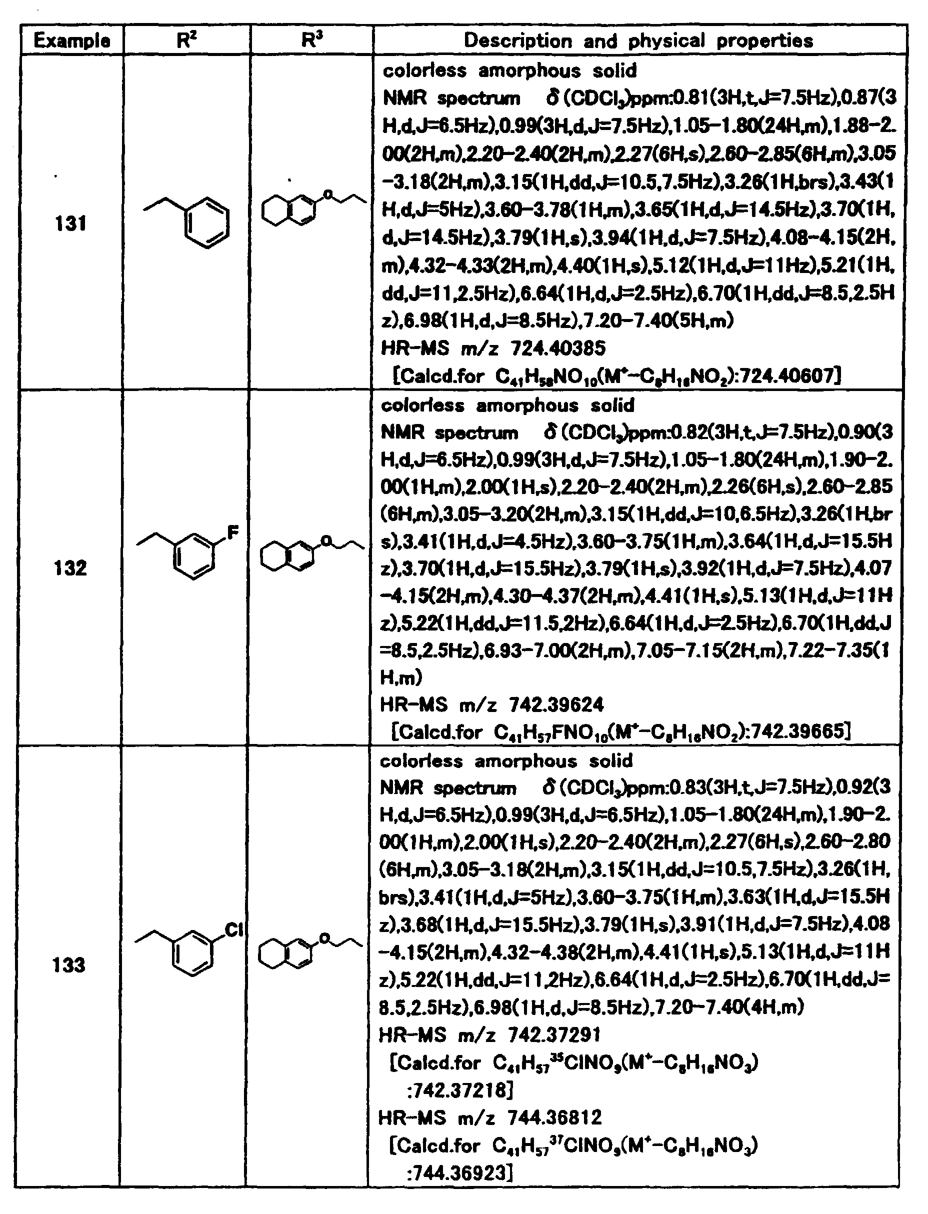

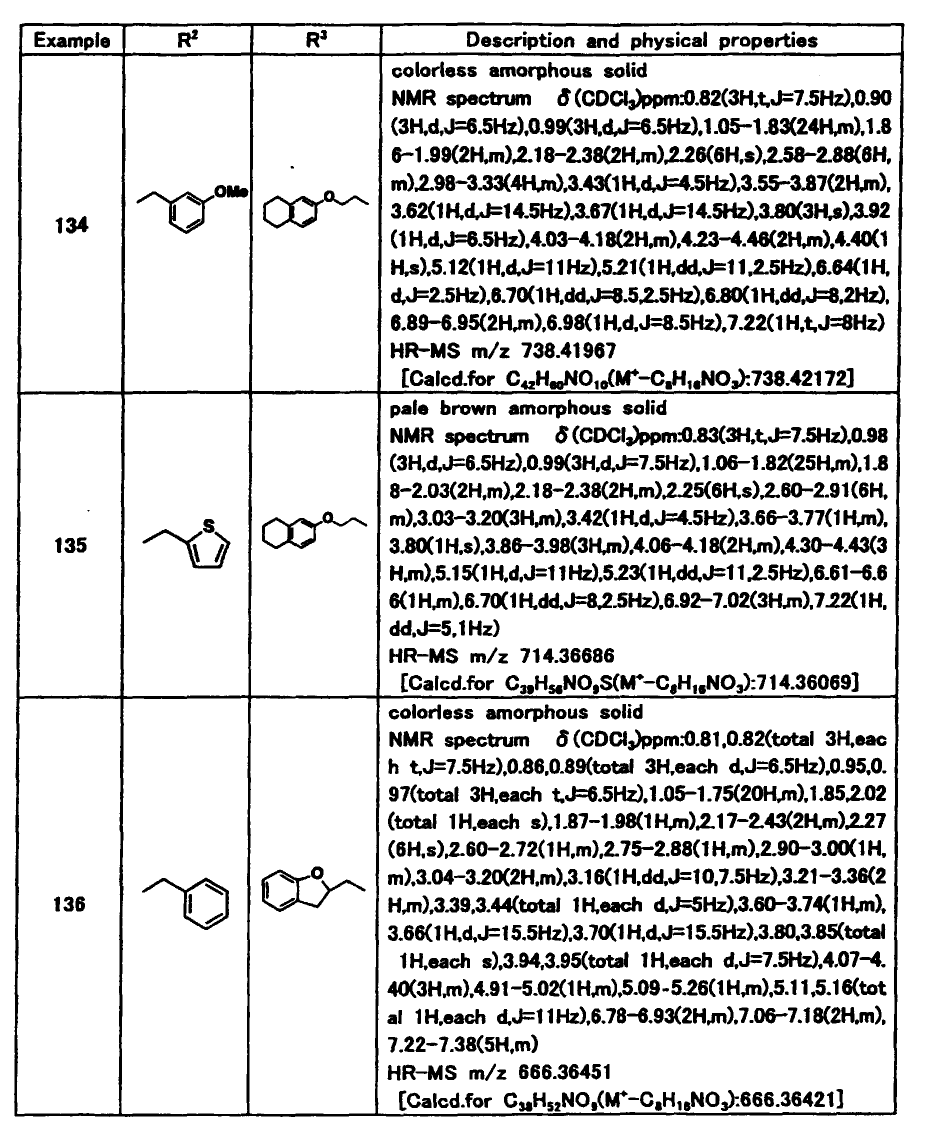

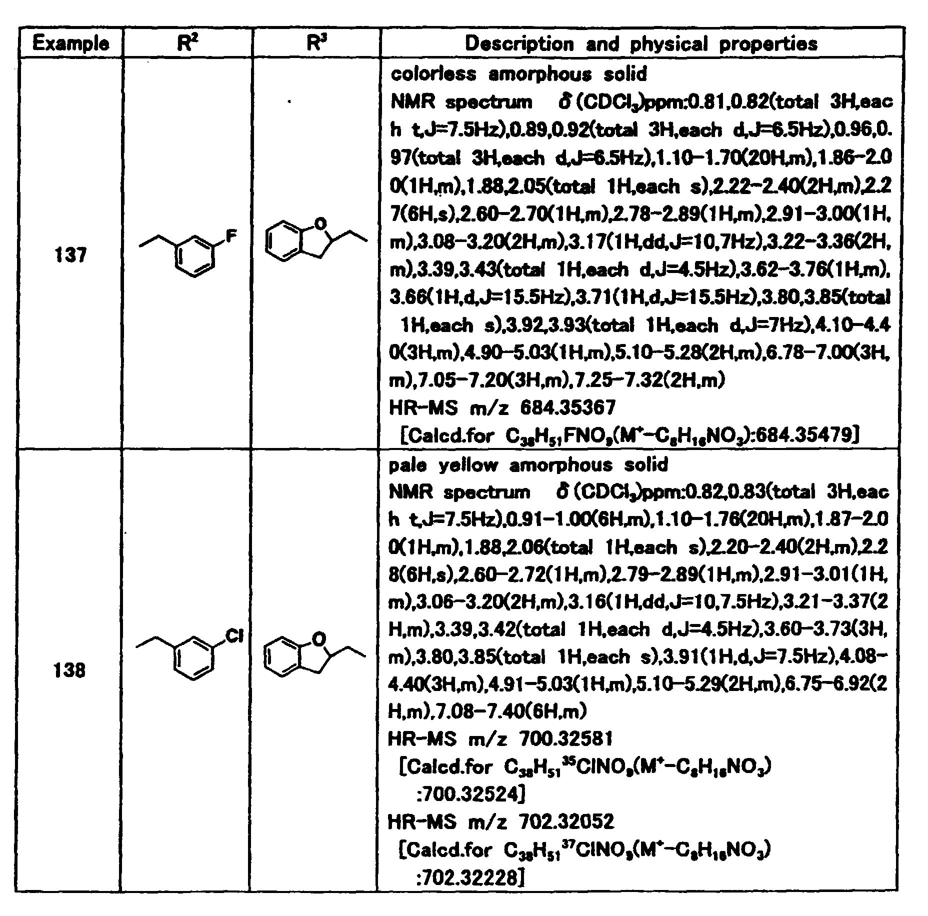

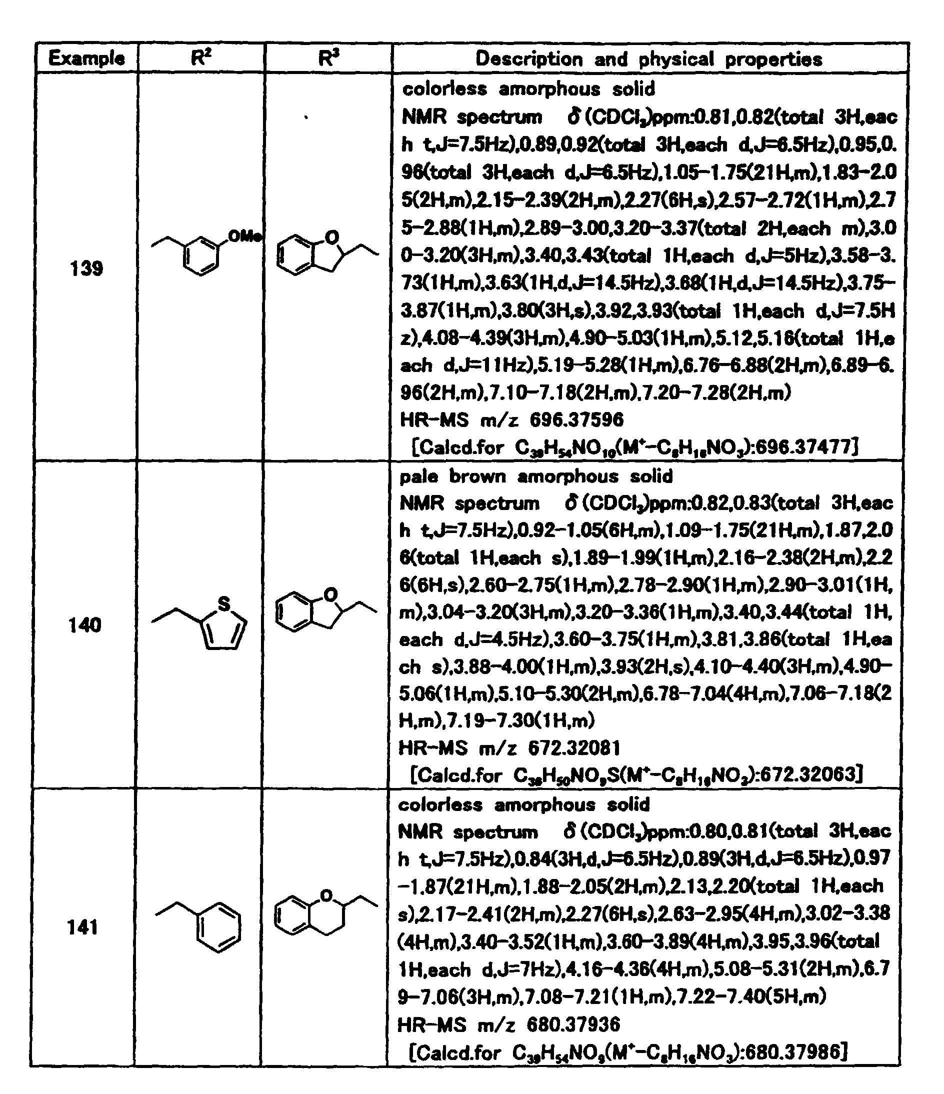

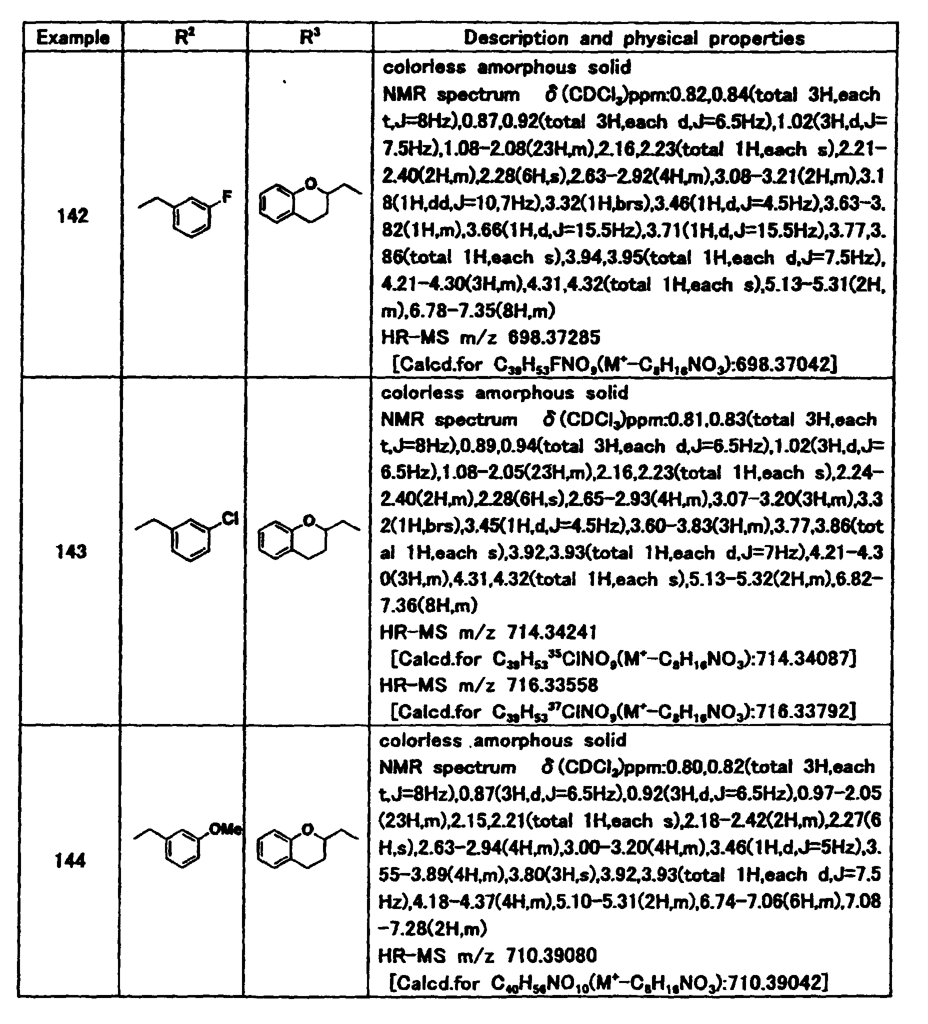

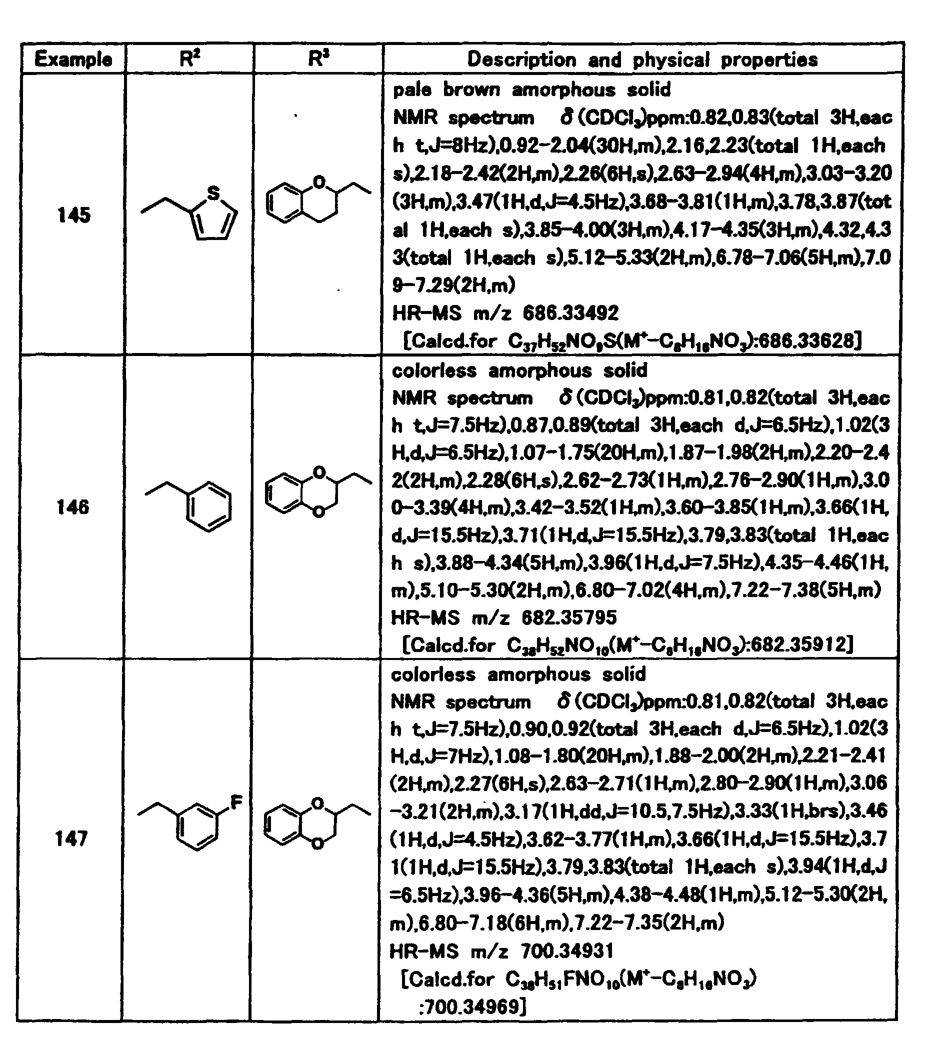

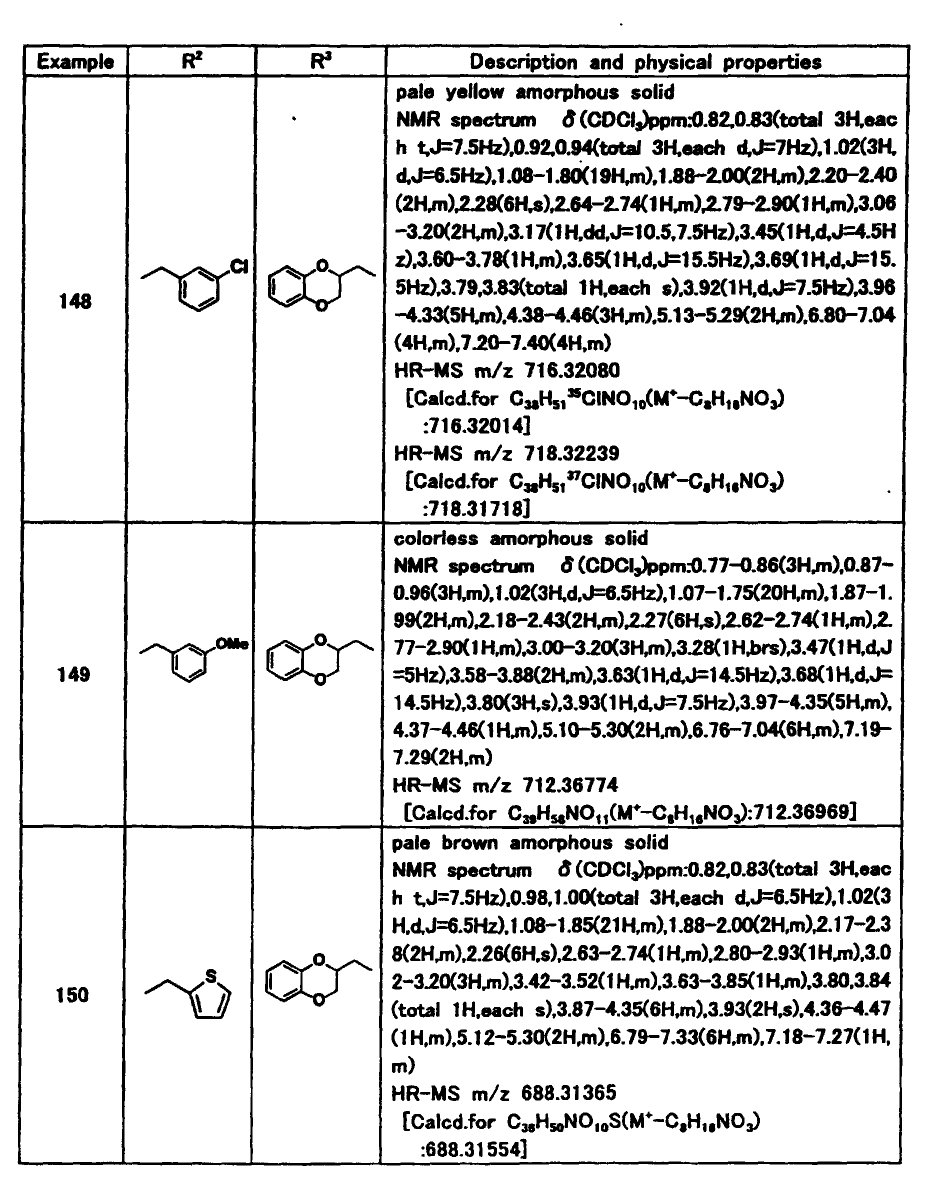

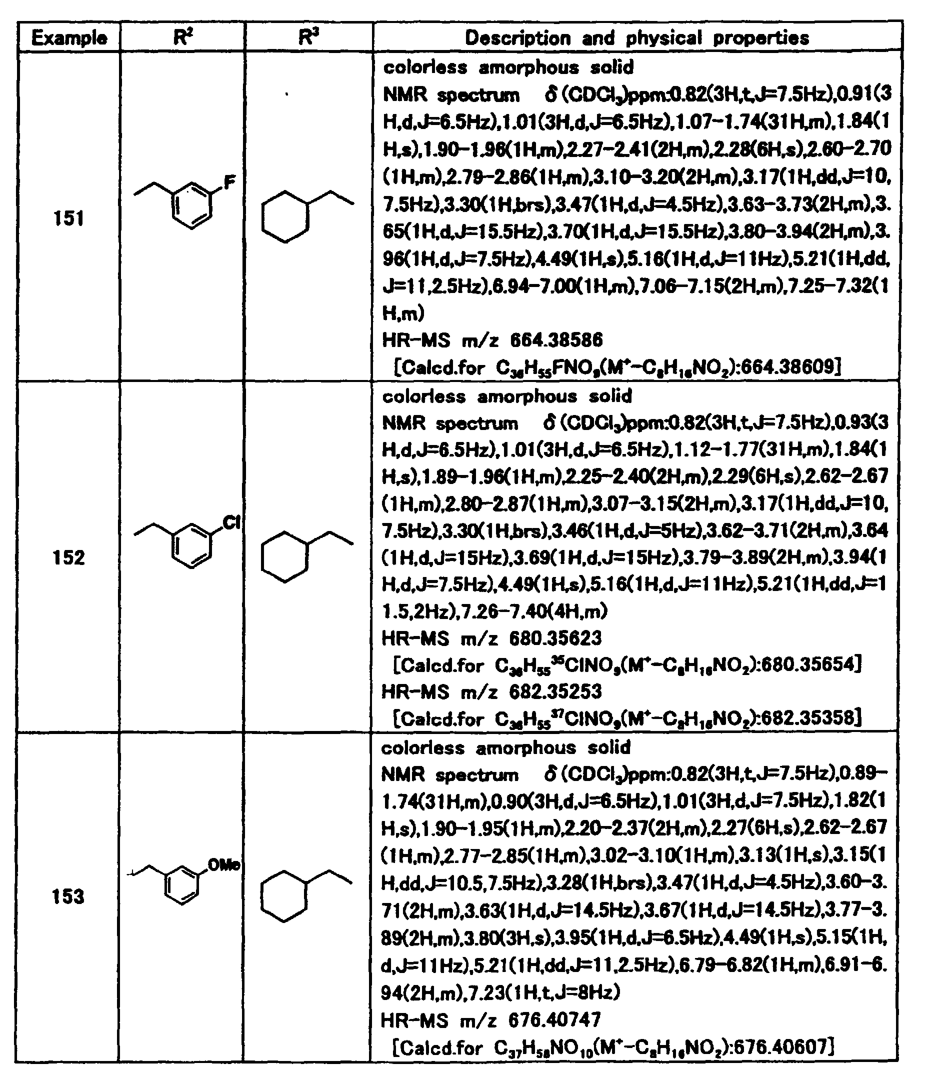

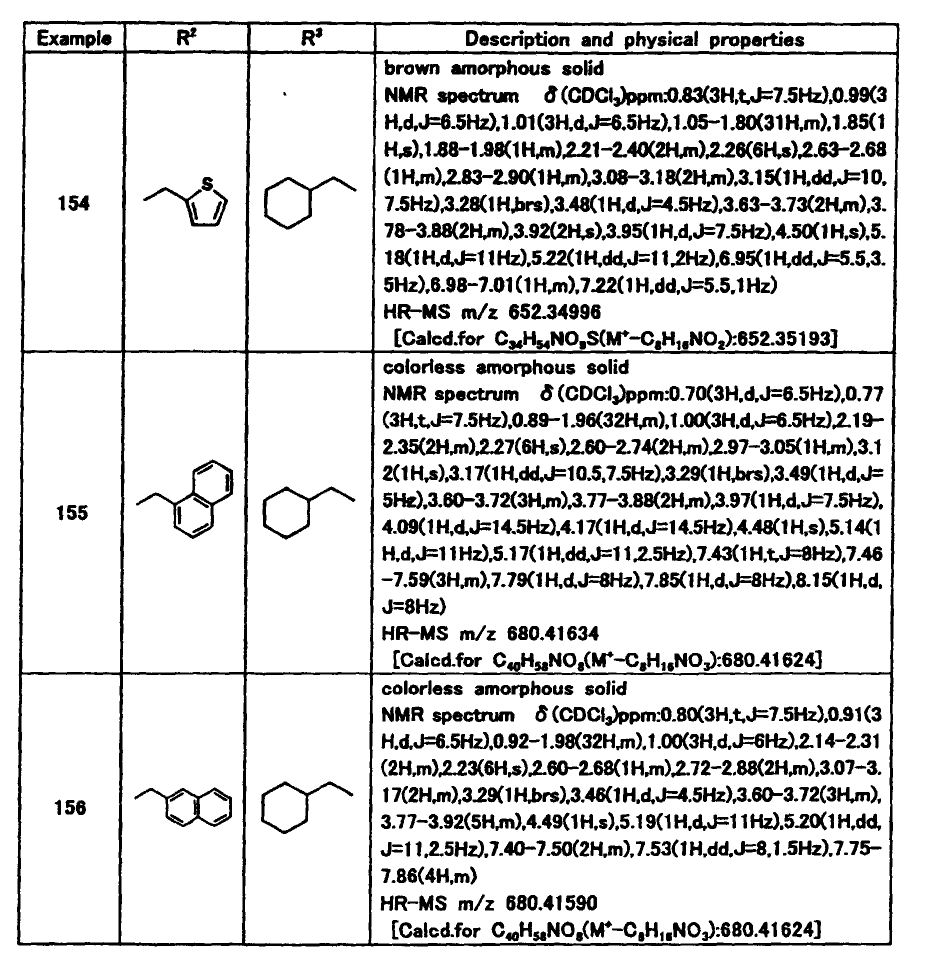

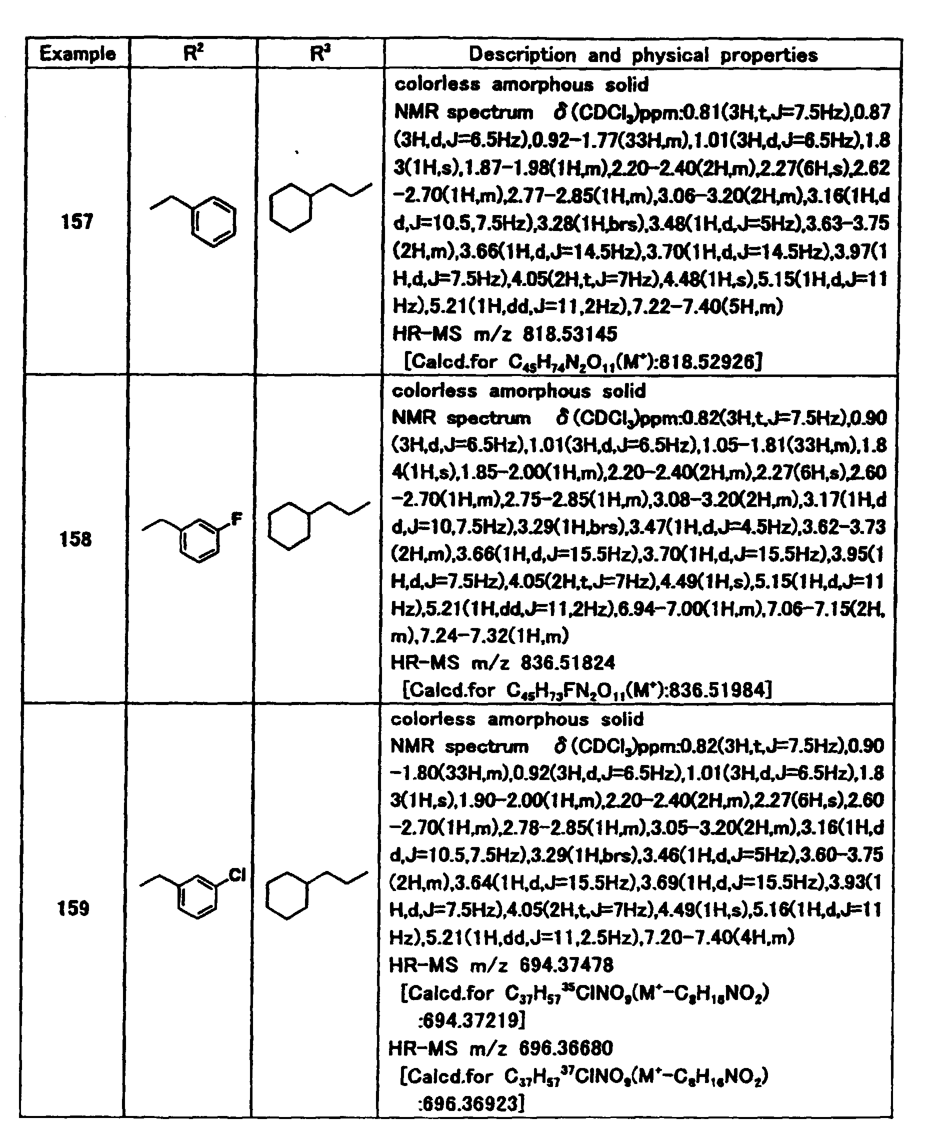

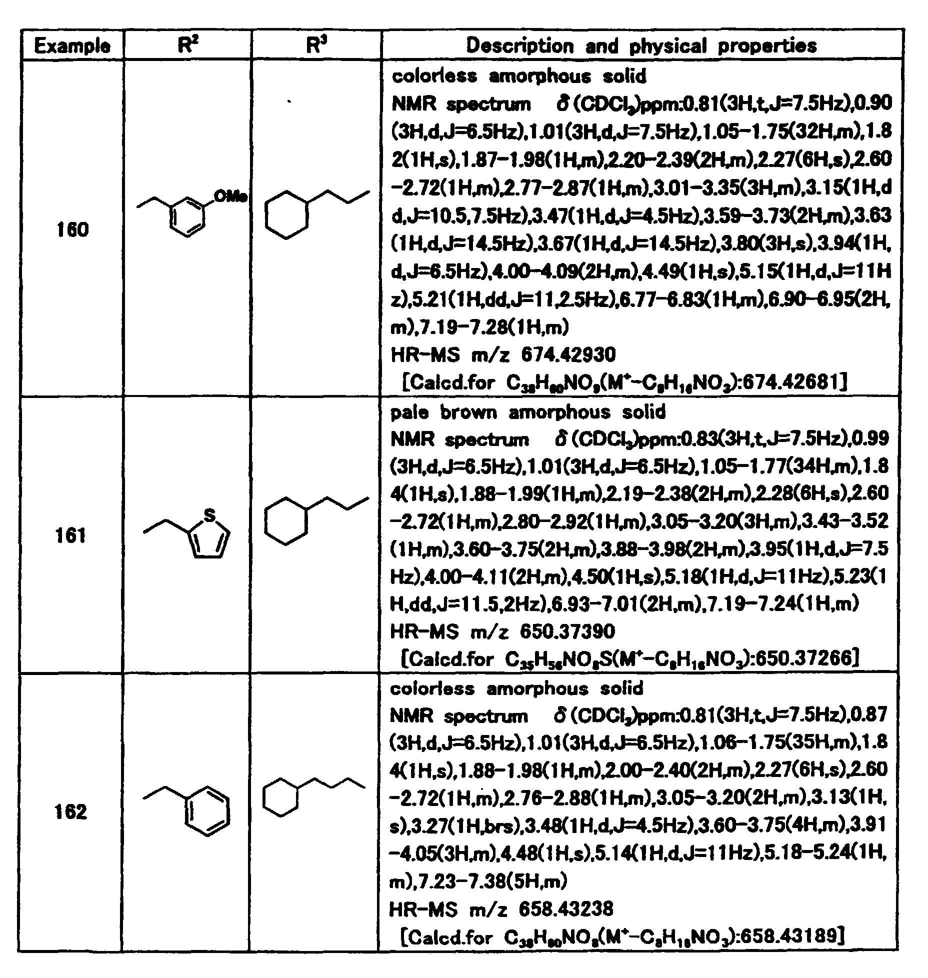

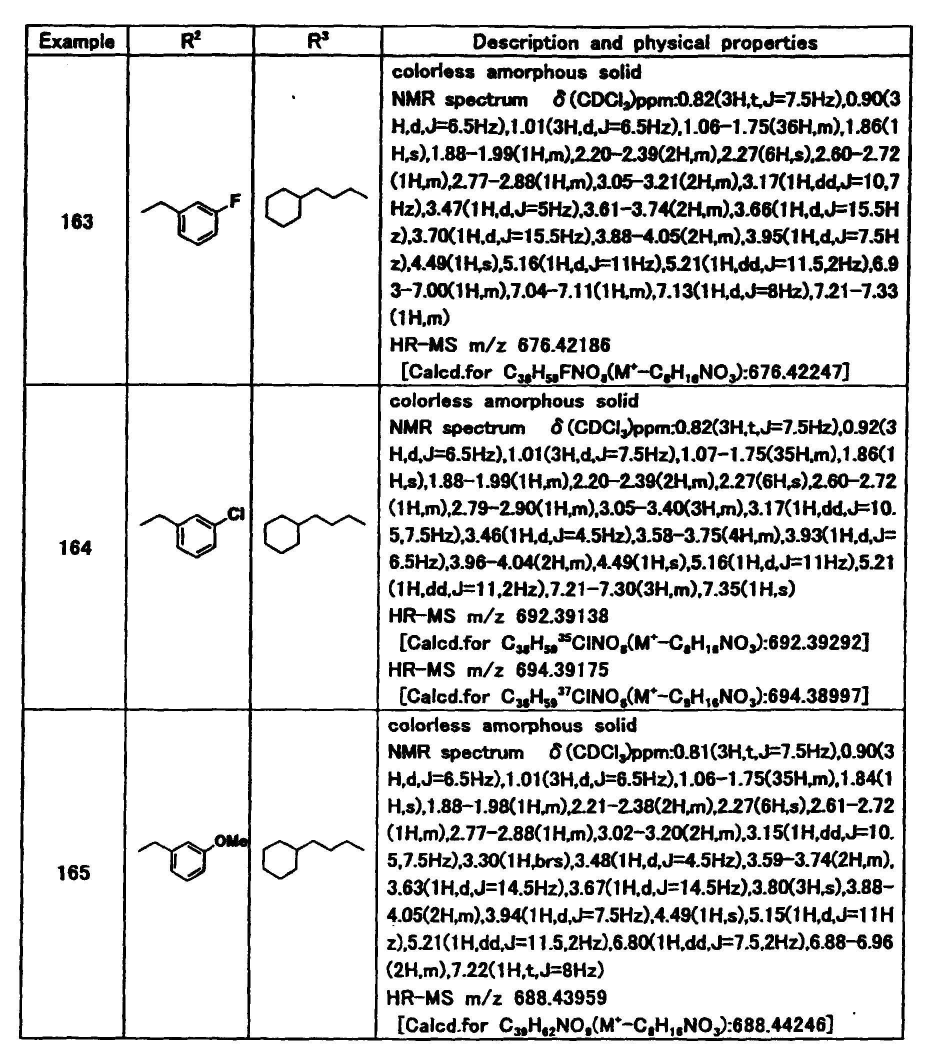

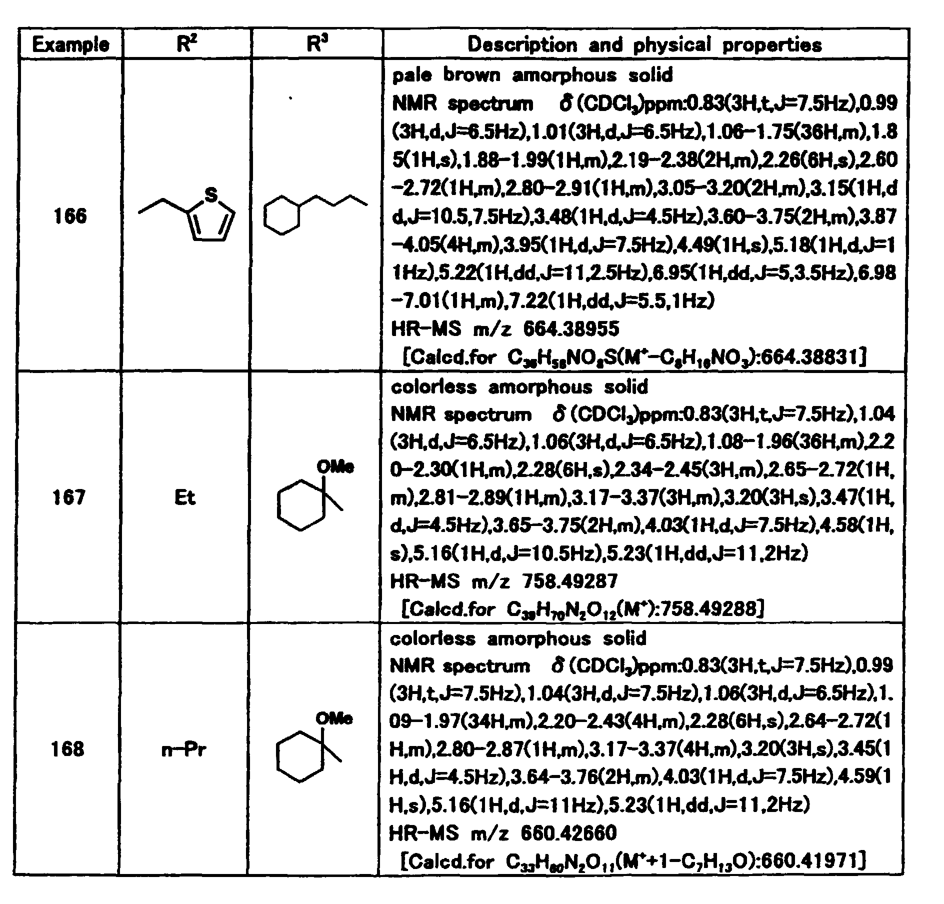

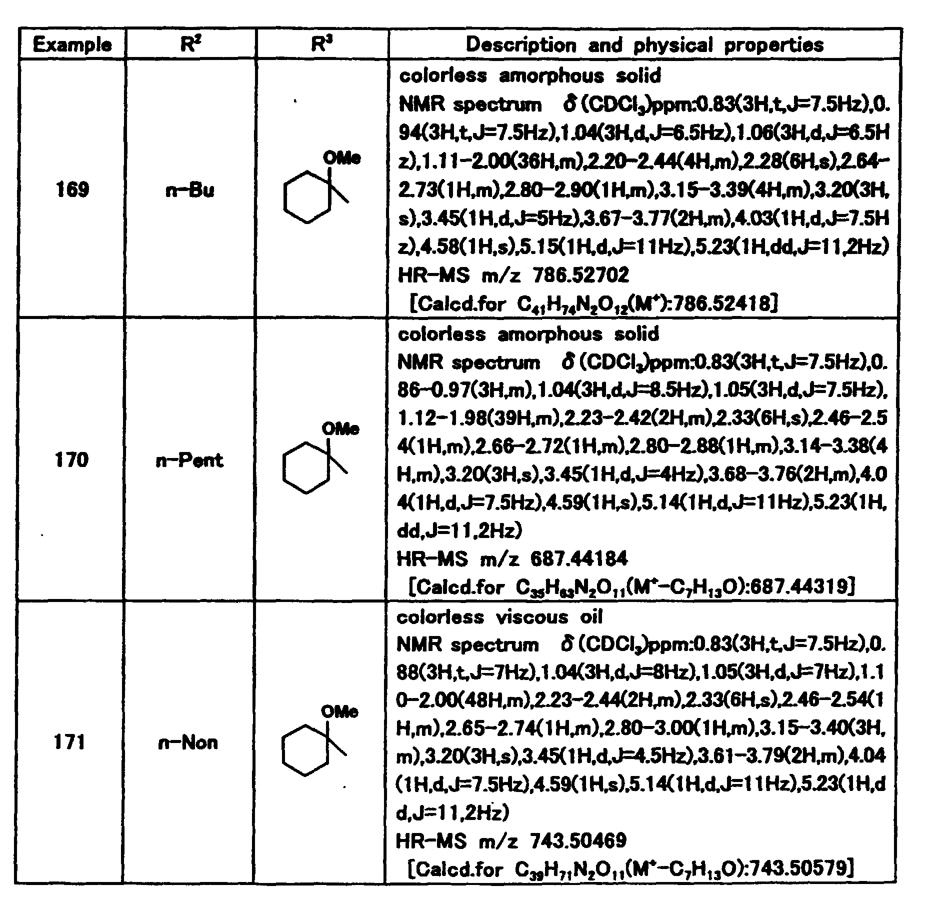

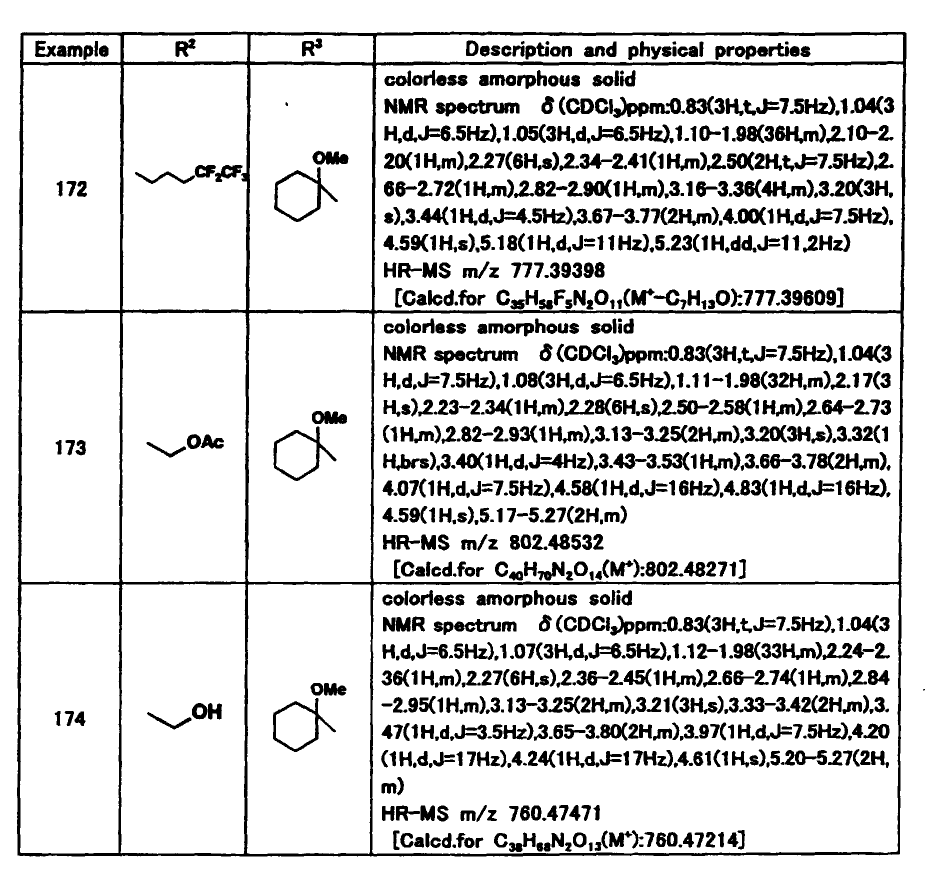

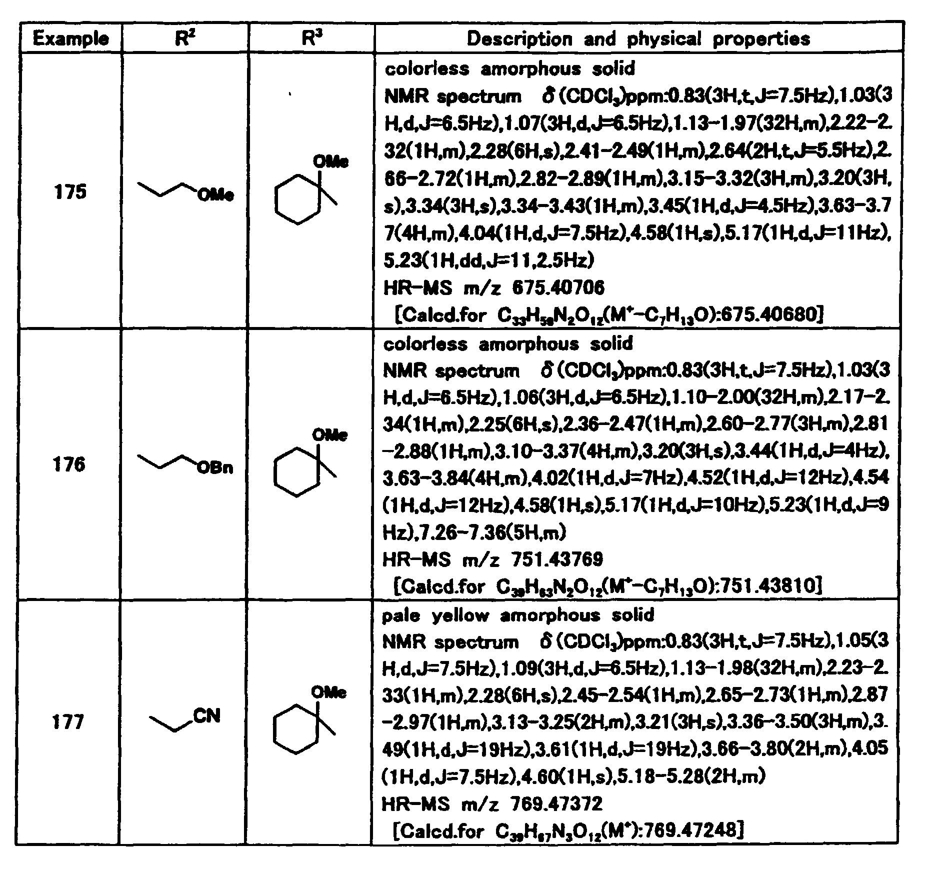

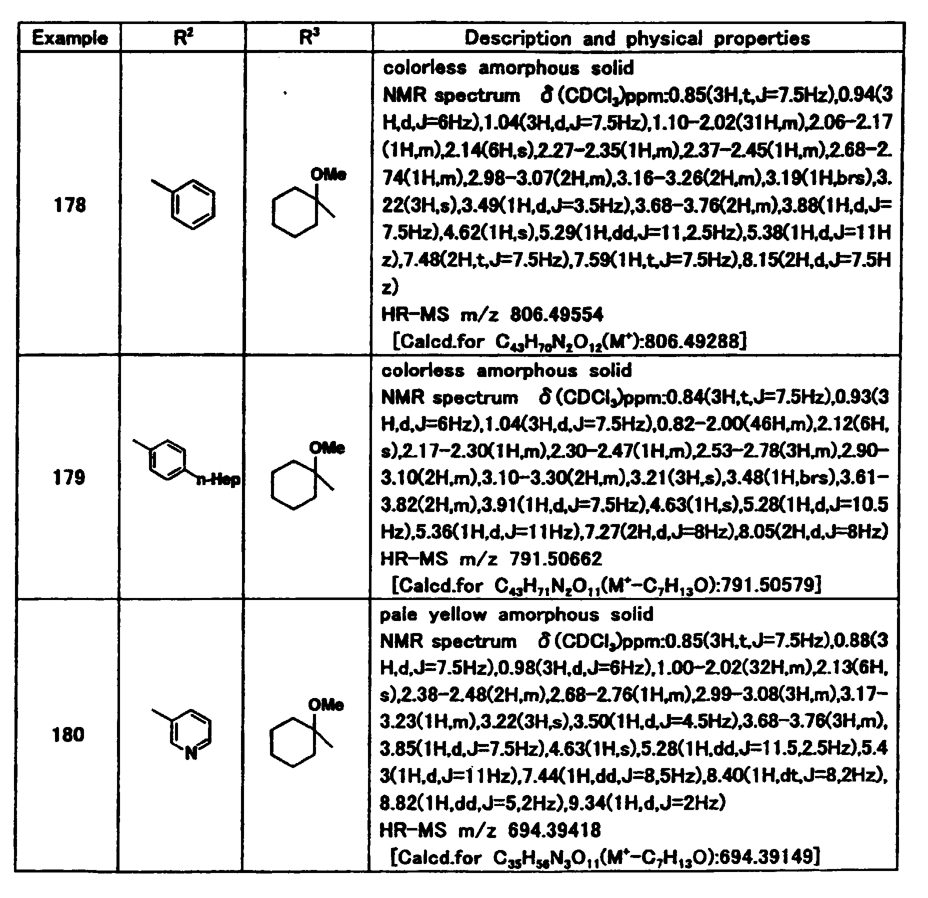

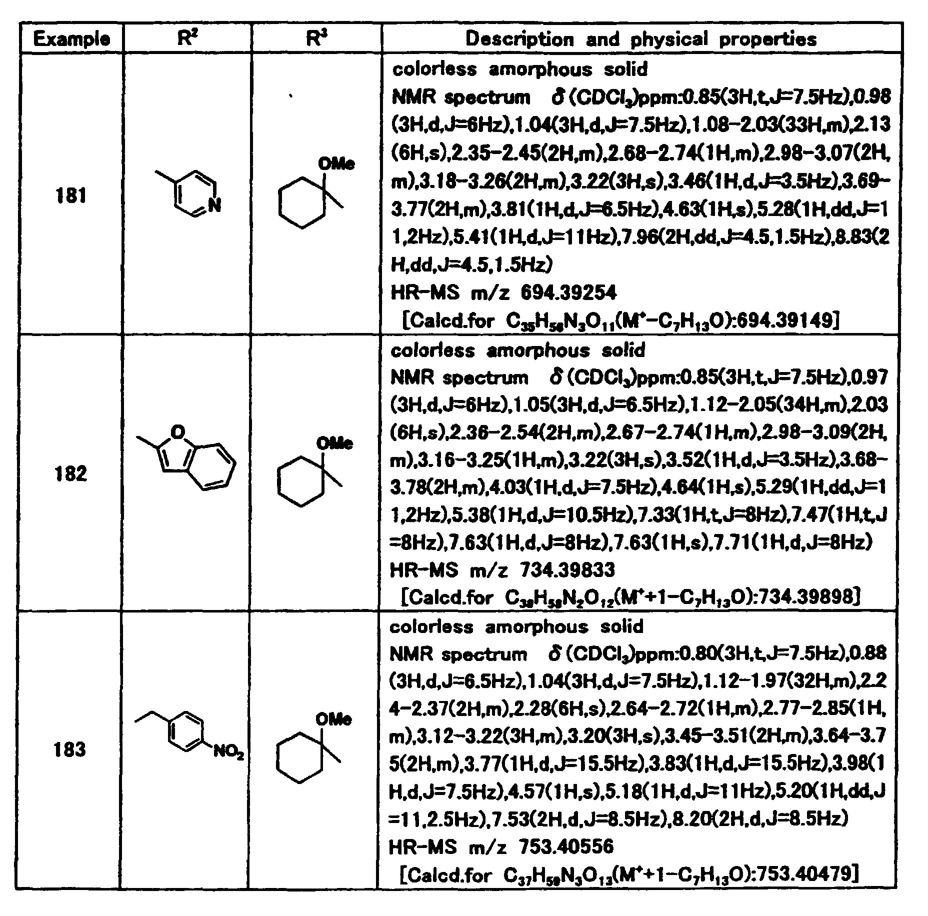

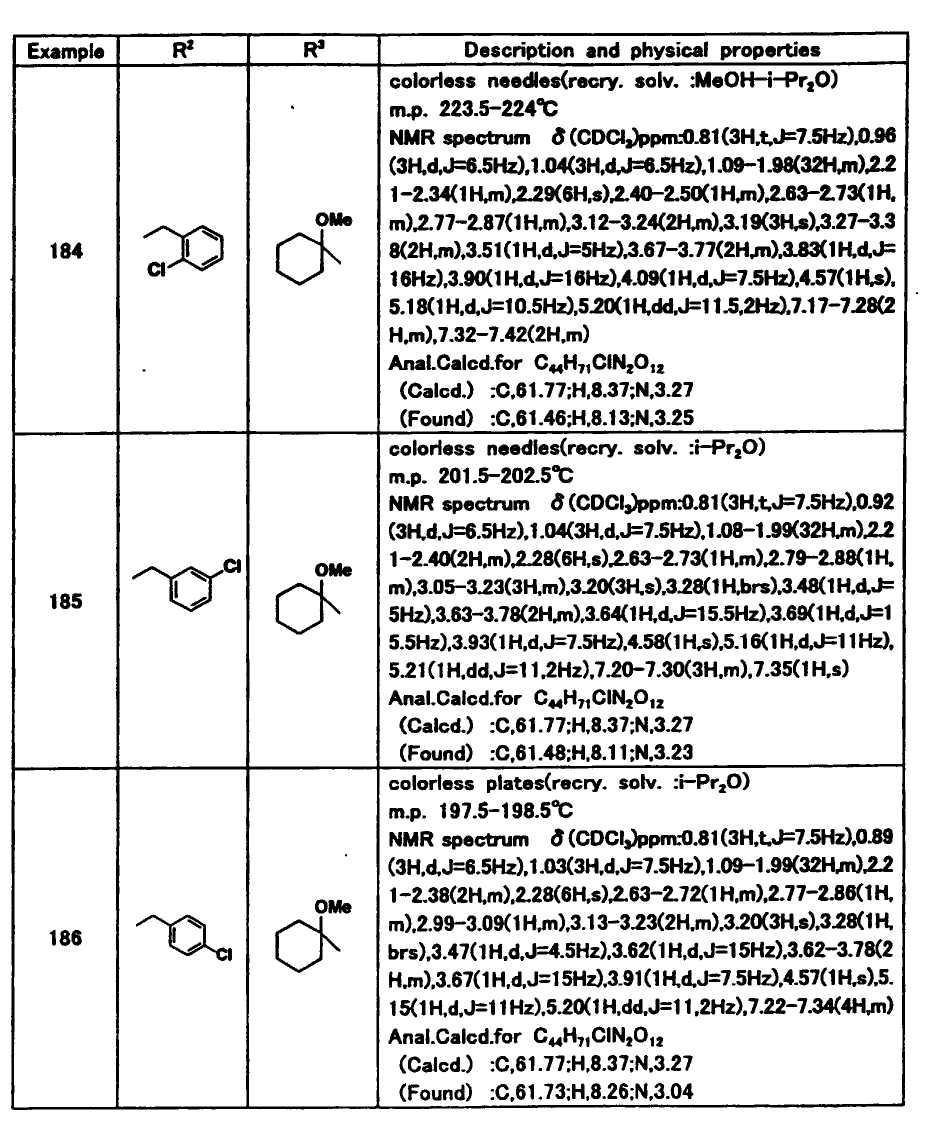

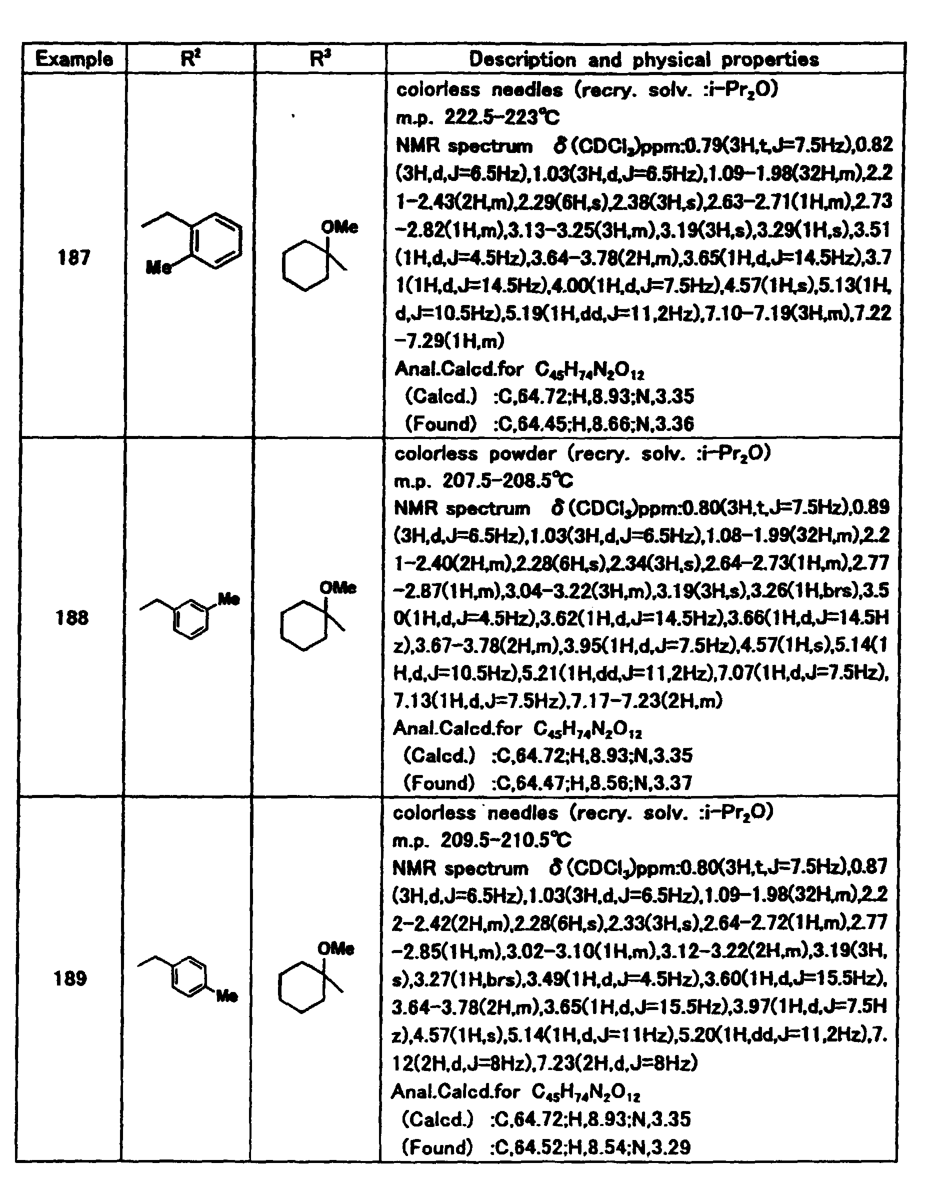

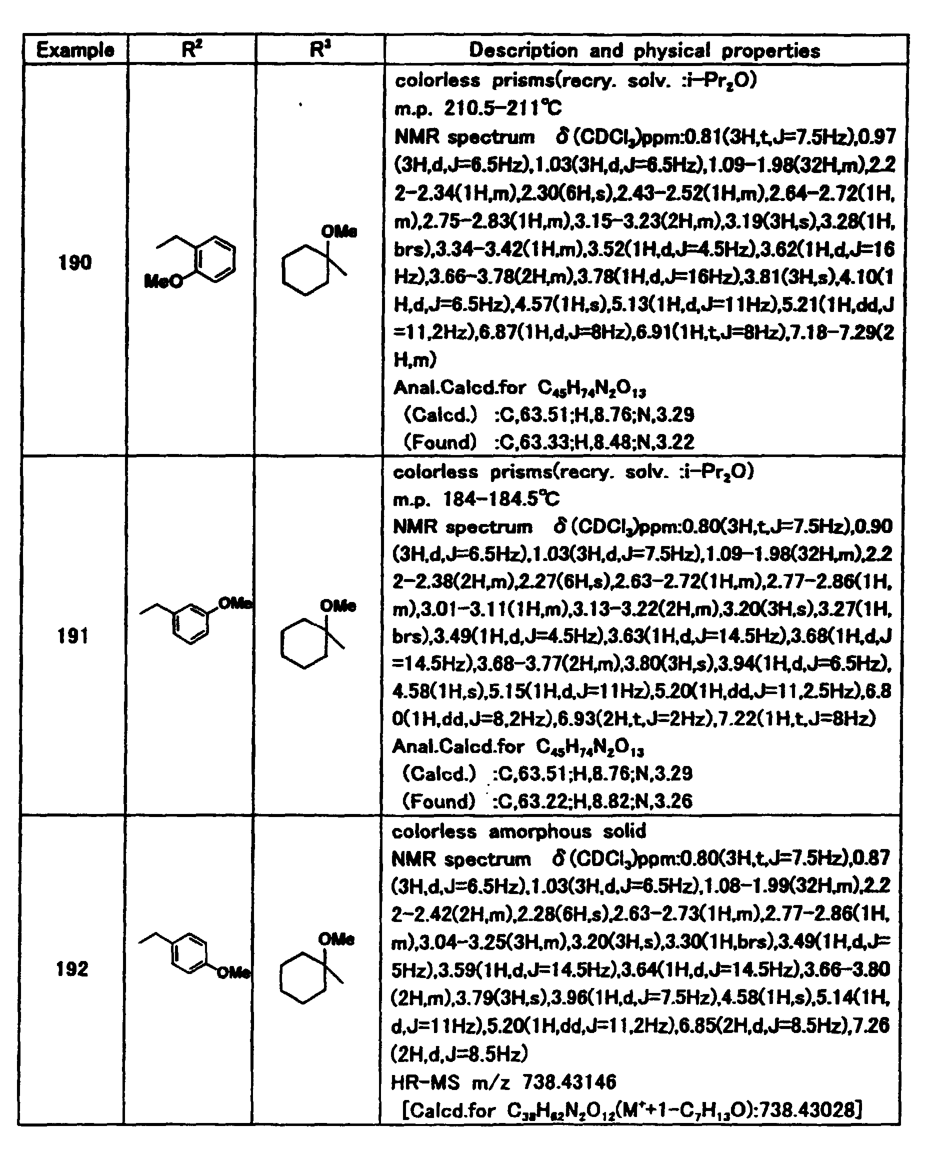

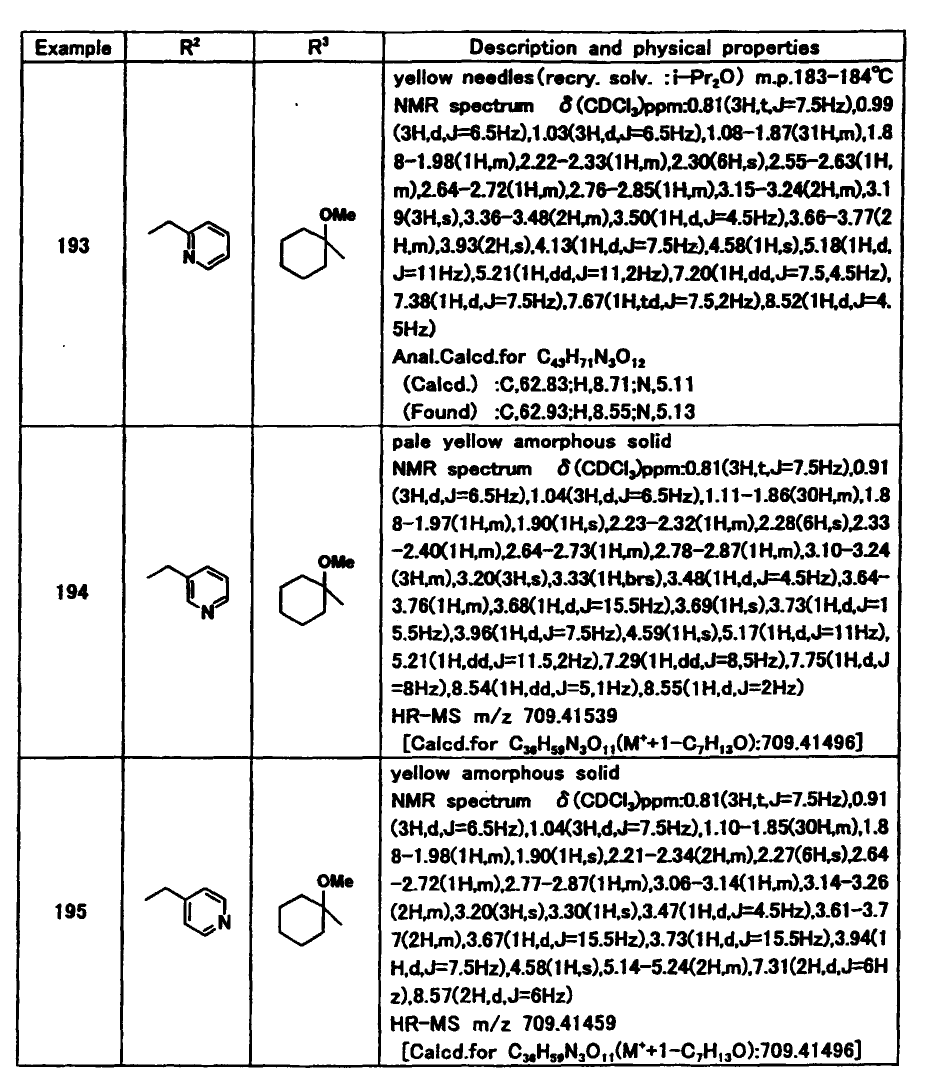

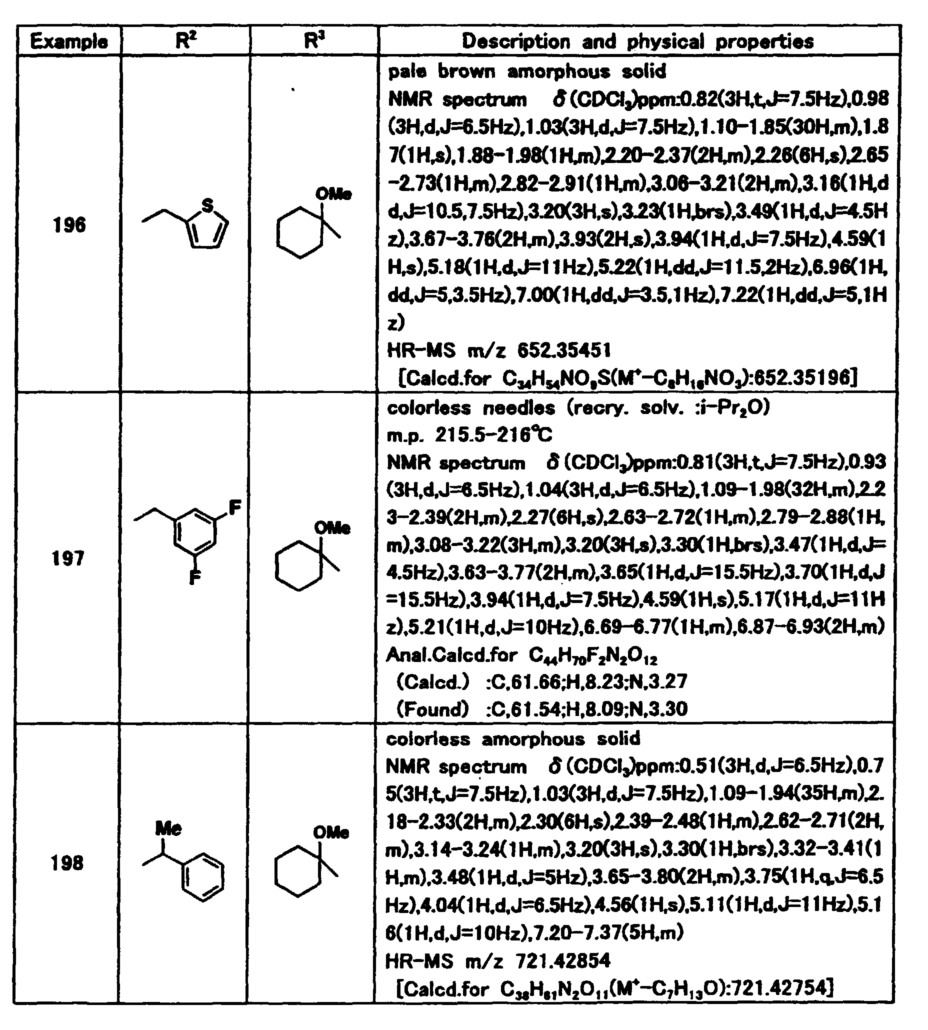

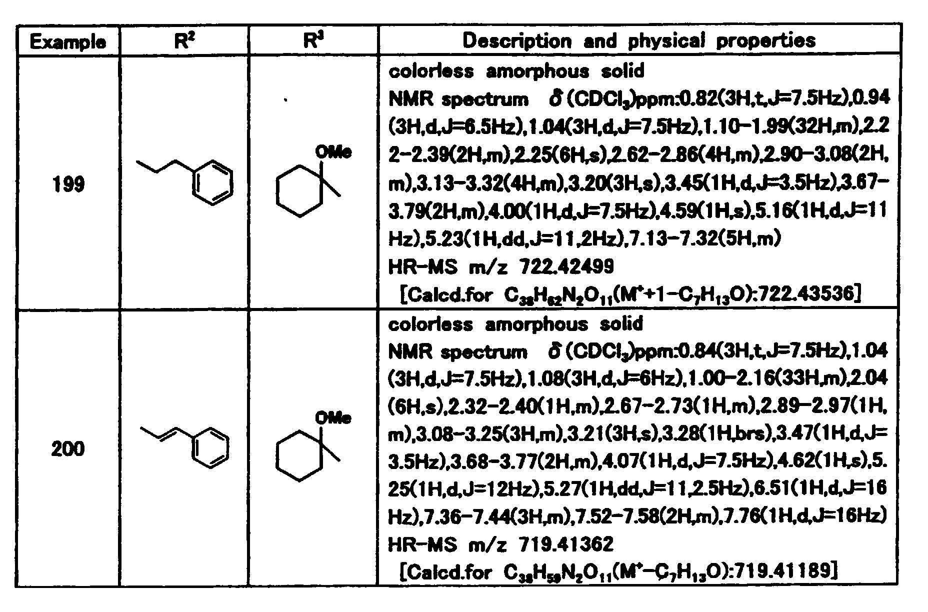

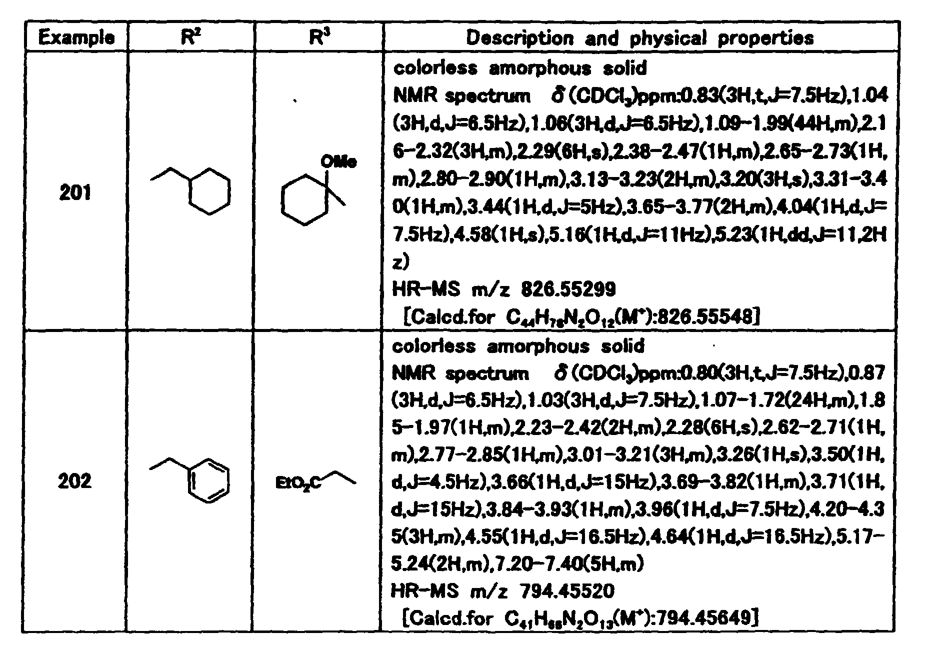

### Example 203: 5-O-Desosaminyl-3-O-imidazolylcarbonylerythronolide A 9-[O-(1-methoxycyclohexyl)oxime]

To a solution of 2.00 g of 2'-O-acetyl-5-O-desosaminylerythronolide A 9-[O-(1-methoxycyclohexyl)oxime] in 30 ml of dichloromethane, 2.18 g of 1,1'-carbonyldiimidazole and 0.36 g of 4-dimethylaminopyridine were successively added at room temperature with stirring, and the reaction mixture was refluxed for 4 days. The reaction mixture was cooled and added with ice-water. The mixture was made alkaline with saturated aqueous sodium hydrogencarbonate solution and extracted with dichloromethane. The extract was washed with water, dried over sodium sulfate, and the solvent was removed under reduced pressure. The residue was purified by column chromatography (silica gel, dichloromethane : methanol = 33:1) to give 2.39 g of a pale yellow amorphous solid. A solution of 1.32 g of the resulting pale yellow amorphous solid in 50 ml of methanol was stirred at room temperature for 1 day. The reaction mixture was concentrated under reduced pressure to give 1.20 g of a pale yellow amorphous solid.
NMR spectrum δ (CDCl₃) ppm: 0.85 (3H, t, J=7.5Hz), 1.05 (3H, d, J=7.5Hz), 1.06 (3H, d, J=6.5Hz), 1.12-2.00 (31H, m), 2.08 (1H, s), 2.18 (6H, s), 2.37-2.44 (1H, m), 2.49-2.58 (1H, m), 2.67-2.77 (1H, m), 3.00-3.08 (2H, m), 3.18-3.24 (1H, m), 3.21 (3H, s), 3.31 (1H, s), 3.53 (1H, d, J=3.5Hz), 3.63-3.77 (3H, m), 3.80 (1H, d, J=6.5Hz), 4.64 (1H, s), 5.25-5.33 (2H, m), 7.11 (1H, s), 7.50 (1H, s), 8.22 (1H, s)
HR-MS m/z 684.39400 [Calcd. for C₃₃H₅₆N₄O₁₁ M⁺+1-C₇H₁₃O): 684.39456]

### Example 204: 5-O-Desosaminyl-3-O-phenylacetylerythronolide A 9-[O-(2-methoxyphenethyl)oxime]

A mixture of 0.40 g of 5-O-desosaminyl-3-O-phenylacetylerythronolide A 9-oxime, 11 mg of tetrabutylammomnium iodide, 0.20 g of 2-methoxyphenethylmethanesulfonate and 45 mg of powdered potassium hydroxide in 4 ml of tetrahydrofuran was stirred at room temperature for 5 days. The reaction mixture was added with water, and the mixture was extracted with diethyl ether. The extract was washed with saturated brine, dried over sodium sulfate, and the solvent was removed under reduced pressure. The residue was purified by column chromatography (silica gel, dichloromethane:methanol:aqueous ammonia = 100:3:1.5) to give 0.50 g of a colorless amorphous solid.
NMR spectrum δ (CDCl₃) ppm: 0.81 (3H, t, J=7.5Hz), 0.87 (3H, d, J=6.5Hz), 0.97 (3H, d, J=7.5Hz), 1.10-1.67 (21H, m), 1.88-1.99 (1H, m), 2.22-2.30 (1H, m), 2.27 (6H, s), 2.31-2.38 (1H, m), 2.59-2.68 (1H, m), 2.77-2.85 (1H, m), 2.87-3.04 (2H, m), 3.06-3.20 (3H, m), 3.28 (1H, brs), 3.41 (1H, d, J=5Hz), 3.54-3.63 (1H, m), 3.66 (1H, d, J=14.5Hz), 3.70 (1H, d, J=14.5Hz), 3.73 (1H, s), 3.84 (3H, s), 3.95 (1H, d, J=7.5Hz), 4.20-4.28 (2H, m), 4.51 (1H, s), 5.14 (1H, d, J=11Hz), 5.22 (1H, dd, J=11.5, 2Hz), 6.81-6.93 (2H, m), 7.08-7.14 (1H, m), 7.17-7.40 (6H, m)
HR-MS m/z 668.38103 [Calcd. for C₃₈H₅₄NO₉ (M⁺+1-C₈H₁₆NO₃): 668.37986]

Compounds of Examples 205 through 207 were obtained in the same manner as that described in Example 204.

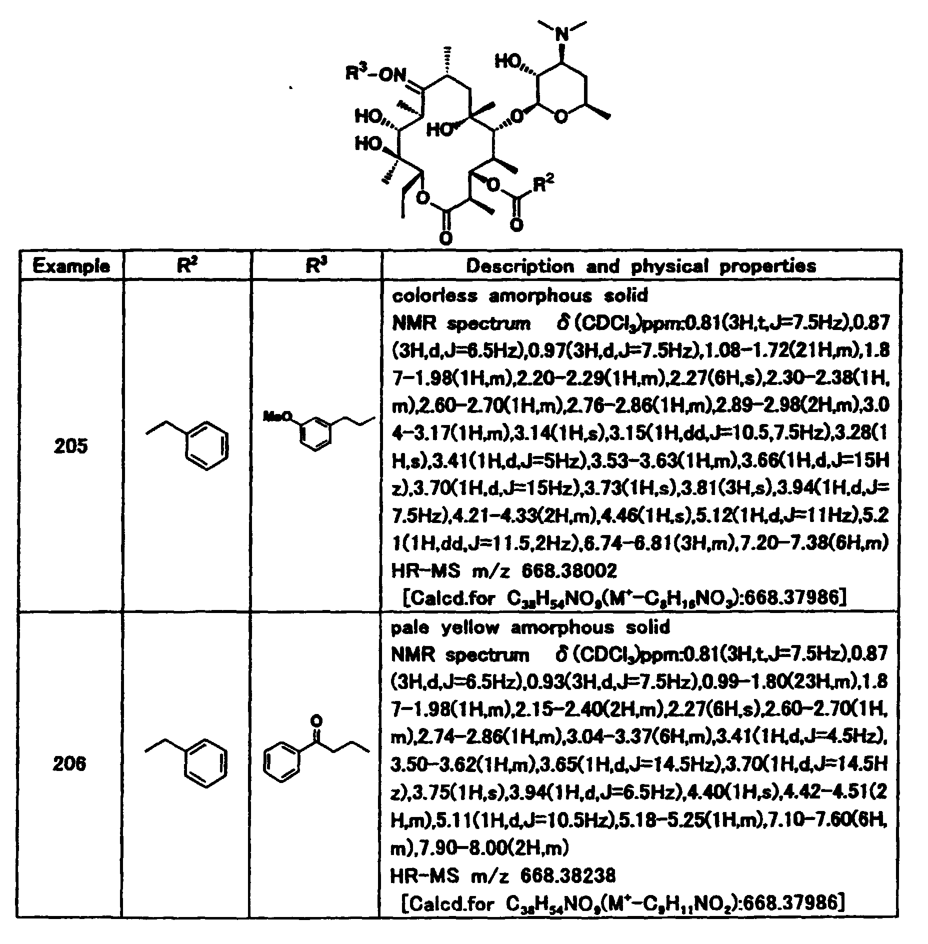

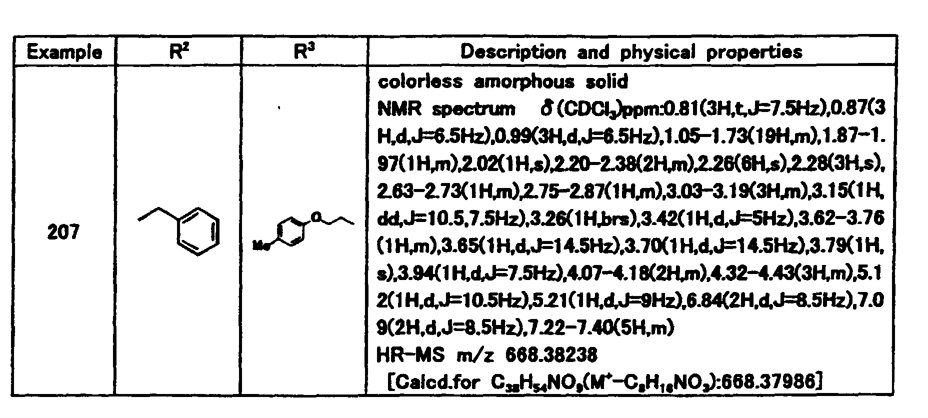

### Example 208: 5-O-Desosaminyl-3-O-methoxycarbonylerythronolide A 9-[O-(3-phenylpropyl)oxime]

To a solution of 0.80 g of 2'-O-acetyl-5-O-desosaminylerythronolide A 9-[O-(3-phenylpropyl)oxime] in 8 ml of pyridine, 1.3 ml of phenyl chloroformate was added dropwise, and the reaction mixture was stirred at room temperature for 24 hours. The reaction mixture was added with water, and the mixture was extracted with diethyl ether. The extract was washed with saturated brine, dried over sodium sulfate, and the solvent was removed under reduced pressure. The residue was purified by column chromatography (silica gel, ethyl acetate) to give 0.38 g of a colorless amorphous solid. A solution of 0.38 g of the resulting colorless amorphous solid in 5 ml of methanol was stirred at room temperature for 160 hours. The reaction mixture was concentrated under reduced pressure, and the residue was purified by column chromatography (silica gel, ethyl acetate) to give 0.22 g of a colorless amorphous solid.
NMR spectrum δ (CDCl₃) ppm: 0.84 (3H, t, J=7.5Hz), 1.04 (3H, d, J=6.5Hz), 1.19-1.80 (23H, m), 1.90-2.03 (4H, m), 2.20-2.40 (7H, m), 2.47-2.60 (1H, m), 2.60-2.75 (3H, m), 2.85-2.95 (1H, m), 3.12 (1H, s), 3.15-3.30 (2H, m), 3.30-3.40 (1H, m), 3.52 (1H, d, J=3.5Hz), 3.62-3.72 (2H, m), 3.80 (3H, s), 4.06 (2H, t, J=6.5Hz), 4.19 (1H, d, J=7.5Hz), 4.45 (1H, s), 4.89 (1H, d, J=11Hz), 5.26 (1H, dd, J=11, 2Hz), 7.10-7.31 (5H, m)
HR-MS m/z 766.46354 [Calcd. for C₄₀H₆₆N₂O₁₂ (M⁺): 766.46158]

Compound of Example 209 was obtained in the same manner as that described in Example 208.

### Example 209: 5-O-Desosaminyl-3-O-methoxycarbonylerythronolide A 9-[O-(2-phenoxyethyl)oxime]

Appearance: a colorless amorphous solid
NMR spectrum δ (CDCl₃) ppm: 0.85 (3H, t, J=7.5Hz), 1.00 (3H, d, J=6.5Hz), 1.05-1.70 (23H, m), 1.90-2.00 (1H, m), 2.12 (1H, s), 2.20-2.35 (1H, m), 2.27 (6H, s), 2.40-2.50 (1H, m), 2.62-2.75 (1H, m), 2.85-2.95 (1H, m), 3.12 (1H, s), 3.15 (1H, dd, J=10.5, 7.5Hz), 3.24 (1H, brs), 3.30-3.40 (1H, m), 3.46 (1H, d, J=4.5Hz), 3.65-3.85 (2H, m), 3.79 (3H, s), 4.13-4.20 (3H, m), 4.37-4.43 (3H, m), 4.87 (1H, d, J=10Hz), 5.26 (1H, dd, J=11, 2.5Hz), 6.90-7.00 (3H, m), 7.25-7.35 (2H, m)
HR-MS m/z 768.43949 [Calcd. for C₃₉H₆₄N₂O₁₃ (M⁺): 768.44084]

### Example 210: 5-O-Desosaminyl-3-O-phenylcarbamoylerythronolide A 9-[O-(3-phenylpropyl)oxime]

A solution of 0.40 g of 2'-O-acetyl-5-O-desosaminylerythronolide A 9-[O-(3-phenylpropyl)oxime], 0.34 ml of phenyl isocyanate and 0.12 ml of pyridine in 4 ml of tetrahydrofuran was stirred at room temperature for 28 hours. The reaction mixture was added with water, and the mixture was extracted with diethyl ether. The extract was washed with saturated brine, dried over sodium sulfate, and the solvent was removed under reduced pressure. A solution of the resulting residue in 15 ml of methanol was stirred at room temperature for 24 hours. The reaction mixture was concentrated under reduced pressure, and the residue was purified by column chromatography (silica gel, ethyl acetate) to give 0.31 g of a colorless amorphous solid.
NMR spectrum δ (CDCl₃) ppm: 0.85 (3H, t, J=7.5Hz), 0.97-1.68 (27H, m), 1.90-2.03 (4H, m), 2.13-2.38 (2H, m), 2.19 (6H, s), 2.62-2.77 (3H, m), 2.82-2.95 (1H, m), 3.05-3.25 (3H, m), 3.55 (1H, d, J=3.5Hz), 3.63-3.79 (2H, m), 3.98-4.17 (3H, m), 4.48 (1H, s), 5.03 (1H, d, J=10.5Hz), 5.27 (1H, dd, J=11.5, 2Hz), 7.05 (1H, t, J=7.5Hz), 7.11 (1H, brs), 7.16-7.22 (3H, m), 7.25-7.34 (4H, m), 7.40-7.48 (2H, m)
HR-MS m/z 827.49261 [Calcd. for C₄₅H₆₉N₃O₁₁ (M⁺): 827.49321]

Compounds of Examples 211 through 212 were obtained in the same manner as that described in Example 210.

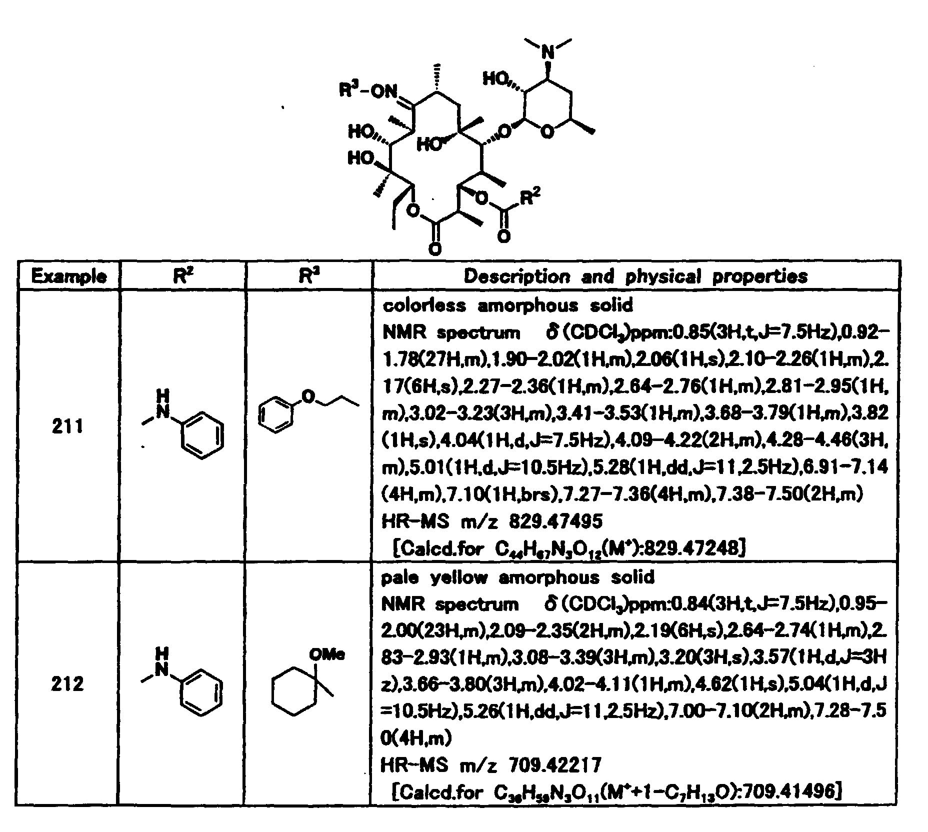

In order to evaluate excellent efficacy of the compounds of the present invention, their antibacterial spectrums against atypical acid-fast mycobacteria (MAC) were measured. Clarithromycin and rifampicin were used as reference compounds.

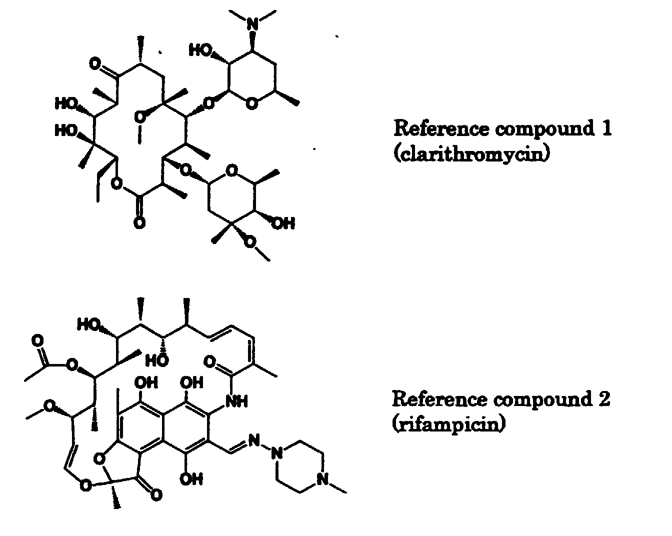

### Antibacterial spectrum against atypical acid-fast mycobacteria

Antibacterial activities (minimum inhibitory concentration: MIC) were measured according to the standard method of the Japan Society of Chemotherapy [Chemotherapy, 29 (1), 76, 1981] by using clinical isolates of atypical acid-fast mycobacteria and applying 10⁶ CFU/ml of live bacteria. The results are shown in Table 126 and Table 127. The compounds of the present invention had more potent antibacterial activities than the reference compounds against atypical acid-fast mycobacteria including erythromycin-resistant strains (*M. avium* 20092 and other bacteria).

Names of the bacteria in the tables are as follows:
*Mycobacterium avium (M. avium)*
*Mycobacterium intracellulare (M. intracellulare)*

| Antibacterial spectrum (Minimum inhibitory concentration µg/ml) | | | | | |
|---|---|---|---|---|---|
| Strain | Example | | | Reference compound | |
| | 23 | 40 | 78 | 1 | 2 |
| *M. avium* 20034 | 3.13 | 3.13 | 3.13 | 3.13 | 12.5 |
| *M. avium* 20045 | 3.13 | 3.13 | 1.56 | 1.56 | 3.13 |
| *M. avium* 20092 | 6.25 | 3.13 | 3.13 | >50 | 50 |
| *M. avium* 20096 | 3.13 | 3.13 | 1.56 | >50 | 3.13 |
| *M. intracellulare* 20066 | 3.13 | 3.13 | 0.78 | 3.13 | 3.13 |
| *M. intracellulare* 20067 | 1.56 | 1.56 | 0.78 | 1.56 | 1.56 |
| *M. intracellulare* 20073 | 3.13 | 1.56 | 0.78 | 1.56 | 3.13 |
| *M. intracellulare* 20075 | 3.13 | 3.13 | 0.78 | 3.13 | 3.13 |

| Distribution of susceptibility (Minimum inhibitory concentration µg/ml) | | | | | |
|---|---|---|---|---|---|
| Strain (Number of strain) | Compound | Minimum inhibitory concentration µg/ml | | | |
| | | Range | 50% | 80% | 90% |
| *M. avium* (27) | Example 23 | 1.56 ∼ 6.25 | 3.13 | 3.13 | 3.13 |
| | Example 40 | 1.56 ∼ 3.13 | 3.13 | 3.13 | 3.13 |
| | Example 78 | 1.56 ∼ 3.13 | 1.56 | 1.56 | 3.13 |
| | Reference compound 1 | 0.78 ∼ >100 | 3.13 | 6.25 | 12.5 |
| | Reference compound 2 | 0.39 ∼ 50 | 25 | 50 | 50 |

### Industrial Applicability

The erythromycin derivatives and salts thereof of the present invention have excellent antibacterial activity against atypical acid-fast mycobacteria including multiple drug-resistant bacteria and are extremely useful as antibacterial agents.

## Claims

1. An erythromycin derivative represented by the following general formula or a salt thereof: 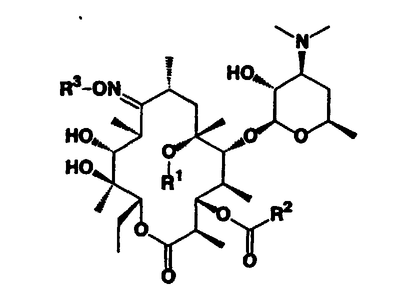 wherein R¹ represents a hydrogen atom or a lower alkyl group; R² represents an alkyl group which may be substituted, a cycloalkyl group which may be substituted, a (cycloalkyl)alkyl group which may be substituted, an aryl group which may be substituted, an aralkyl group which may be substituted, a styryl group which may be substituted, or a group represented by the formula -X-R⁴ wherein X represents an oxygen atom or an amino group, and R⁴ represents an alkyl group which may be substituted or an aryl group which may be substituted; and R³ represents an alkyl group which is substituted with a carboxyl group, an alkoxycarbonyl group, an aryloxycarbonyl group, or an aralkyloxycarbonyl group, a cycloalkyl group which may be substituted, a (cycloalkyl)alkyl group which may be substituted, an alkenyl group which may be substituted, or a group represented by the formula -(CH₂)ₙ-Y-R⁵ wherein Y represents a methylene group which may be substituted, an oxygen atom, a sulfur atom, a sulfinyl group, a sulfonyl group, an amino group which may be substituted with an alkyl group, or a carbonyl group, R⁵ represents an aryl group which may be substituted, and n represents an integer of from 1 to 5.

2. The compound or a salt thereof according to claim 1, wherein R¹ is a hydrogen atom.

3. A medicament which comprises a compound or a physiologically acceptable salt thereof according to claim 1 or 2 as an active ingredient.

4. The medicament according to claim 3, which is used as an agent for therapeutic treatment of an infectious disease.

5. The medicament according to claim 4, which is used as an agent for therapeutic treatment of an infection by atypical acid-fast mycobacteria.

6. The medicament according to claim 5, wherein the atypical acid-fast mycobacteria are *Mycobacterium avium* complex.

7. Use of a compound represented by the general formula (I) or a salt thereof according to claim 1 for the manufacture of a medicament according to any one of claims 3 to 6.

8. A method for therapeutic treatment of an infectious disease, which comprises the step of administering to a patient a therapeutically effective amount of a compound represented by the general formula (I) or a physiologically acceptable salt thereof according to claim 1.
